(19) Europäisches Patentamt
European Patent Office
Office européen des brevets

(11) EP 3 458 074 B1

# (12) EUROPEAN PATENT SPECIFICATION

(45) Date of publication and mention of the grant of the patent:
**17.04.2024 Bulletin 2024/16**

(21) Application number: **17800042.8**

(22) Date of filing: **16.05.2017**

(51) International Patent Classification (IPC):
*A61K 9/51* (2006.01) *A61K 9/127* (2006.01)
*A61K 31/7105* (2006.01) *A61K 31/7088* (2006.01)
*A61P 25/08* (2006.01) *A61P 35/00* (2006.01)
*A61K 9/00* (2006.01) *A61P 21/04* (2006.01)
*A61P 39/02* (2006.01) *A61P 43/00* (2006.01)

(52) Cooperative Patent Classification (CPC):
**A61K 31/7105; A61K 9/0019; A61K 9/127; A61K 9/5123; A61K 31/7088; A61P 21/04; A61P 25/08; A61P 35/00; A61P 39/02; A61P 43/00**

(86) International application number:
**PCT/US2017/032967**

(87) International publication number:
**WO 2017/201091 (23.11.2017 Gazette 2017/47)**

## (54) COMPOSITIONS FOR THE DELIVERY OF tRNA AS NANOPARTICLES AND METHODS OF USE THEREWITH

ZUSAMMENSETZUNGEN ZUR AUSGABE VON TRNA-NANOPARTIKELN UND VERFAHREN ZUR VERWENDUNG DARIN

COMPOSITIONS DESTINÉES À L'ADMINISTRATION D'ARNT SOUS LA FORME DE NANOPARTICULES ET PROCÉDÉ D'UTILISATION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.05.2016 US 201662337096 P**

(43) Date of publication of application:
**27.03.2019 Bulletin 2019/13**

(60) Divisional application:
**24161024.5**

(73) Proprietor: **Board of Regents of the University of Texas System**
**Austin, TX 78701 (US)**

(72) Inventors:
- **THOMAS, Philip, J.**
  **Dallas, TX 75248 (US)**
- **SIEGWART, Daniel, J.**
  **Dallas, TX 75229 (US)**
- **JOHNSON, Arthur, E.**
  **Florence, NJ 08518 (US)**
- **TORRES, Michael, J.**
  **Irving, TX 75060 (US)**
- **MILLER, Jason, B.**
  **Dallas, TX 75206 (US)**

(74) Representative: **Potter Clarkson**
**Chapel Quarter**
**Mount Street**
**Nottingham NG1 6HQ (GB)**

(56) References cited:
**WO-A2-2010/053329**
**KR-A- 20110 090 661**
**US-A1- 2008 242 626**
**US-A1- 2010 048 888**
**US-A1- 2010 196 277**
**US-A1- 2010 196 277**
**US-A1- 2015 297 749**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

## BACKGROUND

### 1. Field

The present disclosure relates generally to the field of genetic diseases and disorders and pharmaceutical formulations. More particularly, it concerns methods of delivering tRNA as a nanoparticle composition.

### 2. Description of Related Art

Protein synthesis *in vivo* is directed by a genetic code that includes 61 three-base-pair codons that encode for different amino acids and 3 three-base-pair codons that terminate the synthesis of a protein. When a nucleic acid sequence that encodes a protein is mutated to contain a stop codon instead a codon for the next amino acid, the resultant protein is prematurely terminated and usually nonfunctional. Such mutations, termed nonsense mutations, result in numerous different genetic diseases, including many cases of cystic fibrosis.

One potential treatment option would be to treat these nonsense mutations with a tRNA that allows the production of the complete protein. Unfortunately, efforts to develop tRNAs and tRNA compositions that can be administered to a patient have met with limited success. Therefore, new compositions and methods for delivering tRNA to a patient are of clinical importance.

US 2015/0297749 discloses a low density lipoprotein-like cationic solid lipid nanoparticle targeting liver cells including parenchyma cells and non-parenchyma cells, a composition for liver target delivery, a composition for diagnosis and/or treatment of liver disease comprising the same, and a method for liver targeting of an active ingredient.

## SUMMARY

In some aspects, the present disclosure provides compositions as defined in the claims that may be used to deliver tRNA or tRNA derivatives to a cell. The disclosure also provides a composition for use in medicine comprising:

(A) a transfer ribonucleic acid (tRNA);

(B) an aminolipid delivery compound;

wherein the aminolipid delivery compound forms a nanoparticle. In some embodiments, the aminolipid delivery compound is a dendrimer of the formula:

$$\text{Core-(Repeating Unit)}_n\text{-Terminating Group (I)}$$

wherein the core is linked to the repeating unit by removing one or more hydrogen atoms from the core and replacing with the repeating unit and wherein:

the core has the formula:

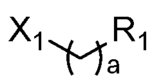

(II)

wherein:

$X_1$ is amino or alkylamino$_{(C\leq12)}$, dialkylamino$_{(C\leq12)}$, heterocycloalkyl$_{(C\leq12)}$, heteroaryl$_{(C\leq12)}$, or a substituted version thereof;

$R_1$ is amino, hydroxy, or mercapto, or alkylamino$_{(C\leq12)}$, dialkylamino$_{(C\leq12)}$, or a substituted version of either of these groups; and

a is 1, 2, 3, 4, 5, or 6; or

the core has the formula:

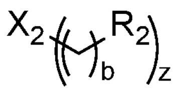 (III)

wherein:

$X_2$ is $N(R_5)_y$;

$R_5$ is hydrogen, $alkyl_{(C\leq18)}$, or substituted $alkyl_{(C\leq18)}$; and

y is 0, 1, or 2, provided that the sum of y and z is 3;

$R_2$ is amino, hydroxy, or mercapto, or $alkylamino_{(C\leq12)}$, $dialkylamino_{(C\leq12)}$, or a substituted version of either of these groups;

b is 1, 2, 3, 4, 5, or 6; and

z is 1, 2, 3; provided that the sum of z and y is 3; or

the core has the formula:

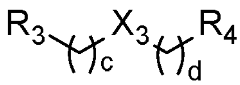 (IV)

wherein:

$X_3$ is $-NR_6-$, -O-, or $alkylaminodiyl_{(C\leq8)}$, $alkoxydiyl_{(C\leq8)}$, $arenediyl_{(C\leq8)}$, $heteroarenediyl_{(C\leq8)}$, $heterocycloalkanediyl_{(C\leq8)}$, or a substituted version of any of these groups, wherein $R_6$ is hydrogen, $alkyl_{(C\leq8)}$, or substituted $alkyl_{(C\leq8)}$;

$R_3$ and $R_4$ are each independently amino, hydroxy, or mercapto, or $alkylamino_{(C\leq12)}$, $dialkylamino_{(C\leq12)}$, wherein, optionally, either of these groups is substituted by one or more substituents selected from the group consisting of: -OH, -F, -Cl, - Br, -I, $-NH_2$, $-NO_2$, $-CO_2H$, $-CO_2CH_3$, -CN, -SH, $-OCH_3$, $-OCH_2CH_3$, - $C(O)CH_3$, $-NHCH_3$, $-NHCH_2CH_3$, $-N(CH_3)_2$, $-C(O)NH_2$, $-C(O)NHCH_3$, - $C(O)N(CH_3)_2$, $-OC(O)CH_3$, $-NHC(O)CH_3$, $-S(O)_2OH$, and $-S(O)_2NH_2$;

wherein:
c and d are each independently 1, 2, 3, 4, 5, or 6; or

the core is $alkylamine_{(C\leq18)}$, $dialkylamine_{(C\leq36)}$, $heterocycloalkane_{(C\leq12)}$, or a substituted version of any of these groups;

wherein the repeating unit comprises a degradable diacyl group and a linker group;

the degradable diacyl group has the formula:

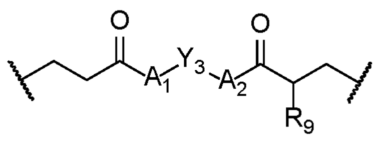 (VII)

wherein:

$A_1$ and $A_2$ are each independently -O-, -S-, or -$NR_a$-, wherein:

$R_a$ is hydrogen, alkyl$_{(C\leq6)}$, or substituted alkyl$_{(C\leq6)}$;

$Y_3$ is alkanediyl$_{(C\leq12)}$, alkenediyl$_{(C\leq12)}$, arenediyl$_{(C\leq12)}$, or a substituted version of any of these groups; or a group of the formula:

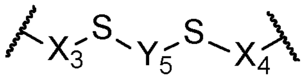

or

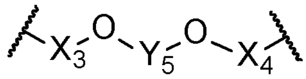

wherein:

$X_3$ and $X_4$ are alkanediyl$_{(C\leq12)}$, alkenediyl$_{(C\leq12)}$, arenediyl$_{(C\leq12)}$, or a substituted version of any of these groups;

$Y_5$ is a covalent bond, alkanediyl$_{(C\leq12)}$, alkenediyl$_{(C\leq12)}$, arenediyl$_{(C\leq12)}$, or a substituted version of any of these groups; and

$R_9$ is alkyl$_{(C\leq8)}$ or substituted alkyl$_{(C\leq8)}$;

the linker group has the formula:

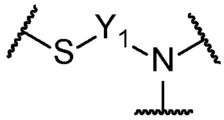

(VI)

wherein:

$Y_1$ is alkanediyl$_{(C\leq12)}$, alkenediyl$_{(C\leq12)}$, arenediyl$_{(C\leq12)}$, or a substituted version of any of these groups; and

wherein when the repeating unit comprises a linker group, then the linker group is attached to a degradable diacyl group on both the nitrogen and the sulfur atoms of the linker group, wherein the first group in the repeating unit is a degradable diacyl group, wherein for each linker group, the next group comprises two degradable diacyl groups attached to the nitrogen atom of the linker group; and wherein n is the number of linker groups present in the repeating unit; and

the terminating group has the formula:

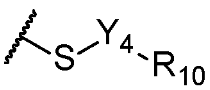

(VIII)

wherein:

$Y_4$ is alkanediyl$_{(C\leq18)}$, alkenediyl$_{(C\leq18)}$, or a substituted version of either group;

$R_{10}$ is hydrogen, carboxy, hydroxy, or

aryl$_{(C\leq12)}$, alkylamino$_{(C\leq12)}$, dialkylamino$_{(C\leq12)}$, *N*-heterocycloalkyl$_{(C\leq12)}$, -C(O)N($R_{11}$)-alkanediyl$_{(C\leq6)}$-heterocycloalkyl$_{(C\leq12)}$, -C(O)-alkylamino$_{(C\leq12)}$, -C(O)-dialkylamino$_{(C\leq12)}$, -C(O)-*N*-heterocycloalkyl$_{(C\leq12)}$, wherein:

$R_{11}$ is hydrogen, alkyl$_{(C\leq6)}$, or substituted alkyl$_{(C\leq6)}$;

wherein the final degradable diacyl group in the chain is attached to a terminating group;

n is 0, 1, 2, 3, 4, 5, or 6;

or a pharmaceutically acceptable salt thereof.

The aminolipid delivery compound may be a dendrimer of the formula:

the core has the formula:

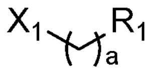

(II)

wherein:

$X_1$ is amino or alkylamino$_{(C\leq 12)}$, dialkylamino$_{(C\leq 12)}$, heterocycloalkyl$_{(C\leq 12)}$, heteroaryl$_{(C\leq 12)}$, or a substituted version thereof;

$R_1$ is amino, hydroxy, or mercapto, or alkylamino$_{(C\leq 12)}$, dialkylamino$_{(C\leq 12)}$, or a substituted version of either of these groups; and

a is 1, 2, 3, 4, 5, or 6; and

wherein the repeating unit comprises a degradable diacyl group and a linker group;

the degradable diacyl group has the formula:

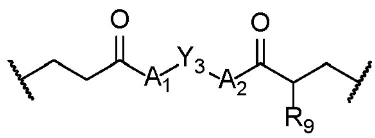

(VII)

wherein:

$A_1$ and $A_2$ are each independently -O- or -NR$_a$-, wherein:
$R_a$ is hydrogen, alkyl$_{(C\leq 6)}$, or substituted alkyl$_{(C\leq 6)}$;

$Y_3$ is alkanediyl$_{(C\leq 12)}$, alkenediyl$_{(C\leq 12)}$, arenediyl$_{(C\leq 12)}$, or a substituted version of any of these groups; or a group of the formula:

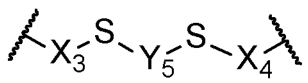

or

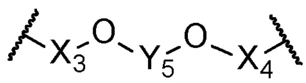

wherein:

$X_3$ and $X_4$ are alkanediyl$_{(C\leq 12)}$, alkenediyl$_{(C\leq 12)}$, arenediyl$_{(C\leq 12)}$, or a substituted version of any of these groups;

$Y_5$ is a covalent bond, alkanediyl$_{(C\leq 12)}$, alkenediyl$_{(C\leq 12)}$, arenediyl$_{(C\leq 12)}$, or a substituted version of any of these groups; and

$R_9$ is alkyl$_{(C\leq 8)}$ or substituted alkyl$_{(C\leq 8)}$;

the linker group has the formula:

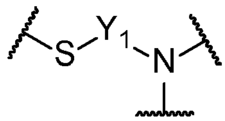
(VI)

wherein:

$Y_1$ is alkanediyl$_{(C\leq12)}$, alkenediyl$_{(C\leq12)}$, arenediyl$_{(C\leq12)}$, or a substituted version of any of these groups; and

wherein when the repeating unit comprises a linker group, then the linker group is attached to a degradable diacyl group on both the nitrogen and the sulfur atoms of the linker group, wherein the first group in the repeating unit is a degradable diacyl group, wherein for each linker group, the next group comprises two degradable diacyl groups attached to the nitrogen atom of the linker group; and wherein n is the number of linker groups present in the repeating unit; and

the terminating group, wherein the terminating group has the formula:

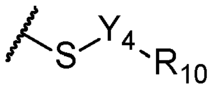
(VIII)

wherein:

$Y_4$ is alkanediyl$_{(C\leq18)}$, alkenediyl$_{(C\leq18)}$, or a substituted version of either group;

$R_{10}$ is hydrogen, carboxy, hydroxy, or

aryl$_{(C\leq12)}$, alkylamino$_{(C\leq12)}$, dialkylamino$_{(C\leq12)}$, *N*-heterocycloalkyl$_{(C\leq12)}$, -C(O)N($R_{11}$)-alkanediyl$_{(C\leq6)}$-heterocycloalkyl$_{(C\leq12)}$, -C(O)-alkylamino$_{(C\leq12)}$, -C(O)-dialkylamino$_{(C\leq12)}$, -C(O)-*N*-heterocyclo-alkyl$_{(C\leq12)}$, wherein: $R_{11}$ is hydrogen, alkyl$_{(C\leq6)}$, or substituted alkyl$_{(C\leq6)}$;

wherein the final degradable diacyl group in the chain is attached to a terminating group;

n is 0, 1, 2, 3, 4, 5, or 6;

or a pharmaceutically acceptable salt thereof. In some embodiments, the aminolipid delivery compound is a compound of the formula:

$$\text{Core-(Repeating Unit)}_n\text{-Terminating Group (I)}$$

wherein the core is linked to the repeating unit by removing one or more hydrogen atoms from the core and replacing with the repeating unit and wherein:

the core has the formula:

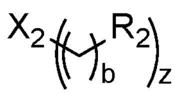
(III)

wherein:

$X_2$ is $N(R_5)_y$;

$R_5$ is hydrogen or alkyl$_{(C\leq8)}$, or substituted alkyl$_{(C\leq18)}$; and

y is 0, 1, or 2, provided that the sum of y and z is 3;

$R_2$ is amino, hydroxy, or mercapto, or alkylamino$_{(C\leq12)}$, dialkylamino$_{(C\leq12)}$, or a substituted version of either of these groups;

b is 1, 2, 3, 4, 5, or 6; and

z is 1, 2, 3; provided that the sum of z and y is 3;

wherein the repeating unit comprises a degradable diacyl group and a linker group;

the degradable diacyl group has the formula:

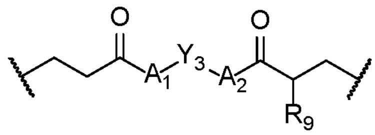
(VII)

wherein:

$A_1$ and $A_2$ are each independently -O- or -NR$_a$-, wherein: $R_a$ is hydrogen, alkyl$_{(C\leq6)}$, or substituted alkyl$_{(C\leq6)}$;

$Y_3$ is alkanediyl$_{(C\leq12)}$, alkenediyl$_{(C\leq12)}$, arenediyl$_{(C\leq12)}$, or a substituted version of any of these groups; or a group of the formula:

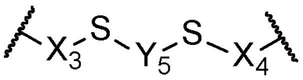
or
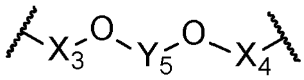

wherein:

$X_3$ and $X_4$ are alkanediyl$_{(C\leq12)}$, alkenediyl$_{(C\leq12)}$, arenediyl$_{(C\leq12)}$, or a substituted version of any of these groups;

$Y_5$ is a covalent bond, alkanediyl$_{(C\leq12)}$, alkenediyl$_{(C\leq12)}$, arenediyl$_{(C\leq12)}$, or a substituted version of any of these groups; and

$R_9$ is alkyl$_{(C\leq8)}$ or substituted alkyl$_{(C\leq8)}$;

the linker group has the formula:

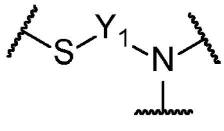
(VI)

wherein:
$Y_1$ is alkanediyl$_{(C\leq12)}$, alkenediyl$_{(C\leq12)}$, arenediyl$_{(C\leq12)}$, or a substituted version of any of these groups; and

wherein when the repeating unit comprises a linker group, then the linker group is attached to a degradable diacyl group on both the nitrogen and the sulfur atoms of the linker group, wherein the first group in the repeating unit is a degradable diacyl group, wherein for each linker group, the next group comprises two degradable diacyl groups attached to the nitrogen atom of the linker group; and wherein n is the number of linker groups present in the repeating unit; and

the terminating group, wherein the terminating group has the formula:

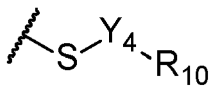 (VIII)

wherein:

$Y_4$ is alkanediyl$_{(C\leq18)}$, alkenediyl$_{(C\leq18)}$, or a substituted version of either group;

$R_{10}$ is hydrogen, carboxy, hydroxy, or

aryl$_{(C\leq12)}$, alkylamino$_{(C\leq12)}$, dialkylamino$_{(C\leq12)}$, *N*-heterocycloalkyl$_{(C\leq12)}$, -C(O)N($R_{11}$)-alkanediyl$_{(C\leq6)}$-heterocycloalkyl$_{(C\leq12)}$, -C(O)-alkylamino$_{(C\leq12)}$, -C(O)-dialkylamino$_{(C\leq12)}$, -C(O)-*N*-heterocyclo-alkyl$_{(C\leq12)}$, wherein:

wherein the final degradable diacyl group in the chain is attached to a terminating group;
n is 0, 1, 2, 3, 4, 5, or 6;

or a pharmaceutically acceptable salt thereof. In some embodiments, the aminolipid delivery compound is a compound of the formula:

Core-(Repeating Unit)$_n$-Terminating Group (I)

wherein the core is linked to the repeating unit by removing one or more hydrogen atoms from

the core and replacing with the repeating unit and wherein:

the core has the formula:

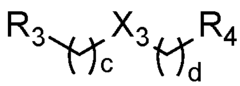 (IV)

wherein:

$X_3$ is -$NR_6$-, -O-, or alkylaminodiyl$_{(C\leq8)}$, alkoxydiyl$_{(C\leq8)}$, arenediyl$_{(C\leq8)}$, heteroarenediyl$_{(C\leq8)}$, heterocycloalkanediyl$_{(C\leq8)}$, or a substituted version of any of these groups, wherein $R_6$ is hydrogen, alkyl$_{(C\leq8)}$, or substituted alkyl$_{(C\leq8)}$;

$R_3$ and $R_4$ are each independently amino, hydroxy, or mercapto, or alkylamino$_{(C\leq12)}$, dialkylamino$_{(C\leq12)}$, wherein, optionally, either of these groups is substituted by one or more substituents selected from the group consisting of: -OH, -F, -Cl, -Br, -I, -$NH_2$, -$NO_2$, -$CO_2H$, -$CO_2CH_3$, -CN, -SH, -$OCH_3$, -$OCH_2CH_3$, -C(O)$CH_3$, -$NHCH_3$, -$NHCH_2CH_3$, -$N(CH_3)_2$, -C(O)$NH_2$, -C(O)$NHCH_3$, -C(O)$N(CH_3)_2$, -OC(O)$CH_3$, -NHC(O)$CH_3$, - S(O)$_2$OH, and -S(O)$_2NH_2$;

wherein:
c and d are each independently 1, 2, 3, 4, 5, or 6; and

wherein the repeating unit comprises a degradable diacyl group and a linker group;

the degradable diacyl group has the formula:

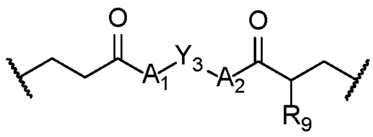
(VII)

wherein:

$A_1$ and $A_2$ are each independently -O- or -$NR_a$-, wherein: $R_a$ is hydrogen, alkyl$_{(C\leq6)}$, or substituted alkyl$_{(C\leq6)}$;

$Y_3$ is alkanediyl$_{(C\leq12)}$, alkenediyl$_{(C\leq12)}$, arenediyl$_{(C\leq12)}$, or a substituted version of any of these groups; or a group of the formula:

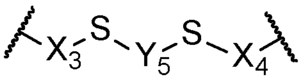
or
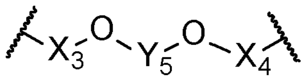

wherein:

$X_3$ and $X_4$ are alkanediyl$_{(C\leq12)}$, alkenediyl$_{(C\leq12)}$, arenediyl$_{(C\leq12)}$, or a substituted version of any of these groups;

$Y_5$ is a covalent bond, alkanediyl$_{(C\leq12)}$, alkenediyl$_{(C\leq12)}$, arenediyl$_{(C\leq12)}$, or a substituted version of any of these groups; and

$R_9$ is alkyl$_{(C\leq8)}$ or substituted alkyl$_{(C\leq8)}$;

the linker group has the formula:

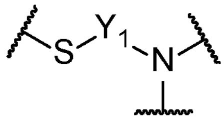
(VI)

wherein: $Y_1$ is alkanediyl$_{(C<12)}$, alkenediyl$_{(C\leq12)}$, arenediyl$_{(C\leq12)}$, or a substituted version of any of these groups; and

wherein when the repeating unit comprises a linker group, then the linker group is attached to a degradable diacyl group on both the nitrogen and the sulfur atoms of the linker group, wherein the first group in the repeating unit is a degradable diacyl group, wherein for each linker group, the next group comprises two degradable diacyl groups attached to the nitrogen atom of the linker group; and wherein n is the number of linker groups present in the repeating unit; and

the terminating group, wherein the terminating group has the formula:

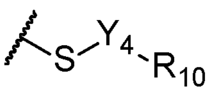
(VIII)

wherein:

$Y_4$ is alkanediyl$_{(C\leq18)}$, alkenediyl$_{(C\leq18)}$, or a substituted version of either group;

$R_{10}$ is hydrogen, carboxy, hydroxy, or

aryl$_{(C\leq12)}$, alkylamino$_{(C\leq12)}$, dialkylamino$_{(C\leq12)}$, *N*-heterocycloalkyl$_{(C\leq12)}$, -C(O)N($R_{11}$)-alkanediyl$_{(C\leq6)}$-heterocycloalkyl$_{(C\leq12)}$, -C(O)-alkylamino$_{(C\leq12)}$, -C(O)-dialkylamino$_{(C\leq12)}$, -C(O)-*N*-heterocyclo-alkyl$_{(C\leq12)}$, wherein:

$R_{11}$ is hydrogen, alkyl$_{(C\leq6)}$, or substituted alkyl$_{(C\leq6)}$;

wherein the final degradable diacyl group in the chain is attached to a terminating group;

n is 0, 1, 2, 3, 4, 5, or 6;

or a pharmaceutically acceptable salt thereof.

$Y_4$ may be an alkanediyl$_{(C\leq18)}$ or an alkanediyl$_{(C\leq18)}$ wherein one or more of the hydrogen atoms on the alkanediyl$_{(C\leq18)}$ has been replaced with -OH, -F, -Cl, -Br, -I, -SH, $-OCH_3$, $-OCH_2CH_3$, $-SCH_3$, or $-OC(O)CH_3$.

In some embodiments, the aminolipid delivery compound is further defined as:

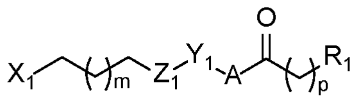
(I)

wherein:

$X_1$ is $-S(O)_2O^-$, $-OP(O)OR_eO^-$, $-(CHR_f)_zC(O)O^-$, or $-NR_gR_hR_i^+$, wherein:

$R_e$, $R_g$, $R_h$, and $R_i$ are each independently hydrogen, alkyl$_{(C\leq6)}$, or substituted alkyl$_{(C\leq6)}$;

$R_f$ is hydrogen, amino, hydroxy, or alkyl$_{(C\leq12)}$, aryl$_{(C\leq12)}$, aralkyl$_{(C\leq12)}$, heteroaryl$_{(C\leq12)}$, acyl$_{(C\leq12)}$, alkoxy$_{(C\leq12)}$, acyloxy$_{(C\leq12)}$, amido$_{(C\leq12)}$, alkoxy$_{(C\leq12)}$, alkoxy$_{(C\leq12)}$, or a substituted version of any of the last ten groups; and

z is 1, 2, 3, or 4;

$Y_1$ is alkanediyl$_{(C\leq12)}$, alkenediyl$_{(C\leq12)}$, arenediyl$_{(C\leq12)}$, heteroarenediyl$_{(C\leq12)}$, heterocycloalkanediyl$_{(C\leq12)}$, -alkanediyl$_{(C\leq8)}$-heterocycloalkanediyl$_{(C\leq12)}$, -alkanediyl$_{(C\leq8)}$-heterocycloalkanediyl$_{(C\leq12)}$-alkanediyl$_{(C\leq8)}$, -alkane-diyl$_{(C\leq8)}$-heteroarenediyl$_{(C<12)}$, -alkanediyl$_{(C\leq8)}$-heteroarene-diyl$_{(C\leq12)}$-alkanediyl$_{(C\leq8)}$, or a substituted version of any of these groups;

$Z_1$ is $-N^+R_3R_4-$ or $-OP(O)O^-O-$

A is $-NR_a-$, -S-, or -O-; wherein:

$R_a$ is hydrogen, alkyl$_{(C\leq6)}$, or substituted alkyl$_{(C\leq6)}$, or $R_a$ is taken together with either $R_3$ or $R_4$ and is alkanediyl$_{(C\leq8)}$, alkenediyl$_{(C\leq8)}$, alkoxydiyl$_{(C\leq8)}$, alkylaminodiyl$_{(C\leq8)}$, or a substituted version of any of these groups;

$R_1$ is a group of the formula:

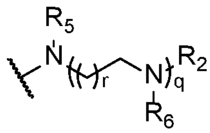

wherein:

$R_5$, $R_6$, and $R_2$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:

R' and R" are each independently hydrogen, alkyl$_{(C\leq8)}$, substituted alkyl$_{(C\leq8)}$, or $-Z_2A'R_7$; wherein:

$Z_2$ is alkanediyl$_{(C\leq6)}$, substituted alkanediyl$_{(C\leq6)}$, or a group of the formula:

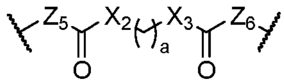

wherein:

$Z_5$ and $Z_6$ are each independently alkanediyl$_{(C\leq6)}$ or substituted alkanediyl$_{(C\leq6)}$;

$X_2$ and $X_3$ are each independently -O-, -S-, or -$NR_m$-; wherein:
$R_m$ is hydrogen, alkyl$_{(C\leq6)}$, or substituted alkyl$_{(C\leq6)}$; and

a is 0, 1, 2, 3, 4, 5, or 6;

A' is -$CHR_j$-, -C(O)O-, or -C(O)$NR_b$-;

$R_b$ is hydrogen, alkyl$_{(C\leq6)}$, or substituted alkyl$_{(C\leq6)}$; and

$R_j$ is hydrogen, halo, hydroxy, acyloxy$_{(C\leq24)}$, or substituted acyloxy$_{(C\leq24)}$;

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $R_2$ are each independently -$Z_3$A"$R_8$; wherein:

$Z_3$ is alkanediyl$_{(C\leq6)}$, substituted alkanediyl$_{(C\leq6)}$, or a group of the formula:

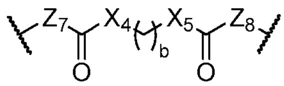

wherein:

$Z_7$ and $Z_8$ are each independently alkanediyl$_{(C\leq6)}$ or substituted alkanediyl$_{(C\leq6)}$;
$X_4$ and $X_5$ are each independently -O-, -S-, or -$NR_n$-; wherein:
$R_n$ is hydrogen, alkyl$_{(C\leq6)}$, or substituted alkyl$_{(C\leq6)}$; and b is 0, 1, 2, 3, 4, 5, or 6;

A" is -$CHR_k$-, -S-, -C(O)O-, or -C(O)NRi-;

$R_1$ is hydrogen, alkyl$_{(C\leq6)}$, or substituted alkyl$_{(C\leq6)}$; and
$R_k$ is hydrogen, halo, hydroxy, acyloxy$_{(C\leq24)}$, or substituted acyloxy$_{(C\leq24)}$; and

$R_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and
r is 1, 2, 3, or 4;

$R_1$ is a group of the formula:

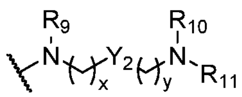

wherein:

$Y_2$ is arenediyl$_{(C\leq12)}$, heterocycloalkanediyl$_{(C\leq12)}$, heteroarenediyl$_{(C\leq12)}$, alkoxydiyl$_{(C\leq12)}$, or a substituted version of any of these groups;
$R_9$, $R_{10}$, and $R_{11}$ are each independently hydrogen, alkyl$_{(C\leq8)}$, substituted alkyl$_{(C\leq8)}$, or -$Z_4$A‴$R_{12}$; wherein:

$Z_4$ is alkanediyl$_{(C\leq6)}$, substituted alkanediyl$_{(C\leq6)}$, or a group of the formula:

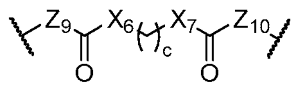

wherein:

$Z_9$ and $Z_{10}$ are each independently alkanediyl$_{(C\leq6)}$ or substituted alkanediyl$_{(C\leq6)}$;
$X_6$ and $X_7$ are each independently -O-, -S-, or -$NR_o$-; wherein:
$R_o$ is hydrogen, alkyl$_{(C\leq6)}$, or substituted alkyl$_{(C\leq6)}$; and c is 0, 1, 2, 3, 4, 5, or 6;

A‴ is -$CHR_k$-, -S-, -C(O)O-, or -C(O)$NR_1$-;

Ri is hydrogen, alkyl$_{(C\leq6)}$, or substituted alkyl$_{(C\leq6)}$; and
$R_k$ is hydrogen, halo, hydroxy, acyloxy$_{(C\leq24)}$, or substituted acyloxy$_{(C\leq24)}$; and

$R_{12}$ is alkyl$_{(C6-24)}$, substituted alkyl$_{(C6-24)}$, alkenyl$_{(C6-24)}$, substituted alkenyl$_{(C6-24)}$; and

x and y are 0, 1, 2, 3, or 4;

$R_3$ and $R_4$ are each independently hydrogen, alkyl$_{(C\leq6)}$, or substituted alkyl$_{(C\leq6)}$, or $R_3$ or $R_4$ are taken together with $R_a$ and is alkanediyl$_{(C\leq8)}$, alkenediyl$_{(C\leq8)}$, alkoxydiyl$_{(C\leq8)}$, alkylaminodiyl$_{(C\leq8)}$, or a substituted version of any of these groups; and

m, n, and p are each independently an integer selected from 0, 1, 2, 3, 4, 5, or 6;

provided that if $X_1$ is positively charged then $Z_1$ is negatively charged, and if $X_1$ is negatively charged, then $Z_1$ is positively charged;

or a pharmaceutically acceptable salt thereof.

In some embodiments, the aminolipid delivery compound is a compound of the formula:

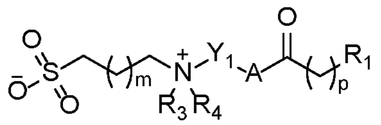

(II)

wherein:

$Y_1$ is alkanediyl$_{(C<12)}$, alkenediyl$_{(C\leq12)}$, arenediyl$_{(C\leq12)}$, heteroarenediyl$_{(C\leq12)}$, heterocycloalkanediyl$_{(C\leq12)}$, -alkanediyl$_{(C\leq8)}$-heterocycloalkanediyl$_{(C\leq12)}$, -alkanediyl$_{(C\leq8)}$-heterocycloalkanediyl$_{(C\leq12)}$-alkanediyl$_{(C\leq8)}$, -alkane-diyl$_{(C\leq8)}$-heteroarenediyl$_{(C\leq12)}$, -alkanediyl$_{(C\leq8)}$-heteroarene-diyl$_{(C\leq12)}$-alkanediyl$_{(C\leq8)}$, or a substituted version of any of these groups;

A is -$NR_a$-, -S-, or -O-; wherein:
$R_a$ is hydrogen, alkyl$_{(C\leq6)}$, or substituted alkyl$_{(C\leq6)}$, or $R_a$ is taken together with either $R_3$ or $R_4$ and is alkanediyl$_{(C\leq8)}$, alkenediyl$_{(C\leq8)}$, alkoxydiyl$_{(C\leq8)}$, alkylaminodiyl$_{(C\leq8)}$, or a substituted version of any of these groups;

$R_1$ is a group of the formula:

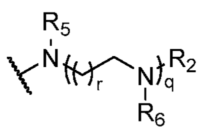

wherein:

$R_5$, $R_6$, and $R_2$ are each independently hydrogen or alkyl$_{(C\leq8)}$, -alkanediyl$_{(C\leq6)}$-$NH_2$, -alkanediyl$_{(C\leq6)}$-alkylami-

no$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:

R' and R" are each independently hydrogen, alkyl$_{(C\leq8)}$, substituted alkyl$_{(C\leq8)}$, or -$Z_2$A'$R_7$; wherein:

$Z_2$ is alkanediyl$_{(C\leq4)}$ or substituted alkanedilyl$_{(C\leq4)}$;

A' is -CH$R_j$-, -C(O)O-, or -C(O)N$R_b$-;

$R_b$ is hydrogen, alkyl$_{(C\leq6)}$, or substituted alkyl$_{(C\leq6)}$; and

$R_j$ is hydrogen, halo, hydroxy, acyloxy$_{(C\leq24)}$, or substituted acyloxy$_{(C\leq24)}$;

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $R_2$ are each independently -$Z_3$A"$R_8$; wherein:

$Z_3$ is alkanediyl$_{(C\leq4)}$ or substituted alkanedilyl$_{(C\leq4)}$;

A" is -CH$R_k$-, -C(O)O-, or -C(O)NRi-;

$R_1$ is hydrogen, alkyl$_{(C\leq6)}$, or substituted alkyl$_{(C\leq6)}$; and

$R_k$ is hydrogen, halo, hydroxy, acyloxy$_{(C\leq24)}$, or substituted acyloxy$_{(C\leq24)}$; and

$R_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and

r is 1, 2, 3, or 4;

$R_1$ is a group of the formula:

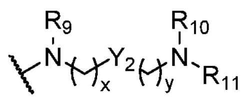

wherein:

$Y_2$ is arenediyl$_{(C\leq12)}$, heterocycloalkanediyl$_{(C\leq12)}$, heteroarenediyl$_{(C\leq12)}$, alkoxydiyl$_{(C\leq12)}$, or a substituted version of any of these groups;

$R_9$, $R_{10}$, and $R_{11}$ are each independently selected from hydrogen, alkyl$_{(C\leq8)}$, substituted alkyl$_{(C\leq8)}$, or -$Z_4$A‴$R_{12}$; wherein:

$Z_4$ is alkanediyl$_{(C\leq4)}$ or substituted alkanedilyl$_{(C\leq4)}$;

A‴ is -CH$R_k$-, -C(O)O-, or -C(O)NRi-;

$R_1$ is hydrogen, alkyl$_{(C\leq6)}$, or substituted alkyl$_{(C\leq6)}$; and

$R_k$ is hydrogen, halo, hydroxy, acyloxy$_{(C\leq24)}$, or substituted acyloxy$_{(C\leq24)}$; and

$R_{12}$ is alkyl$_{(C6-24)}$, substituted alkyl$_{(C6-24)}$, alkenyl$_{(C6-24)}$, substituted alkenyl$_{(C6-24)}$; and

x and y are 1, 2, 3, or 4;

$R_3$ and $R_4$ are each independently hydrogen, alkyl$_{(C\leq6)}$, or substituted alkyl$_{(C\leq6)}$, or $R_3$ or $R_4$ are taken together with $R_a$ and is alkanediyl$_{(C\leq8)}$, alkenediyl$_{(C\leq8)}$, alkoxydiyl$_{(C\leq8)}$, alkylaminodiyl$_{(C\leq8)}$, or a substituted version of any of these groups; and

m, n, and p are each independently an integer selected from 0, 1, 2, 3, 4, 5, or 6;

or alternatively a compound of the formula:

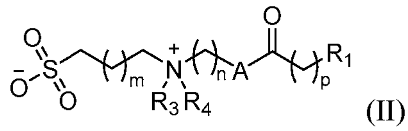
(II)

wherein:

A is -O- or -$NR_2$-, wherein:
$R_2$ is hydrogen, alkyl$_{(C\leq6)}$, or substituted alkyl$_{(C\leq6)}$;

$R_1$ is a group of the formula:

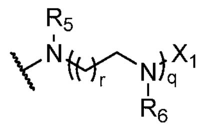

wherein:

$R_5$, $R_6$, and $X_1$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:
R' and R" are each independently $-(CH_2)_sCH(OH)R_7$, $-(CH_2)_sC(O)OR_7$, $-(CH_2)_sC(O)(NR_a)R_7$; wherein:

s is 1, 2, 3, or 4;

$R_a$ is hydrogen, alkyl$_{(C\leq6)}$, or substituted alkyl$_{(C\leq6)}$; and

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $X_1$ are each independently $-(CH_2)_tCH(OH)R_8$, $-(CH_2)_tC(O)OR_8$, $-(CH_2)_tC(O)(NR_b)R_8$; wherein:

t is 1, 2, 3, or 4;

$R_b$ is hydrogen, alkyl$_{(C\leq6)}$, or substituted alkyl$_{(C\leq6)}$; and

$R_8$ is alkyl$_{(C6-24)}$, substituted alkyl$_{(C6-24)}$, alkenyl$_{(C6-24)}$, substituted alkenyl$_{(C6-24)}$; or

q is 1, 2, or 3; and

r is 1, 2, 3, or 4;

$R_1$ is a group of the formula:

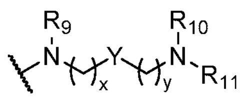

wherein:

Y is arenediyl$_{(C\leq12)}$, heterocycloalkanediyl$_{(C\leq12)}$, heteroarenediyl$_{(C\leq12)}$, or a substituted version of any of these groups;

$R_9$, $R_{10}$, and $R_{11}$ are each independently selected from hydrogen, alkyl$_{(C\leq8)}$, substituted alkyl$_{(C\leq8)}$, $-(CH_2)_uCH(OH)R_{12}$, $-(CH_2)_uC(O)OR_{12}$, $-(CH_2)_uC(O)(NR_c)R_{12}$; wherein:

u is 1, 2, 3, or 4;

$R_c$ is hydrogen, alkyl$_{(C\leq6)}$, or substituted alkyl$_{(C\leq6)}$; and

$R_{12}$ is alkyl$_{(C6-24)}$, substituted alkyl$_{(C6-24)}$, alkenyl$_{(C6-24)}$, substituted alkenyl$_{(C6-24)}$;

$R_3$ and $R_4$ are each independently hydrogen, alkyl$_{(C\leq6)}$, or substituted alkyl$_{(C\leq6)}$; and

m, n, and p are each independently an integer selected from 0, 1, 2, 3, 4, 5, or 6;

or a pharmaceutically acceptable salt thereof. The aminolipid delivery compound may be a compound of the formula:

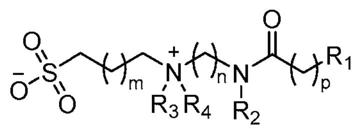

(II)

wherein:

$R_1$ is a group of the formula:

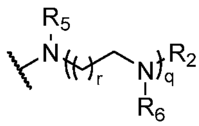

wherein:

$R_5$, $R_6$, and $R_2$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:

R' and R" are each independently hydrogen, alkyl$_{(C\leq8)}$, substituted alkyl$_{(C\leq8)}$, or $-Z_2A'R_7$; wherein:

$Z_2$ is alkanedlyl$_{(C\leq4)}$ or substituted alkanedilyl$_{(C\leq4)}$;

A' is $-CHR_j-$, -C(O)O-, or $-C(O)NR_b-$;

$R_b$ is hydrogen, alkyl$_{(C\leq6)}$, or substituted alkyl$_{(C\leq6)}$; and

$R_j$ is hydrogen, halo, hydroxy, acyloxy$_{(C\leq24)}$, or substituted acyloxy$_{(C\leq24)}$;

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $R_2$ are each independently $-Z_3A''R_8$; wherein:

$Z_3$ is alkanediyl$_{(C\leq4)}$ or substituted alkanedilyl$_{(C\leq4)}$;

A" is $-CHR_k-$, -C(O)O-, or -C(O)NRi-;

$R_1$ is hydrogen, alkyl$_{(C\leq6)}$, or substituted alkyl$_{(C\leq6)}$; and

$R_k$ is hydrogen, halo, hydroxy, acyloxy$_{(C\leq24)}$, or substituted acyloxy$_{(C\leq24)}$; and

$R_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and

r is 1, 2, 3, or 4;

$R_a$, $R_3$, and $R_4$ are each independently hydrogen, alkyl$_{(C\leq6)}$, or substituted alkyl$_{(C\leq6)}$; and

m, n, and p are each independently an integer selected from 0, 1, 2, 3, 4, 5, or 6;

or alternatively, a compound of the formula:

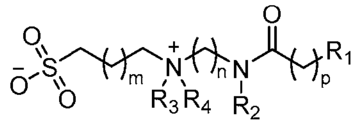

(II)

wherein:

$R_1$ is a group of the formula:

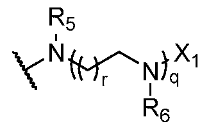

wherein:

$R_5$, $R_6$, and $X_1$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:
R' and R" are each independently $-(CH_2)_sCH(OH)R_7$, $-(CH_2)_sC(O)OR_7$, $-(CH_2)_sC(O)(NR_a)R_7$; wherein:

s is 1, 2, 3, or 4;

$R_a$ is hydrogen, alkyl$_{(C\leq6)}$, or substituted alkyl$_{(C\leq6)}$, and

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $X_1$ are each independently $-(CH_2)_tCH(OH)R_8$, $-(CH_2)_tC(O)OR_8$, $-(CH_2)_tC(O)(NR_b)R_8$; wherein:

t is 1, 2, 3, or 4;

$R_b$ is hydrogen, alkyl$_{(C\leq6)}$, or substituted alkyl$_{(C\leq6)}$; and

$R_8$ is alkyl$_{(C6-24)}$, substituted alkyl$_{(C6-24)}$, alkenyl$_{(C6-24)}$, substituted alkenyl$_{(C6-24)}$; or

q is 1, 2, or 3; and

r is 1, 2, 3, or 4;

$R_2$, $R_3$, and $R_4$ are each independently hydrogen, alkyl$_{(C\leq6)}$, or substituted alkyl$_{(C\leq6)}$; and

m, n, and p are each independently an integer selected from 0, 1, 2, 3, 4, 5, or 6;

or a pharmaceutically acceptable salt thereof. In one embodiment, the aminolipid delivery compound is a compound of the formula:

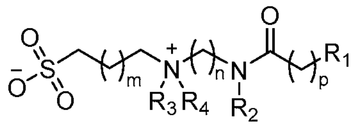
(II)

wherein:

$R_1$ is a group of the formula:

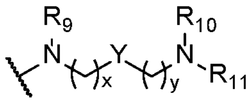

wherein:

Y is arenediyl$_{(C\leq12)}$, heterocycloalkanediyl$_{(C\leq12)}$, heteroarenediyl$_{(C\leq12)}$, or a substituted version of any of these groups;

$R_9$, $R_{10}$, and $R_{11}$ are each independently selected from hydrogen, alkyl$_{(C\leq8)}$, substituted alkyl$_{(C\leq8)}$,-$(CH_1)_uCH(OH)R_{12}$, -$(CH_2)_uC(O)OR_{12}$, -$(CH_2)_uC(O)(NR_c)R_{12}$; wherein:

u is 1, 2, 3, or 4;

$R_c$ is hydrogen, alkyl$_{(C\leq6)}$, or substituted alkyl$_{(C\leq6)}$; and

$R_{12}$ is alkyl$_{(C6-24)}$, substituted alkyl$_{(C6-24)}$, alkenyl$_{(C6-24)}$, substituted alkenyl$_{(C6-24)}$;

$R_2$, $R_3$, and $R_4$ are each independently hydrogen, alkyl$_{(C\leq6)}$, or substituted alkyl$_{(C\leq6)}$; and

m, n, and p are each independently an integer selected from 0, 1, 2, 3, 4, 5, or 6; or a pharmaceutically acceptable salt thereof.

In other embodiments, the aminolipid delivery compound is a compound of the formula:

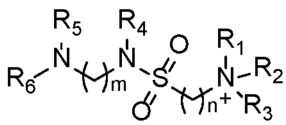
(III)

wherein:

$R_1$ is a group of the formula:

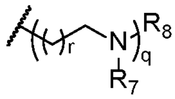

wherein:

$R_7$ and $R_8$ are each independently hydrogen, alkyl$_{(C\leq6)}$, substituted alkyl$_{(C\leq6)}$, or a group of the formula:

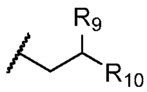

wherein:

$R_9$ is hydrogen, halo, or hydroxy, or alkoxy$_{(C\leq24)}$, acyloxy$_{(C\leq24)}$, or a substituted version of either of these groups; and

$R_{10}$ is alkyl$_{(C\leq24)}$, alkenyl$_{(C\leq24)}$, or a substituted version of either group;

q is 1, 2, or 3; and

r is 0, 1, 2, 3, or 4;

$R_2$, $R_3$, $R_4$, $R_5$, and $R_6$ are each independently hydrogen, alkyl$_{(C\leq6)}$, or substituted alkyl$_{(C\leq6)}$; and

m and n are each independently 1, 2, 3, 4, or 5;

or a pharmaceutically acceptable salt thereof. The aminolipid delivery compound may be a compound of the formula:

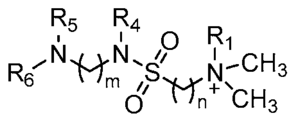 (V)

wherein:

$R_1$, $R_2$, and $R_3$ are each independently hydrogen, alkyl$_{(C\leq6)}$, substituted alkyl$_{(C\leq6)}$, or a group of the formula:

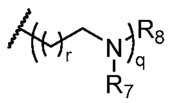

wherein:

$R_7$ and $R_8$ are each independently hydrogen, alkyl$_{(C\leq6)}$, substituted alkyl$_{(C\leq6)}$, or a group of the formula:

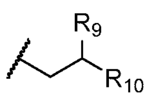

wherein:

$R_9$ is hydrogen, halo, or hydroxy, or alkoxy$_{(C\leq24)}$, acyloxy$_{(C\leq24)}$, or a substituted version of either of these groups; and
$R_{10}$ is alkyl$_{(C\leq24)}$, alkenyl$_{(C\leq24)}$, or a substituted version of either group;

q is 1, 2, or 3; and
r is 0, 1, 2, 3, or 4;

$R_4$, $R_5$, and $R_6$ are each independently hydrogen, alkyl$_{(C\leq6)}$, or substituted alkyl$_{(C\leq6)}$; and

m and n are each independently 1, 2, 3, 4, or 5;

or a pharmaceutically acceptable salt thereof. In some embodiments, the aminolipid comprises a polyester group of the formula:

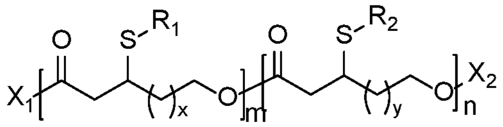

(IV)

wherein:

$X_1$ is alkyl$_{(C\leq18)}$ or substituted alkyl$_{(C\leq18)}$;

$X_2$ is hydrogen, alkyl$_{(C\leq18)}$, or substituted alkyl$_{(C\leq18)}$;

$R_1$ is -A-Z;

wherein:

A is an alkanediyl$_{(C\leq18)}$ or substituted alkanediyl$_{(C\leq18)}$;

Z is $-NR_3R_4$;

wherein:
$R_3$ and $R_4$ are each independently selected from hydrogen, alkyl$_{(C\leq18)}$, substituted alkyl$_{(C\leq18)}$; or $R_3$ and $R_4$ are taken together and are alkanediyl$_{(C\leq18)}$ or substituted alkanediyl$_{(C\leq18)}$;

$R_2$ is alkyl$_{(C\leq24)}$, alkenyl$_{(C\leq24)}$, substituted alkyl$_{(C\leq24)}$, or substituted alkenyl$_{(C\leq24)}$;

x and y are each independently 0, 1, 2, 3, 4, or 5;

m and n are each independently an integer between 0 and 250, provided that at least one of m and n is greater than 1; and

the repeating unit defined by m and n are randomly distributed throughout the polymer;

or a pharmaceutically acceptable salt thereof.

In some embodiments, A is $-CH_2CH_2-$. Z may be $-NR_3R_4$; wherein: $R_3$ and $R_4$ are each independently alkyl$_{(C\leq18)}$ or substituted alkyl$_{(C\leq18)}$. In some embodiments, $R_3$ is alkyl$_{(C\leq18)}$ such as methyl, ethyl, propyl, or butyl. Similarly, $R_4$ may be alkyl$_{(C\leq18)}$ such as methyl, ethyl, propyl, or butyl. In other embodiments, Z is $-NR_3R_4$; wherein: $R_3$ and $R_4$ are taken together and are alkanediyl$_{(C\leq18)}$ or substituted alkanediyl$_{(C\leq18)}$ such as $-CH_2CH_2CH_2CH_2-$, $-CH_2CH_2CH_2CH_2CH_2-$, or $-CH_2CH_2CH_2CH_2CH_2CH_2-$. In some embodiments, $X_1$ is alkyl$_{(C\leq18)}$ or substituted alkyl$_{(C\leq18)}$. $X_1$ may be alkyl$_{(C\leq6)}$ or substituted alkyl$_{(C\leq6)}$ such as $X_1$ is methyl. In some embodiments, $X_2$ is hydrogen. In some embodiments, $R_2$ is alkyl$_{(C\leq24)}$ such as butyl, hexyl, octyl, decyl, dodecyl, tetradecyl, hexadecyl, or octadecyl.

In one embodiment, x is 1. In one embodiment, y is 1. In some embodiments, m is an integer between 1 and 100. Additionally, m may be an integer between 1 and 50. In one embodiment, m is 1, 2, 3, 4, 5, 6, 7, 8, 9, 10, 11, 12, 13, 14, 15, 16, 17, 18, 19, or 20. In some embodiments, n is an integer between 1 and 100. Additionally, n may be an integer between 1 and 50. In one embodiments, n is 1, 2, 3, 4, 5, 6, 7, 8, 9, 10, 11, 12, 13, 14, 15, 16, 17, 18, 19, or 20. In some embodiments, the polymer comprises a molar ratio of the m repeating unit and the n repeating unit between about 10:1 to about 1:10. The molar ratio between the m repeating unit and the n repeating unit may be between about 5:1 to about 1:5 such as 3:1, 1:1, or 1:3.

In some embodiments, polymer has an average molecular weight from about 1,000 to about 100,000 as measured by gel permeation chromatography such as about 2,000 to about 10,000.

In some embodiments, the compound is further defined as:

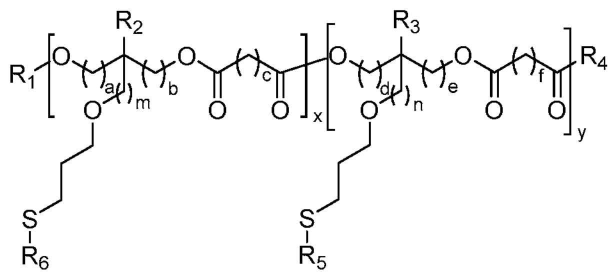

(III)

wherein:

$R_1$, $R_2$, and $R_3$ are each independently hydrogen, alkyl$_{(C\leq8)}$,or substituted alkyl$_{(C\leq8)}$;

$R_4$ is hydrogen, halo, hydroxy, alkyl$_{(C\leq8)}$,or substituted alkyl$_{(C\leq8)}$;

$R_5$ is alkyl$_{(C\leq24)}$, alkenyl$_{(C\leq24)}$, or a substituted version of either of these groups;

$R_6$ is $Y_1$-$R_7$;

wherein:

$Y_1$ is alkanediyl$_{(C\leq8)}$, substituted alkanediyl$_{(C\leq8)}$, $-(CH_2)_rC(O)-$, or $-(CH_2)_rC(O)NR_a(CH_2)_s-$;

wherein:

r and s are each independently 1, 2, or 3; and

$R_a$ is hydrogen, alkyl$_{(C\leq6)}$, or substituted alkyl$_{(C\leq6)}$;

$R_7$ is amino or heteroaryl$_{(C\leq12)}$, heterocycloalkyl$_{(C\leq12)}$, alkylamino$_{(C\leq12)}$, dialkylamino$_{(C\leq12)}$, arylalkylamino$_{(C\leq12)}$, or a substituted version of any of these groups;

a, b, m, d, e, and n are each independently 1, 2, 3, or 4;

c and f are each independently 1-10; and

x and y are each independently an integer between 0 and 250; provided that at least one of x and y is greater than 1;

or a pharmaceutically acceptable salt thereof.

In some embodiments, a is 1 or 2. In one instance, a may be 1. In some embodiments, b is 1 or 2. In one instance, b may be 1. In some embodiments, m is 1 or 2. In one instance, m may be 1. In some embodiments, d is 1 or 2. In one instance, d may be 1. In some embodiments, e is 1 or 2. In one instance, e may be 1. In some embodiments, n is 1 or 2. In one instance, n may be 1. In some embodiments, c is 5-9 such as 6 or 8. In some embodiments, f is 5-9 such as 6 or 8.

In some embodiments, $R_1$ is hydrogen. $R_2$ may be alkyl$_{(C\leq8)}$ such as ethyl. In some embodiments, $R_3$ is alkyl$_{(C\leq8)}$ such as ethyl. In some embodiments, $R_4$ is hydroxy. In other embodiments, $R_4$ is halo. In some embodiments, $R_5$ is alkyl$_{(C\leq24)}$ or substituted alkyl$_{(C\leq24)}$. $R_5$ may be alkyl$_{(C\leq24)}$ such as hexyl, decyl, or dodecyl.

In some embodiments, $R_6$ is $Y_1$-$R_7$;
wherein:

$Y_1$ is alkanediyl$_{(C\leq8)}$ or substituted alkanediyl$_{(C\leq8)}$; and
$R_7$ is amino or heteroaryl$_{(C\leq12)}$, heterocycloalkyl$_{(C\leq12)}$, alkylamino$_{(C\leq12)}$, dialkylamino$_{(C\leq12)}$, arylalkylamino$_{(C\leq12)}$, or a substituted version of any of these groups.

$Y_1$ may be $-CH_2CH_2-$. In some embodiments, $R_7$ is amino. In other embodiments, $R_7$ is alkylamino$_{(C\leq12)}$, dialkylamino$_{(C\leq12)}$, arylalkylamino$_{(C\leq12)}$, or a substituted version of any of these groups. $R_7$ may be $-NH_2$, $-NHCH_2CH_2CH_2CH_3$, $-N(CH_3)_2$, or $-N(CH_2CH_3)_2$.

In other embodiments, $R_6$ is $Y_1$-$R_7$;
wherein:

$Y_1$ is $-(CH_2)_rC(O)-$ or $-(CH_2)_rC(O)NR_a(CH_2)_s-$;

wherein:

r and s are each independently 1, 2, 3, or 4; and

$R_a$ is hydrogen, alkyl$_{(C\leq6)}$, or substituted alkyl$_{(C\leq6)}$; and

$R_7$ is amino or heteroaryl$_{(C\leq12)}$, heterocycloalkyl$_{(C\leq12)}$, alkylamino$_{(C\leq12)}$, dialkylamino$_{(C\leq12)}$, arylalkylamino$_{(C\leq12)}$, or a substituted version of any of these groups.

In some embodiments, r is 2 or 3. In one instance, r may be 3. In some embodiments, s is 2 or 3. In one instance, s may be 3. In some embodiments, $R_a$ is hydrogen. In some embodiments, $R_7$ is heteroaryl$_{(C\leq12)}$ or substituted heteroaryl$_{(C\leq12)}$ such as pyridinyl or imidazolyl. In other embodiments, $R_7$ is heterocycloalkyl$_{(C\leq12)}$ or substituted heterocycloalkyl$_{(C\leq12)}$ such as morpholyl, pyrrolidyl, piperidyl, piperazyl, 4-*N*-methylpiperazyl, 4-*N*-(2-hydroxyethyl)piperazyl, or 4-*N*-(2-dimethylaminoethyl)piperazyl. In other embodiments, $R_7$ is dialkylamino$_{(C\leq12)}$ or substituted dialkylamino$_{(C\leq12)}$ such as dimethylamino, diethylamino, dibutylamino, di(2-hydroxyethyl)amino. In other embodiments, $R_7$ is arylalkylamino$_{(C\leq12)}$ or substituted arylalkylamino$_{(C\leq12)}$ such as (*N*-methyl,*N*-phenyl)amino.

In other embodiments, the dendrimer has the formula:

Core-(Repeating Unit)$_n$-Terminating Group (I)

wherein the core is linked to the repeating unit by removing one or more hydrogen atoms from the core and replacing with the repeating unit and wherein:

the core is alkylamine$_{(C\leq18)}$, dialkylamine$_{(C\leq36)}$, heterocycloalkane$_{(C\leq12)}$, or a substituted version of any of these groups; and

wherein the repeating unit comprises a degradable diacyl group and a linker group;

the degradable diacyl group has the formula:

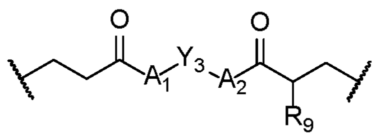
(VII)

wherein:

$A_1$ and $A_2$ are each independently -O- or $-NR_a-$, wherein: $R_a$ is hydrogen, alkyl$_{(C\leq6)}$, or substituted alkyl$_{(C\leq6)}$;

$Y_3$ is alkanediyl$_{(C<12)}$, alkenediyl$_{(C\leq12)}$, arenediyl$_{(C\leq12)}$, or a substituted version of any of these groups; or a group of the formula:

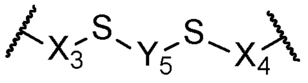
or
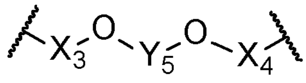

wherein:

$X_3$ and $X_4$ are alkanediyl$_{(C<12)}$, alkenediyl$_{(C\leq12)}$, arenediyl$_{(C\leq12)}$, or a substituted version of any of these groups;

$Y_5$ is a covalent bond, alkanediyl$_{(C<12)}$, alkenediyl$_{(C\leq12)}$, arenediyl$_{(C\leq12)}$, or a substituted version of any of these groups; and

$R_9$ is alkyl$_{(C\leq8)}$ or substituted alkyl$_{(C\leq8)}$;

the linker group has the formula:

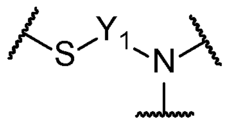

(VI)

wherein:
$Y_1$ is alkanediyl$_{(C<12)}$, alkenediyl$_{(C\leq12)}$, arenediyl$_{(C\leq12)}$, or a substituted version of any of these groups; and

wherein when the repeating unit comprises a linker group, then the linker group is attached to a degradable diacyl group on both the nitrogen and the sulfur atoms of the linker group, wherein the first group in the repeating unit is a degradable diacyl group, wherein for each linker group, the next group comprises two degradable diacyl groups attached to the nitrogen atom of the linker group; and wherein n is the number of linker groups present in the repeating unit; and

the terminating group, wherein the terminating group has the formula:

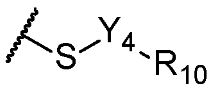

(VIII)

wherein:

$Y_4$ is alkanediyl$_{(C\leq18)}$, alkenediyl$_{(C\leq18)}$, or a substituted version of either of these groups;

$R_{10}$ is hydrogen, carboxy, hydroxy, or

aryl$_{(C\leq12)}$, alkylamino$_{(C\leq12)}$, dialkylamino$_{(C\leq12)}$, *N*-heterocycloalkyl$_{(C\leq12)}$, -C(O)N($R_{11}$)-alkanediyl$_{(C\leq6)}$-heterocycloalkyl$_{(C\leq12)}$, -C(O)-alkylamino$_{(C\leq12)}$, -C(O)-dialkylamino$_{(C\leq12)}$, -C(O)-*N*-heterocyclo-alkyl$_{(C\leq12)}$, wherein:
$R_{11}$ is hydrogen, alkyl$_{(C\leq6)}$, or substituted alkyl$_{(C\leq6)}$;

wherein the final degradable diacyl group in the chain is attached to a terminating group;

n is 0, 1, 2, 3, 4, 5, or 6;

or a pharmaceutically acceptable salt thereof.

In some embodiments, the terminating group is further defined by the formula:

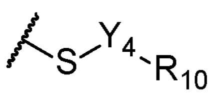

(VIII)

wherein:

$Y_4$ is alkanediyl$_{(C\leq18)}$, alkenediyl$_{(C\leq18)}$, or a substituted version of either of these groups; and

$R_{10}$ is hydrogen.

In other embodiments, the terminating group is further defined by the formula:

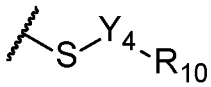 (VIII)

wherein:

$Y_4$ is alkanediyl$_{(C\leq 18)}$; and
$R_{10}$ is hydrogen.

In some embodiments, $Y_4$ is alkanediyl$_{(C4-18)}$. In other embodiments, the terminating group is further defined by the formula:

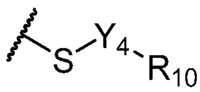 (VIII)

wherein:

$Y_4$ is alkanediyl$_{(C\leq 18)}$, alkenediyl$_{(C\leq 18)}$, or a substituted version of either of these groups; and

$R_{10}$ is alkylamino$_{(C\leq 12)}$, dialkylamino$_{(C\leq 12)}$, *N*-heterocycloalkyl$_{(C\leq 12)}$.

In other embodiments, the terminating group is further defined by the formula:

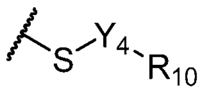 (VIII)

wherein:

$Y_4$ is alkanediyl$_{(C\leq 18)}$, alkenediyl$_{(C\leq 18)}$, or a substituted version of either of these groups; and

$R_{10}$ is hydroxy.

In some embodiments, the core is further defined by the formula:

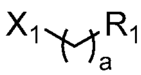 (II)

wherein:

$X_1$ is alkylamino$_{(C\leq 12)}$, dialkylamino$_{(C\leq 12)}$, heterocycloalkyl$_{(C\leq 12)}$, heteroaryl$_{(C\leq 12)}$, or a substituted version thereof;

$R_1$ is amino, hydroxy, or mercapto, or alkylamino$_{(C\leq 12)}$, dialkylamino$_{(C\leq 12)}$, or a substituted version of either of these groups; and

a is 1, 2, 3, 4, 5, or 6.

In some embodiments, $X_1$ is alkylamino$_{(C\leq 12)}$ or substituted alkylamino$_{(C\leq 12)}$ such as ethylamino. In other embodiments, $X_1$ is dialkylamino$_{(C\leq 12)}$ or substituted dialkylamino$_{(C\leq 12)}$ such as dimethylamino. In other embodiments, $X_1$ is heterocycloalkyl$_{(C\leq 12)}$ or substituted heterocycloalkyl$_{(C\leq 12)}$ such as 4-piperidinyl, *N*-piperidinyl, N-morpholinyl, *N*-pyrrolidinyl, 2-pyrrolidinyl, *N*-piperazinyl, or *N*-4-methylpiperadizinyl. In other embodiments, $X_1$ is heteroaryl$_{(C\leq 12)}$ or substituted heteroaryl$_{(C\leq 12)}$ such as 2-pyridinyl or *N*-imidazolyl.

$R_1$ may be hydroxyl or may be amino. In other embodiments, $R_1$ is alkylamino$_{(C\leq 12)}$ or substituted alkylami-

no$_{(C\leq12)}$. In some embodiments, $R_1$ is alkylamino$_{(C\leq12)}$ such as methylamino or ethylamino. In some embodiments, a is 1, 2, 3, or 4. Specifically, a may be 2 or 3. In some embodiments, a is 2. In other embodiments, a is 3. In some embodiments, the core is further defined as a compound of the formula:

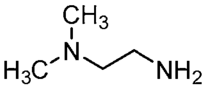, 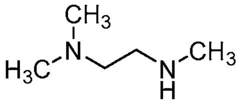, 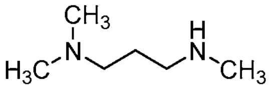, 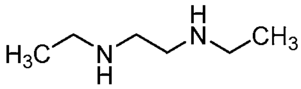,

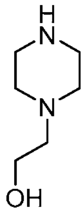, 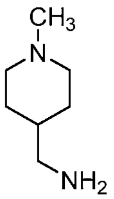, 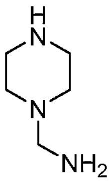, 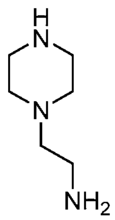, 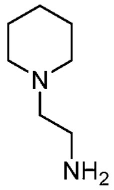, 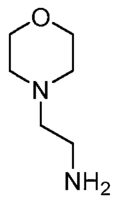, 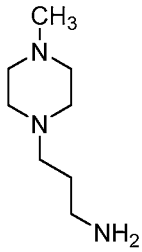, 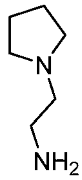,

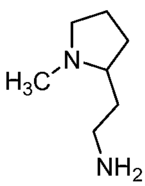, 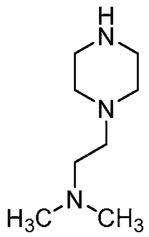, 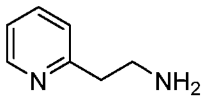, or 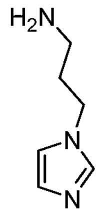.

In other embodiments, the core is further defined by the formula:

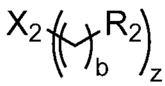 (III)

wherein:

$X_2$ is $N(R_5)_y$;

$R_5$ is hydrogen or alkyl$_{(C\leq8)}$, or substituted alkyl$_{(C\leq18)}$; and

y is 0, 1, or 2, provided that the sum of y and z is 3;

$R_2$ is amino, hydroxy, or mercapto, or alkylamino$_{(C\leq12)}$, dialkylarmno$_{(C\leq12)}$, or a substituted version of either of these groups;

b is 1, 2, 3, 4, 5, or 6; and

z is 1, 2, 3; provided that the sum of z and y is 3.

In some embodiments, $X_2$ is N. In other embodiments, $X_2$ is $NR_5$, wherein $R_5$ is hydrogen or alkyl$_{(C\leq8)}$. In some embodiments, $R_5$ is hydrogen. In other embodiments, $R_5$ is methyl. In some embodiments, z is 3. In other embodiments, z is 2.

$R_2$ may be hydroxyl or may be amino. In other embodiments, $R_2$ is alkylamino$_{(C\leq12)}$ or substituted alkylamino$_{(C\leq12)}$. $R_2$ may be alkylamino$_{(C\leq12)}$ such as methylamino. In other embodiments, $R_2$ is dialkylamino$_{(C\leq12)}$ or substituted dialkylamino$_{(C\leq12)}$. $R_2$ may be dialkylamino$_{(C\leq12)}$ such as dimethylamino. In some embodiments, b is 1, 2, 3, or 4. For example, b may be 2 or 3. In some embodiments, b is 2. In other embodiments, b is 3. In other embodiments, the core is further defined as:

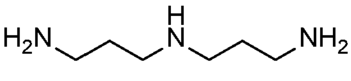, 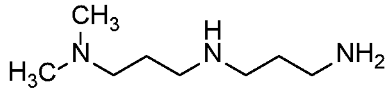, 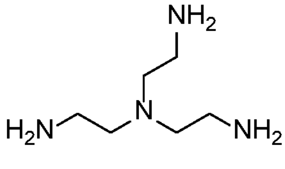,

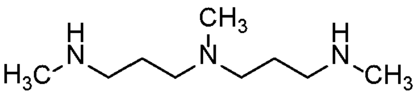, 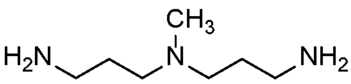, 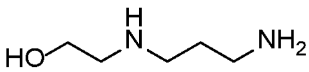, or

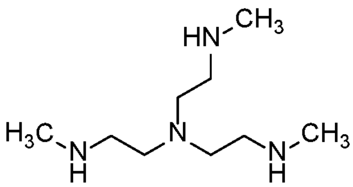.

In other embodiments, the core is further defined as:

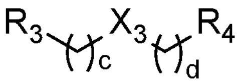 (IV)

wherein:

$X_3$ is $-NR_6-$, -O-, or alkylaminodiyl$_{(C\leq8)}$, alkoxydiyl$_{(C\leq8)}$, arenediyl$_{(C\leq8)}$, heteroarenediyl$_{(C\leq8)}$, heterocycloalkanediyl$_{(C\leq8)}$, or a substituted version of any of these groups, wherein $R_6$ is hydrogen, alkyl$_{(C\leq8)}$, or substituted alkyl$_{(C\leq8)}$;

$R_3$ and $R_4$ are each independently amino, hydroxy, or mercapto, or alkylamino$_{(C\leq12)}$, dialkylamino$_{(C\leq12)}$, wherein, optionally, either of these groups is substituted by one or more substituents selected from the group consisting of: -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, - $S(O)_2OH$, and $-S(O)_2NH_2$; wherein: c and d are each independently 1, 2, 3, 4, 5, or 6.

In some embodiments, $X_3$ is -O-. In other embodiments, $X_3$ is $-NR_6-$, wherein $R_6$ is hydrogen, alkyl$_{(C\leq8)}$, or substituted alkyl$_{(C\leq8)}$ such as -NH- or $-NCH_3-$. In other embodiments, $X_3$ is alkylaminodiyl$_{(C\leq8)}$ or substituted alkylaminodiyl$_{(C\leq8)}$ such as $-NHCH_2CH_2NH-$ or $-NHCH_2CH_2NHCH_2CH_2NH-$. In other embodiments, $X_3$ is alkoxydiyl$_{(C\leq8)}$ or substituted alkoxydiyl$_{(C\leq8)}$ such as $-OCH_2CH_2O-$. In other embodiments, $X_3$ is arenediyl$_{(C\leq8)}$ or substituted arenediyl$_{(C\leq8)}$ such as benzenediyl. In other embodiments, $X_3$ is heterocycloalkanediyl$_{(C\leq8)}$ or substituted heterocycloalkanediyl$_{(C\leq8)}$ such as *N,N'*-piperazindiyl.

In some embodiments, $R_3$ is amino. $R_3$ may be hydroxy. $R_3$ may be alkylamino$_{(C\leq12)}$ or substituted alkylamino$_{(C\leq12)}$. In some embodiments, $R_3$ is alkylamino$_{(C\leq12)}$ such as methylamino. $R_3$ may be dialkylamino$_{(C\leq12)}$ or substituted dialkylamino$_{(C\leq12)}$. In some embodiments, $R_3$ is dialkylamino$_{(C\leq12)}$ such as dimethylamino. $R_4$ may be amino. $R_4$ may be hydroxy. $R_4$ may be alkylamino$_{(C\leq12)}$ or substituted alkylamino$_{(C\leq12)}$. In some embodiments, $R_4$ is alkylamino$_{(C\leq12)}$ such as methylamino. $R_4$ may be dialkylamino$_{(C\leq12)}$ or substituted dialkylamino$_{(C\leq12)}$. In some embodiments, $R_4$ is dialkylamino$_{(C\leq12)}$ such as dimethylamino. In some embodiments, $R_4$ is $-(CH_2CH_2N)_e(R_c)R_d$:
wherein:

e is 1, 2, or 3;

$R_c$ and $R_d$ are each independently hydrogen, alkyl$_{(C\leq6)}$, or substituted alkyl$_{(C\leq6)}$.

In some embodiments, e is 1 or 2. In one instance, e may be 1. In some embodiments, $R_c$ is hydrogen. In some embodiments, $R_d$ is hydrogen. In some embodiments, c is 1, 2, 3, or 4. In one instance, c may be 2 or 3. In some

embodiments, c is 2. In other embodiments, c is 3. In some embodiments, d is 1, 2, 3, or 4. In one instance, d may be 2 or 3. In some embodiments, d is 2. In other embodiments, d is 3. In other embodiments, the core is further defined as:

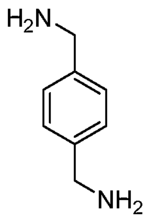,
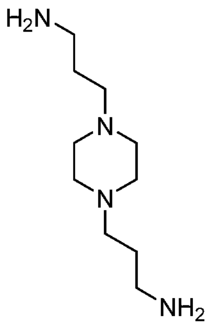,
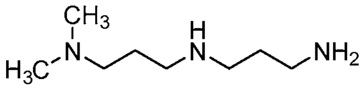,
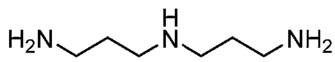,
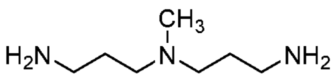,
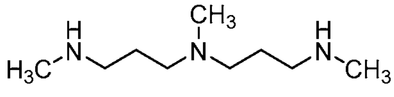,
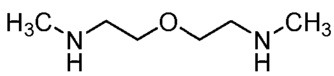,
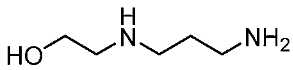,
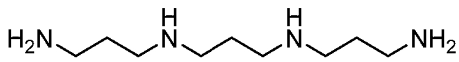,
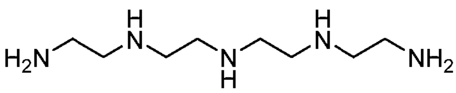,
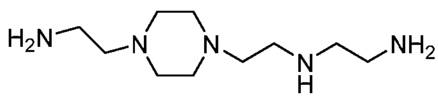,

or

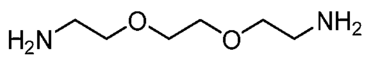.

In other embodiments, the core is alkylamine$_{(C\leq18)}$, dialkylamine$_{(C\leq36)}$, heterocycloalkane$_{(C\leq12)}$, or a substituted version of any of these groups. In some embodiments, the core is an alkylamine$_{(C\leq18)}$ or substituted alkylamine$_{(C\leq18)}$ such as octylamine, decylamine, dodecylamine, tetradecylamine, hexadecylamine, and octadecylamine. In other embodiments, the core is an dialkylamine$_{(C\leq36)}$ or substituted dialkylamine$_{(C\leq36)}$ such as *N*-methyl, *N*-dodecylamine, dioctylamine, or didecylamine. In other embodiments, the core is heterocycloalkane$_{(C\leq12)}$ or substituted heterocycloalkane$_{(C\leq12)}$ such as 4-*N*-methylpiperazinyl.

In some embodiments, $Y_1$ is alkanediyl$_{(C\leq8)}$ or substituted alkanediyl$_{(C\leq8)}$. $Y_1$ may be alkanediyl$_{(C\leq8)}$ such as $-CH_2CH_2-$. In some embodiments, $Y_3$ is alkanediyl$_{(C\leq8)}$ or substituted alkanediyl$_{(C\leq8)}$. $Y_3$ may be alkanediyl$_{(C\leq8)}$ such as $-CH_2CH_2-$. In other embodiments, $Y_3$ is:

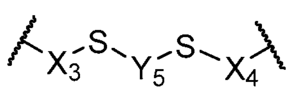

wherein:

$X_3$ and $X_4$ are alkanediyl$_{(C\leq12)}$, alkenediyl$_{(C\leq12)}$, arenediyl$_{(C\leq12)}$, or a substituted version of any of these groups;

$Y_5$ is a covalent bond, alkanediyl$_{(C\leq12)}$, alkenediyl$_{(C\leq12)}$, arenediyl$_{(C\leq12)}$, or a substituted version of any of these groups.

In some embodiments, $X_3$ is alkanediyl$_{(C\leq12)}$ or substituted alkanediyl$_{(C\leq12)}$ such as $-CH_2CH_2-$. In some embodiments, $X_4$ is alkanediyl$_{(C\leq12)}$ or substituted alkanediyl$_{(C\leq12)}$ such as $-CH_2CH_2-$. $Y_5$ may be a covalent bond.

In other embodiments, $Y_3$ is:

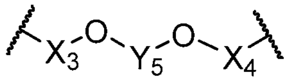

wherein:

$X_3$ and $X_4$ are alkanediyl$_{(C\leq12)}$, alkenediyl$_{(C\leq12)}$, arenediyl$_{(C\leq12)}$, or a substituted version of any of these groups;

$Y_5$ is a covalent bond, alkanediyl$_{(C\leq12)}$, alkenediyl$_{(C\leq12)}$, arenediyl$_{(C\leq12)}$, or a substituted version of any of these groups.

In some embodiments, $X_3$ is alkanediyl$_{(C\leq12)}$ or substituted alkanediyl$_{(C\leq12)}$ such as $X_3$ is $-CH_2CH_2-$. In some embodiments, $X_4$ is alkanediyl$_{(C\leq12)}$ or substituted alkanediyl$_{(C\leq12)}$ such as $-CH_2CH_2-$. $Y_5$ may be a covalent bond. In other embodiments, $Y_5$ is $-CH_2-$ or $-C(CH_3)_2-$

In some embodiments, $A_1$ is -O-. In other embodiments, $A_1$ is $-NR_a-$. $R_a$ may be hydrogen. In some embodiments, $A_2$ is -O-. In other embodiments, $A_2$ is $-NR_a-$. $R_a$ may be hydrogen. In some embodiments, $R_9$ is alkyl$_{(C\leq8)}$ such as methyl. In some embodiments, n is 0, 1, 2, 3 or 4. n may be 0, 1, 2, or 3. In some embodiments, n is 0. In other embodiments, n is 1. In other embodiments, n is 2. In other embodiments, n is 3.

In other embodiments, the compounds are further defined as:

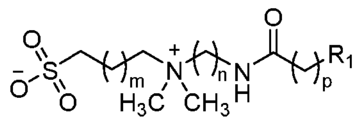
(II)

wherein:

$R_1$ is a group of the formula:

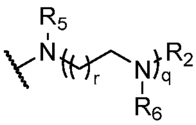

wherein:

$R_5$, $R_6$, and $R_2$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:

R' and R" are each independently hydrogen, alkyl$_{(C\leq8)}$, substituted alkyl$_{(C\leq8)}$, or $-Z_2A'R_7$; wherein:

$Z_2$ is alkanediyl$_{(C\leq4)}$ or substituted alkanediyl$_{(C\leq4)}$;

A' is $-CHR_j-$, -C(O)O-, or $-C(O)NR_b-$;

$R_b$ is hydrogen, alkyl$_{(C\leq6)}$, or substituted alkyl$_{(\leq6)}$; and

$R_j$ is hydrogen, halo, hydroxy, acyloxy$_{(C\leq24)}$, or substituted acyloxy$_{(C\leq24)}$;

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $R_2$ are each independently $-Z_3A''R_8$; wherein:

$Z_3$ is alkanediyl$_{(C\leq4)}$ or substituted alkanediyl$_{(C\leq4)}$;

A" is $-CHR_k-$, -C(O)O-, or -C(O)NRi-;

$R_l$ is hydrogen, alkyl$_{(C\leq 6)}$, or substituted alkyl$_{(C\leq 6)}$; and

$R_k$ is hydrogen, halo, hydroxy, acyloxy$_{(C\leq 24)}$, or substituted acyloxy$_{(C\leq 24)}$; and

$R_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and

r is 1, 2, 3, or 4; and

m, n, and p are each independently an integer selected from 0, 1, 2, 3, 4, 5, or 6;

or alternatively as a compound of the formula:

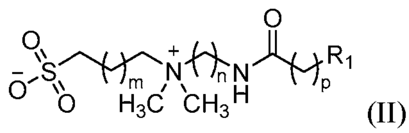
(II)

wherein:

$R_1$ is a group of the formula:

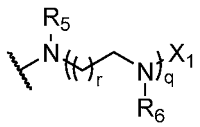

wherein:

$R_5$, $R_6$, and $X_1$ are each independently hydrogen or alkyl$_{(C\leq 8)}$, -alkanediyl$_{(C\leq 6)}$-$NH_2$, -alkanediyl$_{(C\leq 6)}$-alkylamino$_{(C\leq 8)}$, -alkanediyl$_{(C\leq 6)}$-dialkylamino$_{(C\leq 12)}$, -alkanediyl$_{(C\leq 6)}$-NR'R", or a substituted version of any of these groups wherein:
R' and R" are each independently hydrogen, alkyl$_{(C\leq 8)}$, substituted alkyl$_{(C\leq 8)}$, $-(CH_2)_sCH(OH)R_7$, $-(CH_2)_sC(O)OR_7$, or $-(CH_2)_sC(O)(NR_a)R_7$; wherein:

s is 1, 2, 3, or 4;

$R_a$ is hydrogen, alkyl$_{(C\leq 6)}$, or substituted alkyl$_{(\leq 6)}$; and

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $X_1$ are each independently $-(CH_2)_tCH(OH)R_8$, $-(CH_2)_tC(O)OR_8$, $-(CH_2)_tC(O)(NR_b)R_8$; wherein:

t is 1, 2, 3, or 4;

$R_b$ is hydrogen, alkyl$_{(C\leq 6)}$, or substituted alkyl$_{(\leq 6)}$; and

$R_8$ is alkyl$_{(C6-24)}$, substituted alkyl$_{(C6-24)}$, alkenyl$_{(C6-24)}$, substituted alkenyl$_{(C6-24)}$; or

q is 1, 2, or 3; and

r is 1, 2, 3, or 4; and

m, n, and p are each independently an integer selected from 0, 1, 2, 3, 4, 5, or 6;

or a pharmaceutically acceptable salt thereof. In some embodiments, the compounds are further defined as:

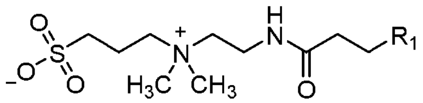

(III)

wherein:

$R_1$ is a group of the formula:

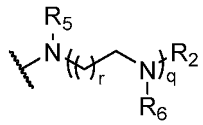

wherein:

$R_5$, $R_6$, and $R_2$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein: R' and R" are each independently are each independently hydrogen, alkyl$_{(C\leq8)}$, substituted alkyl$_{(C\leq8)}$, or -$Z_2$A'$R_7$; wherein:

$Z_2$ is alkanediyl$_{(C\leq4)}$ or substituted alkanediyl$_{(C\leq4)}$;

A' is -CH$R_j$-, -C(O)O-, or -C(O)N$R_b$-;

$R_b$ is hydrogen, alkyl$_{(C\leq6)}$, or substituted alkyl$_{(\leq6)}$; and

$R_j$ is hydrogen, halo, hydroxy, acyloxy$_{(C\leq24)}$, or substituted acyloxy$_{(C\leq24)}$;

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $R_2$ are each independently -$Z_3$A"$R_8$; wherein:

$Z_3$ is alkanediyl$_{(C\leq4)}$ or substituted alkanediyl$_{(C\leq4)}$;

A" is -CH$R_k$-, -C(O)O-, or -C(O)NRi-;

$R_l$ is hydrogen, alkyl$_{(C\leq6)}$, or substituted alkyl$_{(\leq6)}$; and

$R_k$ is hydrogen, halo, hydroxy, acyloxy$_{(C\leq24)}$, or substituted acyloxy$_{(C\leq24)}$; and

$R_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and

r is 1, 2, 3, or 4;

or alternatively as a compound of the formula:

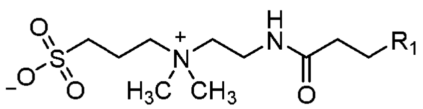

(III)

wherein:

$R_1$ is a group of the formula:

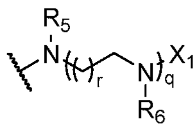

wherein:

$R_5$, $R_6$, and $X_1$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:
R' and R" are each independently are each independently hydrogen, alkyl$_{(C\leq8)}$, substituted alkyl$_{(C\leq8)}$, $-(CH_2)_sCH(OH)R_7$, $-(CH_2)_sC(O)OR_7$, or $-(CH_2)_sC(O)(NR_a)R_7$; wherein:

s is 1, 2, 3, or 4;

$R_a$ is hydrogen, alkyl$_{(C\leq6)}$, or substituted alkyl$_{(\leq6)}$; and

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $X_1$ are each independently $-(CH_2)_tCH(OH)R_8$, $-(CH_2)_tC(O)OR_8$, $-(CH_2)_tC(O)(NR_b)R_8$; wherein:

t is 1, 2, 3, or 4;

$R_b$ is hydrogen, alkyl$_{(C\leq6)}$, or substituted alkyl$_{(\leq6)}$; and

$R_8$ is alkyl$_{(C6-24)}$, substituted alkyl$_{(C6-24)}$, alkenyl$_{(C6-24)}$, substituted alkenyl$_{(C6-24)}$; or

q is 1, 2, or 3; and

r is 1, 2, 3, or 4;

or a pharmaceutically acceptable salt thereof. In some embodiments, the compounds are further defined as:

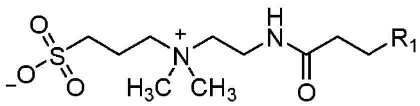
(III)

wherein:

$R_1$ is a group of the formula:

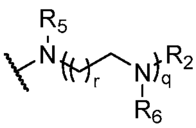

wherein:
$R_5$, $R_6$, and $R_2$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:

R' and R" are each independently are each independently hydrogen, alkyl$_{(C\leq8)}$, substituted alkyl$_{(C\leq8)}$, or $-Z_2A'R_7$; wherein:

$Z_2$ is alkanediyl$_{(C\leq4)}$ or substituted alkanediyl$_{(C\leq4)}$;

A' is $-CHR_j-$, -C(O)O-, or $-C(O)NR_b-$;

$R_b$ is hydrogen, alkyl$_{(C\leq6)}$, or substituted alkyl$_{(\leq6)}$; and

$R_j$ is hydrogen, halo, hydroxy, acyloxy$_{(C\leq24)}$, or substituted acyloxy$_{(C\leq24)}$;

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $R_2$ are each independently -$Z_3$A"$R_8$; wherein:

$Z_3$ is alkanediyl$_{(C\leq4)}$ or substituted alkanediyl$_{(C\leq4)}$;

A" is -CHR$_k$-, -C(O)O-, or -C(O)NRi-;

$R_l$ is hydrogen, alkyl$_{(C\leq6)}$, or substituted alkyl$_{(\leq6)}$; and

$R_k$ is hydrogen, halo, hydroxy, acyloxy$_{(C\leq24)}$, or substituted acyloxy$_{(C\leq24)}$; and

$R_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and

r is 1, 2, 3, or 4;

or alternatively as a compound of the formula:

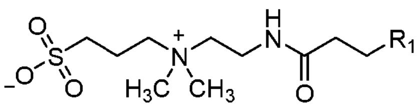

(III)

wherein:

$R_1$ is a group of the formula:

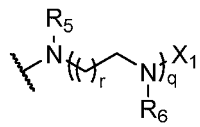

wherein:

$R_5$ is -(CH$_2$)$_t$CH(OH)$R_8$, -(CH$_2$)$_t$C(O)O$R_8$, -(CH$_2$)$_t$C(O)(NH)$R_8$; wherein:

t is 1 or 2; and

$R_8$ is alkyl$_{(C6-24)}$, substituted alkyl$_{(C6-24)}$, alkenyl$_{(C6-24)}$, substituted alkenyl$_{(C6-24)}$;

$R_6$ is alkyl$_{(C\leq8)}$ or substituted alkyl$_{(C\leq8)}$; and

$X_1$ is -alkanediyl$_{(C\leq6)}$-NR'R" or a substituted version of this group wherein:
R' and R" are each independently -(CH$_2$)$_s$CH(OH)$R_7$, -(CH$_2$)$_s$C(O)O$R_7$, or -(CH$_2$)$_s$C(O)(NH)$R_7$; wherein:

s is 1 or 2; and

$R_7$ is alkyl$_{(C6-24)}$, substituted alkyl$_{(C6-24)}$, alkenyl$_{(C6-24)}$, substituted alkenyl$_{(C6-24)}$; or

q is 1 or 2; and

r is 1 or 2;

or a pharmaceutically acceptable salt thereof. In other embodiments, the compounds are further defined as:

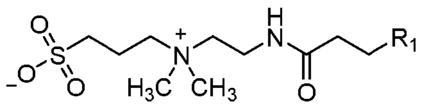
(III)

wherein:

$R_1$ is a group of the formula:

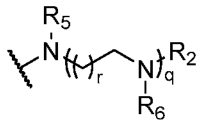

wherein:

$R_5$ is $-Z_3A''R_8$; wherein:

$Z_3$ is alkanediyl$_{(C\leq 2)}$ or substituted alkanediyl$_{(C\leq 2)}$;

A" is $-CHR_k-$, -C(O)O-, or -C(O)NRi-;

$R_l$ is hydrogen, alkyl$_{(C\leq 6)}$, or substituted alkyl$_{(C\leq 6)}$; and

$R_k$ is hydrogen, halo, hydroxy, acyloxy$_{(C\leq 24)}$, or substituted acyloxy$_{(C\leq 24)}$; and

$R_8$ is alkyl$_{(C6-24)}$, substituted alkyl$_{(C6-24)}$, alkenyl$_{(C6-24)}$, substituted alkenyl$_{(C6-24)}$;

$R_6$ is alkyl$_{(C\leq 8)}$ or substituted alkyl$_{(C\leq 8)}$; and

$R_2$ is -alkanediyl$_{(C\leq 6)}$-NR'R" or a substituted version of this group wherein:

R' and R" are each independently $-Z_2A'R_7$; wherein:

$Z_2$ is alkanediyl$_{(C\leq 2)}$ or substituted alkanediyl$_{(C\leq 2)}$;

A' is $-CHR_j-$, -C(O)O-, or $-C(O)NR_b-$;

$R_b$ is hydrogen, alkyl$_{(C\leq 6)}$, or substituted alkyl$_{(C\leq 6)}$; and

$R_j$ is hydrogen, halo, hydroxy, acyloxy$_{(C\leq 24)}$, or substituted acyloxy$_{(C\leq 24)}$;

$R_7$ is alkyl$_{(C6-24)}$, substituted alkyl$_{(C6-24)}$, alkenyl$_{(C6-24)}$, substituted alkenyl$_{(C6-24)}$; or

q is 1 or 2; and

r is 1 or 2;

or alternatively as a compound of the formula:

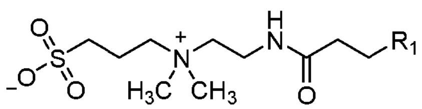
(III)

wherein:

$R_1$ is a group of the formula:

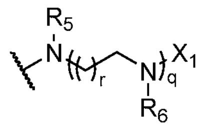

wherein:

$R_5$ is alkyl$_{(C\leq8)}$ or substituted alkyl$_{(C\leq8)}$;

$R_6$ is -alkanediyl$_{(C\leq6)}$-NR'R" or a substituted version of this group wherein: R' and R" are each independently alkyl$_{(C\leq8)}$, substituted alkyl$_{(C\leq8)}$, $-(CH_2)_sCH(OH)R_7$, $-(CH_2)_sC(O)OR_7$, or $-(CH_2)_sC(O)(NH)R_7$; wherein:

s is 1 or 2; and

$R_7$ is alkyl$_{(C6-24)}$, substituted alkyl$_{(C6-24)}$, alkenyl$_{(C6-24)}$, substituted alkenyl$_{(C6-24)}$; and

$X_1$ is -alkanediyl$_{(C\leq6)}$-NR'R" or a substituted version of this group wherein: R' and R" are each independently alkyl$_{(C\leq8)}$, substituted alkyl$_{(C\leq8)}$, $-(CH_2)_sCH(OH)R_7$, $-(CH_2)_sC(O)OR_7$, or $-(CH_2)_sC(O)(NH)R_7$; wherein:

s is 1 or 2; and

$R_7$ is alkyl$_{(C6-24)}$, substituted alkyl$_{(C6-24)}$, alkenyl$_{(C6-24)}$, substituted alkenyl$_{(C6-24)}$;

q is 1 or 2; and

r is 1 or 2;

or a pharmaceutically acceptable salt thereof. In other embodiments, the compounds are further defined as:

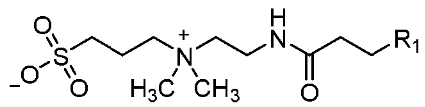
(III)

wherein:

$R_1$ is a group of the formula:

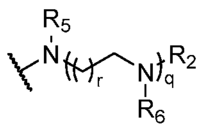

wherein:

$R_5$ is alkyl$_{(C\leq8)}$ or substituted alkyl$_{(C\leq8)}$;

$R_6$ is -alkanediyl$_{(C\leq6)}$-NR'R" or a substituted version of this group wherein:

R' and R" are each independently alkyl$_{(C\leq8)}$, substituted alkyl$_{(C\leq8)}$, or $-Z_2A'R_7$; wherein:

$Z_2$ is alkanediyl$_{(C\leq2)}$ or substituted alkanediyl$_{(C\leq2)}$;

A' is $-CHR_j-$, $-C(O)O-$, or $-C(O)NR_b-$;

$R_b$ is hydrogen, $alkyl_{(C\leq6)}$, or substituted $alkyl_{(\leq6)}$; and

$R_j$ is hydrogen, hydroxy, $acyloxy_{(C\leq6)}$, or substituted $acyloxy_{(C\leq6)}$;

$R_7$ is $alkyl_{(C6-24)}$, substituted $alkyl_{(C6-24)}$, $alkenyl_{(C6-24)}$, substituted $alkenyl_{(C6-24)}$; or

$R_2$ is $-alkanediyl_{(C\leq6)}$-NR'R" or a substituted version of this group wherein:

R' and R" are each independently $alkyl_{(C\leq8)}$, substituted $alkyl_{(C\leq8)}$, or $-Z_2A'R_7$; wherein:

$Z_2$ is $alkanediyl_{(C\leq2)}$ or substituted $alkanediyl_{(C\leq2)}$;

A' is $-CHR_j-$, -C(O)O-, or $-C(O)NR_b-$;

$R_b$ is hydrogen, $alkyl_{(C\leq6)}$, or substituted $alkyl_{(\leq6)}$; and

$R_j$ is hydrogen, hydroxy, $acyloxy_{(C\leq6)}$, or substituted $acyloxy_{(C\leq6)}$;

q is 1 or 2; and

r is 1 or 2;

or alternatively as a compound of the formula:

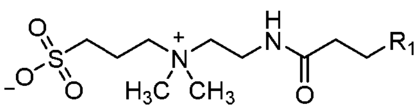

(III)

wherein:

$R_1$ is a group of the formula:

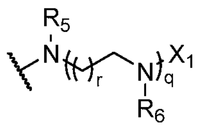

wherein:

$R_5$ is $-(CH_2)_tCH(OH)R_8$, $-(CH_2)_tC(O)OR_8$, $-(CH_2)_tC(O)(NH)R_8$; wherein:

t is 1 or 2; and

$R_8$ is $alkyl_{(C6-24)}$, substituted $alkyl_{(C6-24)}$, $alkenyl_{(C6-24)}$, substituted $alkenyl_{(C6-24)}$;

$R_6$ is $-alkanediyl_{(C\leq6)}$-NR'R" or a substituted version of this group; wherein:

R' and R" are each independently $-(CH_2)_sCH(OH)R_7$, $-(CH_2)_sC(O)OR_7$, or $-(CH_2)_sC(O)(NH)R_7$; wherein:

s is 1 or 2; and

$R_7$ is $alkyl_{(C6-24)}$, substituted $alkyl_{(C6-24)}$, $alkenyl_{(C6-24)}$, substituted $alkenyl_{(C6-24)}$; and

$X_1$ is $-alkanediyl_{(C\leq6)}$-NR'R" or a substituted version of this group; wherein:
R' and R" are each independently $-(CH_2)_sCH(OH)R_7$, $-(CH_2)_sC(O)OR_7$, or $-(CH_2)_sC(O)(NH)R_7$; wherein:

s is 1 or 2; and

$R_7$ is alkyl$_{(C6-24)}$, substituted alkyl$_{(C6-24)}$, alkenyl$_{(C6-24)}$, substituted alkenyl$_{(C6-24)}$;

q is 1 or 2; and

r is 1 or 2;

or a pharmaceutically acceptable salt thereof. In some embodiments, the compounds are further defined as:

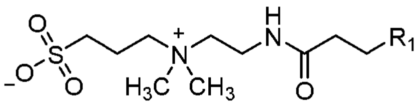

(III)

wherein:

$R_1$ is a group of the formula:

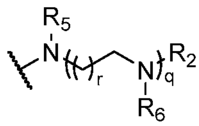

wherein:

$R_5$ is $-Z_3A''R_8$; wherein:

$Z_3$ is alkanediyl$_{(C\leq2)}$ or substituted alkanediyl$_{(C\leq2)}$;

A" is -CHR$_k$-, -C(O)O-, or -C(O)NRi-;

$R_l$ is hydrogen, alkyl$_{(C\leq6)}$, or substituted alkyl$_{(\leq6)}$; and

$R_k$ is hydrogen, hydroxy, acyloxy$_{(C\leq6)}$, or substituted acyloxy$_{(C\leq6)}$; and

$R_8$ is alkyl$_{(C6-24)}$, substituted alkyl$_{(C6-24)}$, alkenyl$_{(C6-24)}$, substituted alkenyl$_{(C6-24)}$;

$R_6$ is -alkanediyl$_{(C\leq6)}$-NR'R" or a substituted version of this group; wherein:

R' and R" are each independently $-Z_2A'R_7$; wherein:

$Z_2$ is alkanediyl$_{(C\leq2)}$ or substituted alkanediyl$_{(C\leq2)}$;

A' is -CHR$_j$-, -C(O)O-, or -C(O)NR$_b$-;

$R_b$ is hydrogen, alkyl$_{(C\leq6)}$, or substituted alkyl$_{(C\leq6)}$; and

$R_j$ is hydrogen, hydroxy, acyloxy$_{(C\leq6)}$, or substituted acyloxy $_{(C\leq6)}$;

$R_7$ is alkyl$_{(C6-24)}$, substituted alkyl$_{(C6-24)}$, alkenyl$_{(C6-24)}$, substituted alkenyl$_{(C6-24)}$; and

$R_2$ is -alkanediyl$_{(C\leq6)}$-NR'R" or a substituted version of this group; wherein:

R' and R" are each independently $-Z_2A'R_7$; wherein:

$Z_2$ is alkanediyl$_{(C\leq2)}$ or substituted alkanediyl$_{(C\leq2)}$;

A' is $-CHR_j-$, $-C(O)O-$, or $-C(O)NR_b-$;

$R_b$ is hydrogen, $alkyl_{(C\leq6)}$, or substituted $alkyl_{(\leq6)}$; and

$R_j$ is hydrogen, hydroxy, $acyloxy_{(C\leq6)}$, or substituted acyloxy $_{(C\leq6)}$;

$R_7$ is $alkyl_{(C6-24)}$, substituted $alkyl_{(C6-24)}$, $alkenyl_{(C6-24)}$, substituted $alkenyl_{(C6-24)}$;

q is 1 or 2; and

r is 1 or 2;

or alternatively as a compound of the formula:

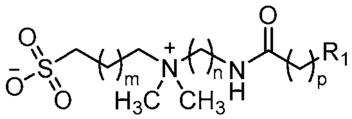

(II)

wherein:

$R_1$ is a group of the formula:

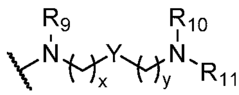

wherein:

Y is $arenediyl_{(C\leq12)}$, $heterocycloalkanediyl_{(C\leq12)}$, $heteroarenediyl_{(C\leq12)}$, or a substituted version of any of these groups;

$R_9$, $R_{10}$, and $R_{11}$ are each independently selected from hydrogen, $alkyl_{(C\leq8)}$, substituted $alkyl_{(C\leq8)}$, $-(CH_2)_uCH(OH)R_{12}$, $-(CH_2)_uC(O)OR_{12}$, $-(CH_2)_uC(O)(NR_c)R_{12}$; wherein:

u is 1, 2, 3, or 4;

$R_c$ is hydrogen, $alkyl_{(C\leq6)}$, or substituted $alkyl_{(\leq6)}$; and

$R_{12}$ is $alkyl_{(C6-24)}$, substituted $alkyl_{(C6-24)}$, $alkenyl_{(C6-24)}$, substituted $alkenyl_{(C6-24)}$; and

x and y are 1, 2, 3, or 4; and

m, n, and p are each independently an integer selected from 0, 1, 2, 3, 4, 5, or 6;

or a pharmaceutically acceptable salt thereof. In other embodiments, the compounds are further defined as:

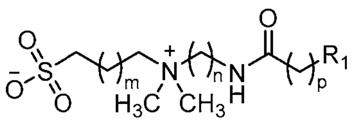

(II)

wherein:

$R_1$ is a group of the formula:

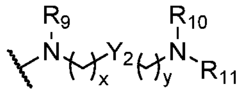

wherein:

$Y_2$ is arenediyl$_{(C\leq12)}$, heterocycloalkanediyl$_{(C\leq12)}$, heteroarenediyl$_{(C\leq12)}$, alkoxydiyl$_{(C\leq12)}$, or a substituted version of any of these groups;

$R_9$, $R_{10}$, and $R_{11}$ are each independently selected from hydrogen, alkyl$_{(C\leq8)}$, substituted alkyl$_{(C\leq8)}$, or -$Z_4$A''' $R_{12}$; wherein:

$Z_4$ is alkanediyl$_{(C\leq4)}$ or substituted alkanediyl$_{(C\leq4)}$;

A''' is -CHR$_k$-, -C(O)O-, or -C(O)NRi-;

$R_l$ is hydrogen, alkyl$_{(C\leq6)}$, or substituted alkyl$_{(\leq6)}$; and

$R_k$ is hydrogen, halo, hydroxy, acyloxy$_{(C\leq24)}$, or substituted acyloxy$_{(C\leq24)}$; and

$R_{12}$ is alkyl$_{(C6-24)}$, substituted alkyl$_{(C6-24)}$, alkenyl$_{(C6-24)}$, substituted alkenyl$_{(C6-24)}$; and

x and y are 1, 2, 3, or 4;

$R_a$, $R_3$, and $R_4$ are each independently hydrogen, alkyl$_{(C\leq6)}$, or substituted alkyl$_{(\leq6)}$; and

m, n, and p are each independently an integer selected from 0, 1, 2, 3, 4, 5, or 6;

or alternatively as a compound of the formula:

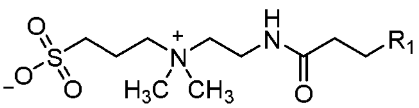
(III)

wherein:

$R_1$ is a group of the formula:

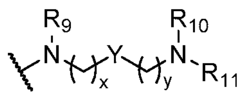

wherein:

Y is arenediyl$_{(C\leq12)}$, heterocycloalkanediyl$_{(C\leq12)}$, heteroarenediyl$_{(C\leq12)}$, or a substituted version of any of these groups;
$R_9$, $R_{10}$, and $R_{11}$ are each independently selected from hydrogen, alkyl$_{(C\leq8)}$, substituted alkyl$_{(C\leq8)}$, -$Z_4$A''' $R_{12}$; wherein:

$Z_4$ is alkanediyl$_{(C\leq4)}$ or substituted alkanediyl$_{(C\leq4)}$;

A''' is -CHR$_k$-, -C(O)O-, or -C(O)NRi-;

$R_l$ is hydrogen, alkyl$_{(C\leq6)}$, or substituted alkyl$_{(\leq6)}$; and

$R_k$ is hydrogen, halo, hydroxy, acyloxy$_{(C\leq24)}$, or substituted acyloxy$_{(C\leq24)}$; and

$R_{12}$ is alkyl$_{(C6-24)}$, substituted alkyl$_{(C6-24)}$, alkenyl$_{(C6-24)}$, substituted alkenyl$_{(C6-24)}$; and

x and y are 1, 2, 3, or 4;

or a pharmaceutically acceptable salt thereof. In other embodiments, the compounds are further defined as:

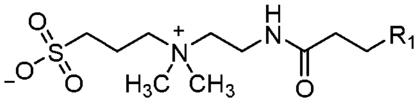
(III)

wherein:

$R_1$ is a group of the formula:

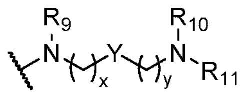

wherein:
Y is heterocycloalkanediyl$_{(C\leq12)}$ or substituted heterocycloalkanediyl$_{(C\leq12)}$; $R_9$, $R_{10}$, and $R_{11}$ are each independently selected from hydrogen, alkyl$_{(C\leq8)}$, substituted alkyl$_{(C\leq8)}$, $-Z_4A'''R_{12}$; wherein:

$Z_4$ is alkanediyl$_{(C\leq4)}$ or substituted alkanediyl$_{(C\leq4)}$;

A‴ is $-CHR_k-$, -C(O)O-, or -C(O)NRi-;

$R_l$ is hydrogen, alkyl$_{(C\leq6)}$, or substituted alkyl$_{(\leq6)}$; and

$R_k$ is hydrogen, halo, hydroxy, acyloxy$_{(C\leq24)}$, or substituted acyloxy$_{(C\leq24)}$; and

$R_{12}$ is alkyl$_{(C6-24)}$, substituted alkyl$_{(C6-24)}$, alkenyl$_{(C6-24)}$, substituted alkenyl$_{(C6-24)}$; and

x and y are 1, 2, 3, or 4;

or a pharmaceutically acceptable salt thereof. In other embodiments, the compounds are further defined as:

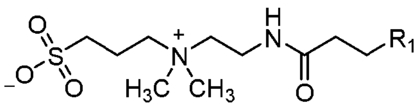
(III)

wherein:

$R_1$ is a group of the formula:

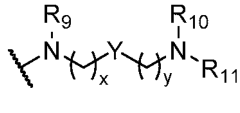

wherein:

Y is heterocycloalkanediyl$_{(C\leq12)}$ or substituted heterocycloalkanediyl$_{(C\leq12)}$;

$R_9$, $R_{10}$, and $R_{11}$ are each independently selected from hydrogen, $-Z_4A'''R_{12}$; wherein:

$Z_4$ is alkanediyl$_{(C\leq4)}$ or substituted alkanediyl$_{(C\leq4)}$;

A''' is -CHR$_k$-, -C(O)O-, or -C(O)NRi-;

R$_l$ is hydrogen, alkyl$_{(C\leq6)}$, or substituted alkyl$_{(\leq6)}$; and

R$_k$ is hydrogen, halo, hydroxy, acyloxy$_{(C\leq24)}$, or substituted acyloxy$_{(C\leq24)}$; and

R$_{12}$ is alkyl$_{(C6-24)}$, substituted alkyl$_{(C6-24)}$, alkenyl$_{(C6-24)}$, substituted alkenyl$_{(C6-24)}$; and

x and y are 1, 2, 3, or 4;

or a pharmaceutically acceptable salt thereof.

R$_2$ may be hydrogen. In other embodiments, R$_2$ is alkyl$_{(C\leq8)}$ or substituted alkyl$_{(C\leq8)}$. R$_3$ may be hydrogen. In other embodiments, R$_3$ is alkyl$_{(C\leq8)}$ or substituted alkyl$_{(C\leq8)}$. In some embodiments, R$_3$ is alkyl$_{(C\leq8)}$ such as methyl. In some embodiments, R$_3$ is hydrogen. In other embodiments, R$_4$ is alkyl$_{(C\leq8)}$ or substituted alkyl$_{(C\leq8)}$. R$_4$ may be alkyl$_{(C\leq8)}$ such as methyl.

In some embodiments, m is 1 or 2. In some embodiments, m is 1. In other embodiments, m is 2. In some embodiments, n is 2 or 3. In some embodiments, n is 2. In other embodiments, n is 3. In some embodiments, p is 1, 2, or 3. In some embodiments, p is 1. In other embodiments, p is 2. In other embodiments, p is 3.

In some embodiments, R1 is a group of the formula:
R$_1$ is a group of the formula:

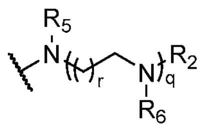

wherein:

R$_5$, R$_6$, and R$_2$ are each independently hydrogen or alkyl$_{(C\leq8)}$, -alkanediyl$_{(C\leq6)}$-NH$_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein: R' and R" are each independently hydrogen, alkyl$_{(C\leq8)}$, substituted alkyl$_{(C\leq8)}$, -Z$_2$A'R$_7$; wherein:

Z$_2$ is alkanediyl$_{(C\leq4)}$ or substituted alkanediyl$_{(C\leq4)}$;

A' is -CHR$_j$-, -C(O)O-, or -C(O)NR$_b$-;

R$_b$ is hydrogen, alkyl$_{(C\leq6)}$, or substituted alkyl$_{(C\leq6)}$; and
R$_j$ is hydrogen, halo, hydroxy, acyloxy$_{(C\leq24)}$, or substituted acyloxy$_{(C\leq24)}$;

R$_7$ is alkyl$_{(C6-24)}$, substituted alkyl$_{(C6-24)}$, alkenyl$_{(C6-24)}$, substituted alkenyl$_{(C6-24)}$; or

R$_5$, R$_6$, and X$_1$ are each independently -Z$_3$A"R$_8$; wherein:

Z$_3$ is alkanediyl$_{(C\leq4)}$ or substituted alkanediyl$_{(C\leq4)}$;

A" is -CHR$_k$-, -C(O)O-, or -C(O)NR$_1$-;

R$_l$ is hydrogen, alkyl$_{(C\leq6)}$, or substituted alkyl$_{(C\leq6)}$; and

R$_k$ is hydrogen, halo, hydroxy, acyloxy$_{(C\leq24)}$, or substituted acyloxy$_{(C\leq24)}$; and

R$_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and

r is 1, 2, 3, or 4.

In some embodiments, $R_1$ is a group of the formula:

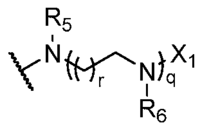

wherein:

$R_5$, $R_6$, and $X_1$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein: R' and R" are each independently hydrogen, alkyl$_{(C\leq8)}$, substituted alkyl$_{(C\leq8)}$, $-(CH_2)_sCH(OH)R_7$, $-(CH_2)_sC(O)OR_7$, or $-(CH_2)_sC(O)(NR_a)R_7$; wherein:

s is 1, 2, 3, or 4;

$R_a$ is hydrogen, alkyl$_{(C\leq6)}$, or substituted alkyl$_{(\leq6)}$; and

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $X_1$ are each independently $-(CH_2)_tCH(OH)R_8$, $-(CH_2)_tC(O)OR_8$, $-(CH_2)_tC(O)(NR_b)R_8$; wherein:

t is 1, 2, 3, or 4;

$R_b$ is hydrogen, alkyl$_{(C\leq6)}$, or substituted alkyl$_{(C\leq6)}$; and

$R_8$ is alkyl$_{(C6-24)}$, substituted alkyl$_{(C6-24)}$, alkenyl$_{(C6-24)}$, substituted alkenyl$_{(C6-24)}$; or

q is 1, 2, or 3; and

r is 1, 2, 3, or 4.

In some embodiments, q is 1 or 2. In some embodiments, q is 1. In other embodiments, q is 2. In some embodiments, r is 1 or 2. In some embodiments, r is 1. In other embodiments, r is 2. In some embodiments, $R_5$ is hydrogen. In other embodiments, $R_5$ is alkyl$_{(C\leq8)}$ or substituted alkyl$_{(C\leq8)}$. In some embodiments, $R_5$ is alkyl$_{(C\leq8)}$ such as methyl or isopropyl.

In some embodiments, $R_5$ is further defined as $-Z_3A''R_8$ wherein:

$Z_3$ is alkanediyl$_{(C\leq4)}$ or substituted alkanediyl$_{(C\leq4)}$;

A" is $-CHR_k-$, -C(O)O-, or $-C(O)NR_1-$;

$R_l$ is hydrogen, alkyl$_{(C\leq6)}$, or substituted alkyl$_{(\leq6)}$; and

$R_k$ is hydrogen, halo, hydroxy, acyloxy$_{(C\leq24)}$, or substituted acyloxy$_{(C\leq24)}$; and $R_8$ is alkyl$_{(C6-24)}$, substituted alkyl$_{(C6-24)}$, alkenyl$_{(C6-24)}$, substituted alkenyl$_{(C6-24)}$.

In some embodiments, $Z_3$ is alkanediyl$_{(C1-2)}$. In one instance, $Z_3$ is $-CH_2-$. In some embodiments, $Z_3$ is substituted alkanediyl$_{(C1-2)}$. In one instance, $Z_3$ is $-CH_2CH(OH)$. In some embodiments, A" is $-CHR_k-$. In one instance, $R_k$ is hydroxy. In some embodiments, $R_k$ is acyloxy$_{(C\leq24)}$ or substituted acyloxy$_{(C\leq24)}$. In some embodiments, $R_k$ is acyloxy$_{(C1-8)}$ or substituted acyloxy$_{(C1-8)}$. In some embodiments, $R_k$ is acyloxy$_{(C\leq12-24)}$ or substituted acyloxy$_{(C\leq12-24)}$. In one instance, A" is -C(O)O-. In another instance, A" is -C(O)NH-.

In other embodiments, $R_5$ is $-(CH_2)_tCH(OH)R_8$, $-(CH_2)_tC(O)OR_8$, or $-(CH_2)_tC(O)(NR_b)R_8$; wherein:

t is 1, 2, 3, or 4;

$R_b$ is hydrogen, alkyl$_{(C\leq6)}$, or substituted alkyl$_{(\leq6)}$; and

$R_8$ is alkyl$_{(C6-24)}$, substituted alkyl$_{(C6-24)}$, alkenyl$_{(C6-24)}$, substituted alkenyl$_{(C6-24)}$.

In some embodiments, Rs is $-(CH_2)_tCH(OH)R_8$. In other embodiments, Rs is $-(CH_2)_tC(O)OR_8$. In other embodiments, Rs is $-(CH_2)_tC(O)(NR_b)R_8$. t may be 1 or 2. In some embodiments, t is 1. In other embodiments, t is 2. In some embodiments, $R_b$ is hydrogen. In other embodiments, $R_b$ is alkyl$_{(C\leq6)}$ or substituted alkyl$_{(C\leq6)}$ such as alkyl$_{(C6-24)}$ or substituted alkyl$_{(C6-24)}$.

In some embodiments, $R_8$ is alkyl$_{(C6-24)}$ such as octyl, decyl, dodecyl, tetradecyl, hexadecyl, or octadecyl. In other embodiments, $R_8$ is alkenyl$_{(C6-24)}$ or substituted alkenyl$_{(C6-24)}$. In some embodiments, $R_6$ is hydrogen. In other embodiments, $R_6$ is alkyl$_{(C\leq8)}$ or substituted alkyl$_{(C\leq8)}$. $R_6$ may be alkyl$_{(C\leq8)}$ such as methyl or isopropyl.

In some embodiments, $R_6$ is $-Z_3A''R_8$; wherein:

$Z_3$ is alkanediyl$_{(C\leq4)}$ or substituted alkanediyl$_{(C\leq4)}$;

A" is $-CHR_k-$, -C(O)O-, or -C(O)NRi-;

$R_l$ is hydrogen, alkyl$_{(C\leq6)}$, or substituted alkyl$_{(\leq6)}$; and

$R_k$ is hydrogen, halo, hydroxy, acyloxy$_{(C\leq24)}$, or substituted acyloxy$_{(C\leq24)}$; and $R_8$ is alkyl$_{(C6-24)}$, substituted alkyl$_{(C6-24)}$, alkenyl$_{(C6-24)}$, substituted alkenyl$_{(C6-24)}$.

In some embodiments, $Z_3$ is alkanediyl$_{(C1-2)}$. In one instance, $Z_3$ is $-CH_2-$. In some embodiments, $Z_3$ is substituted alkanediyl$_{(C1-2)}$. In one instance, $Z_3$ is $-CH_2CH(OH)$. In some embodiments, A" is $-CHR_k-$. In one instance, $R_k$ is hydroxy. In some embodiments, $R_k$ is acyloxy$_{(C\leq24)}$ or substituted acyloxy$_{(C\leq24)}$. In some embodiments, $R_k$ is acyloxy$_{(C1-8)}$ or substituted acyloxy$_{(C1-8)}$. In some embodiments, $R_k$ is acyloxy$_{(C\leq12-24)}$ or substituted acyloxy$_{(C\leq12-24)}$. In one instance, A" is -C(O)O-. In another instance, A" is -C(O)NH-. In some embodiments, $R_8$ is alkyl$_{(C6-24)}$ or substituted alkyl$_{(C6-24)}$. $R_8$ may be alkyl$_{(C6-24)}$ such as octyl, decyl, dodecyl, tetradecyl, hexadecyl, or octadecyl. In some embodiments, $R_8$ is alkenyl$_{(C6-24)}$ or substituted alkenyl$_{(C6-24)}$.

In other embodiments, $R_6$ is $-(CH_2)_tCH(OH)R_8$, $-(CH_2)_tC(O)OR_8$, or $-(CH_2)_tC(O)(NR_b)R_8$; wherein:

t is 1, 2, 3, or 4;

$R_b$ is hydrogen, alkyl$_{(C\leq6)}$, or substituted alkyl$_{(\leq6)}$; and

$R_8$ is alkyl$_{(C6-24)}$, substituted alkyl$_{(C6-24)}$, alkenyl$_{(C6-24)}$, substituted alkenyl$_{(C6-24)}$.

In some embodiments, $R_6$ is $-(CH_2)tCH(OH)R_8$. In other embodiments, $R_6$ is $-(CH_2)tC(O)OR_8$. In other embodiments, $R_6$ is $-(CH_2)tC(O)(NR_b)R_8$. In some embodiments, t is 1 or 2. In some embodiments, t is 1. In other embodiments, t is 2. In some embodiments, $R_b$ is hydrogen. In other embodiments, $R_b$ is alkyl$_{(C\leq6)}$ or substituted alkyl$_{(C\leq6)}$. $R_8$ may be alkyl$_{(C6-24)}$ or substituted alkyl$_{(C6-24)}$. In some embodiments, $R_8$ is alkyl$_{(C6-24)}$ such as octyl, decyl, dodecyl, tetradecyl, hexadecyl, or octadecyl. In other embodiments, $R_8$ is alkenyl$_{(C6-24)}$ or substituted alkenyl$_{(C6-24)}$.

In some embodiments, $R_6$ is -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, or a substituted version of any of these groups. In some embodiments, $R_6$ is -alkanediyl$_{(C\leq6)}$-$NH_2$ or a substituted version of this group such as $-CH_2CH_2NH_2$. In other embodiments, $R_6$ is -alkanediyl$_{(C\leq6)}$-alkylamino$_{(C\leq8)}$ or a substituted version of this group such as $-CH_2CH_2NHMe$ or $-CH_2CH_2NH$*i*Pr. In other embodiments, $R_6$ is -alkanediyl$_{(C\leq6)}$-dialkylamino$_{(C\leq8)}$ or a substituted version of this group.

In some embodiments, $R_2$ is hydrogen. In other embodiments, $R_2$ is alkyl$_{(C\leq8)}$ or substituted alkyl$_{(C\leq8)}$. In some embodiments, $R_2$ is alkyl$_{(C\leq8)}$ such as methyl or isopropyl. In some embodiments, $R_2$ is $-Z_3A''R_8$; wherein:

$Z_3$ is alkanediyl$_{(C\leq4)}$ or substituted alkanediyl$_{(C\leq4)}$;

A" is $-CHR_k-$, -C(O)O-, or -C(O)NRi-;

$R_l$ is hydrogen, alkyl$_{(C\leq6)}$, or substituted alkyl$_{(\leq6)}$; and

$R_k$ is hydrogen, halo, hydroxy, acyloxy$_{(C\leq24)}$, or substituted acyloxy$_{(C\leq24)}$; and $R_8$ is alkyl$_{(C6-24)}$, substituted alkyl$_{(C6-24)}$, alkenyl$_{(C6-24)}$, substituted alkenyl$_{(C6-24)}$.

In some embodiments, $Z_3$ is alkanediyl$_{(C1-2)}$. In one instance, $Z_3$ is $-CH_2-$. In some embodiments, $Z_3$ is substituted

alkanediyl$_{(C1-2)}$. In one instance, $Z_3$ is $-CH_2CH(OH)$. In some embodiments, A" is $-CHR_k-$. In one instance, $R_k$ is hydroxy. In some embodiments, $R_k$ is acyloxy$_{(C\leq24)}$ or substituted acyloxy$_{(C\leq24)}$. In some embodiments, $R_k$ is acyloxy$_{(C1-8)}$ or substituted acyloxy$_{(C1-8)}$. In some embodiments, $R_k$ is acyloxy$_{(C\leq12-24)}$ or substituted acyloxy$_{(C\leq12-24)}$. In one instance, A" is -C(O)O-. In another instance, A" is -C(O)NH-. In some embodiments, $R_8$ is alkyl$_{(C6-24)}$ or substituted alkyl$_{(C6-24)}$. $R_8$ may be alkyl$_{(C6-24)}$ such as octyl, decyl, dodecyl, tetradecyl, hexadecyl, or octadecyl. In some embodiments, $R_8$ is alkenyl$_{(C6-24)}$ or substituted alkenyl$_{(C6-24)}$.

In other embodiments, $R_2$ is $-(CH_2)_tCH(OH)R_8$, $-(CH_2)_tC(O)OR_8$, or $-(CH_2)_tC(O)(NR_b)R_8$; wherein:

t is 1, 2, 3, or 4;

$R_b$ is hydrogen, alkyl$_{(C\leq6)}$, or substituted alkyl$_{(C\leq6)}$; and

$R_8$ is alkyl$_{(C6-24)}$, substituted alkyl$_{(C6-24)}$, alkenyl$_{(C6-24)}$, substituted alkenyl$_{(C6-24)}$.

In some embodiments, $R_2$ is $-(CH_2)_tCH(OH)R_8$. In other embodiments, $R_2$ is $-(CH_2)_tC(O)OR_8$. In other embodiments, $R_2$ is $-(CH_2)_tC(O)(NR_b)R_8$. t may be 1 or 2. In some embodiments, t is 1. In other embodiments, t is 2. In some embodiments, $R_b$ is hydrogen. In other embodiments, $R_b$ is alkyl$_{(C\leq6)}$ or substituted alkyl$_{(C\leq6)}$. $R_8$ may be alkyl$_{(C6-24)}$ or substituted alkyl$_{(C6-24)}$. In some embodiments, $R_8$ is alkyl$_{(C6-24)}$ such as octyl, decyl, dodecyl, tetradecyl, hexadecyl, or octadecyl. In some embodiments, $R_8$ is alkenyl$_{(C6-24)}$ or substituted alkenyl$_{(C6-24)}$.

In other embodiments, $R_2$ is -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, or a substituted version of any of these groups. In some embodiments, $R_2$ is -alkanediyl$_{(C\leq6)}$-$NH_2$ or a substituted version of this group such as $-CH_2CH_2NH_2$. In other embodiments, $R_2$ is -alkanediyl$_{(C\leq6)}$-alkylamino$_{(C\leq8)}$ or a substituted version of this group such as $-CH_2CH_2NHMe$ or $-CH_2CH_2NH$*i*Pr. In other embodiments, $R_2$ is -alkanediyl$_{(C\leq6)}$-dialkylamino$_{(C\leq8)}$ or a substituted version of this group.

In other aspects, $R_1$ is a group of the formula:

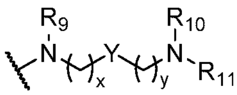

wherein:

Y is arenediyl$_{(C\leq12)}$, heterocycloalkanediyl$_{(6\leq12)}$, heteroarenediyl$_{(C\leq12)}$, or a substituted version of any of these groups;

$R_9$, $R_{10}$, and $R_{11}$ are each independently selected from hydrogen, alkyl$_{(C\leq8)}$, substituted alkyl$_{(C\leq8)}$, $-(CH_2)_uCH(OH)R_{12}$, $-(CH_2)_uC(O)OR_{12}$, $-(CH_2)_uC(O)(NR_c)R_{12}$; wherein:

u is 1, 2, 3, or 4;

$R_c$ is hydrogen, alkyl$_{(C\leq6)}$, or substituted alkyl$_{(C\leq6)}$; and

$R_{12}$ is alkyl$_{(C6-24)}$, substituted alkyl$_{(C6-24)}$, alkenyl$_{(C6-24)}$, substituted alkenyl$_{(C6-24)}$; and

x and y are 1, 2, 3, or 4.

In other aspects, $R_1$ is a group of the formula:

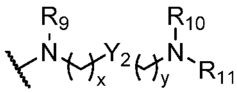

wherein:

$Y_2$ is arenediyl$_{(C\leq12)}$, heterocycloalkanediyl$_{(C\leq12)}$, heteroarenediyl$_{(C\leq12)}$, alkoxydiyl$_{(C\leq12)}$, or a substituted version of any of these groups;

$R_9$, $R_{10}$, and $R_{11}$ are each independently selected from hydrogen, alkyl$_{(C\leq8)}$, substituted alkyl$_{(C\leq8)}$, or $-Z_4A'''R_{12}$; wherein:

$Z_4$ is alkanediyl$_{(C\leq4)}$ or substituted alkanediyl$_{(C\leq4)}$;

A‴ is $-CHR_k-$, -C(O)O-, or $-C(O)NR_1-$;

$R_1$ is hydrogen, alkyl$_{(C\leq6)}$, or substituted alkyl$_{(C\leq6)}$; and

$R_k$ is hydrogen, halo, hydroxy, acyloxy$_{(C\leq24)}$, or substituted acyloxy$_{(C\leq24)}$; and

$R_{12}$ is alkyl$_{(C6\text{-}24)}$, substituted alkyl$_{(C6\text{-}24)}$, alkenyl$_{(C6\text{-}24)}$, substituted alkenyl$_{(C6\text{-}24)}$; and

x and y are 1, 2, 3, or 4.

In some embodiments, Y is heterocycloalkanediyl$_{(C\leq12)}$ or substituted heterocycloalkanediyl$_{(C\leq12)}$. In some embodiments, Y is heterocycloalkanediyl$_{(C\leq12)}$ such as piperazindiyl. In other embodiments, Y is heteroarenediyl$_{(C\leq12)}$ or substituted heteroarenediyl$_{(C\leq12)}$. In other embodiments, Y is arenediyl$_{(C\leq12)}$ or substituted arenediyl$_{(C\leq12)}$. In some embodiments, x is 2 or 3. In one embodiment, x is 2. In another embodiment, x is 3. In some embodiments, y is 2 or 3. In one embodiment, y is 2. In another embodiment, y is 3.

In one embodiment, $R_9$ is $-(CH_2)_uCH(OH)R_{12}$, $-(CH_2)_uC(O)OR_{12}$, $-(CH_2)_uC(O)(NR_c)R_{12}$; wherein:

u is 1, 2, 3, or 4;

$R_c$ is hydrogen, alkyl$_{(C\leq6)}$, or substituted alkyl$_{(C\leq6)}$; and

$R_{12}$ is alkyl$_{(C6\text{-}24)}$, substituted alkyl$_{(C6\text{-}24)}$, alkenyl$_{(C6\text{-}24)}$, substituted alkenyl$_{(C6\text{-}24)}$.

In some embodiments, $R_9$ is $-(CH_2)_uCH(OH)R_{12}$. In other embodiment, $R_9$ is $-(CH_2)_uC(O)OR_{12}$. In some embodiments, $R_9$ is $-(CH_2)_uC(O)(NR_c)R_{12}$. u may be 1, 2, or 3. In some embodiments, u is 1 or 2. In one embodiment, u is 1. In another embodiment, u is 2. In some embodiments, $R_c$ is hydrogen. In other embodiments, $R_c$ is alkyl$_{(C\leq6)}$ or substituted alkyl$_{(C\leq6)}$. $R_{12}$ may be alkyl$_{(C6\text{-}24)}$ or substituted alkyl$_{(C6\text{-}24)}$. In some embodiments, $R_{12}$ is alkyl$_{(C6\text{-}24)}$ such as octyl, decyl, dodecyl, tetradecyl, hexadecyl, or octadecyl. In other embodiments, $R_{12}$ is alkenyl$_{(C6\text{-}24)}$ or substituted alkenyl$_{(C6\text{-}24)}$.

In some embodiments, $R_{10}$ is $-(CH_2)_uCH(OH)R_{12}$, $-(CH_2)_uC(O)OR_{12}$, $-(CH_2)_uC(O)(NR_c)R_{12}$; wherein:

u is 1, 2, 3, or 4;

$R_c$ is hydrogen, alkyl$_{(C\leq6)}$, or substituted alkyl$_{(C\leq6)}$; and

$R_{12}$ is alkyl$_{(C6\text{-}24)}$, substituted alkyl$_{(C6\text{-}24)}$, alkenyl$_{(C6\text{-}24)}$, substituted alkenyl$_{(C6\text{-}24)}$.

In some embodiments, $R_{10}$ is $-(CH_2)_uCH(OH)R_{12}$. In other embodiments, $R_{10}$ is $-(CH_2)_uC(O)OR_{12}$. In other embodiments, $R_{10}$ is $-(CH_2)_uC(O)(NR_c)R_{12}$. u may be 1, 2, or 3. In some embodiments, u is 1 or 2. In one embodiment, u is 1. In another embodiment, u is 2. In some embodiments, $R_c$ is hydrogen. In other embodiments, $R_c$ is alkyl$_{(C\leq6)}$ or substituted alkyl$_{(C\leq6)}$. $R_{12}$ may be alkyl$_{(C6\text{-}24)}$ or substituted alkyl$_{(C6\text{-}24)}$. In some embodiments, $R_{12}$ is alkyl$_{(C6\text{-}24)}$ such as octyl, decyl, dodecyl, tetradecyl, hexadecyl, or octadecyl. In other embodiments, $R_{12}$ is alkenyl$_{(C6\text{-}24)}$ or substituted alkenyl$_{(C6\text{-}24)}$.

In some embodiments, $R_{11}$ is $-(CH_2)_uCH(OH)R_{12}$, $-(CH_2)_uC(O)OR_{12}$, $-(CH_2)_uC(O)(NR_c)R_{12}$; wherein:

u is 1, 2, 3, or 4;

$R_c$ is hydrogen, alkyl$_{(C\leq6)}$, or substituted alkyl$_{(C\leq6)}$; and

$R_{12}$ is alkyl$_{(C6\text{-}24)}$, substituted alkyl$_{(C6\text{-}24)}$, alkenyl$_{(C6\text{-}24)}$, substituted alkenyl$_{(C6\text{-}24)}$.

In some embodiments, $R_{11}$ is $-(CH_2)_uCH(OH)R_{12}$. In other embodiments, $-(CH_2)_uC(O)OR_{12}$. In other embodiments, $R_{11}$ is $-(CH_2)_uC(O)(NR_c)R_{12}$. u may be 1, 2, or 3. In some embodiments, u is 1 or 2. In one embodiment, u is 1. In another embodiment, u is 2. In some embodiments, $R_c$ is hydrogen. In other embodiments, $R_c$ is alkyl$_{(C\leq6)}$ or substituted alkyl$_{(C\leq6)}$. $R_{12}$ may be alkyl$_{(C6\text{-}24)}$ or substituted alkyl$_{(C6\text{-}24)}$. In some embodiments, $R_{12}$ is alkyl$_{(C6\text{-}24)}$ such as octyl, decyl, dodecyl, tetradecyl, hexadecyl, or octadecyl. In other embodiments, $R_{12}$ is alkenyl$_{(C6\text{-}24)}$ or substituted alkenyl$_{(C6\text{-}24)}$.

In other embodiments, the compounds are further defined as:

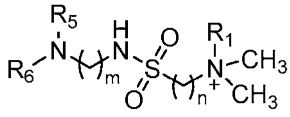 (V)

wherein:

$R_1$, $R_2$, and $R_3$ are each independently hydrogen, alkyl$_{(C\leq6)}$, substituted alkyl$_{(C\leq6)}$, or a group of the formula:

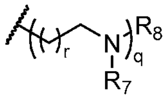

wherein:

$R_7$ and $R_8$ are each independently hydrogen, alkyl$_{(C\leq6)}$, substituted alkyl$_{(C\leq6)}$, or a group of the formula:

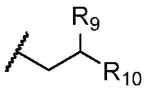

wherein:

$R_9$ is hydrogen, halo, or hydroxy, or alkoxy$_{(C\leq8)}$, acyloxy$_{(C\leq8)}$, or a substituted version of either of these groups; and

$R_{10}$ is alkyl$_{(C\leq24)}$, alkenyl$_{(C\leq24)}$, or a substituted version of either group;

q is 1, 2, or 3; and

r is 0, 1, 2, 3, or 4;

$R_5$ and $R_6$ are each independently hydrogen, alkyl$_{(C\leq6)}$, or substituted alkyl$_{(C\leq6)}$; and

m and n are each independently 1, 2, 3, 4, or 5;

or a pharmaceutically acceptable salt thereof. In some embodiments, the compounds are further defined as:

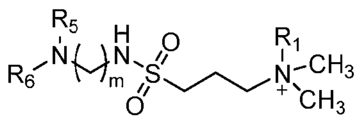 (VI)

wherein:

$R_1$, $R_2$, and $R_3$ are each independently hydrogen, alkyl$_{(C\leq6)}$, substituted alkyl$_{(C\leq6)}$, or a group of the formula:

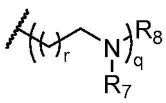

wherein:

$R_7$ and $R_8$ are each independently hydrogen, alkyl$_{(C\leq6)}$, substituted alkyl$_{(C\leq6)}$, or a group of the formula:

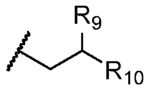

wherein:

$R_9$ is hydrogen, halo, or hydroxy, or alkoxy$_{(C\leq8)}$, acyloxy$_{(C\leq8)}$, or a substituted version of either of these groups; and

$R_{10}$ is alkyl$_{(C\leq24)}$, alkenyl$_{(C\leq24)}$, or a substituted version of either group;

q is 1, 2, or 3; and

r is 0, 1, 2, 3, or 4;

$R_5$ and $R_6$ are each independently hydrogen, alkyl$_{(C\leq6)}$, or substituted alkyl$_{(C\leq6)}$; and

m is 1, 2, 3, 4, or 5;

or a pharmaceutically acceptable salt thereof.

In some embodiments, $R_2$ is hydrogen. $R_2$ may be alkyl$_{(C\leq6)}$ or substituted alkyl$_{(C\leq6)}$. In some embodiments, $R_2$ is alkyl$_{(C\leq6)}$ such as methyl or ethyl. In other embodiment, $R_2$ is a group of the formula:

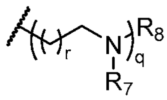

wherein:

$R_7$ and $R_8$ are each independently hydrogen, alkyl$_{(C\leq6)}$, substituted alkyl$_{(C\leq6)}$, or a group of the formula:

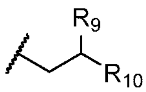

wherein:

$R_9$ is hydrogen, halo, or hydroxy, or alkoxy$_{(C\leq8)}$, acyloxy$_{(C\leq8)}$, or a substituted version of either of these groups; and

$R_{10}$ is alkyl$_{(C\leq24)}$, alkenyl$_{(C\leq24)}$, or a substituted version of either group;

q is 1, 2, or 3; and

r is 0, 1, 2, 3, or 4.

In some embodiments, $R_7$ is hydrogen. In other embodiment, $R_7$ is alkyl$_{(C\leq6)}$ or substituted alkyl$_{(C\leq6)}$. $R_7$ may be alkyl$_{(C\leq6)}$ such as methyl or ethyl. In other embodiments, $R_7$

is 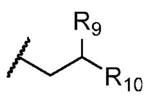

wherein:

$R_9$ is hydrogen, halo, or hydroxy, or alkoxy$_{(C\leq8)}$, acyloxy$_{(C\leq8)}$, or a substituted version of either of these groups; and

$R_{10}$ is alkyl$_{(C\leq24)}$, alkenyl$_{(C\leq24)}$, or a substituted version of either group.

In some embodiments, $R_9$ is halo such as chloro or bromo. In other embodiments, $R_9$ is hydroxy. In other embodiments, $R_9$ is alkoxy$_{(C\leq8)}$ or substituted alkoxy$_{(C\leq8)}$. $R_9$ may be alkoxy$_{(C\leq8)}$ such as methoxy. In other embodiments, $R_9$ is acyloxy$_{(C\leq8)}$ or substituted acyloxy$_{(C\leq8)}$. $R_9$ may be acyloxy$_{(C\leq8)}$ such as acetoxy or pivaloyloxy. In other embodiments, $R_{10}$ is alkyl$_{(C\leq24)}$ or substituted alkyl$_{(C\leq24)}$ such as octyl, decyl, dodecyl, tetradecyl, hexadecyl, or octadecyl. In other embodiments, $R_{10}$ is alkenyl$_{(C\leq24)}$ or substituted alkenyl$_{(C\leq24)}$.

In some embodiments, $R_8$ is hydrogen. In other embodiments, $R_8$ is alkyl$_{(C\leq6)}$ or substituted alkyl$_{(C\leq6)}$. $R_8$ may be alkyl$_{(C\leq6)}$ such as methyl or ethyl. In other embodiment,

$R_8$ is 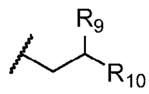

wherein:

$R_9$ is hydrogen, halo, or hydroxy, or alkoxy$_{(C\leq8)}$, acyloxy$_{(C\leq8)}$, or a substituted version of either of these groups; and

$R_{10}$ is alkyl$_{(C\leq24)}$, alkenyl$_{(C\leq24)}$, or a substituted version of either group.

In some embodiments, $R_9$ is halo such as chloro or bromo. In other embodiments, $R_9$ is hydroxy. In other embodiments, $R_9$ is alkoxy$_{(C\leq8)}$ or substituted alkoxy$_{(C\leq8)}$. $R_9$ may be alkoxy$_{(C\leq8)}$ such as methoxy. In other embodiments, $R_9$ is acyloxy$_{(C\leq8)}$ or substituted acyloxy$_{(C\leq8)}$. $R_9$ may be acyloxy$_{(C\leq8)}$ such as acetoxy or pivaloyloxy. In other embodiments, $R_{10}$ is alkyl$_{(C\leq24)}$ or substituted alkyl$_{(C\leq24)}$ such as octyl, decyl, dodecyl, tetradecyl, hexadecyl, or octadecyl. In other embodiments, $R_{10}$ is alkenyl$_{(C\leq24)}$ or substituted alkenyl$_{(C\leq24)}$.

Alternatively, q may be 1 or 2. In some embodiments, q is 1. In other embodiments, q is 2. Similarly, r may be 1, 2, or 3. In some embodiments, r is 1. In other embodiments, r is 2. In other embodiments, r is 3. In some embodiments, $R_3$ is hydrogen. In some embodiments, $R_3$ is alkyl$_{(C\leq6)}$ or substituted alkyl$_{(C\leq6)}$. $R_3$ may be alkyl$_{(C\leq6)}$ such as methyl or ethyl.

In some embodiments, $R_4$ is hydrogen. In other embodiments, $R_4$ is alkyl$_{(C\leq6)}$ or substituted alkyl$_{(C\leq6)}$. In some embodiments, $R_5$ is hydrogen. In other embodiments, $R_5$ is alkyl$_{(C\leq6)}$ or substituted alkyl$_{(C\leq6)}$. $R_5$ may be alkyl$_{(C\leq6)}$ such as methyl or ethyl. In some embodiments, $R_6$ is hydrogen. In other embodiments, $R_6$ is alkyl$_{(C\leq6)}$ or substituted alkyl$_{(C\leq6)}$. $R_6$ may be alkyl$_{(C\leq6)}$ such as methyl or ethyl. In some embodiments, m is 2, 3, or 4. In some embodiments, m is 2. In In some embodiments, n is 3.

In some embodiments, $R_1$ is hydrogen. In other embodiments, $R_1$ is alkyl$_{(C\leq6)}$ or substituted alkyl$_{(C\leq6)}$. $R_1$ may be alkyl$_{(C\leq6)}$ such as methyl or ethyl. In other embodiments, $R_1$ is a group of the formula:

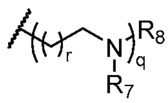

wherein:

$R_7$ and $R_8$ are each independently hydrogen, alkyl$_{(C\leq6)}$, substituted alkyl$_{(C\leq6)}$, or a group of the formula:

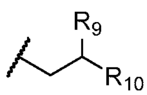

wherein:

$R_9$ is hydrogen, halo, or hydroxy, or alkoxy$_{(C\leq8)}$, acyloxy$_{(C\leq8)}$, or a substituted version of either of these groups; and

$R_{10}$ is alkyl$_{(C\leq24)}$, alkenyl$_{(C\leq24)}$, or a substituted version of either group;

q is 1, 2, or 3; and

r is 0, 1, 2, 3, or 4.

In some embodiments, $R_7$ is hydrogen. In other embodiments, $R_7$ is alkyl$_{(C\leq6)}$ or substituted alkyl$_{(C\leq6)}$. $R_7$ may be alkyl$_{(C\leq6)}$ such as methyl or ethyl. In other embodiments,

$R_7$ is 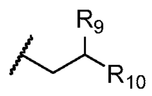

wherein:

$R_9$ is hydrogen, halo, or hydroxy, or alkoxy$_{(C\leq8)}$, acyloxy$_{(C\leq8)}$, or a substituted version of either of these groups; and

$R_{10}$ is alkyl$_{(C\leq24)}$, alkenyl$_{(C\leq24)}$, or a substituted version of either group.

In some embodiments, $R_9$ is halo such as chloro or bromo. In other embodiments, $R_9$ is hydroxy. In other embodiments, $R_9$ is alkoxy$_{(C\leq8)}$ or substituted alkoxy$_{(C\leq8)}$. $R_9$ may be alkoxy$_{(C\leq8)}$ such as methoxy. In other embodiments, $R_9$ is acyloxy$_{(C\leq8)}$ or substituted acyloxy$_{(C\leq8)}$. $R_9$ may be acyloxy$_{(C\leq8)}$ such as acetoxy or pivaloyloxy. In other embodiments, $R_{10}$ is alkyl$_{(C\leq24)}$ or substituted alkyl$_{(C\leq24)}$ such as octyl, decyl, dodecyl, tetradecyl, hexadecyl, or octadecyl. In other embodiments, $R_{10}$ is alkenyl$_{(C\leq24)}$ or substituted alkenyl$_{(C\leq24)}$.

In some embodiments, $R_8$ is hydrogen. In other embodiments, $R_8$ is alkyl$_{(C\leq6)}$ or substituted alkyl$_{(C\leq6)}$. $R_8$ may be alkyl$_{(C\leq6)}$ such as methyl or ethyl. In other embodiments,

$R_8$ is 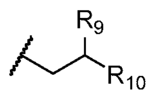

wherein:

$R_9$ is hydrogen, halo, or hydroxy, or alkoxy$_{(C\leq8)}$, acyloxy$_{(C\leq8)}$, or a substituted version of either of these groups; and

$R_{10}$ is alkyl$_{(C\leq24)}$, alkenyl$_{(C\leq24)}$, or a substituted version of either group.

In some embodiments, $R_9$ is halo such as chloro or bromo. In other embodiments, $R_9$ is hydroxy. In other embodiments, $R_9$ is alkoxy$_{(C\leq8)}$ or substituted alkoxy$_{(C\leq8)}$. $R_9$ may be alkoxy$_{(C\leq8)}$ such as methoxy. In other embodiments, $R_9$ is acyloxy$_{(C\leq8)}$ or substituted acyloxy$_{(C\leq8)}$. $R_9$ may be acyloxy$_{(C\leq8)}$ such as acetoxy or pivaloyloxy. In other embodiments, $R_{10}$ is alkyl$_{(C\leq24)}$ or substituted alkyl$_{(C\leq24)}$ such as octyl, decyl, dodecyl, tetradecyl, hexadecyl, or octadecyl. In other embodiments, $R_{10}$ is alkenyl$_{(C\leq24)}$ or substituted alkenyl$_{(C\leq24)}$.

In some embodiments, q is 1 or 2. In some embodiments, q is 1. In other embodiments, q is 2. In some embodiments, r is 1, 2, or 3. In some embodiments, r is 1. In other embodiments, r is 2. In other embodiments, r is 3.

In some embodiments, the tRNA is an unmodified tRNA. In other embodiments, the tRNA is a modified tRNA. In some embodiments, the tRNA is a suppressor tRNA. In some embodiments, the tRNA is a tRNA that delivers an amino acid into a protein instead of terminating translation. In some embodiments, the tRNA is a tRNA amber suppressor. In other embodiments, the tRNA is a tRNA opal suppressor. In other embodiments, the tRNA is a tRNA ochre suppressor. In some embodiments, the tRNA is a tRNA frameshift suppressor. The tRNA, such as tRNA$^{arg/op}$, may be used to add an arginine residue to a growing polypeptide.

In some embodiments, the compositions further comprise a steroid or steroid derivative. In some embodiments, the steroid or steroid derivative is a sterol such as cholesterol. In some embodiments, the compositions further comprise a phospholipid. In some embodiments, the phospholipid is a phosphatidylcholine. In other embodiments, the phospholipid is distearoylphosphatidyl-choline. In some embodiments, the compositions further comprise a PEG lipid. In some embodiments, the PEG lipid is a PEGylated diacylglycerol such as PEGylated dimyristoyl-sn-glycerol. In other embodiments, the PEG lipid is:

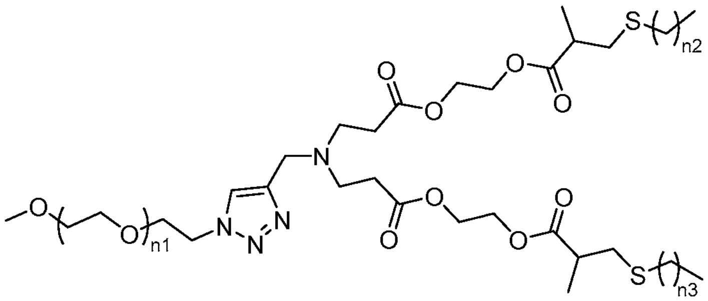

wherein:

$n_1$ is an integer from 1 to 250; and

$n_2$ and $n_3$ are each independently selected from 5, 6, 7, 8, 9, 10, 11, 12, 13, 14, 15, 16, 17, 18, 19, 20, 21, 22, or 23.

In some embodiments, m is 5 to 100. In some embodiments, m is 45. In some embodiments, $n_2$ is 11, 12, 13, 14, 15, 16, or 17. In some embodiments, $n_2$ is 15. In some embodiments, $n_3$ is 11, 12, 13, 14, 15, 16, or 17. In some embodiments, $n_3$ is 15.

In some embodiments, the compositions comprise a mole ratio of the compound to the nucleic acid from about 5:1 to about 1000:1. In some embodiments, the mole ratio of the compound to the nucleic acid is from about 100:1 to about 1000:1. In some embodiments, the mole ratio is about 166:1. In other embodiments, the mole ratio is from about 250:1 to about 750:1. In some embodiments, the compositions comprise a ratio of the compound to the steroid or steroid derivative from about 1:1 to about 20:1 such as from about 1:1 to about 6:1. In some embodiments, the compositions comprise a ratio of the compound to the phospholipid is from about 1:1 to about 9:1 such as from about 2.5:1 to about 7.5:1. In some embodiments, the compositions comprise a ratio of the compound to the PEG-lipid is from about 2.5:1 to about 100:1 such as from about 7.5:1 to about 50:1.

In some embodiments, the tRNA is 5'-GGCCGCGTGGCCTAATGGAtAAGGCGTCTGACTTCAGATCAGAAGAtTGCAGGTT CGAGTCCTGCCGCGGTCGCCA-3' (SEQ ID NO: 1). In other embodiments, the tRNA is:

5′−GGCCGCGUGGCCUAAUGGAUAAGGCGUCUGACUUCAGAUCAGAAGAUUGCA GGUUCGAGUCCUGCCGCGGUCGCCA−3′ (SEQ ID NO: 2);

5′−GGCCUCGUGGCGCAACGGUAGCGCGUCUGACUUCAGAUCAGAAGGUUGCGU GUUCAAAUCACGUCGGGGUCACCA−3′ (SEQ ID NO: 3);

5′−GCGUUGGUGGUAUAGUGGUUAGCAUAGCUGCCUUCAAAGCAGUUGACCCGG GUUCGAUUCCCGGCCAACGCACCA−3′ (SEQ ID NO: 4);

5′−GCAGCGAUGGCCGAGUGGUUAAGGCGUUGGACUUCAAAUCCAAUGGGGUCU CCCCGCGCAGGUUCGAACCCUGCUCGCUGCGCCA−3′ (SEQ ID NO: 5);

5′−GGUCCCAUGGUGUAAUGGUUAGCACUCUGGACUUUAAAUCCAGCGAUCCGA GUUCAAAUCUCGGUGGGACCUCCA−3′ (SEQ ID NO: 6);

5′−GCCCGGAUAGCUCAGUCGGUAGAGCAUCAGACUUUAAAUCUGAGGGUCCAG GGUUCAAGUCCCUGUUCGGGCGCCA−3′ (SEQ ID NO: 7);

5′−ACCAGGAUGGCCGAGUGGUUAAGGCGUUGGACUUUAGAUCCAAUGGACAU AUGUCCGCGUGGGUUCGAACCCCACUCCUGGUACCA−3′ (SEQ ID NO: 8);

5′−GCCCGGCUAGCUCAGUCGGUAGAGCAUGGGACUCUAAAUCCCAGGGUCGUG GGUUCGAGCCCCACGUUGGGCGCCA−3′ (SEQ ID NO: 9);

5′−UCCCUGGUGGUCUAGUGGUUAGGAUUCGGCGCUAUCACCGCCGCGGCCCGG GUUCGAUUCCCGGUCAGGGAACCA−3′ (SEQ ID NO: 10);

5′−UCCCUGGUGGUCUAGUGGUUAGGAUUCGGCGCUAUCACCGCCGCGGCCCGG GUUCGAUUCCCGGUCAGGGAACCA−3′ (SEQ ID NO: 11);

5′−GUCAGGAUGGCCGAGUGGUCUAAGGCGCCAGACUCUAGUUCUGGUCUCCGU AUGGAGGCGUGGGUUCGAAUCCCACUUCUGACACCA−3′ (SEQ ID NO: 12);

5′−ACCAGGAUGGCCGAGUGGUUAAGGCGUUGGACUUCAGAUCCAAUGGACAUA UGUCCGCGUGGGUUCGAACCCCACUCCUGGUACCA−3′ (SEQ ID NO: 13);

5′−CCUUCAAUAGUUCAGCUGGUAGAGCAGAGGACUCUAGGUCCUUAGGUUGCU GGUUCGAUUCCAGCUUGAAGGACCA−3′ (SEQ ID NO: 14);

5′−CCUUCAAUAGUUCAGCUGGUAGAGCAGAGGACUUUAGGUCCUUAGGUUGCU GGUUCGAUUCCAGCUUGAAGGACCA−3′ (SEQ ID NO: 15);

5′−GGCCUCGUGGCGCAACGGUAGCGCGUCUGACUCUAGAUCAGAAGGUUGCGU GUUCAAAUCACGUCGGGGUCACCA−3′ (SEQ ID NO: 16);

5′−GCUGUGAUGGCCGAGUGGUUAAGGCGUUGGACUCGAAAUCCAAUGGGGUC UCCCCGCGCAGGUUCGAAUCCUGCUCACAGCGCCA−3′ (SEQ ID NO: 17);

5′−GCAGCGAUGGCCGAGUGGUUAAGGCGUUGGACUUUAAAUCCAAUGGGGUC UCCCCGCGCAGGUUCGAACCCUGCUCGCUGCGCCA−3′ (SEQ ID NO: 18);

5′−GCGUUGGUGGUAUAGUGGUGAGCAUAGCUGCCUUCAAAGCAGUUGACCCGG GUUCGAUUCCCGGCCAACGCACCA-3’(SEQ ID NO: 19);

or

5′−GCGUUGGUGGUAUAGUGGUAAGCAUAGCUGCCUUCAAAGCAGUUGACCCGG GUUCGAUUCCCGGCCAACGCACCA-3’ (SEQ ID NO: 20).

In some embodiments, the tRNA corrects a nonsense mutation in cystic fibrosis transmembrane conductance regulator protein.

In another aspect, the present disclosure provides pharmaceutical compositions comprising:

(A) a composition described herein; and

(B) an excipient.

In some embodiments, the pharmaceutical compositions are formulated for administration: orally, intraadiposally, intraarterially, intraarticularly, intracranially, intradermally, intralesionally, intramuscularly, intranasally, intraocularly, intrapericardially, intraperitoneally, intrapleurally, intraprostatically, intrarectally, intrathecally, intratracheally, intratumorally, intraumbilically, intravaginally, intravenously, intravesicularlly, intravitreally, liposomally, locally, mucosally, parenterally, rectally, subconjunctivally, subcutaneously, sublingually, topically, transbuccally, transdermally, vaginally, in crèmes, in lipid compositions, via a catheter, via a lavage, via continuous infusion, via infusion, via inhalation, via injection, via local delivery, or via localized perfusion. In some embodiments, the pharmaceutical compositions are formulated for administration via injection. In some embodiments, the pharmaceutical compositions are formulated as a unit dose.

In yet another aspect, the present disclosure provides a composition as described herein for use in methods of treating a disease or disorder in a patient comprising administering to a patient in need thereof a therapeutically effective amount of a composition described herein. In some embodiments, the disease or disorder is a genetic disease or disorder. In some embodiments, the genetic disease or disorder is cystic fibrosis. In some embodiments, the gene for the cystic fibrosis transmembrane conductance protein (CFTR) has a nonsense mutation. In some embodiments, the methods result in 25% increase in CFTR activity. In some embodiments, the methods further comprise administering a second cystic fibrosis therapy.

In other embodiments, the genetic disease or disorder is Duchene muscular dystrophy (DMD), congenital disorder of glycosylation, Dravet syndrome, mucopolysaccharidosis I (MPS I) disorder, NGLY1 deficiency (N-glycanase deficiency) disorder, Rett syndrome, or cancer.

In some embodiments, the disease or disorder is cancer. In some embodiments, the cancer is a carcinoma, sarcoma, lymphoma, leukemia, melanoma, mesothelioma, multiple myeloma, or seminoma. In some embodiments, the cancer is of the bladder, blood, bone, brain, breast, central nervous system, cervix, colon, endometrium, esophagus, gall bladder, gastrointestinal tract, genitalia, genitourinary tract, head, kidney, larynx, liver, lung, muscle tissue, neck, oral or nasal mucosa, ovary, pancreas, prostate, skin, spleen, small intestine, large intestine, stomach, testicle, or thyroid. In some embodiments, the cancer is lung cancer or colorectal cancer. In some embodiments, the lung cancer has a nonsense genetic mutation of the p53 gene. In other embodiments, the colorectal cancer has a nonsense genetic mutation of the APC gene. In some embodiments, the cancer comprises a genetic mutation in one or more tumor suppressor protein such as p53, APC, LKB1, ERCC3, WRN, BRCA2, IDH1, or ARID1A. In some embodiments, the cancer is a liver cancer such as hepatitis B derived hepatocellular carcinoma (HCC).

In other embodiments, the methods further comprise administering a second cancer therapy such as a chemotherapy, surgery, immunotherapy, or radiotherapy. In some embodiments, the patient is a mammal such as a human. In some embodiments, the composition is administered once. In other embodiments, the composition is administered two or more times.

As used herein, the specification "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present disclosure will become apparent from the following detailed description. It should be understood, however, that the detailed description and the specific examples, while indicating preferred embodiments of the disclosure, are given by way of illustration only, since various changes and modifications within the spirit and scope of the disclosure will become apparent to those skilled in the art from this detailed description.

## BRIEF DESCRIPTION OF THE DRAWINGS

The following drawings form part of the present specification and are included to further demonstrate certain aspects of the present disclosure. The disclosure may be better understood by reference to one or more of these drawings in combination with the detailed description of specific embodiments presented herein.

**FIG. 1** shows a schematic of the result of a nonsense mutation.

**FIGS. 2A-2C** show the **(FIG. 2A)** Western blot analysis of CFTR mutation after 72 hour transfection in 293 cells. G418 and PTC124 were added 24 hours post transfection. **FIG. 2B** shows the Western blot analysis of CFTR in IB3-1 cells after 48 hour of increasing concentration of PTC124 and G418. Calu-3 cells were used as an antibody control. **FIG. 2C** shows the schematic of read through agent effect on nonsense mutations.

**FIGS. 3A & 3B** show the **(FIG. 3A)** Western blot analysis of CFTR mutants after 48 hours transfection in 293 cells. **FIG. 3B** shows the transepithelial resistance measurements across monolayers of transiently transfected FRT were performed using a TECC 24 (EP Devices) after addition of indicated compounds.

**FIGS. 4A & 4B** show the **(FIG. 4A)** representative single-channel recordings of wild-type (WT) and W1282L-CFTR in excised inside-out membrane patches from transiently transfected CHO cells. ATP (1 mM) and PKA (75 nM) were continuously present in the intracellular solution. Dotted lines indicate the closed channel state and downward deflections correspond to channel openings. The bar graphs below show the single-channel current amplitude (i), single-channel slope conductance ($\gamma$), and open probability ($P_o$) of WT and W1282L-CFTR. Data are presented as means $\pm$ SEM (n > 3); * > 0.05 vs. WT. **FIG. 4B** shows that representative recordings shown the effects of VX-770 (0.05, 0.2, and 1 $\mu$M) on the single-channel activity of W1282L-CFTR in excised inside-out membrane patches from CHO cells. ATP (1 mM) and PKA (75 nM) were continuously present in the intracellular solution.

**FIGS. 5A-5C** show the **(FIG. 5A)** Western blot analysis of CFTR mutants after 48 hour transfection with indicated plasmids in 293 cells. **FIG. 5B** show quantification of full-length CFTR normalized to WT CFTR levels after expression of Sup-tRNA$^{Arg}$ by LiCOR Image Studio software. **FIG. 5C** shows representative whole cell current recordings from 293 cells transfected with indicated plasmids under basal and flow-stimulated conditions.

**FIGS. 6A-6C** show **(FIG. 6A)** shows representative images of fluorescently labeled tRNA delivered by nanoparticles in Hela cells after 24 hour exposure. **FIG. 6B** shows the Western blot analysis of nmP53 (R196X) rescue with indicated carriers after 48 hour exposure in Calu6 cells. **FIG. 6C** shows the schematic of nonsense mutation outcome after delivery of modified tRNAs.

**FIG. 7** shows the fraction of tRNA bound for a variety of different nanoparticle compositions.

**FIG. 8** shows the particle size and polydispersity index of a variety of different nanoparticle compositions.

**FIG. 9** shows the amount of p53 rescued by Western blot analysis of a variety of different nanoparticle compositions.

**FIG. 10** shows that composition RCT-Z100 results in p53 expression event at a half dose. G418 and PTC124 both showed only minimal p53 expression compared to RCT-Z100.

**FIG. 11** shows that the RCT-Z100 nanoparticles are readily taken up into Calu6 cells after incubation as monitored by fluorescence microscopy.

**FIG. 12** shows the zwitterionic amino lipids and cationic sulfonamide amino lipids are able to deliver suppressor tRNA and thus restore p53 expression.

**FIG. 13** shows the results of a luciferase response assay and viability of cells treated with tRNA containing nanoparticles which is prepared using manual mixing or microfluidic mixing and dilution or dilation.

**FIG. 14** shows the components of the ZALs used in these studies.

**FIG. 15** shows the components of the CSALs used in these studies.

## DESCRIPTION OF ILLUSTRATIVE EMBODIMENTS

In some aspects, the present disclosure provides compositions as defined in the claims comprising tRNA, a modified tRNA, or a tRNA derivative and an aminolipid delivery compound which compound forms a nanoparticle. The aminolipid delivery compound assists in the delivery of the tRNA to a cell. These compositions are for use in medicine, where the composition is used to deliver a transfer RNA (tRNA) into a cell, and may be administered to a patient to correct a nonsense mutation in the genome. Thus, in some embodiments, the compositions may be administered to treat a genetic disorder such as, but not limited to, cystic fibrosis, cancer, or Duchene muscular dystrophy.

### I. Aminolipid Delivery Compound

In some aspects, the present disclosure provides compositions containing an aminolipid delivery compound, which forms a nanoparticle. Aminolipid delivery compounds in accordance with the invention are defined in the claims.

In some embodiments, the aminolipid delivery compound may be a dendrimer (also known as a dendron or a dendritic lipid). In other embodiments, the aminolipid delivery compounds is a polymer with a positively charged amine group and one or more hydrophobic components. In some embodiments, the polymer may be degradable *in vivo.* Some non-limiting examples of polymers that may be used in the compositions include polyamides and polyesters. In other embodiments, the aminolipid delivery compound is a zwitterionic compound with a negatively charged group, a positively charged amine group, and one or more hydrophobic components. Some non-limiting examples of possible negatively charged groups that the present compositions may contain include sulfonic acid, phosphorous acid, or carboxylic acid. In some embodiments, the negatively charged group is a sulfonic acid. In still another embodiment, the aminolipid delivery is a positively charged amine group, a sulfonamide functional group, a tetraalkylamine functional group, and one or more hydrophobic component. These compounds have a net positive charge, a dimethyl quarternary amine functional group, and one or more lipid components.

In some embodiments, the present disclosure relates to compounds such as the polyesters of Table 1 and the dendrimers of Table 2. Within the polyesters, the number of repeating units is measured based upon the average molecular weight of the compounds. More information about these compounds can be found in Yan *et al.,* 2016. Additional details about the amino lipid components can be found in WO 2016/094342, WO 2017/048789, and International Application No. PCT/US2017/032950. Additional structures include zwitterionic and cationic compositions prepared using the components described in FIGS. 14 and 15, respectively.

**Table 1: Polyester Polymers Used in the Compositions Described herein**

| Name | Structure |
|---|---|
| **A13-0.2C6-8K** | 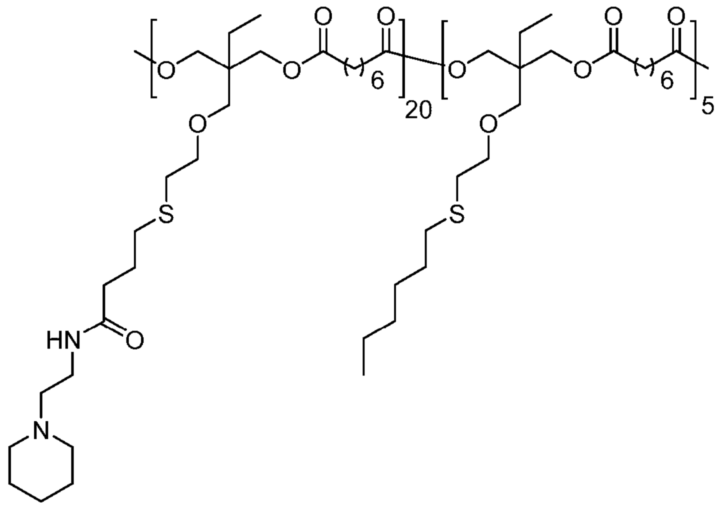 |
| **A13-0.2C10-8K** | 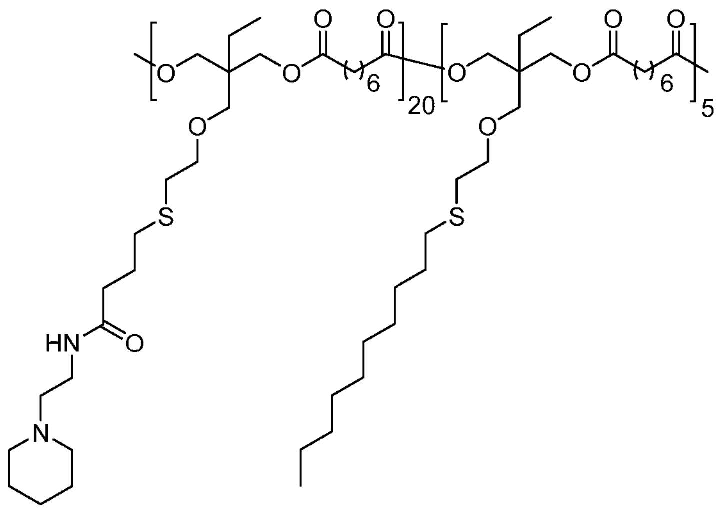 |

(continued)

| Name | Structure |
|---|---|
| **A13-0.2C6-4K** | 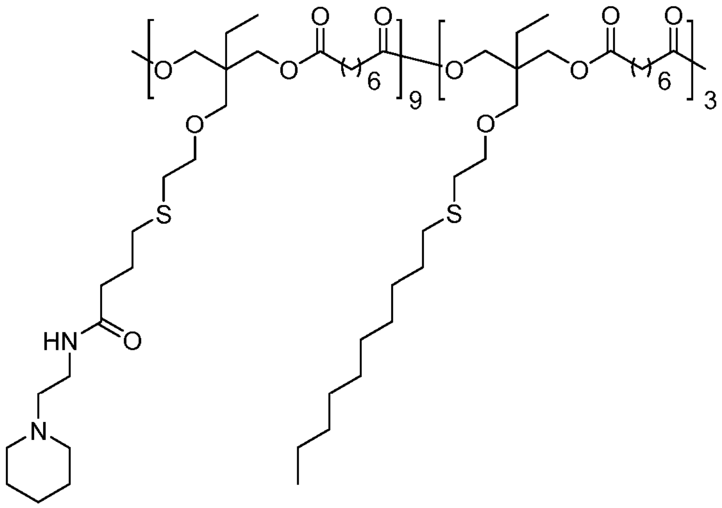 |
| **A13-0.33C6-4K** | 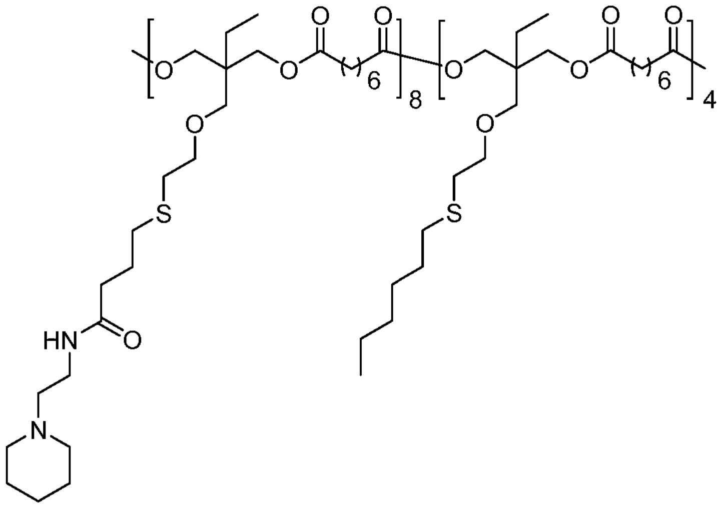 |
| **A13-0.5C10-4K** | 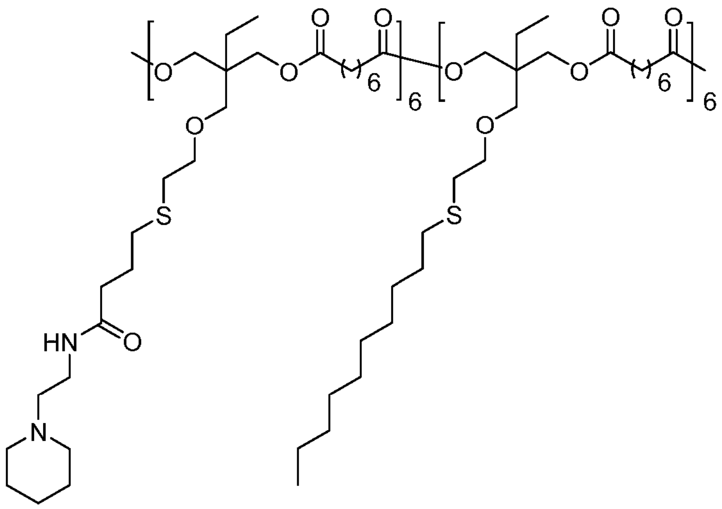 |

(continued)

| Name | Structure |
|---|---|
| **A13-0.2C12-4K** | 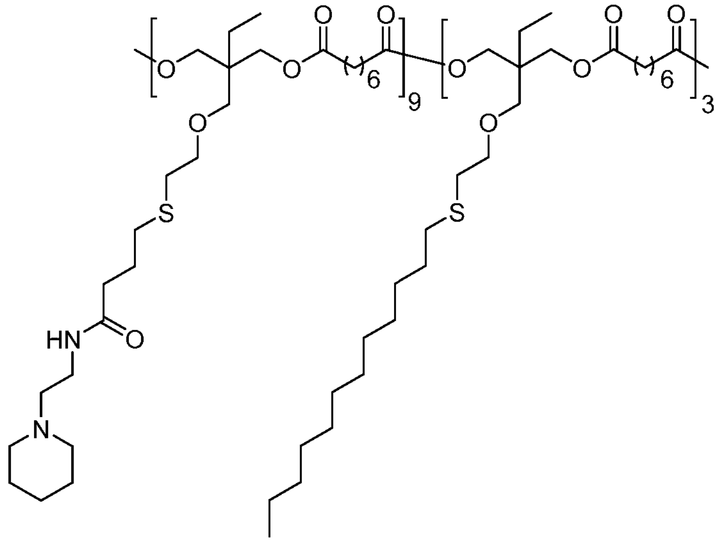 |
| **A13-0.5C6-4K** | 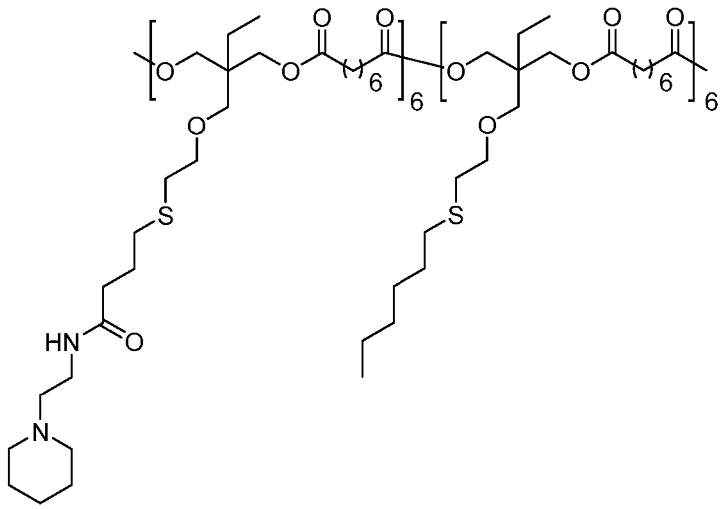 |
| **A13-0.33C10-4K** | 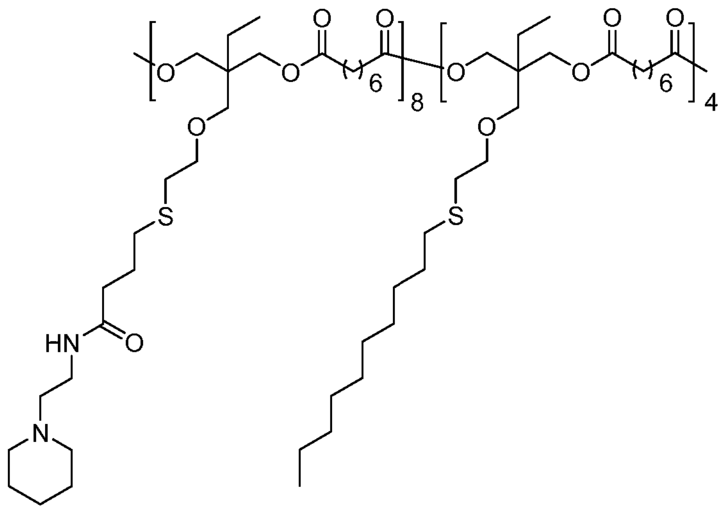 |

**Table 2: Dendrimers Used in the Compositions Described herein**

| ID | Structure |
|---|---|
| **2A1SC14** | 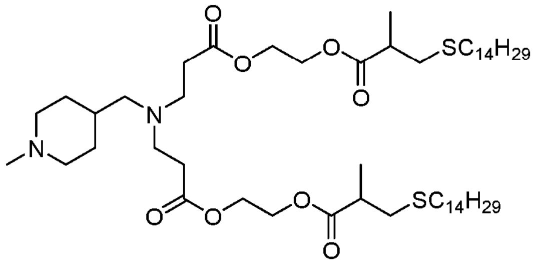 |
| **2A2SC14** | 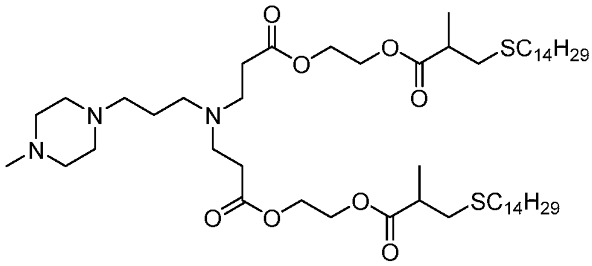 |
| **3A5SC12** | 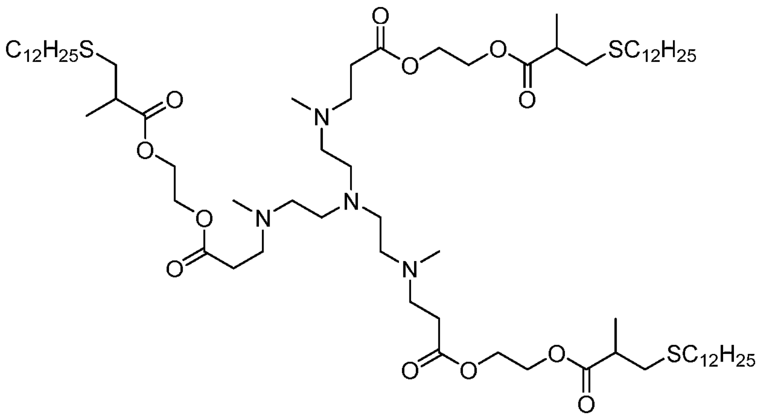 |
| **3A5SC14** | 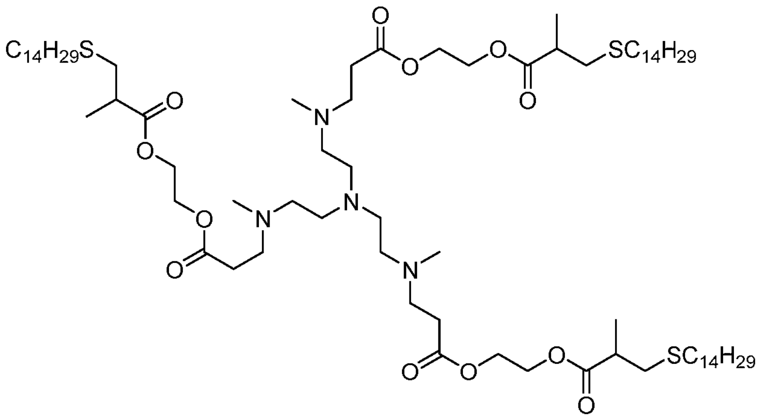 |

(continued)

| ID | Structure |
|---|---|
| **2A5SC14** | 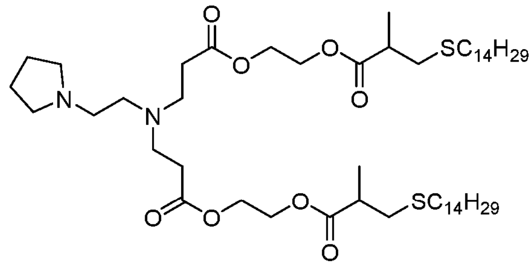 |
| **2A6SC14** | 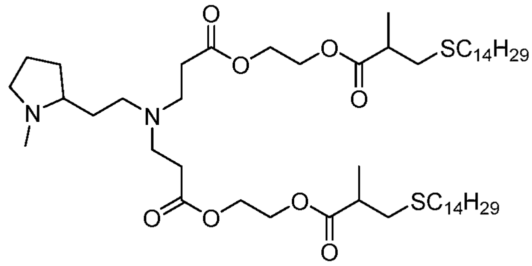 |
| **4A1SC12** | 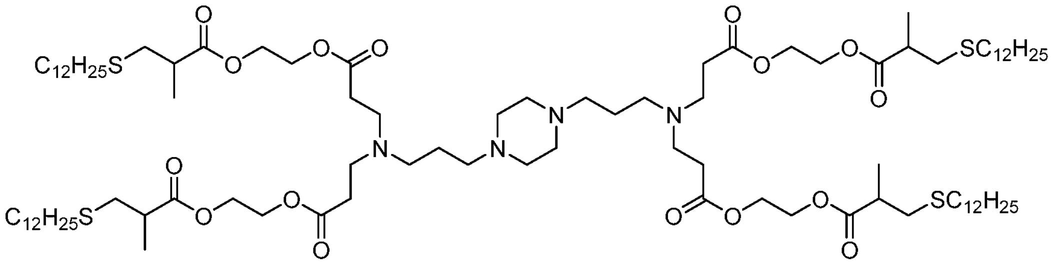 |
| **6A1SC8** | 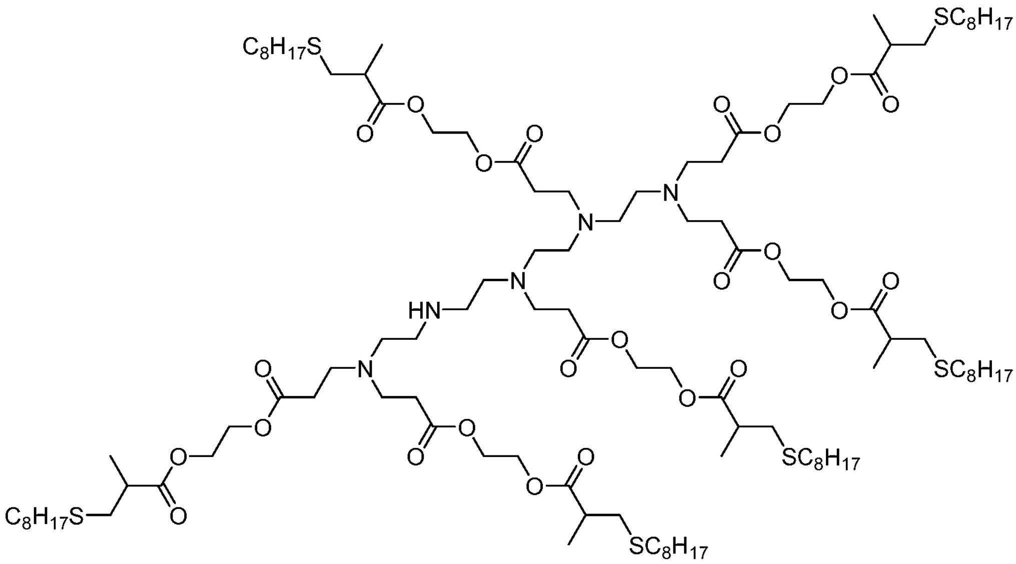 |

(continued)

| ID | Structure |
|---|---|
| 3A3SC6 | 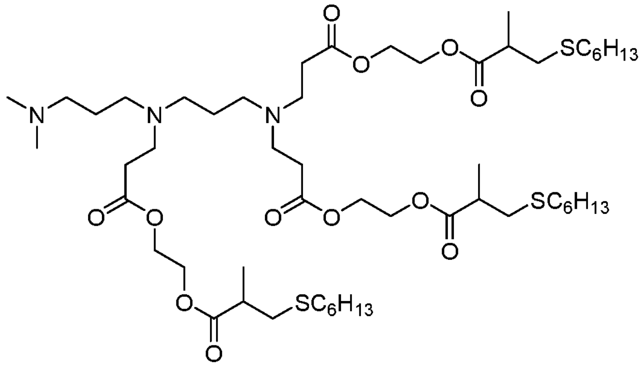 |
| 3A3SC12 | 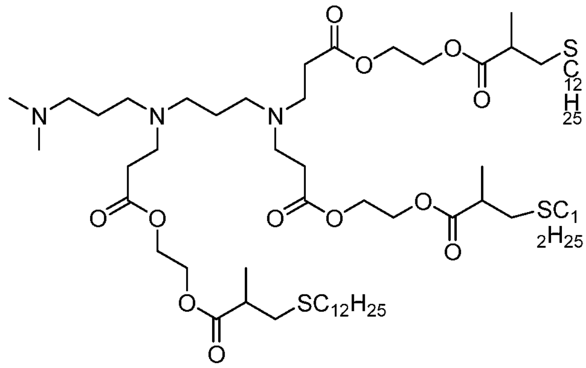 |
| 4A3SC12 | 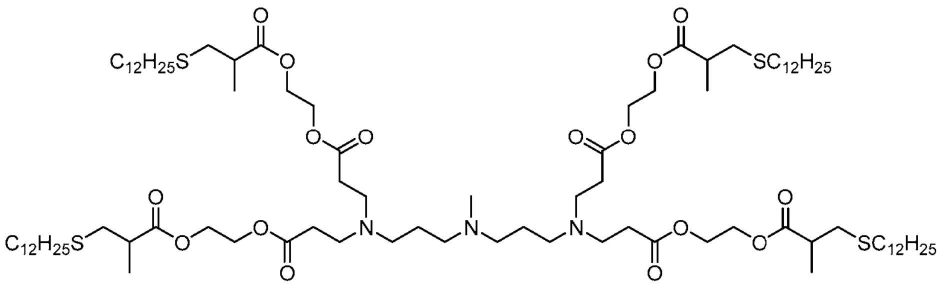 |
| 5A2SC8 | 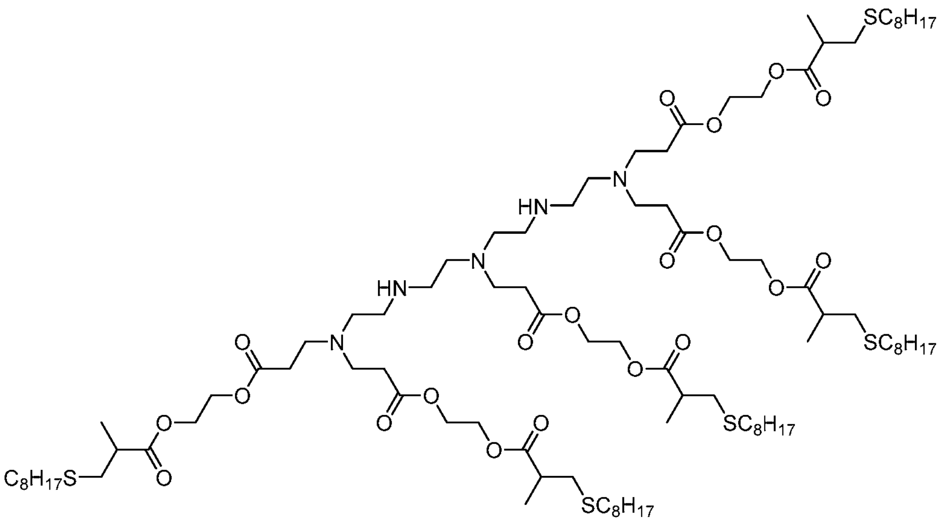 |

(continued)

| ID | Structure |
|---|---|
| **6A4SC12** | 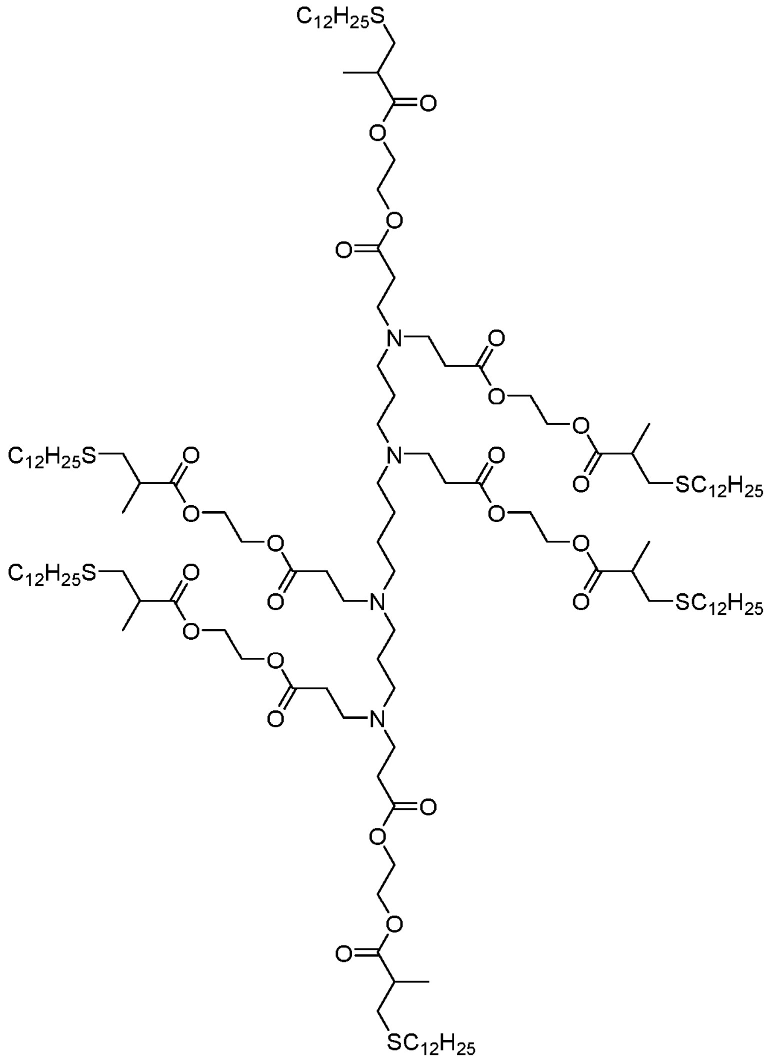 |
| **3A3SC8** | 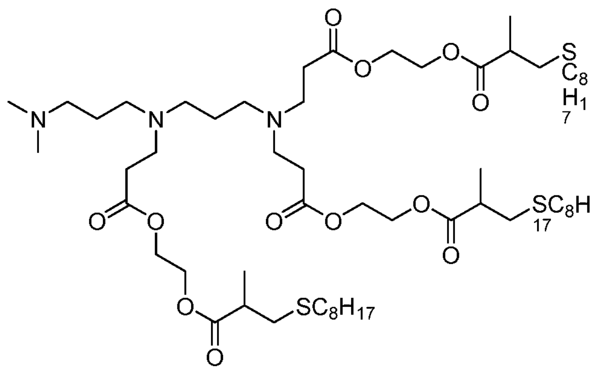 |

(continued)

| ID | Structure |
|---|---|
| **4A3SC8** | 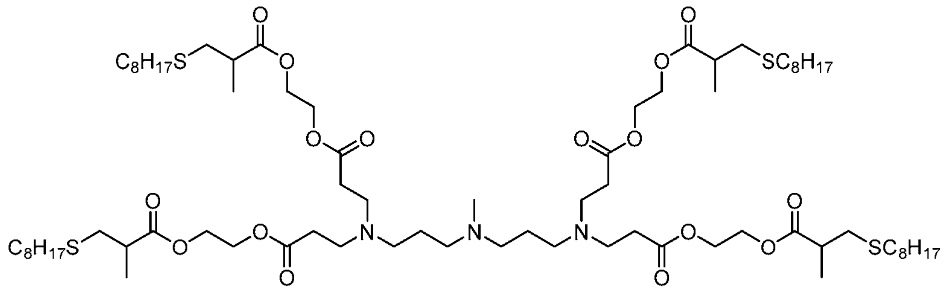 |
| **6A1SC8** | 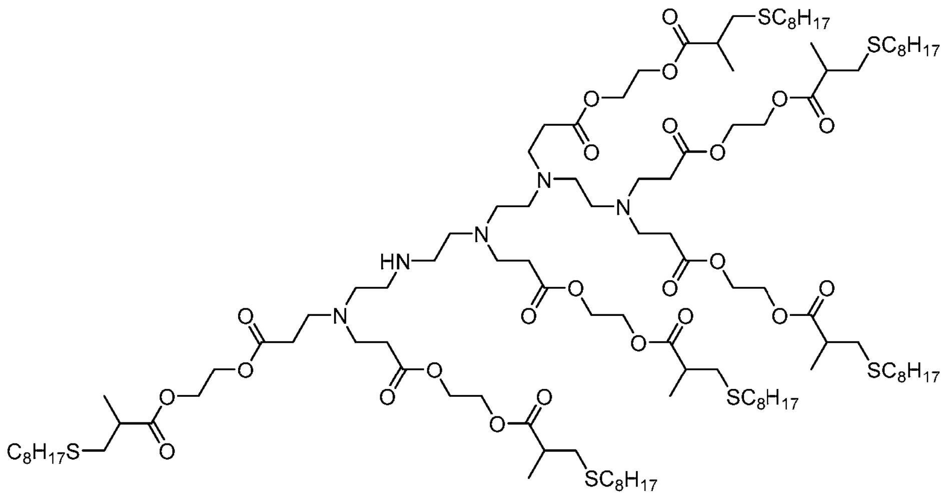 |
| **6A3SC8** | 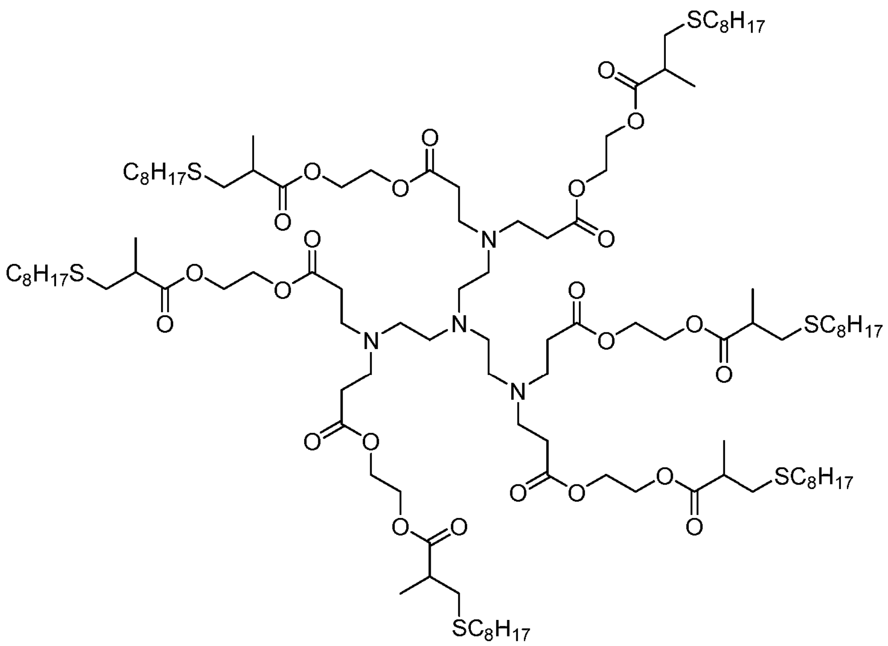 |

(continued)

| ID | Structure |
|---|---|
| **6A4SC8** | 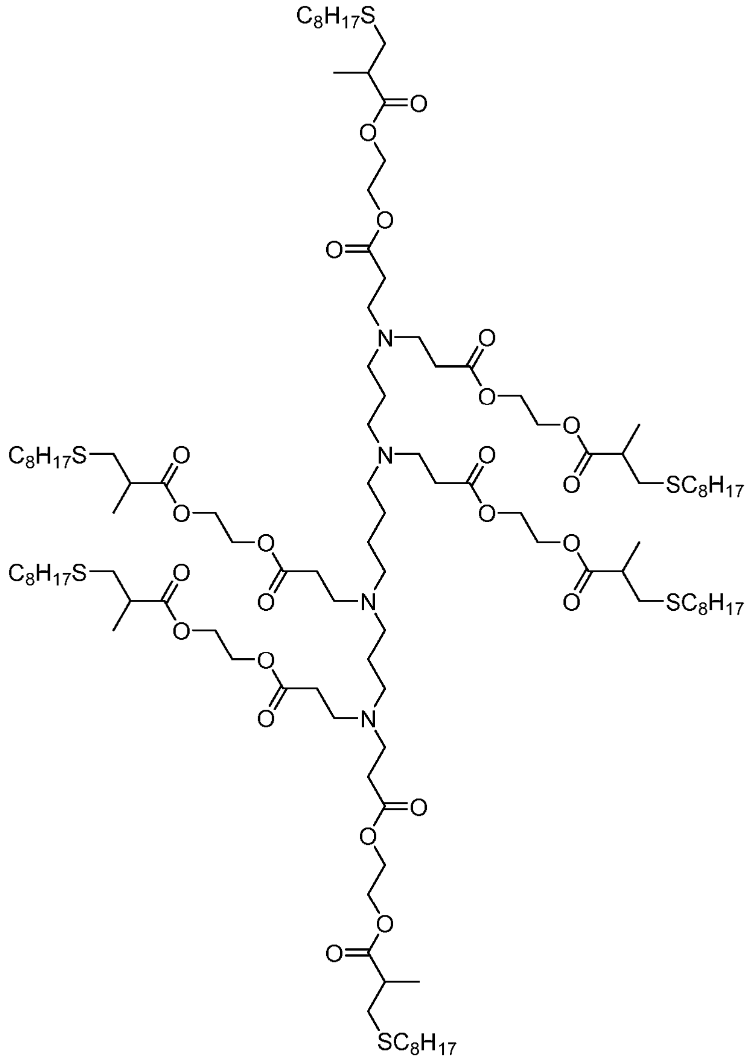 |
| **4A1SC8** | 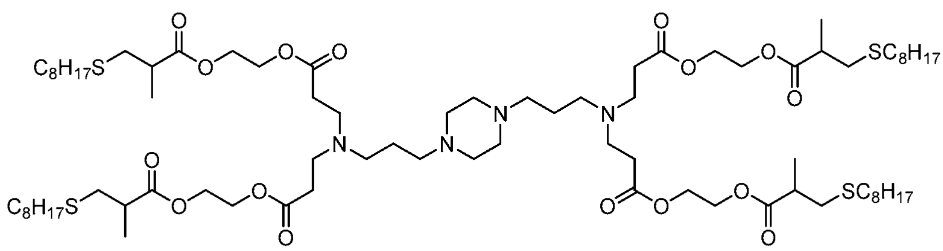 |

The aminolipid delivery compounds provided by the present disclosure are shown, for example, above in the summary section and in the claims below. They may be made using the methods outlined in the Examples section. These methods can be further modified and optimized using the principles and techniques of organic chemistry as applied by a person skilled in the art. Such principles and techniques are taught, for example, in March's Advanced Organic Chemistry: Reactions, Mechanisms, and Structure (2007), which is incorporated by reference herein.

Compounds described herein may contain one or more asymmetrically-substituted carbon or nitrogen atoms, and may be isolated in optically active or racemic form. Thus, all chiral, diastereomeric, racemic form, epimeric form, and all geometric isomeric forms of a chemical formula are intended, unless the specific stereochemistry or isomeric form is specifically indicated. Compounds may occur as racemates and racemic mixtures, single enantiomers, diastereomeric mixtures and individual diastereomers. In some embodiments, a single diastereomer is obtained. The chiral

centers of the compounds of the present disclosure can have the *S* or the *R* configuration.

Chemical formulas used to represent the aminolipid delivery compounds of the disclosure will typically only show one of possibly several different tautomers. For example, many types of ketone groups are known to exist in equilibrium with corresponding enol groups. Similarly, many types of imine groups exist in equilibrium with enamine groups. Regardless of which tautomer is depicted for a given compound, and regardless of which one is most prevalent, all tautomers of a given chemical formula are intended.

Compounds of the disclosure may also have the advantage that they may be more efficacious than, be less toxic than, be longer acting than, be more potent than, produce fewer side effects than, be more easily absorbed than, and/or have a better pharmacokinetic profile (*e.g.,* higher oral bioavailability and/or lower clearance) than, and/or have other useful pharmacological, physical, or chemical properties over, compounds known in the prior art, whether for use in the indications stated herein or otherwise.

In addition, atoms making up the compounds of the present disclosure are intended to include all isotopic forms of such atoms. Isotopes, as used herein, include those atoms having the same atomic number but different mass numbers. By way of general example and without limitation, isotopes of hydrogen include tritium and deuterium, and isotopes of carbon include $^{13}C$ and $^{14}C$.

It should be recognized that the particular anion or cation forming a part of any salt form of a compound provided herein is not critical, so long as the salt, as a whole, is pharmacologically acceptable. Additional examples of pharmaceutically acceptable salts and their methods of preparation and use are presented in Handbook of Pharmaceutical Salts: Properties, and Use (2002).

## II. **tRNA**

The present composition comprises a transfer RNA (known as a tRNA). As used herein, the term transfer RNA or tRNA refers to both traditional tRNA molecules as well as tRNA molecules with one or more modifications unless specifically noted otherwise. Transfer RNA is an RNA polymer that is about 70 to 100 nucleotides in length. During protein synthesis, a tRNA delivers an amino acid to the ribosome for addition to the growing peptide chain. Active tRNAs have a 3' CCA tail that may be transcribed into the tRNA during its synthesis or may be added later during post-transcriptional processing. The amino acid is covalently attached to the 2' or 3' hydroxyl group of the 3'-terminal ribose to form an aminoacyl-tRNA (aa-tRNA); an amino acid can spontaneously migrate from the 2'-OH to the 3'-OH and vice versa, but it is incorporated into a growing protein chain at the ribosome from the 3'-OH position. A loop at the other end of the folded aa-tRNA molecule contains a sequence of three bases known as the anticodon. When this anticodon sequence base-pairs with a three-base codon sequence in a ribosome-bound messenger RNA (mRNA), the aa-tRNA binds to the ribosome and its amino acid is incorporated into the nascent protein chain. Since all tRNAs that base-pair with a specific codon are aminoacylated with a single specific amino acid, the translation of the genetic code is effected by tRNAs: each of the 61 non-termination codons in an mRNA directs the binding of its cognate aa-tRNA and the addition of a single specific amino acid to the growing protein polymer. In some embodiments, the tRNA may comprise a mutation in the anticodon region of the tRNA such that the aa-tRNA base-pairs with a different codon on the mRNA. In certain embodiments, the mutated tRNA introduces a different amino acid into the growing protein chain than the amino acid encoded by the mRNA. In other embodiments, the mutated tRNA base-pairs with a stop codon and introduces an amino acid instead of terminating protein synthesis, thereby allowing the nascent peptide to continue to grow. In some embodiments, a tRNA, wild-type or mutated, may read through a stop codon and introduce an amino acid instead of terminating protein synthesis. In some embodiments, the tRNA may comprise a full-length tRNA with the 3'-terminal-CCA nucleotides included. In other embodiments, tRNAs lacking the 3'-terminal -A, -CA, or -CCA are made full-length *in vivo* by the CCA-adding enzyme.

In other aspects, the present compositions may further comprise one or more modified tRNA molecules including: acylated tRNA; alkylated tRNA; a tRNA containing one or more bases other than adenine, cytosine, guanine, or uracil; a tRNA covalently modified by the attachment of a specific ligand or antigenic, fluorescent, affinity, reactive, spectral, or other probe moiety; a tRNA containing one or more ribose moieties that are methylated or otherwise modified; aa-tRNAs that are aminoacylated with an amino acid other than the 20 natural amino acids, including non-natural amino acids that function as a carrier for reagents, specific ligands, or as an antigentic, fluorescent, reactive, affinity, spectral, or other probe; or any combination of these compositions. Some examples of modified tRNA molecules are taught by Söll, *et al.,* 1995; El Yacoubi, *et al.,* 2012; Grosjean and Benne, *et al.,* 1998; Hendrickson, *et al.,* 2004; Ibba and Söll, 2000; Johnson, *et al.,* 1995; Johnson, *et al.,* 1982; Crowley, *et al.,* 1994; Beier and Grimm, 2001; Torres, *et al.,* 2014; and Björk, *et al.,* 1987.

## III. **Genetic Disorders**

In some aspects, the present disclosure provides compositions that may be used to treat one or more genetic

disorders. One example of a genetic disorder is cystic fibrosis. While some of the cases of cystic fibrosis are the result of a mutation that results in the deletion of a phenylalanine at position 508, about 10% of the cases results from a mutation which generates a stop codon that replaces the codon for the next amino acid in the protein sequence. This mutation, termed a nonsense mutation, results in a truncated and non-functional cystic fibrosis transmembrance conductance regulator (CFTR) protein.

A mutation in the CFTR protein results in impaired chloride ion transport and thus patients with cystic fibrosis present with salty tasting skin, poor growth, poor weight gain, the accumulation of mucus particularly in the lungs, frequent chest infections, and a persistent cough. As cystic fibrosis is a genetic disorder, treatment options have often related to treating the symptoms rather than the underlying pathology. Such treatments include treating infections, therapies that remove the mucus building up, and organ transplants such as a lung transplant. Thus, efforts that can address the underlying pathology are of critical importance.

In another aspect, the present disclosure contemplates the treatment of cancer, especially cancers with a nonsense mutation in one or more tumor suppressor genes. Some non-limiting types of cancer include a cancer of the bladder, blood, bone, brain, breast, central nervous system, cervix, colon, endometrium, esophagus, gall bladder, gastrointestinal tract, genitalia, genitourinary tract, head, kidney, larynx, liver, lung, muscle tissue, neck, oral or nasal mucosa, ovary, pancreas, prostate, skin, spleen, small intestine, large intestine, stomach, testicle, or thyroid or is a carcinoma, sarcoma, lymphoma, leukemia, melanoma, mesothelioma, multiple myeloma, or seminoma. A non-limiting list of possible tumor suppressor proteins includes p53, pRb, PTEN, SWI, SNF, pVHL, APC, CD95, ST5, YPEL3, ST7, and ST14. In addition to the composition described herein, the patient may be treated with one or more additional anti-cancer therapies, including chemotherapy, surgery, immunotherapy, or radiotherapy.

## IV. Therapies

### A. Pharmaceutical Formulations and Routes of Administration

Where clinical applications are contemplated, it will be necessary to prepare pharmaceutical compositions in a form appropriate for the intended application. In some embodiments, such formulation with the compositions of the present disclosure is contemplated. Generally, this will entail preparing compositions, as defined in the claims, that are essentially free of pyrogens, as well as other impurities that could be harmful to humans or animals.

One will generally desire to employ appropriate salts and buffers to render delivery vectors stable and allow for uptake by target cells. Buffers also will be employed when recombinant cells are introduced into a patient. Aqueous compositions of the present disclosure comprise an effective amount of the vector to cells, dissolved or dispersed in a pharmaceutically acceptable carrier or aqueous medium. Such compositions also are referred to as inocula. The phrase "pharmaceutically or pharmacologically acceptable" refers to molecular entities and compositions that do not produce adverse, allergic, or other untoward reactions when administered to an animal or a human. As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the vectors or cells of the present disclosure, its use in therapeutic compositions is contemplated. Supplementary active ingredients also can be incorporated into the compositions.

The active compositions of the present disclosure may include classic pharmaceutical preparations. Administration of these compositions according to the present disclosure will be via any common route so long as the target tissue is available via that route. Such routes include oral, nasal, buccal, rectal, vaginal or topical route. Alternatively, administration may be by orthotopic, intradermal, subcutaneous, intramuscular, intratumoral, intraperitoneal, or intravenous injection. Such compositions would normally be administered as pharmaceutically acceptable compositions, described *supra.*

The active compositions may also be administered parenterally or intraperitoneally. Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required

particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

For oral administration the compositions described herein may be incorporated with excipients and used in the form of non-ingestible mouthwashes and dentifrices. A mouthwash may be prepared incorporating the active ingredient in the required amount in an appropriate solvent, such as a sodium borate solution (Dobell's Solution). Alternatively, the active ingredient may be incorporated into an antiseptic wash containing sodium borate, glycerin and potassium bicarbonate. The active ingredient may also be dispersed in dentifrices, including: gels, pastes, powders and slurries. The active ingredient may be added in a therapeutically effective amount to a paste dentifrice that may include water, binders, abrasives, flavoring agents, foaming agents, and humectants.

The compositions of the present disclosure may be formulated in a neutral or salt form. Pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Upon formulation, solutions will be administered in a manner compatible with the dosage formulation and in such amount as is therapeutically effective. The formulations are easily administered in a variety of dosage forms such as injectable solutions, drug release capsules and the like. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 mL of hypodermoclysis fluid or injected at the proposed site of infusion, (see for example, "Remington's Pharmaceutical Sciences," 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

### I. *Steroids and Steroid Derivatives*

In some aspects of the present disclosure, the composition further comprises one or more steroid or a steroid derivative to create a nanoparticle composition. In some embodiments, the steroid or steroid derivative comprises any steroid or steroid derivative. As used herein, in some embodiments, the term "steroid" is a class of compounds with a four ring 17 carbon cyclic structure which can further comprises one or more substitutions including alkyl groups, alkoxy groups, hydroxy groups, oxo groups, acyl groups, or a double bond between two or more carbon atoms. In one aspect, the ring structure of a steroid comprises three fused cyclohexyl rings and a fused cyclopentyl ring as shown in the formula below:

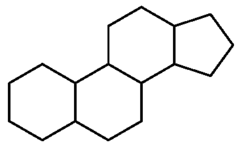

.

In some embodiments, a steroid derivative comprises the ring structure above with one or more non-alkyl substitutions. In some embodiments, the steroid or steroid derivative is a sterol wherein the formula is further defined as:

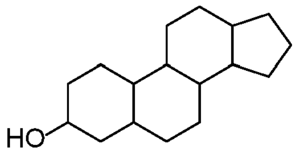

.

In some embodiments of the present disclosure, the steroid or steroid derivative is a cholestane or cholestane derivative. In a cholestane, the ring structure is further defined by the formula:

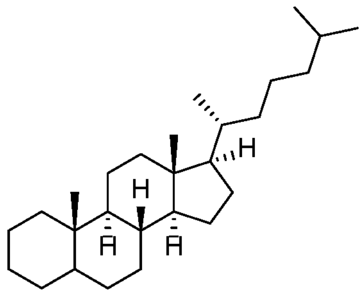

As described above, a cholestane derivative includes one or more non-alkyl substitution of the above ring system. In some embodiments, the cholestane or cholestane derivative is a cholestene or cholestene derivative or a sterol or a sterol derivative. In other embodiments, the cholestane or cholestane derivative is both a cholestere and a sterol or a derivative thereof.

In some embodiments, the present composition comprises a ratio of the compound or amino lipids to the steroid or steroid derivative from about 1:3 to about 30:1 or from about 1:1 to about 20:1. The ratio may be from about 1:1-6:1 such as a ratio of about 1.3:1. In some embodiments, the ratio is from about 1:3, 1:2, 1:1, 1.25:1, 1.5:1, 2:1, 3:1, 5:1, 8:1, 10:1, 12.5:1, 15:1, 17.5:1, 20:1, 25:1, to about 30:1, or any range derivable therein.

## II. *PEG or PEGylated lipid*

In some aspects of the present disclosure, composition further comprises one or more PEGylated lipids (or PEG lipid) to create a nanoparticle composition. In some embodiments, the present disclosure comprises using any lipid to which a PEG group has been attached. In some embodiments, the PEG lipid is a diglyceride which also comprises a PEG chain attached to the glycerol group. In other embodiments, the PEG lipid is a compound which contains one or more C6-C24 long chain alkyl or alkenyl group or a C6-C24 fatty acid group attached to a linker group with a PEG chain. Some non-limiting examples of a PEG lipid includes a PEG modified phosphatidylethanolamine and phosphatidic acid, a PEG ceramide conjugated, PEG modified dialkylamines and PEG modified 1,2-diacyloxypropan-3-amines, PEG modified diacylglycerols and dialkylglycerols. In some embodiments, PEG modified diastearoylphosphatidylethanolamine or PEG modified dimyristoyl-sn-glycerol. In some embodiments, the PEG modification is measured by the molecular weight of PEG component of the lipid. In some embodiments, the PEG modification has a molecular weight from about 100 to about 5,000. In some embodiments, the molecular weight is from about 200 to about 500 or from about 1,200 to about 3,000. Some non-limiting examples of lipids that may be used in the present disclosure are taught by U.S. Patent 5,820,873, WO 2010/141069, or U.S. Patent 8,450,298.

In another aspect, the PEG lipid has the formula:

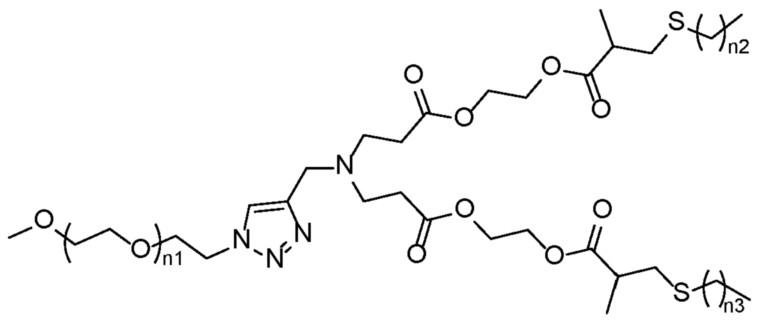

wherein: $n_1$ is an integer between 1 and 100 and $n_2$ and $n_3$ are each independently selected from an integer between 1 and 29. In some embodiments, $n_1$ is 5, 10, 15, 20, 25, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, or 100, or any range derivable therein. In some embodiments, $n_1$ is from about 30 to about 50. In some embodiments, $n_2$ is from 5 to 23. In some embodiments, $n_2$ is 11 to about 17. In some embodiments, $n_3$ is from 5 to 23. In some embodiments, $n_3$ is 11 to about 17.

In some embodiments, the present composition comprises a ratio of the compound or amino lipids to the PEG lipid from about 1:1 to about 150:1 or from about 2.5:1 to about 100:1. The ratio may be from about 7.5:1-50:1 such as a ratio of about 33.3:1. In some embodiments, the ratio is from about 5:1, 10:1, 20:1, 25:1, 30:1, 35:1, 40:1, 50:1, 60:1, 70:1, 80:1, 90:1, 100:1, 120:1, 140:1, to about 150:1, or any range derivable therein.

### III. *Phospholipid*

In some aspects of the present disclosure, the composition further comprises one or more phospholipids to create a nanoparticle composition. In some embodiments, any lipid which also comprises a phosphate group. In some embodiments, the phospholipid is a structure which contains one or two long chain C6-C24 alkyl or alkenyl groups, a glycerol or a sphingosine, one or two phosphate groups, and, optionally, a small organic molecule. In some embodiments, the small organic molecule is an amino acid, a sugar, or an amino substituted alkoxy group, such as choline or ethanolamine. In some embodiments, the phospholipid is a phosphatidylcholine. In some embodiments, the phospholipid is distearoylphosphatidylcholine.

In some embodiments, the present composition comprises a ratio of the compound or amino lipids to the phospholipid from about 1:1 to about 15:1 or from about 1:1 to about 9:1. The ratio may be from about 2.5:1-7.5:1 such as a ratio of about 5:1. In some embodiments, the ratio is from about 1:1, 2:1, 3:1, 4:1, 4.5:1, 5:1, 5.5:1, 6:1, 7:1, 8:1, 9:1, 10:1, 12:1, 14:1, to about 15:1, or any range derivable therein.

### B. Methods of Treatment

The compositions defined in the claims are for use in medicine, wherein the composition is used to deliver a transfer RNA (tRNA) into a cell. In particular, the compositions that may be used in treating genetic diseases and cancer in a subject (e.g., a human subject) are disclosed herein. The compositions described above are preferably administered to a mammal (e.g., rodent, human, non-human primates, canine, bovine, ovine, equine, feline, *etc.*) in an effective amount, that is, an amount capable of producing a desirable result in a treated subject. Toxicity and therapeutic efficacy of the compositions utilized in methods of the disclosure can be determined by standard pharmaceutical procedures. As is well known in the medical and veterinary arts, dosage for any one animal depends on many factors, including the subject's size, body surface area, body weight, age, the particular composition to be administered, time and route of administration, general health, the clinical symptoms of the genetic disease or cancer and other drugs being administered concurrently. In some embodiments, these dosings may be reduced or increased based upon the biological factors of a particular patient such as increased or decreased metabolic breakdown of the drug or decreased uptake by the digestive tract if administered orally. Additionally, the active compositions may be more efficacious and thus a smaller dose is required to achieve a similar effect. Such a dose is typically administered once a day for a few weeks or until sufficient reducing in cancer cells has been achieved.

The composition defined in the claims for use in medicine, wherein the composition is used to deliver a transfer RNA (tRNA) into a cell, involves therapeutic methods (which include prophylactic treatment) that in general include administration of a therapeutically effective amount of the compositions described herein to a subject in need thereof, including a mammal, particularly a human. Such treatment will be suitably administered to subjects, particularly humans, suffering from, having, susceptible to, or at risk for a disease, disorder, or symptom thereof. Determination of those subjects "at risk" can be made by any objective or subjective determination by a diagnostic test or opinion of a subject

or health care provider (e.g., genetic test, enzyme or protein marker, marker (as defined herein), family history, and the like).

### C. Combination Therapies

It is envisioned that the active compositions defined in the claims may be used in combination therapies with one or more therapy. In some embodiments, it is common in the field of cancer therapy to combine therapeutic modalities. The following is a general discussion of therapies that may be used in conjunction with the therapies of the present disclosure.

For example, when compositions for use in medicine as defined in the claimed are used to treat, one would generally contact a tumor cell or subject with a compound and at least one other therapy. These therapies would be provided in a combined amount effective to achieve a reduction in one or more disease parameter. This process may involve contacting the cells/subjects with the both agents/therapies at the same time, e.g., using a single composition or pharmacological formulation that includes both agents, or by contacting the cell/subject with two distinct compositions or formulations, at the same time, wherein one composition includes the compound and the other includes the other agent.

Alternatively, the active compositions described herein may precede or follow the other treatment by intervals ranging from minutes to weeks. One would generally ensure that a significant period of time did not expire between the time of each delivery, such that the therapies would still be able to exert an advantageously combined effect on the cell/subject. In such instances, it is contemplated that one would contact the cell with both modalities within about 12-24 hours of each other, within about 6-12 hours of each other, or with a delay time of only about 1-2 hours. In some situations, it may be desirable to extend the time period for treatment significantly; however, where several days (2, 3, 4, 5, 6 or 7) to several weeks (1, 2, 3, 4, 5, 6, 7 or 8) lapse between the respective administrations.

It also is conceivable that more than one administration of either the compound or the other therapy will be desired. Various combinations may be employed, where a compound of the present disclosure is "A," and the other therapy is "B," as exemplified below:

A/B/A B/A/B B/B/A A/A/B B/A/A A/B/B B/B/B/A B/B/A/B
A/A/B/B A/B/A/B A/B/B/A B/B/A/A B/A/B/A B/A/A/B B/B/B/A
A/A/A/B B/A/A/A A/B/A/A A/A/B/A A/B/B/B B/A/B/B B/B/A/B

Other combinations are also contemplated. The following is a general discussion of cancer therapies that may be used combination with the compounds of the present disclosure.

#### 1. Chemotherapy

The term "chemotherapy" refers to the use of drugs to treat cancer. A "chemotherapeutic agent" is used to connote a compound or composition that is administered in the treatment of cancer. These agents or drugs are categorized by their mode of activity within a cell, for example, whether and at what stage they affect the cell cycle. Alternatively, an agent may be characterized based on its ability to directly cross-link DNA, to intercalate into DNA, or to induce chromosomal and mitotic aberrations by affecting nucleic acid synthesis. Most chemotherapeutic agents fall into the following categories: alkylating agents, antimetabolites, antitumor antibiotics, mitotic inhibitors, and nitrosoureas.

Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e.g.*, calicheamicin, especially calicheamicin $\gamma 1$ and calicheamicin $\omega 1$; dynemicin, including dynemicin A; uncialamycin and derivatives thereof; bisphosphonates, such as clodronate; an esperamicin; as well as neocarzinostatin chromophore and related chromoprotein enediyne antiobiotic chromophores, aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, or zorubicin; anti-metabolites such as methotrexate and 5-fluorouracil (5-FU); folic acid analogues such as denopterin,

methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as folinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK polysaccharide complex); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel and docetaxel; chlorambucil; gemcitabine; 6-thioguanine; mercaptopurine; methotrexate; platinum coordination complexes such as cisplatin, oxaliplatin and carboplatin; vinblastine; platinum; etoposide (VP-16); ifosfamide; mitoxantrone; vincristine; vinorelbine; novantrone; teniposide; edatrexate; daunomycin; aminopterin; xeloda; ibandronate; irinotecan (e.g., CPT-11); topoisomerase inhibitor RFS 2000; difluorometlhylomithine (DMFO); retinoids such as retinoic acid; capecitabine; cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, paclitaxel, docetaxel, gemcitabien, navelbine, famesyl-protein tansferase inhibitors, transplatinum, 5-fluorouracil, vincristin, vinblastin and methotrexate and pharmaceutically acceptable salts, acids or derivatives of any of the above.

### 2. Radiotherapy

Radiotherapy, also called radiation therapy, is the treatment of cancer and other diseases with ionizing radiation. Ionizing radiation deposits energy that injures or destroys cells in the area being treated by damaging their genetic material, making it impossible for these cells to continue to grow. Although radiation damages both cancer cells and normal cells, the latter are able to repair themselves and function properly.

Radiation therapy used according to the present disclosure may include, but is not limited to, the use of γ-rays, X-rays, and/or the directed delivery of radioisotopes to tumor cells. Other forms of DNA damaging factors are also contemplated such as microwaves and UV-irradiation. It is most likely that all of these factors induce a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

Radiotherapy may comprise the use of radiolabeled antibodies to deliver doses of radiation directly to the cancer site (radioimmunotherapy). Antibodies are highly specific proteins that are made by the body in response to the presence of antigens (substances recognized as foreign by the immune system). Some tumor cells contain specific antigens that trigger the production of tumor-specific antibodies. Large quantities of these antibodies can be made in the laboratory and attached to radioactive substances (a process known as radiolabeling). Once injected into the body, the antibodies actively seek out the cancer cells, which are destroyed by the cell-killing (cytotoxic) action of the radiation. This approach can minimize the risk of radiation damage to healthy cells.

Conformal radiotherapy uses the same radiotherapy machine, a linear accelerator, as the normal radiotherapy treatment but metal blocks are placed in the path of the x-ray beam to alter its shape to match that of the cancer. This ensures that a higher radiation dose is given to the tumor. Healthy surrounding cells and nearby structures receive a lower dose of radiation, so the possibility of side effects is reduced. A device called a multi-leaf collimator has been developed and may be used as an alternative to the metal blocks. The multi-leaf collimator consists of a number of metal sheets which are fixed to the linear accelerator. Each layer can be adjusted so that the radiotherapy beams can be shaped to the treatment area without the need for metal blocks. Precise positioning of the radiotherapy machine is very important for conformal radiotherapy treatment and a special scanning machine may be used to check the position of internal organs at the beginning of each treatment.

High-resolution intensity modulated radiotherapy also uses a multi-leaf collimator. During this treatment the layers of the multi-leaf collimator are moved while the treatment is being given. This method is likely to achieve even more precise shaping of the treatment beams and allows the dose of radiotherapy to be constant over the whole treatment area.

Although research studies have shown that conformal radiotherapy and intensity modulated radiotherapy may reduce the side effects of radiotherapy treatment, it is possible that shaping the treatment area so precisely could stop microscopic cancer cells just outside the treatment area being destroyed. This means that the risk of the cancer coming back in the future may be higher with these specialized radiotherapy techniques.

Scientists also are looking for ways to increase the effectiveness of radiation therapy. Two types of investigational drugs are being studied for their effect on cells undergoing radiation. Radiosensitizers make the tumor cells more likely to be damaged, and radioprotectors protect normal tissues from the effects of radiation. Hyperthermia, the use of heat, is also being studied for its effectiveness in sensitizing tissue to radiation.

### 3. Immunotherapy

In the context of cancer treatment, immunotherapeutics, generally, rely on the use of immune effector cells and molecules to target and destroy cancer cells. Trastuzumab (Herceptin™) is such an example. The immune effector may be, for example, an antibody specific for some marker on the surface of a tumor cell. The antibody alone may serve as an effector of therapy or it may recruit other cells to actually affect cell killing. The antibody also may be conjugated to a drug or toxin (chemotherapeutic, radionuclide, ricin A chain, cholera toxin, pertussis toxin, *etc.*) and serve merely as a targeting agent. Alternatively, the effector may be a lymphocyte carrying a surface molecule that interacts, either directly or indirectly, with a tumor cell target. Various effector cells include cytotoxic T cells and NK cells. The combination of therapeutic modalities, *i.e.,* direct cytotoxic activity and inhibition or reduction of ErbB2 would provide therapeutic benefit in the treatment of ErbB2 overexpressing cancers.

In one aspect of immunotherapy, the tumor cell must bear some marker that is amenable to targeting, *i.e.,* is not present on the majority of other cells. Many tumor markers exist and any of these may be suitable for targeting in the context of the present disclosure. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, erb B and p155. An alternative aspect of immunotherapy is to combine anticancer effects with immune stimulatory effects. Immune stimulating molecules also exist including: cytokines such as IL-2, IL-4, IL-12, GM-CSF, $\gamma$-IFN, chemokines such as MIP-1, MCP-1, IL-8 and growth factors such as FLT3 ligand. Combining immune stimulating molecules, either as proteins or using gene delivery in combination with a tumor suppressor has been shown to enhance anti-tumor effects (Ju *et al.,* 2000). Moreover, antibodies against any of these compounds may be used to target the anti-cancer agents discussed herein.

Examples of immunotherapies currently under investigation or in use are immune adjuvants *e.g., Mycobacterium bovis, Plasmodium falciparum,* dinitrochlorobenzene and aromatic compounds (U.S. Patents 5,801,005 and 5,739,169; Hui and Hashimoto, 1998; Christodoulides *et al.,* 1998), cytokine therapy, *e.g.,* interferons $\alpha$, $\beta$, and y; IL-1, GM-CSF and TNF (Bukowski *et al.,* 1998; Davidson *et al.,* 1998; Hellstrand *et al.,* 1998) gene therapy, *e.g.,* TNF, IL-1, IL-2, p53 (Qin *et al.,* 1998; Austin-Ward and Villaseca, 1998; U.S. Patents 5,830,880 and 5,846,945) and monoclonal antibodies, e.g., anti-ganglioside GM2, anti-HER-2, anti-p185 (Pietras *et al.,* 1998; Hanibuchi *et al.,* 1998; U.S. Patent 5,824,311). It is contemplated that one or more anti-cancer therapies may be employed with the gene silencing therapies described herein.

In active immunotherapy, an antigenic peptide, polypeptide or protein, or an autologous or allogenic tumor cell composition or "vaccine" is administered, generally with a distinct bacterial adjuvant (Ravindranath and Morton, 1991; Morton *et al.,* 1992; Mitchell *et al.,* 1990; Mitchell *et al.,* 1993).

In adoptive immunotherapy, the patient's circulating lymphocytes, or tumor infiltrated lymphocytes, are isolated *in vitro,* activated by lymphokines such as IL-2 or transduced with genes for tumor necrosis, and readministered (Rosenberg *et al.,* 1988; 1989).

### 4. Surgery

Approximately 60% of persons with cancer will undergo surgery of some type, which includes preventative, diagnostic or staging, curative, and palliative surgery. Curative surgery is a cancer treatment that may be used in conjunction with other therapies, such as the treatment of the present disclosure, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and microscopically controlled surgery (Mohs' surgery). It is further contemplated that the present disclosure may be used in conjunction with removal of superficial cancers, precancers, or incidental amounts of normal tissue.

Upon excision of part or all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

In some particular embodiments, after removal of the tumor, an adjuvant treatment with a compound of the present disclosure is believe to be particularly efficacious in reducing the reoccurance of the tumor. Additionally, the

compounds of the present disclosure can also be used in a neoadjuvant setting.

### 5. Cystic Fibrosis Treatments

There is no cure for cystic fibrosis and thus much of the treatment options treat the symptoms rather than the underlying pathology of the conditions. Some therapies include methods to reduce the deposition of the mucus within the airways. The therapies may be physical therapy, exercise, manual expectoration, or medication. These medicines include the use of saline, a DNase such as domase alfa, a mucolytic such as acetylcysteine, a bronchodilator such as albuterol or salmeterol, or an anticholinergic. In other aspects, the cystic fibrosis treatment is an antibiotic, such as but not limited to, gentamicin, piperacillin, tazobactum, azteonam, ciprofloxacin, tobramycin, ceftazidime, amikacin, meropenem, or azithromycin. In yet another aspect, the cystic fibrosis treatment may be an anti-inflammatory agent such as an NSAID, a membrane stabilizer such as cromolyn, or a corticosteroid such as fluticasone or prednisone. In other aspects, the cystic fibrosis treatment is another therapy such as ivacaftor.

## V. Definitions

When used in the context of a chemical group: "hydrogen" means -H; "hydroxy" means -OH; "oxo" means =O; "carbonyl" means -C(=O)-; "carboxy" means -C(=O)OH (also written as -COOH or $-CO_2H$); "halo" means independently -F, -Cl, -Br or -I; "amino" means $-NH_2$; "hydroxyamino" means -NHOH; "nitro" means $-NO_2$; imino means =NH; "cyano" means -CN; "isocyanate" means -N=C=O; "azido" means $-N_3$; in a monovalent context "phosphate" means $-OP(O)(OH)_2$ or a deprotonated form thereof; in a divalent context "phosphate" means -OP(O)(OH)O- or a deprotonated form thereof; "mercapto" means -SH; and "thio" means =S; "sulfonyl" means $-S(O)_2-$; "hydroxysulfonyl" means $-S(O)_2OH$; "sulfonamido" means $-S(O)_2NH_2$; and "sulfinyl" means -S(O)-.

In the context of chemical formulas, the symbol "-" means a single bond, "=" means a double bond, and "≡" means triple bond. The symbol "- - - -" represents an optional bond, which if present is either single or double. The symbol "- - - -" represents a single bond or a double bond. Thus, for example, the formula

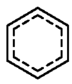

includes

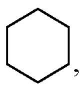, 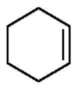,

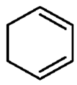, 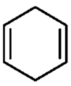 and 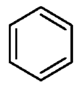.

And it is understood that no one such ring atom forms part of more than one double bond. Furthermore, it is noted that the covalent bond symbol "-", when connecting one or two stereogenic atoms, does not indicate any preferred stereochemistry. Instead, it covers all stereoisomers as well as mixtures thereof. The symbol "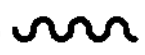", when drawn perpendicularly across a bond (*e.g.*, 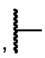$CH_3$ for methyl) indicates a point of attachment of the group. It is noted that the point of attachment is typically only identified in this manner for larger groups in order to assist the reader in unambiguously identifying a point of attachment. The symbol "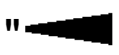" means a single bond where the group attached to the thick end of the wedge is "out of the page." The symbol "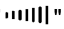" means a single bond where the group attached to the thick end of the wedge is "into the page". The symbol "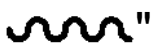" means a single bond where the geometry around a double bond (e.g., either E or Z) is undefined. Both options, as well as combinations thereof are therefore intended. Any undefined valency on an atom of a structure shown in this application implicitly represents a hydrogen atom bonded to that atom. A bold dot on a carbon atom indicates that the hydrogen attached to that carbon is oriented out of the plane of the paper.

When a group "R" is depicted as a "floating group" on a ring system, for example, in the formula:

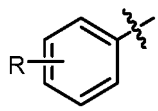

,

then R may replace any hydrogen atom attached to any of the ring atoms, including a depicted, implied, or expressly defined hydrogen, so long as a stable structure is formed. When a group "R" is depicted as a "floating group" on a fused ring system, as for example in the formula:

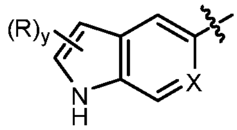

,

then R may replace any hydrogen attached to any of the ring atoms of either of the fused rings unless specified otherwise. Replaceable hydrogens include depicted hydrogens (e.g., the hydrogen attached to the nitrogen in the formula above), implied hydrogens (e.g., a hydrogen of the formula above that is not shown but understood to be present), expressly defined hydrogens, and optional hydrogens whose presence depends on the identity of a ring atom (e.g., a hydrogen attached to group X, when X equals -CH-), so long as a stable structure is formed. In the example depicted, R may reside on either the 5-membered or the 6-membered ring of the fused ring system. In the formula above, the subscript letter "y" immediately following the group "R" enclosed in parentheses, represents a numeric variable. Unless specified otherwise, this variable can be 0, 1, 2, or any integer greater than 2, only limited by the maximum number of replaceable hydrogen atoms of the ring or ring system.

For the chemical groups and compound classes, the number of carbon atoms in the group or class is as indicated as follows: "Cn" defines the exact number (n) of carbon atoms in the group/class. "C≤n" defines the maximum number (n) of carbon atoms that can be in the group/class, with the minimum number as small as possible for the group/class in question, e.g., it is understood that the minimum number of carbon atoms in the group "alkenyl$_{(C\leq 8)}$" or the class "alkene$_{(C\leq 8)}$" is two. Compare with "alkoxy$_{(C\leq 10)}$", which designates alkoxy groups having from 1 to 10 carbon atoms. "Cn-n'" defines both the minimum (n) and maximum number (n') of carbon atoms in the group. Thus, "alkyl$_{(C2-10)}$" designates those alkyl groups having from 2 to 10 carbon atoms. These carbon number indicators may precede or follow the chemical groups or class it modifies and it may or may not be enclosed in parenthesis, without signifying any change in meaning. Thus, the terms "C5 olefin", "C5-olefin", "olefin$_{(C5)}$", and "olefines" are all synonymous. When any of the chemical groups or compound classes defined herein is modified by the term "substituted", any carbon atom(s) in a moiety replacing a hydrogen atom is not counted. Thus methoxyhexyl is an example of a substituted alkyl$_{(C1-6)}$.

The term "saturated" when used to modify a compound or chemical group means the compound or chemical group has no carbon-carbon double and no carbon-carbon triple bonds, except as noted below. When the term is used to modify an atom, it means that the atom is not part of any double or triple bond. In the case of substituted versions of saturated groups, one or more carbon oxygen double bond or a carbon nitrogen double bond may be present. And when such a bond is present, then carbon-carbon double bonds that may occur as part of keto-enol tautomerism or imine/enamine tautomerism are not precluded. When the term "saturated" is used to modify a solution of a substance, it means that no more of that substance can dissolve in that solution.

The term "aliphatic" when used without the "substituted" modifier signifies that the compound or chemical group so modified is an acyclic or cyclic, but non-aromatic hydrocarbon compound or group. In aliphatic compounds/groups, the carbon atoms can be joined together in straight chains, branched chains, or non-aromatic rings (alicyclic). Aliphatic compounds/groups can be saturated, that is joined by single carbon-carbon bonds (alkanes/alkyl), or unsaturated, with one or more carbon-carbon double bonds (alkenes/alkenyl) or with one or more carbon-carbon triple bonds (alkynes/alkynyl).

The term "aromatic" when used to modify a compound or a chemical group refers to a planar unsaturated ring of atoms with 4n +2 electrons in a fully conjugated cyclic $\pi$ system.

The term "alkyl" when used without the "substituted" modifier refers to a monovalent saturated aliphatic group with a carbon atom as the point of attachment, a linear or branched acyclic structure, and no atoms other than carbon and hydrogen. The groups $-CH_3$ (Me), $-CH_2CH_3$ (Et), $-CH_2CH_2CH_3$ (*n*-Pr or propyl), $-CH(CH_3)_2$ (*i*-Pr, $^{i}$Pr or isopropyl), $-CH_2CH_2CH_2CH_3$ (*n*-Bu), $-CH(CH_3)CH_2CH_3$ (*sec*-butyl), $-CH_2CH(CH_3)_2$ (isobutyl), $-C(CH_3)_3$ (*tert*-butyl, *t*-butyl, *t*-Bu or $^{t}$Bu), and $-CH_2C(CH_3)_3$ (*neo*-pentyl) are non-limiting examples of alkyl groups. The term "alkanediyl" when used without the "substituted" modifier refers to a divalent saturated aliphatic group, with one or two saturated carbon atom(s) as the point(s) of attachment, a linear or branched acyclic structure, no carbon-carbon double or triple bonds, and no atoms

other than carbon and hydrogen. The groups $-CH_2-$ (methylene), $-CH_2CH_2-$, $-CH_2C(CH_3)_2CH_2-$, and $-CH_2CH_2CH_2-$ are non-limiting examples of alkanediyl groups. The term "alkylidene" when used without the "substituted" modifier refers to the divalent group =CRR' in which R and R' are independently hydrogen or alkyl. Non-limiting examples of alkylidene groups include: $=CH_2$, $=CH(CH_2CH_3)$, and $=C(CH_3)_2$. An "alkane" refers to the class of compounds having the formula H-R, wherein R is alkyl as this term is defined above. When any of these terms is used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$. The following groups are non-limiting examples of substituted alkyl groups: $-CH_2OH$, $-CH_2Cl$, $-CF_3$, $-CH_2CN$, $-CH_2C(O)OH$, $-CH_2C(O)OCH_3$, $-CH_2C(O)NH_2$, $-CH_2C(O)CH_3$, $-CH_2OCH_3$, $-CH_2OC(O)CH_3$, $-CH_2NH_2$, $-CH_2N(CH_3)_2$, and $-CH_2CH_2Cl$. The term "haloalkyl" is a subset of substituted alkyl, in which the hydrogen atom replacement is limited to halo (*i.e.* -F, -Cl, -Br, or -I) such that no other atoms aside from carbon, hydrogen and halogen are present. The group, $-CH_2Cl$ is a non-limiting example of a haloalkyl. The term "fluoroalkyl" is a subset of substituted alkyl, in which the hydrogen atom replacement is limited to fluoro such that no other atoms aside from carbon, hydrogen and fluorine are present. The groups $-CH_2F$, $-CF_3$, and $-CH_2CF_3$ are non-limiting examples of fluoroalkyl groups.

The term "cycloalkyl" when used without the "substituted" modifier refers to a monovalent saturated aliphatic group with a carbon atom as the point of attachment, said carbon atom forming part of one or more non-aromatic ring structures, no carbon-carbon double or triple bonds, and no atoms other than carbon and hydrogen. Non-limiting examples include: $-CH(CH_2)_2$ (cyclopropyl), cyclobutyl, cyclopentyl, or cyclohexyl (Cy). The term "cycloalkanediyl" when used without the "substituted" modifier refers to a divalent saturated aliphatic group with two carbon atoms as points of attachment, no carbon-carbon double or triple bonds, and no atoms other than carbon and hydrogen. The group

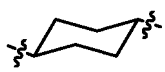

is a non-limiting example of cycloalkanediyl group. A "cycloalkane" refers to the class of compounds having the formula H-R, wherein R is cycloalkyl as this term is defined above. When any of these terms is used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$.

The term "alkenyl" when used without the "substituted" modifier refers to an monovalent unsaturated aliphatic group with a carbon atom as the point of attachment, a linear or branched, acyclic structure, at least one nonaromatic carbon-carbon double bond, no carbon-carbon triple bonds, and no atoms other than carbon and hydrogen. Non-limiting examples include: $-CH=CH_2$ (vinyl), $-CH=CHCH_3$, $-CH=CHCH_2CH_3$, $-CH_2CH=CH_2$ (allyl), $-CH_2CH=CHCH_3$, and $-CH=CHCH=CH_2$. The term "alkenediyl" when used without the "substituted" modifier refers to a divalent unsaturated aliphatic group, with two carbon atoms as points of attachment, a linear or branched, a linear or branched acyclic structure, at least one nonaromatic carbon-carbon double bond, no carbon-carbon triple bonds, and no atoms other than carbon and hydrogen. The groups -CH=CH-, $-CH=C(CH_3)CH_2-$, $-CH=CHCH_2-$, and $-CH_2CH=CHCH_2-$ are non-limiting examples of alkenediyl groups. It is noted that while the alkenediyl group is aliphatic, once connected at both ends, this group is not precluded from forming part of an aromatic structure. The terms "alkene" and "olefin" are synonymous and refer to the class of compounds having the formula H-R, wherein R is alkenyl as this term is defined above. Similarly the terms "terminal alkene" and "$\alpha$-olefin" are synonymous and refer to an alkene having just one carbon-carbon double bond, wherein that bond is part of a vinyl group at an end of the molecule. When any of these terms are used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$. The groups -CH=CHF, -CH=CHCl and -CH=CHBr are non-limiting examples of substituted alkenyl groups.

The term "alkynyl" when used without the "substituted" modifier refers to a monovalent unsaturated aliphatic group with a carbon atom as the point of attachment, a linear or branched acyclic structure, at least one carbon-carbon triple bond, and no atoms other than carbon and hydrogen. As used herein, the term alkynyl does not preclude the presence of one or more non-aromatic carbon-carbon double bonds. The groups $-C{\equiv}CH$, $-C{\equiv}CCH_3$, and $-CH_2C{\equiv}CCH_3$ are non-limiting examples of alkynyl groups. An "alkyne" refers to the class of compounds having the formula H-R, wherein R is alkynyl. When any of these terms are used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$.

The term "aryl" when used without the "substituted" modifier refers to a monovalent unsaturated aromatic group

with an aromatic carbon atom as the point of attachment, said carbon atom forming part of a one or more six-membered aromatic ring structure, wherein the ring atoms are all carbon, and wherein the group consists of no atoms other than carbon and hydrogen. If more than one ring is present, the rings may be fused or unfused. As used herein, the term does not preclude the presence of one or more alkyl or aralkyl groups (carbon number limitation permitting) attached to the first aromatic ring or any additional aromatic ring present. Non-limiting examples of aryl groups include phenyl (Ph), methylphenyl, (dimethyl)phenyl, $-C_6H_4CH_2CH_3$ (ethylphenyl), naphthyl, and a monovalent group derived from biphenyl. The term "arenediyl" when used without the "substituted" modifier refers to a divalent aromatic group with two aromatic carbon atoms as points of attachment, said carbon atoms forming part of one or more six-membered aromatic ring structure(s) wherein the ring atoms are all carbon, and wherein the monovalent group consists of no atoms other than carbon and hydrogen. As used herein, the term does not preclude the presence of one or more alkyl, aryl or aralkyl groups (carbon number limitation permitting) attached to the first aromatic ring or any additional aromatic ring present. If more than one ring is present, the rings may be fused or unfused. Unfused rings may be connected via one or more of the following: a covalent bond, alkanediyl, or alkenediyl groups (carbon number limitation permitting). Non-limiting examples of arenediyl groups include:

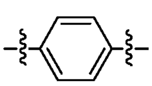, 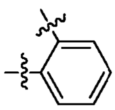, 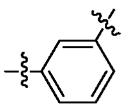, 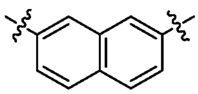 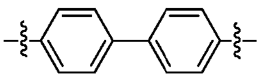,

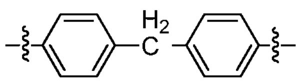, and 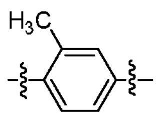.

An "arene" refers to the class of compounds having the formula H-R, wherein R is aryl as that term is defined above. Benzene and toluene are non-limiting examples of arenes. When any of these terms are used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$.

The term "aralkyl" when used without the "substituted" modifier refers to the monovalent group -alkanediyl-aryl, in which the terms alkanediyl and aryl are each used in a manner consistent with the definitions provided above. Non-limiting examples are: phenylmethyl (benzyl, Bn) and 2-phenyl-ethyl. When the term aralkyl is used with the "substituted" modifier one or more hydrogen atom from the alkanediyl and/or the aryl group has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$. Non-limiting examples of substituted aralkyls are: (3-chlorophenyl)-methyl, and 2-chloro-2-phenyl-eth-1-yl.

The term "heteroaryl" when used without the "substituted" modifier refers to a monovalent aromatic group with an aromatic carbon atom or nitrogen atom as the point of attachment, said carbon atom or nitrogen atom forming part of one or more aromatic ring structures wherein at least one of the ring atoms is nitrogen, oxygen or sulfur, and wherein the heteroaryl group consists of no atoms other than carbon, hydrogen, aromatic nitrogen, aromatic oxygen and aromatic sulfur. If more than one ring is present, the rings may be fused or unfused. As used herein, the term does not preclude the presence of one or more alkyl, aryl, and/or aralkyl groups (carbon number limitation permitting) attached to the aromatic ring or aromatic ring system. Non-limiting examples of heteroaryl groups include furanyl, imidazolyl, indolyl, indazolyl (Im), isoxazolyl, methylpyridinyl, oxazolyl, phenylpyridinyl, pyridinyl (pyridyl), pyrrolyl, pyrimidinyl, pyrazinyl, quinolyl, quinazolyl, quinoxalinyl, triazinyl, tetrazolyl, thiazolyl, thienyl, and triazolyl. The term "heteroarenediyl" when used without the "substituted" modifier refers to an divalent aromatic group, with two aromatic carbon atoms, two aromatic nitrogen atoms, or one aromatic carbon atom and one aromatic nitrogen atom as the two points of attachment, said atoms forming part of one or more aromatic ring structure(s) wherein at least one of the ring atoms is nitrogen, oxygen or sulfur, and wherein the divalent group consists of no atoms other than carbon, hydrogen, aromatic nitrogen, aromatic oxygen and aromatic sulfur. If more than one ring is present, the rings may be fused or unfused. Unfused rings may be connected via one or more of the following: a covalent bond, alkanediyl, or alkenediyl groups (carbon number limitation permitting). As used herein, the term does not preclude the presence of one or more alkyl, aryl, and/or aralkyl groups (carbon number limitation permitting) attached to the aromatic ring or aromatic ring system. Non-limiting examples of heteroarenediyl groups include:

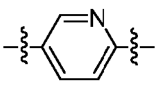, 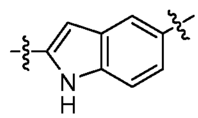 and 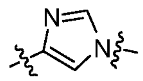.

The term "*N*-heteroaryl" refers to a heteroaryl group with a nitrogen atom as the point of attachment. A "heteroarene" refers to the class of compounds having the formula H-R, wherein R is heteroaryl. Pyridine and quinoline are non-limiting examples of heteroarenes. When these terms are used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$.

The term "heterocycloalkyl" when used without the "substituted" modifier refers to a monovalent non-aromatic group with a carbon atom or nitrogen atom as the point of attachment, said carbon atom or nitrogen atom forming part of one or more non-aromatic ring structures wherein at least one of the ring atoms is nitrogen, oxygen or sulfur, and wherein the heterocycloalkyl group consists of no atoms other than carbon, hydrogen, nitrogen, oxygen and sulfur. If more than one ring is present, the rings may be fused or unfused. As used herein, the term does not preclude the presence of one or more alkyl groups (carbon number limitation permitting) attached to the ring or ring system. Also, the term does not preclude the presence of one or more double bonds in the ring or ring system, provided that the resulting group remains non-aromatic. Non-limiting examples of heterocycloalkyl groups include aziridinyl, azetidinyl, pyrrolidinyl, piperidinyl, piperazinyl, morpholinyl, thiomorpholinyl, tetrahydrofuranyl, tetrahydrothiofuranyl, tetrahydropyranyl, pyranyl, oxiranyl, and oxetanyl. The term "heterocycloalkanediyl" when used without the "substituted" modifier refers to an divalent cyclic group, with two carbon atoms, two nitrogen atoms, or one carbon atom and one nitrogen atom as the two points of attachment, said atoms forming part of one or more ring structure(s) wherein at least one of the ring atoms is nitrogen, oxygen or sulfur, and wherein the divalent group consists of no atoms other than carbon, hydrogen, nitrogen, oxygen and sulfur. If more than one ring is present, the rings may be fused or unfused. Unfused rings may be connected via one or more of the following: a covalent bond, alkanediyl, or alkenediyl groups (carbon number limitation permitting). As used herein, the term does not preclude the presence of one or more alkyl groups (carbon number limitation permitting) attached to the ring or ring system. Also, the term does not preclude the presence of one or more double bonds in the ring or ring system, provided that the resulting group remains non-aromatic. Non-limiting examples of heterocycloalkanediyl groups include:

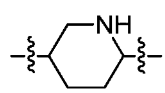, 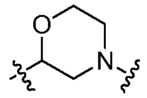, and 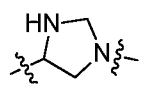.

The term "*N*-heterocycloalkyl" refers to a heterocycloalkyl group with a nitrogen atom as the point of attachment. *N*-pyrrolidinyl is an example of such a group. When these terms are used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$.

The term "acyl" when used without the "substituted" modifier refers to the group -C(O)R, in which R is a hydrogen, alkyl, cycloalkyl, alkenyl, aryl, aralkyl or heteroaryl, as those terms are defined above. The groups, -CHO, $-C(O)CH_3$ (acetyl, Ac), $-C(O)CH_2CH_3$, $-C(O)CH_2CH_2CH_3$, $-C(O)CH(CH_3)_2$, $-C(O)CH(CH_2)_2$, $-C(O)C_6H_5$, $-C(O)C_6H_4CH_3$, $-C(O)CH_2C_6H_5$, -C(O)(imidazolyl) are non-limiting examples of acyl groups. A "thioacyl" is defined in an analogous manner, except that the oxygen atom of the group -C(O)R has been replaced with a sulfur atom, -C(S)R. The term "aldehyde" corresponds to an alkane, as defined above, wherein at least one of the hydrogen atoms has been replaced with a -CHO group. When any of these terms are used with the "substituted" modifier one or more hydrogen atom (including a hydrogen atom directly attached to the carbon atom of the carbonyl or thiocarbonyl group, if any) has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$. The groups, $-C(O)CH_2CF_3$, $-CO_2H$ (carboxyl), $-CO_2CH_3$ (methylcarboxyl), $-CO_2CH_2CH_3$, $-C(O)NH_2$ (carbamoyl), and $-CON(CH_3)_2$, are non-limiting examples of substituted acyl groups.

The term "alkoxy" when used without the "substituted" modifier refers to the group -OR, in which R is an alkyl, as that term is defined above. Non-limiting examples include: $-OCH_3$ (methoxy), $-OCH_2CH_3$ (ethoxy), $-OCH_2CH_2CH_3$, $-OCH(CH_3)_2$ (isopropoxy), $-OC(CH_3)_3$ (*tert*-butoxy), $-OCH(CH_2)_2$, -O-cyclopentyl, and -O-cyclohexyl. The terms "cycloalkoxy", "alkenyloxy", "alkynyloxy", "aryloxy", "aralkoxy", "heteroaryloxy", "heterocycloalkoxy", and "acyloxy", when used without the "substituted" modifier, refers to groups, defined as -OR, in which R is cycloalkyl, alkenyl, alkynyl, aryl,

aralkyl, heteroaryl, heterocycloalkyl, and acyl, respectively. The term "alkoxydiyl" refers to the divalent group -O-alkanediyl-, -O-alkanediyl-O-, or -alkanediyl-O-alkanediyl-. The term "alkylthio" and "acylthio" when used without the "substituted" modifier refers to the group -SR, in which R is an alkyl and acyl, respectively. The term "alcohol" corresponds to an alkane, as defined above, wherein at least one of the hydrogen atoms has been replaced with a hydroxy group. The term "ether" corresponds to an alkane, as defined above, wherein at least one of the hydrogen atoms has been replaced with an alkoxy group. When any of these terms is used with the "substituted" modifier one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$.

The term "alkylamino" when used without the "substituted" modifier refers to the group -NHR, in which R is an alkyl, as that term is defined above. Non-limiting examples include: $-NHCH_3$ and $-NHCH_2CH_3$. The term "dialkylamino" when used without the "substituted" modifier refers to the group -NRR', in which R and R' can be the same or different alkyl groups, or R and R' can be taken together to represent an alkanediyl. Non-limiting examples of dialkylamino groups include: $-N(CH_3)_2$ and $-N(CH_3)(CH_2CH_3)$. The terms "cycloalkylamino", "alkenylamino", "alkynylamino", "arylamino", "aralkylamino", "heteroarylamino", "heterocycloalkylamino", "alkoxyamino", and "alkylsulfonylamino" when used without the "substituted" modifier, refers to groups, defined as -NHR, in which R is cycloalkyl, alkenyl, alkynyl, aryl, aralkyl, heteroaryl, heterocycloalkyl, alkoxy, and alkylsulfonyl, respectively. A non-limiting example of an arylamino group is $-NHC_6H_5$. The term "amido" (acylamino), when used without the "substituted" modifier, refers to the group -NHR, in which R is acyl, as that term is defined above. A non-limiting example of an amido group is $-NHC(O)CH_3$. The term "alkylimino" when used without the "substituted" modifier refers to the divalent group =NR, in which R is an alkyl, as that term is defined above. The term "alkylaminodiyl" refers to the divalent group -NH-alkanediyl-, -NH-alkanediyl-NH-, or -alkanediyl-NH-alkanediyl-. When any of these terms is used with the "substituted" modifier one or more hydrogen atom attached to a carbon atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$. The groups $-NHC(O)OCH_3$ and $-NHC(O)NHCH_3$ are non-limiting examples of substituted amido groups.

The use of the word "a" or "an," when used in conjunction with the term "comprising" in the claims and/or the specification may mean "one," but it is also consistent with the meaning of "one or more," "at least one," and "one or more than one."

Throughout this application, the term "about" is used to indicate that a value includes the inherent variation of error for the device, the method being employed to determine the value, or the variation that exists among the study subjects.

The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and also covers other unlisted steps.

The term "effective," as that term is used in the specification and/or claims, means adequate to accomplish a desired, expected, or intended result. "Effective amount," "Therapeutically effective amount" or "pharmaceutically effective amount" when used in the context of treating a patient or subject with a compound means that amount of the compound which, when administered to a subject or patient for treating a disease, is sufficient to effect such treatment for the disease.

As used herein, the term "$IC_{50}$" refers to an inhibitory dose which is 50% of the maximum response obtained. This quantitative measure indicates how much of a particular drug or other substance (inhibitor) is needed to inhibit a given biological, biochemical or chemical process (or component of a process, i.e. an enzyme, cell, cell receptor or microorganism) by half.

An "isomer" of a first compound is a separate compound in which each molecule contains the same constituent atoms as the first compound, but where the configuration of those atoms in three dimensions differs.

As used herein, the term "patient" or "subj ect" refers to a living mammalian organism, such as a human, monkey, cow, sheep, goat, dog, cat, mouse, rat, guinea pig, or transgenic species thereof. In certain embodiments, the patient or subject is a primate. Non-limiting examples of human subjects are adults, juveniles, infants and fetuses.

As generally used herein "pharmaceutically acceptable" refers to those compounds, materials, compositions, and/or dosage forms which are, within the scope of sound medical judgment, suitable for use in contact with the tissues, organs, and/or bodily fluids of human beings and animals without excessive toxicity, irritation, allergic response, or other problems or complications commensurate with a reasonable benefit/risk ratio.

"Pharmaceutically acceptable salts" means salts of compounds of the present disclosure which are pharmaceutically acceptable, as defined above, and which possess the desired pharmacological activity. Such salts include acid addition salts formed with inorganic acids such as hydrochloric acid, hydrobromic acid, sulfuric acid, nitric acid, phosphoric acid, and the like; or with organic acids such as 1,2-ethanedisulfonic acid, 2-hydroxyethanesulfonic acid, 2-naphthalenesulfonic acid, 3-phenylpropionic acid, 4,4'-methylenebis(3-hydroxy-2-ene-1-carboxylic acid), 4-methylbicyclo[2.2.2]oct-2-ene-1-carboxylic acid, acetic acid, aliphatic mono- and dicarboxylic acids, aliphatic sulfuric acids, aromatic sulfuric acids, benzenesulfonic acid, benzoic acid, camphorsulfonic acid, carbonic acid, cinnamic acid, citric acid, cyclopentanepropionic acid, ethanesulfonic acid, fumaric acid, glucoheptonic acid, gluconic acid, glutamic acid, glycolic acid, heptanoic acid, hexanoic acid, hydroxynaphthoic acid, lactic acid, laurylsulfuric acid, maleic acid, malic acid, malonic acid, mandelic acid, methanesulfonic acid, muconic acid, *o*-(4-hydroxybenzoyl)benzoic acid, oxalic acid, *p*-chlorobenzenesulfonic acid, phenyl-substituted alkanoic acids, propionic acid, *p*-toluenesulfonic acid, pyruvic acid, salicylic acid, stearic acid, succinic acid, tartaric acid, tertiarybutylacetic acid, trimethylacetic acid, and the like. Pharmaceutically acceptable salts also include base addition salts that may be formed when acidic protons present are capable of reacting with inorganic or organic bases. Acceptable inorganic bases include sodium hydroxide, sodium carbonate, potassium hydroxide, aluminum hydroxide and calcium hydroxide. Acceptable organic bases include ethanolamine, diethanolamine, triethanolamine, tromethamine, *N*-methylglucamine and the like. It should be recognized that the particular anion or cation forming a part of any salt of this disclosure is not critical, so long as the salt, as a whole, is pharmacologically acceptable. Additional examples of pharmaceutically acceptable salts and their methods of preparation and use are presented in Handbook of Pharmaceutical Salts: Properties, and Use (P. H. Stahl & C. G. Wermuth eds., Verlag Helvetica Chimica Acta, 2002).

The term "pharmaceutically acceptable carrier," as used herein means a pharmaceutically-acceptable material, composition or vehicle, such as a liquid or solid filler, diluent, excipient, solvent or encapsulating material, involved in carrying or transporting a chemical agent.

"Prevention" or "preventing" includes: (1) inhibiting the onset of a disease in a subject or patient which may be at risk and/or predisposed to the disease but does not yet experience or display any or all of the pathology or symptomatology of the disease, and/or (2) slowing the onset of the pathology or symptomatology of a disease in a subject or patient which may be at risk and/or predisposed to the disease but does not yet experience or display any or all of the pathology or symptomatology of the disease.

A "stereoisomer" or "optical isomer" is an isomer of a given compound in which the same atoms are bonded to the same other atoms, but where the configuration of those atoms in three dimensions differs. "Enantiomers" are stereoisomers of a given compound that are mirror images of each other, like left and right hands. "Diastereomers" are stereoisomers of a given compound that are not enantiomers. Chiral molecules contain a chiral center, also referred to as a stereocenter or stereogenic center, which is any point, though not necessarily an atom, in a molecule bearing groups such that an interchanging of any two groups leads to a stereoisomer. In organic compounds, the chiral center is typically a carbon, phosphorus or sulfur atom, though it is also possible for other atoms to be stereocenters in organic and inorganic compounds. A molecule can have multiple stereocenters, giving it many stereoisomers. In compounds whose stereoisomerism is due to tetrahedral stereogenic centers (*e.g.*, tetrahedral carbon), the total number of hypothetically possible stereoisomers will not exceed $2^n$, where n is the number of tetrahedral stereocenters. Molecules with symmetry frequently have fewer than the maximum possible number of stereoisomers. A 50:50 mixture of enantiomers is referred to as a racemic mixture. Alternatively, a mixture of enantiomers can be enantiomerically enriched so that one enantiomer is present in an amount greater than 50%. Typically, enantiomers and/or diastereomers can be resolved or separated using techniques known in the art. It is contemplated that that for any stereocenter or axis of chirality for which stereochemistry has not been defined, that stereocenter or axis of chirality can be present in its *R* form, *S* form, or as a mixture of the *R* and *S* forms, including racemic and non-racemic mixtures. As used herein, the phrase "substantially free from other stereoisomers" means that the composition contains $\leq$ 15%, more preferably $\leq$ 10%, even more preferably $\leq$ 5%, or most preferably $\leq$ 1% of another stereoisomer(s).

"Treatment" or "treating" includes (1) inhibiting a disease in a subject or patient experiencing or displaying the pathology or symptomatology of the disease (*e.g.*, arresting further development of the pathology and/or symptomatology), (2) ameliorating a disease in a subject or patient that is experiencing or displaying the pathology or symptomatology of the disease (*e.g.*, reversing the pathology and/or symptomatology), and/or (3) effecting any measurable decrease in a disease in a subject or patient that is experiencing or displaying the pathology or symptomatology of the disease.

The above definitions supersede any conflicting definition in any reference that is described herein. The fact that certain terms are defined, however, should not be considered as indicative that any term that is undefined is indefinite. Rather, all terms used are believed to describe the disclosure in terms such that one of ordinary skill can appreciate the scope and practice the present disclosure.

### V. Examples

The following examples are included to demonstrate preferred embodiments of the disclosure. It should be appreciated by those of skill in the art that the techniques disclosed in the examples which follow represent techniques discovered by the inventor to function well in the practice of the disclosure, and thus can be considered to constitute preferred modes for its practice. However, those of skill in the art should, in light of the present disclosure, appreciate

that many changes can be made in the specific embodiments which are disclosed and still obtain a like or similar result without departing from the scope of the claims.

**Example 1 - Methods and Materials**

**A. Cell Culture**

Calu-6 and Calu-3 cells were obtained from the American Type Culture Collection and cultured in RMP1 1640 (Corning) medium with L-Glutamine and 25 mM HEPES supplemented with 5% FBS (Gemini Bio-Products). IB3-1 cells were kindly provided by Harvey Pollard and cultured in serum free LHC-8 (Invitrogen) medium. HEK 293 cells were obtained from the American Type Culture Collection and cultured in DMEM (Invitrogen) supplemented with 10% FBS (Gemini Bio-Products).

**B. Antibodies and Reagents**

p53 DO-1(#sc-126) and GFP C2 (#sc-390394) antibodies were purchased from Santa Cruz Biotechnology, Inc. Actin antibody (MAB1501) was purchased from EMD Millipore. CFTR 596 antibody was purchased from the UNC Antibody Distribution Program. Unacylated *E. coli* $tRNA^{Phe}$-$F^{8}$ (P-05) was prepared by chemically modifying chromatographically purified $tRNA^{Phe}$ with a fluorescein dye reagent as described in Johnson *et al.,* 1982. For other tRNAs, DNA fragments encoding a specific tRNA sequence behind a T7 RNA polymerase promoter sequence were synthesized chemically. The T7 promoter-tRNA DNA sequences were amplified by PCR and then transcribed *in vitro* using T7 RNA polymerase using standard techniques such as those described by Green and Sambrook, 2012; Rio *et al.,* 2011; Flanagan *et al.,* 2003;and Janiak *et al.,* 1992. The resulting tRNA transcripts were extracted with phenol, precipitated in high salt and ethanol, and purified by HPLC using a MonoQ ion exchange column. The purified tRNAs were precipitated, resuspended, and dialyzed into water. RNAiMax and Lipofectamine 2000 were purchased from Invitrogen and used following the supplier's recommended protocols. G418 (sc-29065) was purchased from Santa Cruz. PTC124 (S6003) and VX-770 (S1144) were purchased from Selleck Chemicals. 3-Isobutyl-1-methylxanthine (IBMX) (I5879) and Forskolin (F3917) were purchased from Sigma-Aldrich. CFTR-Inh172 was obtained from CFFT (Cystic Fibrosis Foundation Therapeutics, Inc). The compositions were formulated according to Tables 3-6 shown below.

**Table 3: Formulations using Polyester Polymers**

| Formulation code | PE in formulation | Molecular weight | Polymer:RNA wt ratio |
|---|---|---|---|
| **PE100** | **A13-0.2C6-8K** | 8000 | 30 |
| **PE200** | **A13-0.2C10-8K** | 8000 | 30 |
| **PE300** | **A13-0.2C6-4K** | 4000 | 30 |
| **PE400** | **A13-0.33C6-4K** | 4000 | 30 |
| **PE500** | **A13-0.5C10-4K** | 4000 | 30 |
| **PE600** | **A13-0.2C12-4K** | 4000 | 30 |
| **PE700** | **A13-0.5C6-4K** | 4000 | 30 |
| **PE800** | **A13-0.33C10-4K** | 4000 | 30 |

Table 4: Formulations using Dendrimers

| | | | | Mole ratios in lipid mix | | | | | |
|---|---|---|---|---|---|---|---|---|---|
| **Formulation code** | **Dendrimer** | **MW** | **Class** | **Synth lipid** | **Cholesterol** | **DSPC** | **DOPE** | **PEGlipid** | **Dendrimer:nucleic acid mol ratio** |
| **D100** | 5A2SC8 | 1841.72 | Dendrimer | 50 | 38 | 10 | 0 | 2 | 237 |
| **D101** | 5A2SC8 | 1841.72 | Dendrimer | 50 | 38 | 10 | 0 | 2 | 475 |
| **D102** | 5A2SC8 | 1841.72 | Dendrimer | 50 | 38 | 10 | 0 | 2 | 119 |
| **D103** | 5A2SC8 | 1841.72 | Dendrimer | 50 | 38 | 0 | 10 | 2 | 200 |
| **D104** | 5A2SC8 | 1841.72 | Dendrimer | 50 | 38 | 0 | 10 | 2 | 400 |
| **D105** | 5A2SC8 | 1841.72 | Dendrimer | 50 | 38 | 0 | 10 | 2 | 100 |
| **D200** | 3A3SC12 | 1319.05 | Dendrimer | 50 | 38.5 | 10 | 0 | 2 | 200 |
| **D201** | 3A3SC12 | 1319.05 | Dendrimer | 50 | 38.5 | 0 | 10 | 2 | 400 |
| **D202** | 3A3SC12 | 1319.05 | Dendrimer | 50 | 38.5 | 10 | 0 | 2 | 400 |
| **D203** | 3A3SC12 | 1319.05 | Dendrimer | 50 | 38.5 | 10 | 0 | 2 | 100 |
| **D204** | 3A3SC12 | 1319.05 | Dendrimer | 50 | 38 | 0 | 0 | 2 | 200 |
| **D205** | 3A3SC12 | 1319.05 | Dendrimer | 50 | 38 | 0 | 0 | 2 | 100 |
| **D206** | 3A3SC12 | 1319.05 | Dendrimer | 50 | 38.5 | 10 | 0 | 2 | 50 |
| **D207** | 3A3SC12 | 1319.05 | Dendrimer | 50 | 38.5 | 10 | 0 | 2 | 25 |
| **D208** | 3A3SC12 | 1319.05 | Dendrimer | 50 | 38.5 | 0 | 0 | 2 | 50 |
| **D209** | 3A3SC12 | 1319.05 | Dendrimer | 50 | 38.5 | 0 | 0 | 2 | 25 |
| **D210** | 3A3SC12 | 1319.05 | Dendrimer | 50 | 38.5 | 10 | 10 | 2 | 100 |
| **D211** | 3A3SC12 | 1319.05 | Dendrimer | 50 | 38 | 0 | 10 | 2 | 400 |
| **D300** | 2A1SC14 | 957.509 | Dendrimer | 50 | 38.5 | 10 | 0 | 2 | 200 |
| **D400** | 2A2SC14 | 986.551 | Dendrimer | 50 | 38.5 | 10 | 0 | 2 | 200 |
| **D500** | 2A5SC14 | 943.482 | Dendrimer | 50 | 38.5 | 10 | 0 | 2 | 200 |
| **D600** | 2A6SC14 | 957.509 | Dendrimer | 50 | 38.5 | 10 | 0 | 2 | 200 |
| **D700** | 3A3SC6 | 1066.564 | Dendrimer | 50 | 38.5 | 10 | 0 | 2 | 200 |
| **D800** | 3A5SC12 | 1348.09 | Dendrimer | 50 | 38.5 | 10 | 0 | 2 | 200 |

(continued)

| | | | | Mole ratios in lipid mix | | | | | |
|---|---|---|---|---|---|---|---|---|---|
| **Formulation code** | **Dendrimer** | **MW** | **Class** | **Synth lipid** | **Cholesterol** | **DSPC** | **DOPE** | **PEGlipid** | **Dendrimer:nucleic acid mol ratio** |
| **D801** | 3A5SC12 | 1348.09 | Dendrimer | 50 | 38 | 0 | 0 | 2 | 200 |
| **D900** | 4A1SC12 | 1746.69 | Dendrimer | 50 | 38 | 10 | 0 | 2 | 200 |
| **D901** | 4A1SC12 | 1746.69 | Dendrimer | 50 | 38 | 0 | 0 | 2 | 200 |
| **D902** | 4A1SC12 | 1746.69 | Dendrimer | 50 | 38 | 10 | 0 | 2 | 400 |
| **D903** | 4A1SC12 | 1746.69 | Dendrimer | 50 | 38 | 10 | 0 | 2 | 100 |
| **D904** | 4A1SC12 | 1746.69 | Dendrimer | 50 | 38 | 0 | 10 | 2 | 200 |
| **D905** | 4A1SC12 | 1746.69 | Dendrimer | 50 | 38 | 0 | 10 | 2 | 400 |
| **D906** | 4A1SC12 | 1746.69 | Dendrimer | 50 | 38 | 0 | 10 | 2 | 100 |
| **D1000** | 4A3SC12 | 1691.61 | Dendrimer | 50 | 38 | 10 | 0 | 2 | 200 |
| **D1001** | 4A3SC12 | 1691.61 | Dendrimer | 50 | 38 | 0 | 0 | 2 | 200 |
| **D1002** | 4A3SC12 | 1691.61 | Dendrimer | 50 | 38 | 10 | 0 | 2 | 400 |
| **D1003** | 4A3SC12 | 1691.61 | Dendrimer | 50 | 38 | 10 | 0 | 2 | 100 |
| **D1004** | 4A3SC12 | 1691.61 | Dendrimer | 50 | 38 | 0 | 10 | 2 | 200 |
| **D1005** | 4A3SC12 | 1691.61 | Dendrimer | 50 | 38 | 0 | 10 | 2 | 400 |
| **D1006** | 4A3SC12 | 1691.61 | Dendrimer | 50 | 38 | 0 | 10 | 2 | 100 |
| **D1100** | 3A3SC8 | 1150.73 | Dendrimer | 50 | 38 | 10 | 0 | 2 | 200 |
| **D1101** | 3A3SC8 | 1150.73 | Dendrimer | 50 | 38 | 10 | 0 | 2 | 400 |
| **D1102** | 3A3SC8 | 1150.73 | Dendrimer | 50 | 38 | 10 | 0 | 2 | 100 |
| **D1103** | 3A3SC8 | 1150.73 | Dendrimer | 50 | 38 | 0 | 10 | 2 | 200 |
| **D1104** | 3A3SC8 | 1150.73 | Dendrimer | 50 | 38 | 0 | 10 | 2 | 400 |
| **D1105** | 3A3SC8 | 1150.73 | Dendrimer | 50 | 38 | 0 | 10 | 2 | 100 |
| **D1200** | 4A1SC8 | 1522.26 | Dendrimer | 50 | 38 | 10 | 0 | 2 | 200 |
| **D1201** | 4A1SC8 | 1522.26 | Dendrimer | 50 | 38 | 10 | 0 | 2 | 400 |
| **D1202** | 4A1SC8 | 1522.26 | Dendrimer | 50 | 38 | 10 | 0 | 2 | 100 |

(continued)

| | | | | Mole ratios in lipid mix | | | | | |
|---|---|---|---|---|---|---|---|---|---|
| **Formulation code** | **Dendrimer** | **MW** | **Class** | **Synth lipid** | **Cholesterol** | **DSPC** | **DOPE** | **PEGlipid** | **Dendrimer:nucleic acid mol ratio** |
| **D1203** | 4A1SC8 | 1522.26 | Dendrimer | 50 | 38 | 0 | 10 | 2 | 200 |
| **D1204** | 4A1SC8 | 1522.26 | Dendrimer | 50 | 38 | 0 | 10 | 2 | 400 |
| **D1205** | 4A1SC8 | 1522.26 | Dendrimer | 50 | 38 | 0 | 10 | 2 | 100 |
| **D1300** | 4A3SC8 | 1467.18 | Dendrimer | 50 | 38 | 10 | 0 | 2 | 200 |
| **D1301** | 4A3SC8 | 1467.18 | Dendrimer | 50 | 38 | 10 | 0 | 2 | 400 |
| **D1302** | 4A3SC8 | 1467.18 | Dendrimer | 50 | 38 | 10 | 0 | 2 | 100 |
| **D1303** | 4A3SC8 | 1467.18 | Dendrimer | 50 | 38 | 0 | 10 | 2 | 200 |
| **D1304** | 4A3SC8 | 1467.18 | Dendrimer | 50 | 38 | 0 | 10 | 2 | 400 |
| **D1305** | 4A3SC8 | 1467.18 | Dendrimer | 50 | 38 | 0 | 10 | 2 | 100 |
| **D1400** | 6A4SC12 | 2521.89 | Dendrimer | 50 | 38 | 10 | 0 | 2 | 200 |
| **D1401** | 6A4SC12 | 2521.89 | Dendrimer | 50 | 38 | 10 | 0 | 2 | 400 |
| **D1402** | 6A4SC12 | 2521.89 | Dendrimer | 50 | 38 | 10 | 0 | 2 | 100 |
| **D1403** | 6A4SC12 | 2521.89 | Dendrimer | 50 | 38 | 0 | 10 | 2 | 200 |
| **D1404** | 6A4SC12 | 2521.89 | Dendrimer | 50 | 38 | 0 | 10 | 2 | 400 |
| **D1405** | 6A4SC12 | 2521.89 | Dendrimer | 50 | 38 | 0 | 10 | 2 | 100 |
| **D1500** | 6A4SC8 | 2185.24 | Dendrimer | 50 | 38 | 10 | 0 | 2 | 200 |
| **D1501** | 6A4SC8 | 2185.24 | Dendrimer | 50 | 38 | 10 | 0 | 2 | 400 |
| **D1502** | 6A4SC8 | 2185.24 | Dendrimer | 50 | 38 | 10 | 0 | 2 | 100 |
| **D1503** | 6A4SC8 | 2185.24 | Dendrimer | 50 | 38 | 0 | 10 | 2 | 200 |
| **D1504** | 6A4SC8 | 2185.24 | Dendrimer | 50 | 38 | 0 | 10 | 2 | 400 |
| **D1505** | 6A4SC8 | 2185.24 | Dendrimer | 50 | 38 | 0 | 10 | 2 | 100 |
| **D1600** | 6A1SC8 | 2172.21 | Dendrimer | 50 | 38 | 10 | 0 | 2 | 201 |
| **D1601** | 6A1SC8 | 2172.21 | Dendrimer | 50 | 38 | 10 | 0 | 2 | 402 |
| **D1602** | 6A1SC8 | 2172.21 | Dendrimer | 50 | 38 | 10 | 0 | 2 | 101 |

(continued)

| | | | | Mole ratios in lipid mix | | | | | |
|---|---|---|---|---|---|---|---|---|---|
| **Formulation code** | **Dendrimer** | **MW** | **Class** | **Synth lipid** | **Cholesterol** | **DSPC** | **DOPE** | **PEGlipid** | **Dendrimer:nucleic acid mol ratio** |
| **D1603** | 6A1SC8 | 2172.21 | Dendrimer | 50 | 38 | 0 | 10 | 2 | 200 |
| **D1604** | 6A1SC8 | 2172.21 | Dendrimer | 50 | 38 | 0 | 10 | 2 | 400 |
| **D1605** | 6A1SC8 | 2172.21 | Dendrimer | 50 | 38 | 0 | 10 | 2 | 100 |

**Table 5: Formulations using Zwitterionic Amino Lipids (ZALs)**

| | | | Molar ratios in lipid mix | | | | | |
|---|---|---|---|---|---|---|---|---|
| **Formulation code** | **ZAL in formulation** | *Mol. Wt.* | *ZAL* | *Cholesterol* | *DSPC* | *DOPE* | *PEG Lipid* | **ZAL:nucleic acid wt ratio** |
| **Z100** | ZA3-Ep10 | 1191.92 | 50 | 38.5 | 0 | 0 | 0 | 20.00 |
| **Z101** | ZA3-Ep10 | 1191.92 | 50 | 38.5 | 0 | 0 | 2 | 20.00 |
| **Z103** | ZA3-Ep10 | 1191.92 | 50 | 38.5 | 0 | 0 | 0.5 | 20.00 |
| **Z102** | ZA3-Ep10 | 1191.92 | 50 | 38.5 | 0 | 0 | 1 | 20.00 |
| **Z103** | ZA3-Ep10 | 1191.92 | 50 | 38.5 | 0 | 0 | 0.5 | 20.00 |
| **Z104** | ZA3-Ep10 | 1191.92 | 40 | 38.5 | 10 | 0 | 2 | 15.60 |
| **Z105** | ZA3-Ep10 | 1191.92 | 40 | 38.5 | 0 | 10 | 2 | 15.69 |
| **Z106** | ZA3-Ep10 | 1191.92 | 30 | 38.5 | 20 | 0 | 2 | 12.35 |
| **Z107** | ZA3-Ep10 | 1191.92 | 30 | 38.5 | 0 | 20 | 2 | 12.50 |
| **Z108** | ZA3-Ep10 | 1191.92 | 50 | 38.5 | 10 | 0 | 2 | 20.00 |
| **Z109** | ZA3-Ep10 | 1191.92 | 50 | 38.5 | 0 | 10 | 2 | 20.00 |
| **Z110** | ZA3-Ep10 | 1191.92 | 50 | 38.5 | 0 | 0 | 5 | 20.00 |
| **Z111** | ZA3-Ep10 | 1191.92 | 50 | 38.5 | 0 | 0 | 10 | 20.00 |
| **Z112** | ZA3-Ep10 | 1191.92 | 50 | 38.5 | 0 | 0 | 2 | 10.00 |
| **Z113** | ZA3-Ep10 | 1191.92 | 50 | 77 | 0 | 4 | 2 | 10.00 |
| **Z114** | ZA3-Ep10 | 1191.92 | 50 | 38.5 | 0 | 0 | 2 | 7.50 |
| **Z115** | ZA3-Ep10 | 1191.92 | 50 | 38.5 | 0 | 0 | 2 | 5.00 |
| **Z116** | ZA3-Ep10 | 1191.92 | 50 | 38.5 | 0 | 0 | 1 | 10.00 |
| **Z117** | ZA3-Ep10 | 1191.92 | 50 | 38.5 | 0 | 0 | 0.5 | 10.00 |
| **Z118** | ZA3-Ep10 | 1191.92 | 50 | 38.5 | 0 | 0 | 1 | 7.50 |
| **Z119** | ZA3-Ep10 | 1191.92 | 50 | 38.5 | 0 | 0 | 1 | 5.00 |
| **Z120** | ZA3-Ep10 | 1191.92 | 50 | 38.5 | 0 | 0 | 0.5 | 7.50 |
| **Z121** | ZA3-Ep10 | 1191.92 | 50 | 38.5 | 0 | 0 | 0.5 | 5.00 |
| **Z122** | ZA3-Ep10 | 1191.92 | 50 | 38.5 | 0 | 0 | 5 | 10.00 |
| **Z123** | ZA3-Ep10 | 1191.92 | 50 | 38.5 | 0 | 0 | 5 | 7.50 |
| **Z124** | ZA3-Ep10 | 1191.92 | 50 | 38.5 | 0 | 0 | 5 | 5.00 |
| **Z125** | ZA3-Ep10 | 1191.92 | 50 | 38.5 | 0 | 0 | 0 | 10.00 |
| **Z202** | ZA6-Ep10 | 933.48 | 50 | 38.5 | 0 | 0 | 1 | 20.00 |
| **Z302** | ZA3-Ep8 | 1051.68 | 50 | 38.5 | 0 | 0 | 1 | 20.00 |
| **Z402** | ZA3-Ep12 | 1332.18 | 50 | 38.5 | 0 | 0 | 1 | 20.00 |
| **Z502** | ZA3-Ep14 | 1472.48 | 50 | 38.5 | 0 | 0 | 1 | 20.00 |
| **Z602** | ZA3-Ep16 | 1612.73 | 50 | 38.5 | 0 | 0 | 1 | 20.00 |
| **Z702** | ZA3-Ep18 | 1753.03 | 50 | 38.5 | 0 | 0 | 1 | 20.00 |
| **Z802** | ZA1-Ep10 | 765.2 | 50 | 38.5 | 0 | 0 | 1 | 20.00 |
| **Z902** | ZA4-Ep10 | 850.35 | 50 | 38.5 | 0 | 0 | 1 | 20.00 |
| **Z1002** | ZA6-Ac10 | 1101.66 | 50 | 38.5 | 0 | 0 | 1 | 20.00 |

(continued)

| | | | Molar ratios in lipid mix | | | | | |
|---|---|---|---|---|---|---|---|---|
| **Formulation code** | **ZAL in formulation** | *Mol. Wt.* | *ZAL* | *Cholesterol* | *DSPC* | *DOPE* | *PEG Lipid* | **ZAL:nucleic acid wt ratio** |
| **Z1102** | ZA6-Ac12 | 1185.84 | 50 | 38.5 | 0 | 0 | 1 | 20.00 |

**Table 6: Formulations using Cationic Sulfonamide Amino Lipids (CSALs)**

| | | | Molar ratios in lipid mix | | | | | |
|---|---|---|---|---|---|---|---|---|
| **Formulation code** | **CSAL in formulation** | *Mol. Wt.* | *ZAL* | *Cholesterol* | *DSPC* | *DOPE* | *PEG Lipid* | **CSAL:nucleic acid wt ratio** |
| **CS100** | A3OAcC2Me | 1356.19 | 50 | 38.5 | 10 | 0 | 1.5 | 32.1 |
| **CS101** | A3OAcC2Me | 1356.19 | 50 | 38.5 | 10 | 0 | 1.5 | 14.3 |
| **CS102** | A3OAcC2Me | 1356.19 | 50 | 38.5 | 10 | 0 | 1.5 | 21.4 |
| **CS103** | A3OAcC2M | 1356.19 | 50 | 38.5 | 0 | 0 | 1.5 | 20.0 |
| **CS104** | A3OAcC2M | 1356.19 | 50 | 38.5 | 0 | 0 | 1.5 | 15.0 |
| **CS111** | A3OAcC2M | 1356.19 | 50 | 38.5 | 0 | 0 | 10 | 15.0 |
| **CS200** | A1OAcC2Me | 804.29 | 50 | 38.5 | 10 | 0 | 1.5 | 17.9 |

**C. Plasmids and Site-Directed Mutagenesis of CFTR**

An expression plasmid of full-length, wild-type CFTR, (pBI-CFTR) was purchased from Clontech and was mutagenized using standard protocols for site-directed mutagenesis (Sambrook *et al.,* 1989). Site-directed mutagenesis was performed by PCR techniques using PfuUltra High-Fidelity DNA Polymerase (Stratagene, Santa Clara, CA). All mutations were confirmed by DNA sequencing. Sup-tRNA$^{Arg}$ was a gift from Carla Oliveira (Institute of Molecular Pathology and Immunology of the University of Porto (IPATIMUP), Porto, Portugal).

**D. Quantification Methods of Mature CFTR**

HEK293 cells were seeded ($9\times10^5$ cells) and transfected with CFTR plasmids. 2 $\mu$g of CFTR plasmid and 500 ng of Sup-tRNA$^{Arg}$ were co-transfected using 4 $\mu$l of Lipofectamine 2000 in a 6-well format. G418 (200 $\mu$g) or PTC124 (40 $\mu$M) was added to the media 24hr post-transfection and remained for 48hr. IB3-1 cells were seeded and G418 (0-400 $\mu$G) or PTC124 (0-20 $\mu$M) was added 24hr later. After 48hr, cells were lysed directly in 2x Sample Buffer ((Tris-HCL 250 mM, pH 6.8, 20% Glycerol, 2.5% SDS, 0.1% Bromophenol blue). Cell lysate proteins were separated by electrophoresis on 7%/10% step (wt/vol) polyacrylamide gels using a Tris-glycine buffering system and transferred to polyvinylidene fluoride Immobilon membranes (EMD Millipore). Western blot analysis was performed using primary CFTR antibody (596) (University of North Carolina School of Medicine, Chapel Hill, NC), actin antibody (EMD Millipore), and secondary antibody IRdye-680RD (Li-Cor) and imaged/quantified using a Li-Cor Odyssey CLx (Li-Cor). Data was plotted using Prism 6 (Graphpad).

**E. CFTR-Dependent Whole-Cell Current in HEK293 Cells**

HEK293 cells were transfected with the plasmids used for the CFTR maturation experiments. 2 $\mu$g of CFTR plasmid and 500 ng of Sup-tRNA$^{Arg}$ were cotransfected using 4 $\mu$l of Lipofectamine 2000 in a 6-well format. 24 hr post-transfection, the whole-cell configuration of the patch-clamp technique was used to measure the Cl- current. The pipette solution contained 145 mM NMDG+-Cl- , 1 mM MgCl2, 2 mM EGTA, 5 mM ATP, and 10 mM HEPES (pH 7.3 with Tris). The bath solution was 145 mM NMDG+-Cl-, 1 mM MgCl2, 1 mM Ca C12, 10 mM HEPES and 10 mM glucose (pH 7.4 with Tris). The current was recorded with an Axopatch 200B patch-clamp amplifier and digitized at 2 kHz. The membrane conductance was probed by stepping the membrane potential from a holding potential of 0 mV to membrane potentials -40 and +40 mV steps for 200 ms. Whole-cell current responses were measured in response to 10 $\mu$M forskolin plus 100 $\mu$M IBMX and 10 $\mu$M CFTRInh-172 (Inh-172). Pipettes had resistances between 3 and 5 M$\Omega$ when filled with pipette

solution and seal resistance exceeded 8 GU. Current recording and analysis was performed with pClamp 9.2 software and analyzed with Origin 8 software.

**F. Uptake Studies**

Cellular uptake studies were performed using the top performing materials from the screen. Calu6 cells were seeded at a density of 40,000 cells per well in 8-chambered coverglass slides (Nunc) and allowed to attached for 24 hours. NP formulations were prepared by manual mixing using a similar protocol to the *in vitro* transfection assays above using Fluorescin-labeled tRNA. The formulation was performed in 10 mM citrate buffer pH 4.3 at a final mole ratio of 25:1 carrier:siRNA, and the carrier mixture of the formulation consisted of molar ratios 50:38:10:2 carrier: cholesterol: DSPC: PEG-lipid. The nanoparticles were added to the cells at a final tRNA dose of 0.9 μg/well. After 6h incubation, the medium was aspirated, washed with PBS, and cell membrane staining was performed (Cell Mask Red, Molecular Probes) using the manufacturer's protocol. Cell nuclei were stained with DAPI (Sigma-Aldrich). Confocal microscopy imaging was performed using a Nikon Eclipse TE2000-E and images were analyzed using Imaged (NIH).

**G. Nanoparticle Carrier Screen in Calu6 Cells**

Calu6 cells were seeded at a density of 500,000 cells per well in a 6-well format and allowed to attach over night. For plasmid DNA, 1μg was transfected using 3 μl of Lipofectamine 2000 using manufacturer recommend protocols. For $tRNA^{Arg/OP}$-RNAiMax, 4 μg was transfected using 3 μl of RNAiMax using manufacturer recommended protocols. Particles were diluted in Opti-MEM (Invitrogen). G418 (50 μg) and PTC124 (10 μl M) was added directly to the media. Nanoparticles were prepared by mixing all delivery components in ethanol, followed by rapid mixing with tRNA in 10 mM citrate buffer pH 4.3, such that the final mole ratio was 25:1 carrier: siRNA, and the carrier mixture of the formulation consisted of molar ratios 50:38:10:2 carrier: cholesterol: DSPC: PEG-lipid. The nanoparticles were then diluted in sterile PBS to raise the pH and added to cells. After 48hr, cells were lysed directly in 2x Sample Buffer ((Tris-HCL 250 mM, pH 6.8, 20% Glycerol, 2.5% SDS, 0.1% Bromophenol blue). Cell lysate proteins were separated by electrophoresis on 10% (wt/vol) polyacrylamide gels using a Tris-glycine buffering system and transferred to polyvinylidene fluoride Immobilon membranes (EMD Millipore). Western blot analysis was performed using primary p53 antibody (Santa Cruz Biotechnology, Inc) actin antibody (EMD Millipore), and secondary antibody IRdye-680RD (Li-Cor) and imaged/quantified using a Li-Cor Odyssey CLx (Li-Cor).

**H. NP size analysis**

Particle sizes were measured by Dynamic Light Scattering (DLS) using a Malvern Zetasizer Nano ZS (He-Ne laser, λ = 632 nm).

**Example 2 - tRNA Delivery**

As shown in **FIG. 1,** nonsense mutations result in nonfunctional proteins by terminating the synthesis at a mutation that results in a premature stop codon in the mRNA. Efforts to correct these mutations in a protein by read through agents have not restored activity in cystic fibrosis transmembrane regulator protein as can be shown in **FIGS. 2A-2C.** As shown in **FIGS. 3A & 3B,** correction of the mutation with any amino acid does not result in a functional protein. Similarly, even if the mutation leads to folding, the resultant protein may be functional defective **(FIG. 4A & 4B).** Thus read-through approaches to correcting genetic mutations may not sufficient restore activity of the protein. On the other hand, introduction of a modified tRNA results in restoration of CFTR folding and function as can be seen in **FIGS. 5A-5C.** Similarly, the introduction of the modified tRNA in a nanoparticle formulation results in the delivery of the tRNA into a cell **(FIG. 6A)** and the production of functional p53 by the introduction of the specific amino acid into the growing peptide chain **(FIGS. 6B & 6C).**

A variety of different amino lipid delivery compositions were tested to determine the amount of tRNA bound to the nanoparticles. **FIG. 7** shows that most of the materials tested bound greater than 0.8 fraction of tRNA bound. Similarly, **FIG. 8** shows the characteristics of these tRNA compositions including particle size and the polydispersity index. These compositions were tested for their ability to restore p53 expression in Calu6 cells using exogenous $tRNA^{Arg/OP}$. The western blot analysis of these composition is shown in **FIG. 9.** One of the compositions which showed restoration of p53 expression was tested at a variety of different concentrations as well as tested two other agents which showed only a marginal restoration of p53 expression relative to the composition **(FIG. 10).** To confirm that the tRNA composition was being taken up by cells, fluorescent microscopy was carried out with a labeled tRNA. As can be seen in **FIG. 11,** the tRNA was taken up by the Calu6 cells after 6 hours of incubation. Additionally, **FIG. 12** shows the update of tRNA using zwitterionic and cationic sulfonamide amino lipids. Finally, the data in **FIG. 13** shows different methods of preparing the

nanoparticles on both the response in a luciferase assay as well as viability.

## REFERENCES

The following references, to the extent that they provide exemplary procedural or other details supplementary to those set forth herein.


WO 2016/094342
WO 2017/048789
International Application No. PCT/US2017/032950
Söll, et al., tRNA: Structure, Biosynthesis, and Function (ASM Press), 1995.
El Yacoubi, et al., Annu. Rev. Genet., 46:69-95, 2012.
Grosjean and Benne, Modification and Editing of RNA (ASM Press), 1998.
Hendrickson, et al., Annu. Rev. Biochem., 73:147-176, 2004.
Ibba and Soil, Annu. Rev. Biochem., 69:617-650, 2000.
Johnson et al., Cold Spring Harbor Symp. Quant. Biol., 60:71-82, 1995.
Johnson etal., J. Mol. Biol., 156:113-140, 1982.
Crowley et al., Cell, 78:61-71, 1994.
Beier and Grimm, Nucleic Acids Res., 29:4767-4782, 2001.
Torres, et al., Trends Mol. Med., 20:306-314, 2014.
Björk et al., Annu. Rev. Biochem., 56:263-287, 1987.
Green and Sambrook, Molecular Cloning: A Laboratory Manual (CHSL Press), 2012.
Rio et al., RNA: A Laboratory Manual (CHSL Press), 2011.
Flanagan et al., J. Biol. Chem., 278:18628-18637, 2003.
Janiak, et al., Biochemistry, 31:5830-5840, 1992.
Yan et al., Proc. Natl. Acad. Sci., USA, 113(39):E5702-E5710, 2016.


## Claims

**1.** A composition comprising:

(A) a transfer ribonucleic acid (tRNA); and
(B) an aminolipid delivery compound;
wherein the aminolipid delivery compound forms a nanoparticle and the aminolipid delivery compound is:

(i) a dendrimer of the formula:

$$\text{Core-(Repeating Unit)}_n\text{-Terminating Group (I)}$$

wherein the core is linked to the repeating unit by removing one or more hydrogen atoms from the core and replacing with the repeating unit and wherein:

the core has the formula:

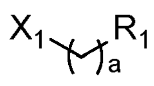

(II)

wherein:

$X_1$ is amino or alkylamino$_{(C\leq12)}$, dialkylamino$_{(C\leq12)}$, heterocycloalkyl$_{(C\leq12)}$, heteroaryl$_{(C\leq12)}$, or a substituted version thereof;
$R_1$ is amino, hydroxy, or mercapto, or alkylamino$_{(C\leq12)}$, dialkylamino$_{(C\leq12)}$, or a substituted version of either of these groups; and
a is 1, 2, 3, 4, 5, or 6; or

the core has the formula:

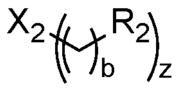
(III)

wherein:

$X_2$ is $N(R_5)_y$;
$R_5$ is hydrogen, $alkyl_{(C\leq 18)}$, or substituted $alkyl_{(C\leq 18)}$; and
y is 0, 1, or 2, provided that the sum of y and z is 3;
$R_2$ is amino, hydroxy, or mercapto, or $alkylamino_{(C\leq 12)}$, $dialkylamino_{(C\leq 12)}$, or a substituted version of either of these groups;
b is 1, 2, 3, 4, 5, or 6; and
z is 1, 2, 3; provided that the sum of z and y is 3; or

the core has the formula:

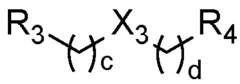
(IV)

wherein:

$X_3$ is $-NR_6-$, -O-, or $alkylaminodiyl_{(C\leq 8)}$, $alkoxydiyl_{(C\leq 8)}$, $arenediyl_{(C\leq 8)}$, $heteroarenediyl_{(C\leq 8)}$, $heterocycloalkanediyl_{(C\leq 8)}$, or a substituted version of any of these groups, wherein $R_6$ is hydrogen, $alkyl_{(C\leq 8)}$, or substituted $alkyl_{(C\leq 8)}$;
$R_3$ and $R_4$ are each independently amino, hydroxy, or mercapto, or $alkylamino_{(C\leq 12)}$, $dialkylamino_{(C\leq 12)}$, wherein, optionally, either of these groups is substituted by one or more substituents selected from the group consisting of: -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, and $-S(O)_2NH_2$;
wherein:
c and d are each independently 1, 2, 3, 4, 5, or 6; or

the core is $alkylamine_{(C\leq 18)}$, $dialkylamine_{(C\leq 36)}$, $heterocycloalkane_{(C\leq 12)}$, or a substituted version of any of these groups;
wherein the repeating unit comprises a degradable diacyl group and a linker group;
the degradable diacyl group has the formula:

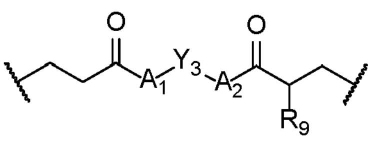
(VII)

wherein:
$A_1$ and $A_2$ are each independently -O-, -S-, or $-NR_a-$, wherein:

$R_a$ is hydrogen, $alkyl_{(C\leq 6)}$, or substituted $alkyl_{(C\leq 6)}$;
$Y_3$ is $alkanediyl_{(C\leq 12)}$, $alkenediyl_{(C\leq 12)}$, $arenediyl_{(C\leq 12)}$, or a substituted version of any of these groups; or a group of the formula:

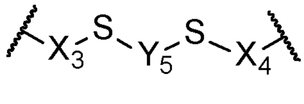
or
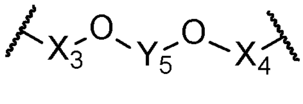

wherein:

$X_3$ and $X_4$ are alkanediyl$_{(C\leq12)}$, alkenediyl$_{(C\leq12)}$, arenediyl$_{(C\leq12)}$, or a substituted version of any of these groups;
$Y_5$ is a covalent bond, alkanediyl$_{(C\leq12)}$, alkenediyl$_{(C\leq12)}$, arenediyl$_{(C\leq12)}$, or a substituted version of any of these groups; and
$R_9$ is alkyl$_{(C\leq8)}$ or substituted alkyl$_{(C\leq8)}$;

the linker group has the formula:

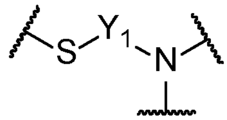
(VI)

wherein:

$Y_1$ is alkanediyl$_{(C\leq12)}$, alkenediyl$_{(C\leq12)}$, arenediyl$_{(C\leq12)}$, or a substituted version of any of these groups; and
wherein when the repeating unit comprises a linker group, then the linker group is attached to a degradable diacyl group on both the nitrogen and the sulfur atoms of the linker group, wherein the first group in the repeating unit is a degradable diacyl group, wherein for each linker group, the next group comprises two degradable diacyl groups attached to the nitrogen atom of the linker group; and wherein n is the number of linker groups present in the repeating unit; and

the terminating group has the formula:

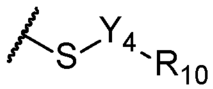
(VIII)

wherein:

$Y_4$ is alkanediyl$_{(C\leq18)}$, alkenediyl$_{(C\leq18)}$, or a substituted version of either group;
$R_{10}$ is hydrogen, carboxy, hydroxy, or
aryl$_{(C\leq12)}$, alkylamino$_{(C\leq12)}$, dialkylamino$_{(C\leq12)}$, *N*-heterocycloalkyl$_{(C\leq12)}$, -C(O)N($R_{11}$)-alkanediyl$_{(C\leq6)}$-heterocycloalkyl$_{(C\leq12)}$, -C(O)-alkylamino$_{(C\leq12)}$, -C(O)-dialkylamino$_{(C\leq12)}$, -C(O)-*N*-heterocycloalkyl$_{(C\leq12)}$, wherein:
$R_{11}$ is hydrogen, alkyl$_{(C\leq6)}$, or substituted alkyl$_{(C\leq6)}$;
wherein the final degradable diacyl group in the chain is attached to a terminating group;
n is 0, 1, 2, 3, 4, 5, or 6; or

(ii) a compound of the formula:

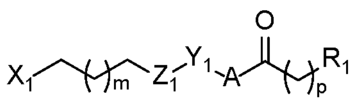
(I)

wherein:

$X_1$ is $-S(O)_2O^-$, $-OP(O)OR_eO^-$, $-(CHR_f)_zC(O)O$ , or $-NR_gR_hR_i^+$, wherein:

$R_e$, $R_g$, $R_h$, and $R_i$ are each independently hydrogen, alkyl$_{(C\leq6)}$, or substituted alkyl$_{(C\leq6)}$;
$R_f$ is hydrogen, amino, hydroxy, or alkyl$_{(C\leq12)}$, aryl$_{(C\leq12)}$, aralkyl$_{(C\leq12)}$, heteroaryl$_{(C\leq12)}$, acyl$_{(C\leq12)}$, alkoxy$_{(C\leq12)}$, acyloxy$_{(C\leq12)}$, amido$_{(C\leq12)}$, alkoxy$_{(C\leq12)}$, alkoxy$_{(C\leq12)}$, or a substituted version of any

of the last ten groups; and
z is 1, 2, 3, or 4;

$Y_1$ is alkanediyl$_{(C\leq12)}$, alkenediyl$_{(C\leq12)}$, arenediyl$_{(C\leq12)}$, heteroarenediyl$_{(C\leq12)}$, heterocycloalkanediyl$_{(C\leq12)}$, -alkanediyl$_{(C\leq8)}$-heterocycloalkanediyl$_{(C\leq12)}$, -alkanediyl$_{(C\leq8)}$-heterocycloalkanediyl$_{(C\leq12)}$-alkanediyl$_{(C\leq8)}$, -alkanediyl$_{(C\leq8)}$-heteroarenediyl$_{(C\leq12)}$, -alkanediyl$_{(C\leq8)}$-heteroarenediyl$_{(C\leq12)}$-alkanediyl$_{(C\leq8)}$, or a substituted version of any of these groups;
$Z_1$ is $-N^+R_3R_4-$ or $-OP(O)O^-O-$
A is $-NR_a-$, -S-, or -O-; wherein:
$R_a$ is hydrogen, alkyl$_{(C\leq6)}$, or substituted alkyl$_{(C\leq6)}$, or $R_a$ is taken together with either $R_3$ or $R_4$ and is alkanediyl$_{(C\leq8)}$, alkenediyl$_{(C\leq8)}$, alkoxydiyl$_{(C\leq8)}$, alkylaminodiyl$_{(C\leq8)}$, or a substituted version of any of these groups;
$R_1$ is a group of the formula:

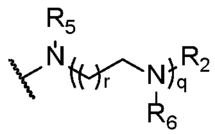

wherein:

$R_5$, $R_6$, and $R_2$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:
R' and R" are each independently hydrogen, alkyl$_{(C\leq8)}$, substituted alkyl$_{(C\leq8)}$, or $-Z_2A'R_7$; wherein:

$Z_2$ is alkanediyl$_{(C\leq6)}$, substituted alkanediyl$_{(C\leq6)}$, or a group of the formula:

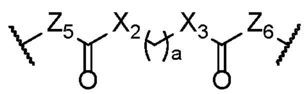

wherein:

$Z_5$ and $Z_6$ are each independently alkanediyl$_{(C\leq6)}$ or substituted alkanediyl$_{(C\leq6)}$;
$X_2$ and $X_3$ are each independently -O-, -S-, or $-NR_m-$; wherein:
$R_m$ is hydrogen, alkyl$_{(C\leq6)}$, or substituted alkyl$_{(C\leq6)}$; and
a is 0, 1, 2, 3, 4, 5, or 6;

A' is $-CHR_j-$, -C(O)O-, or $-C(O)NR_b-$;

$R_b$ is hydrogen, alkyl$_{(C\leq6)}$, or substituted alkyl$_{(C\leq6)}$; and
$R_j$ is hydrogen, halo, hydroxy, acyloxy$_{(C\leq24)}$, or substituted acyloxy(cs24);

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $R_2$ are each independently $-Z_3A''R_8$; wherein:

$Z_3$ is alkanediyl$_{(C\leq6)}$, substituted alkanediyl$_{(C\leq6)}$, or a group of the formula:

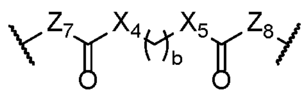

wherein:

$Z_7$ and $Z_8$ are each independently alkanediyl$_{(C\leq6)}$ or substituted alkanediyl$_{(C\leq6)}$;

$X_4$ and $X_5$ are each independently -O-, -S-, or -$NR_n$-; wherein:
$R_n$ is hydrogen, alkyl$_{(C\leq6)}$, or substituted alkyl$_{(C\leq6)}$; and
b is 0, 1, 2, 3, 4, 5, or 6;

A" is -$CHR_k$-, -S-, -C(O)O-, or -C(O)$NR_1$-;

$R_l$ is hydrogen, alkyl$_{(C\leq6)}$, or substituted alkyl$_{(C\leq6)}$; and
$R_k$ is hydrogen, halo, hydroxy, acyloxy$_{(C\leq24)}$, or substituted acyloxy$_{(C\leq24)}$; and

$R_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and
r is 1, 2, 3, or 4;

$R_1$ is a group of the formula:

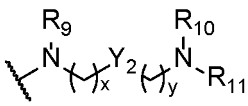

wherein:

$Y_2$ is arenediyl$_{(C\leq12)}$, heterocycloalkanediyl$_{(C\leq12)}$, heteroarenediyl$_{(C\leq12)}$, alkoxydiyl$_{(C\leq12)}$, or a substituted version of any of these groups;
$R_9$, $R_{10}$, and $R_{11}$ are each independently hydrogen, alkyl$_{(C\leq8)}$, substituted alkyl$_{(C\leq8)}$, or -$Z_4A'''R_{12}$; wherein:

$Z_4$ is alkanediyl$_{(C\leq6)}$, substituted alkanediyl$_{(C\leq6)}$, or a group of the formula:

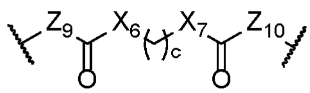

wherein:

$Z_9$ and $Z_{10}$ are each independently alkanediyl$_{(C\leq6)}$ or substituted alkanediyl$_{(C\leq6)}$;
$X_6$ and $X_7$ are each independently -O-, -S-, or -$NR_o$-; wherein:
$R_o$ is hydrogen, alkyl$_{(C\leq6)}$, or substituted alkyl$_{(C\leq6)}$; and
c is 0, 1, 2, 3, 4, 5, or 6;

A''' is -$CHR_k$-, -S-, -C(O)O-, or -C(O)NRi-;

$R_l$ is hydrogen, alkyl$_{(C\leq6)}$, or substituted alkyl$_{(C\leq6)}$; and
$R_k$ is hydrogen, halo, hydroxy, acyloxy$_{(C\leq24)}$, or substituted acyloxy$_{(C\leq24)}$; and

$R_{12}$ is alkyl$_{(C6-24)}$, substituted alkyl$_{(C6-24)}$, alkenyl$_{(C6-24)}$, substituted alkenyl$_{(C6-24)}$; and
x and y are 0, 1, 2, 3, or 4;

$R_3$ and $R_4$ are each independently hydrogen, alkyl$_{(C\leq6)}$, or substituted alkyl$_{(C\leq6)}$, or $R_3$ or $R_4$ are taken together with $R_a$ and is alkanediyl$_{(C\leq8)}$, alkenediyl$_{(C\leq8)}$, alkoxydiyl$_{(C\leq8)}$, alkylaminodiyl$_{(C\leq8)}$, or a substituted version of any of these groups; and
m, n, and p are each independently an integer selected from 0, 1, 2, 3, 4, 5, or 6;
provided that if $X_1$ is positively charged then $Z_1$ is negatively charged, and if $X_1$ is negatively charged, then $Z_1$ is positively charged;

or a pharmaceutically acceptable salt thereof;
wherein when a term is used with the "substituted" modifier, or the phrase "substituted version" is used, one or

more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$.

**2.** A composition for use in medicine, wherein the composition is used to deliver a transfer RNA (tRNA) into a cell, the composition comprising:

(a) the tRNA; and
(b) an aminolipid delivery compound;

wherein the aminolipid delivery compound forms a nanoparticle.

**3.** The composition for use of claim 2, wherein the aminolipid delivery compound is a dendrimer of the formula:

$$\text{Core-(Repeating Unit)}_n\text{-Terminating Group (I)}$$

wherein the core is linked to the repeating unit by removing one or more hydrogen atoms from the core and replacing with the repeating unit and wherein:
the core has the formula:

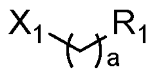
(II)

wherein:

$X_1$ is amino or alkylamino$_{(C\leq12)}$, dialkylamino$_{(C\leq12)}$, heterocycloalkyl$_{(C\leq12)}$, heteroaryl$_{(C\leq12)}$, or a substituted version thereof;
$R_1$ is amino, hydroxy, or mercapto, or alkylamino$_{(C\leq12)}$, dialkylamino$_{(C\leq12)}$, or a substituted version of either of these groups; and
a is 1, 2, 3, 4, 5, or 6; or

the core has the formula:

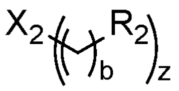
(III)

wherein:

$X_2$ is $N(R_5)_y$;
$R_5$ is hydrogen, alkyl$_{(C\leq18)}$, or substituted alkyl$_{(C\leq18)}$; and
y is 0, 1, or 2, provided that the sum of y and z is 3;
$R_2$ is amino, hydroxy, or mercapto, or alkylamino$_{(C\leq12)}$, dialkylamino$_{(C\leq12)}$, or a substituted version of either of these groups;
b is 1, 2, 3, 4, 5, or 6; and
z is 1, 2, 3; provided that the sum of z and y is 3; or

the core has the formula:

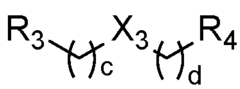
(IV)

wherein:

$X_3$ is $-NR_6-$, -O-, or alkylaminodiyl$_{(C\leq 8)}$, alkoxydiyl$_{(C\leq 8)}$, arenediyl$_{(C\leq 8)}$, heteroarenediyl$_{(C\leq 8)}$, heterocycloalkanediyl$_{(C\leq 8)}$, or a substituted version of any of these groups, wherein $R_6$ is hydrogen, alkyl$_{(C\leq 8)}$, or substituted alkyl$_{(C\leq 8)}$;
$R_3$ and $R_4$ are each independently amino, hydroxy, or mercapto, or alkylamino$_{(C\leq 12)}$, dialkylamino$_{(C\leq 12)}$, wherein, optionally, either of these groups is substituted by one or more substituents selected from the group consisting of: -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, - $C(O)N(CH_3)_2$, $-OC(O)CH_3$, $-NHC(O)CH_3$, $-S(O)_2OH$, and $-S(O)_2NH_2$;
wherein:
c and d are each independently 1, 2, 3, 4, 5, or 6; or

the core is alkylamine$_{(C\leq 18)}$, dialkylamine$_{(C\leq 36)}$, heterocycloalkane$_{(C\leq 12)}$, or a substituted version of any of these groups;
wherein the repeating unit comprises a degradable diacyl group and a linker group;
the degradable diacyl group has the formula:

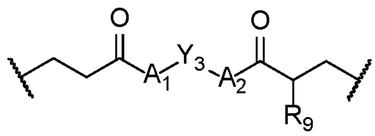
(VII)

wherein:
$A_1$ and $A_2$ are each independently -O-, -S-, or $-NR_a-$, wherein:

$R_a$ is hydrogen, alkyl$_{(C\leq 6)}$, or substituted alkyl$_{(C\leq 6)}$;
$Y_3$ is alkanediyl$_{(C\leq 12)}$, alkenediyl$_{(C\leq 12)}$, arenediyl$_{(C\leq 12)}$, or a substituted version of any of these groups; or a group of the formula:

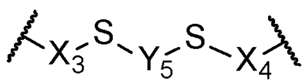
or
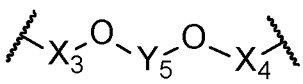

wherein:

$X_3$ and $X_4$ are alkanediyl$_{(C\leq 12)}$, alkenediyl$_{(C\leq 12)}$, arenediyl$_{(C\leq 12)}$, or a substituted version of any of these groups;
Ys is a covalent bond, alkanediyl$_{(C\leq 12)}$, alkenediyl$_{(C\leq 12)}$, arenediyl$_{(C\leq 12)}$, or a substituted version of any of these groups; and
$R_9$ is alkyl$_{(C\leq 8)}$ or substituted alkyl$_{(C\leq 8)}$;

the linker group has the formula:

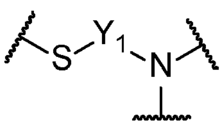
(VI)

wherein:

$Y_1$ is alkanediyl$_{(C\leq 12)}$, alkenediyl$_{(C\leq 12)}$, arenediyl$_{(C\leq 12)}$, or a substituted version of any of these groups; and wherein when the repeating unit comprises a linker group, then the linker group is attached to a degradable diacyl group on both the nitrogen and the sulfur atoms of the linker group, wherein the first group in the repeating unit is a degradable diacyl group, wherein for each linker group, the next group comprises two degradable diacyl groups attached to the nitrogen atom of the linker group; and wherein n is the number

of linker groups present in the repeating unit; and

the terminating group has the formula:

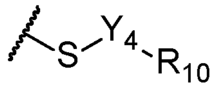 (VIII)

wherein:

$Y_4$ is alkanediyl$_{(C\leq18)}$, alkenediyl$_{(C\leq18)}$, or a substituted version of either group;
$R_{10}$ is hydrogen, carboxy, hydroxy, or aryl$_{(C\leq12)}$, alkylamino$_{(C\leq12)}$, dialkylamino$_{(C\leq12)}$, *N*-heterocycloalkyl$_{(C\leq12)}$, -C(O)N($R_{11}$)-alkanediyl$_{(C\leq6)}$-heterocycloalkyl$_{(C\leq12)}$, -C(O)-alkylamino$_{(C\leq12)}$, -C(O)-dialkylamino$_{(C\leq12)}$, -C(O)-*N*-heterocycloalkyl$_{(C\leq12)}$, wherein:
$R_{11}$ is hydrogen, alkyl$_{(C\leq6)}$, or substituted alkyl$_{(C\leq6)}$;
wherein the final degradable diacyl group in the chain is attached to a terminating group;
n is 0, 1, 2, 3, 4, 5, or 6;

or a pharmaceutically acceptable salt thereof;
wherein when a term is used with the "substituted" modifier, or the phrase "substituted version" is used, one or more hydrogen atom has been independently replaced by -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, or $-S(O)_2NH_2$.

**4.** The composition for use of claim 3, wherein the aminolipid delivery compound is a dendrimer of the formula:

$$\text{Core-(Repeating Unit)}_n\text{-Terminating Group (I)}$$

wherein the core is linked to the repeating unit by removing one or more hydrogen atoms from the core and replacing with the repeating unit and wherein:

the core has the formula:

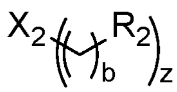 (III)

wherein:

$X_2$ is $N(R_5)_y$;

$R_5$ is hydrogen or alkyl$_{(C\leq8)}$, or substituted alkyl$_{(C\leq18)}$; and
y is 0, 1, or 2, provided that the sum of y and z is 3;

$R_2$ is amino, hydroxy, or mercapto, or alkylamino$_{(C\leq12)}$, dialkylamino$_{(C\leq12)}$, or a substituted version of either of these groups;
b is 1, 2, 3, 4, 5, or 6; and
z is 1, 2, 3; provided that the sum of z and y is 3;

wherein the repeating unit comprises a degradable diacyl group and a linker group;

the degradable diacyl group has the formula:

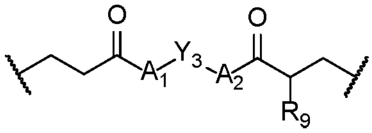
(VII)

wherein:

$A_1$ and $A_2$ are each independently -O- or -$NR_a$-, wherein:
$R_a$ is hydrogen, alkyl$_{(C\leq 6)}$, or substituted alkyl$_{(C\leq 6)}$;
$Y_3$ is alkanediyl$_{(C\leq 12)}$, alkenediyl$_{(C\leq 12)}$, arenediyl$_{(C\leq 12)}$, or a substituted version of any of these groups; or a group of the formula:

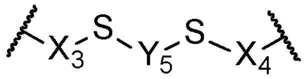
or
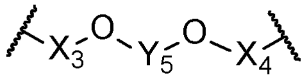

wherein:

$X_3$ and $X_4$ are alkanediyl$_{(C\leq 12)}$, alkenediyl$_{(C\leq 12)}$, arenediyl$_{(C\leq 12)}$, or a substituted version of any of these groups;
$Y_5$ is a covalent bond, alkanediyl$_{(C\leq 12)}$, alkenediyl$_{(C\leq 12)}$, arenediyl$_{(C\leq 12)}$, or a substituted version of any of these groups; and

$R_9$ is alkyl$_{(C\leq 8)}$ or substituted alkyl$_{(C\leq 8)}$;

the linker group has the formula:

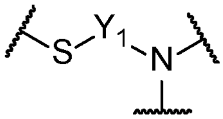
(VI)

wherein: $Y_1$ is alkanediyl$_{(C\leq 12)}$, alkenediyl$_{(C\leq 12)}$, arenediyl$_{(C\leq 12)}$, or a substituted version of any of these groups; and

wherein when the repeating unit comprises a linker group, then the linker group is attached to a degradable diacyl group on both the nitrogen and the sulfur atoms of the linker group, wherein the first group in the repeating unit is a degradable diacyl group, wherein for each linker group, the next group comprises two degradable diacyl groups attached to the nitrogen atom of the linker group; and wherein n is the number of linker groups present in the repeating unit; and
the terminating group, wherein the terminating group has the formula:

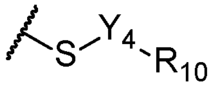
(VIII)

wherein:

$Y_4$ is alkanediyl$_{(C\leq 18)}$, alkenediyl$_{(C\leq 18)}$, or a substituted version of either group;
$R_{10}$ is hydrogen, carboxy, hydroxy, or
aryl$_{(C\leq 12)}$, alkylamino$_{(C\leq 12)}$, dialkylamino$_{(C\leq 12)}$, *N*-heterocycloalkyl$_{(C\leq 12)}$, -C(O)N($R_{11}$)-alkanediyl$_{(C\leq 6)}$-heterocycloalkyl$_{(C\leq 12)}$, -C(O)-alkylamino$_{(C\leq 12)}$, -C(O)-dialkylamino$_{(C\leq 12)}$, -C(O)-*N*-heterocycloalkyl$_{(C\leq 12)}$, wherein:

wherein the final degradable diacyl group in the chain is attached to a terminating group;

n is 0, 1, 2, 3, 4, 5, or 6;

or a pharmaceutically acceptable salt thereof;
or wherein the aminolipid delivery compound is a dendrimer of the formula:

$$\text{Core-(Repeating Unit)}_n\text{-Terminating Group (I)}$$

wherein the core is linked to the repeating unit by removing one or more hydrogen atoms from the core and replacing with the repeating unit and wherein:

the core has the formula:

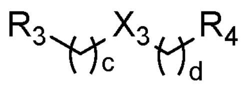
(IV)

wherein:

$X_3$ is $-NR_6-$, -O-, or alkylaminodiyl$_{(C\leq8)}$, alkoxydiyl$_{(C\leq8)}$, arenediyl$_{(C\leq8)}$, heteroarenediyl$_{(C\leq8)}$, heterocycloalkanediyl$_{(C\leq8)}$, or a substituted version of any of these groups, wherein $R_6$ is hydrogen, alkyl$_{(C\leq8)}$, or substituted alkyl$_{(C\leq8)}$;
$R_3$ and $R_4$ are each independently amino, hydroxy, or mercapto, or alkylamino$_{(C\leq12)}$, dialkylamino$_{(C\leq12)}$, wherein, optionally, either of these groups is substituted by one or more substituents selected from the group consisting of: -OH, -F, -Cl, -Br, -I, $-NH_2$, $-NO_2$, $-CO_2H$, - $CO_2CH_3$, -CN, -SH, $-OCH_3$, $-OCH_2CH_3$, $-C(O)CH_3$, $-NHCH_3$, - $NHCH_2CH_3$, $-N(CH_3)_2$, $-C(O)NH_2$, $-C(O)NHCH_3$, $-C(O)N(CH_3)_2$, - $OC(O)CH_3$, $-NHC(O)CH_3$, $-S(O)_2OH$, and $-S(O)_2NH_2$;
wherein: c and d are each independently 1, 2, 3, 4, 5, or 6; and

wherein the repeating unit comprises a degradable diacyl group and a linker group;

the degradable diacyl group has the formula:

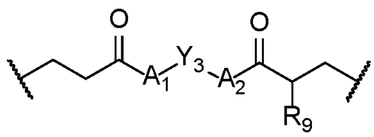
(VII)

wherein:

$A_1$ and $A_2$ are each independently -O- or $-NR_a-$, wherein: $R_a$ is hydrogen, alkyl$_{(C\leq6)}$, or substituted alkyl$_{(C\leq6)}$;
$Y_3$ is alkanediyl$_{(C\leq12)}$, alkenediyl$_{(C\leq12)}$, arenediyl$_{(C\leq12)}$, or a substituted version of any of these groups; or a group of the formula:

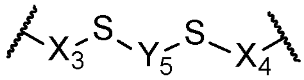
or
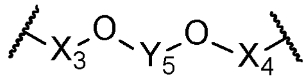

wherein:

$X_3$ and $X_4$ are alkanediyl$_{(C\leq12)}$, alkenediyl$_{(C\leq12)}$, arenediyl$_{(C\leq12)}$, or a substituted version of any of these groups;
$Y_5$ is a covalent bond, alkanediyl$_{(C\leq12)}$, alkenediyl$_{(C\leq12)}$, arenediyl$_{(C\leq12)}$, or a substituted version of any of these groups; and

$R_9$ is alkyl$_{(C\leq8)}$ or substituted alkyl$_{(C\leq8)}$;

the linker group has the formula:

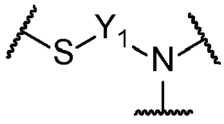

(VI)

wherein:

$Y_1$ is alkanediyl$_{(C\leq12)}$, alkenediyl$_{(C\leq12)}$, arenediyl$_{(C\leq12)}$, or a substituted version of any of these groups; and

wherein when the repeating unit comprises a linker group, then the linker group is attached to a degradable diacyl group on both the nitrogen and the sulfur atoms of the linker group, wherein the first group in the repeating unit is a degradable diacyl group, wherein for each linker group, the next group comprises two degradable diacyl groups attached to the nitrogen atom of the linker group; and wherein n is the number of linker groups present in the repeating unit; and
the terminating group, wherein the terminating group has the formula:

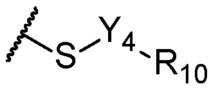

(VIII)

wherein:

$Y_4$ is alkanediyl$_{(C\leq18)}$, alkenediyl$_{(C\leq18)}$, or a substituted version of either group;
$R_{10}$ is hydrogen, carboxy, hydroxy, or
aryl$_{(C\leq12)}$, alkylamino$_{(C\leq12)}$, dialkylamino$_{(C\leq12)}$, *N*-heterocycloalkyl$_{(C\leq12)}$, -C(O)N($R_{11}$)-alkanediyl$_{(C\leq6)}$-heterocycloalkyl$_{(C\leq12)}$, -C(O)-alkylamino$_{(C\leq12)}$, -C(O)-dialkylamino$_{(C\leq12)}$, -C(O)-*N*-heterocycloalkyl$_{(C\leq12)}$, wherein:
$R_{11}$ is hydrogen, alkyl$_{(C\leq6)}$, or substituted alkyl$_{(C\leq6)}$;

wherein the final degradable diacyl group in the chain is attached to a terminating group;
n is 0, 1, 2, 3, 4, 5, or 6;

or a pharmaceutically acceptable salt thereof.

**5.** The composition for use of claim 2, wherein the aminolipid delivery compound is further defined as:

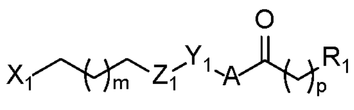

(I)

wherein:

$X_1$ is $-S(O)_2O^-$, $-OP(O)OR_eO^-$, $-(CHR_f)_zC(O)O^-$, or $-NR_gR_hR_i^+$, wherein:

$R_e$, $R_g$, $R_h$, and $R_i$ are each independently hydrogen, alkyl$_{(C\leq6)}$, or substituted alkyl$_{(C\leq6)}$;
$R_f$ is hydrogen, amino, hydroxy, or alkyl$_{(C\leq12)}$, aryl$_{(C\leq12)}$, aralkyl$_{(C\leq12)}$, heteroaryl$_{(C\leq12)}$, acyl$_{(C\leq12)}$, alkoxy$_{(C\leq12)}$, acyloxy$_{(C\leq12)}$, amido$_{(C\leq12)}$, alkoxy$_{(C\leq12)}$, alkoxy$_{(C\leq12)}$, or a substituted version of any of the last ten groups; and
z is 1, 2, 3, or 4;

$Y_1$ is alkanediyl$_{(C\leq 12)}$, alkenediyl$_{(C\leq 12)}$, arenediyl$_{(C\leq 12)}$, heteroarenediyl$_{(C\leq 12)}$, heterocycloalkanediyl$_{(C\leq 12)}$, -alkanediyl$_{(C\leq 18)}$-heterocycloalkanediyl$_{(C\leq 12)}$, -alkanediyl$_{(C\leq 8)}$-heterocycloalkanediyl$_{(C\leq 12)}$-alkanediyl$_{(C\leq 8)}$, -alkanediyl$_{(C\leq 8)}$-heteroarenediyl$_{(C\leq 12)}$, -alkanediyl$_{(C\leq 8)}$-heteroarene-diyl$_{(C\leq 12)}$-alkanediyl$_{(C\leq 8)}$, or a substituted version of any of these groups;
$Z_1$ is $-N^+R_3R_4-$ or $-OP(O)O^-O-$
A is $-NR_a-$, -S-, or -O-; wherein:
$R_a$ is hydrogen, alkyl$_{(C\leq 6)}$, or substituted alkyl$_{(C\leq 6)}$, or $R_a$ is taken together with either $R_3$ or $R_4$ and is alkanediyl$_{(C\leq 8)}$, alkenediyl$_{(C\leq 8)}$, alkoxydiyl$_{(C\leq 8)}$, alkylaminodiyl$_{(C\leq 8)}$, or a substituted version of any of these groups;
$R_1$ is a group of the formula:

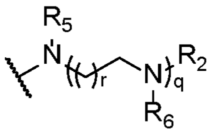

wherein:
$R_5$, $R_6$, and $R_2$ are each independently hydrogen or alkyl$_{(C\leq 8)}$, -alkanediyl$_{(C\leq 6)}$-$NH_2$, -alkanediyl$_{(C\leq 6)}$-alkylamino$_{(C\leq 8)}$, -alkanediyl$_{(C\leq 6)}$-dialkylamino$_{(C\leq 12)}$, -alkanediyl$_{(C\leq 6)}$-NR'R", or a substituted version of any of these groups wherein:

R' and R" are each independently hydrogen, alkyl$_{(C\leq 8)}$, substituted alkyl$_{(C\leq 8)}$, or $-Z_2A'R_7$; wherein:

$Z_2$ is alkanediyl$_{(C\leq 6)}$, substituted alkanediyl$_{(C\leq 6)}$, or a group of the formula:

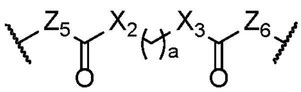

wherein:

$Z_5$ and $Z_6$ are each independently alkanediyl$_{(C\leq 6)}$ or substituted alkanediyl$_{(C\leq 6)}$;
$X_2$ and $X_3$ are each independently -O-, -S-, or $-NR_m-$; wherein:
$R_m$ is hydrogen, alkyl$_{(C\leq 6)}$, or substituted alkyl$_{(C\leq 6)}$; and
a is 0, 1, 2, 3, 4, 5, or 6;

A' is $-CHR_j-$, -C(O)O-, or $-C(O)NR_b-$;

$R_b$ is hydrogen, alkyl$_{(C\leq 6)}$, or substituted alkyl$_{(C\leq 6)}$; and
$R_j$ is hydrogen, halo, hydroxy, acyloxy$_{(C\leq 24)}$, or substituted acyloxy$_{(C\leq 24)}$;

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $R_2$ are each independently $-Z_3A"R_8$; wherein:

$Z_3$ is alkanediyl$_{(C\leq 6)}$, substituted alkanediyl$_{(C\leq 6)}$, or a group of the formula:

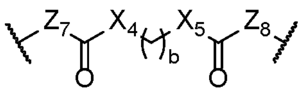

wherein:

$Z_7$ and $Z_8$ are each independently alkanediyl$_{(C\leq 6)}$ or substituted alkanediyl$_{(C\leq 6)}$;
$X_4$ and $X_5$ are each independently -O-, -S-, or $-NR_n-$; wherein:
$R_n$ is hydrogen, alkyl$_{(C\leq 6)}$, or substituted alkyl$_{(C\leq 6)}$; and
b is 0, 1, 2, 3, 4, 5, or 6;

A" is -CHR$_k$-, -S-, -C(O)O-, or -C(O)NR$_l$-;

R$_l$ is hydrogen, alkyl$_{(C\leq6)}$, or substituted alkyl$_{(C\leq6)}$; and
R$_k$ is hydrogen, halo, hydroxy, acyloxy$_{(C\leq24)}$, or substituted acyloxy$_{(C\leq24)}$; and

R$_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and
r is 1, 2, 3, or 4;

R$_1$ is a group of the formula:

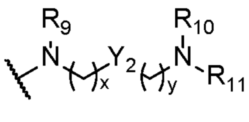

wherein:

Y$_2$ is arenediyl$_{(C\leq12)}$, heterocycloalkanediyl$_{(C\leq12)}$, heteroarenediyl$_{(C\leq12)}$, alkoxydiyl$_{(C\leq12)}$, or a substituted version of any of these groups;
R$_9$, R$_{10}$, and R$_{11}$ are each independently hydrogen, alkyl$_{(C\leq8)}$, substituted alkyl$_{(C\leq8)}$, or -Z$_4$A‴R$_{12}$; wherein:

Z$_4$ is alkanediyl$_{(C\leq6)}$, substituted alkanediyl$_{(C\leq6)}$, or a group of the formula:

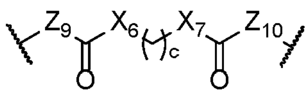

wherein:

Z$_9$ and Z$_{10}$ are each independently alkanediyl$_{(C\leq6)}$ or substituted alkanediyl$_{(C\leq6)}$;
X$_6$ and X$_7$ are each independently -0-, -S-, or -NR$_o$-; wherein:
R$_o$ is hydrogen, alkyl$_{(C\leq6)}$, or substituted alkyl$_{(C\leq6)}$; and
c is 0, 1, 2, 3, 4, 5, or 6;

A‴ is -CHR$_k$-, -S-, -C(O)O-, or -C(0)NRi-;

R$_l$ is hydrogen, alkyl$_{(C\leq6)}$, or substituted alkyl$_{(C\leq6)}$; and
R$_k$ is hydrogen, halo, hydroxy, acyloxy$_{(C\leq24)}$, or substituted acyloxy$_{(C\leq24)}$; and

R$_{12}$ is alkyl$_{(C6-24)}$, substituted alkyl$_{(C6-24)}$, alkenyl$_{(C6-24)}$, substituted alkenyl$_{(C6-24)}$; and

x and y are 0, 1, 2, 3, or 4;

R$_3$ and R$_4$ are each independently hydrogen, alkyl$_{(C\leq6)}$, or substituted alkyl$_{(C\leq6)}$, or R$_3$ or R$_4$ are taken together with R$_a$ and is alkanediyl$_{(C\leq8)}$, alkenediyl$_{(C\leq8)}$, alkoxydiyl$_{(C\leq8)}$, alkylaminodiyl$_{(C\leq8)}$, or a substituted version of any of these groups; and
m, n, and p are each independently an integer selected from 0, 1, 2, 3, 4, 5, or 6;
provided that if X$_1$ is positively charged then Z$_1$ is negatively charged, and if X$_1$ is negatively charged, then Z$_1$ is positively charged;

or a pharmaceutically acceptable salt thereof;
wherein when a term is used with the "substituted" modifier, or the phrase "substituted version" is used, one or more hydrogen atom has been independently replaced by -OH, -F, -Cl, -Br, -I, -NH$_2$, -NO$_2$, -CO$_2$H, -CO$_2$CH$_3$, -CN, -SH, -OCH$_3$, -OCH$_2$CH$_3$, -C(O)CH$_3$, -NHCH$_3$, -NHCH$_2$CH$_3$, -N(CH$_3$)$_2$, -C(O)NH$_2$, -C(O)NHCH$_3$, -C(O)N(CH$_3$)$_2$, -OC(O)CH$_3$, -NHC(O)CH$_3$, -S(O)$_2$OH, or -S(O)$_2$NH$_2$.

**6.** The composition for use of claim 2, wherein the aminolipid delivery compound is a compound of the formula:

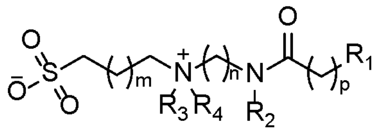

(II)

wherein:

$R_1$ is a group of the formula:

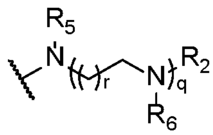

wherein:
$R_5$, $R_6$, and $R_2$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:

R' and R" are each independently hydrogen, alkyl$_{(C\leq8)}$, substituted alkyl$_{(C\leq8)}$, or -$Z_2$A'$R_7$; wherein:

$Z_2$ is alkanediyl$_{(C\leq4)}$ or substituted alkanediyl$_{(C\leq4)}$;
A' is -$CHR_j$-, -C(O)O-, or -C(O)$NR_b$-;

$R_b$ is hydrogen, alkyl$_{(C\leq6)}$, or substituted alkyl$_{(C\leq6)}$; and
$R_j$ is hydrogen, halo, hydroxy, acyloxy$_{(C\leq24)}$, or substituted acyloxy$_{(C\leq24)}$;

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $R_2$ are each independently -$Z_3$A"$R_8$; wherein:

$Z_3$ is alkanediyl$_{(C\leq4)}$ or substituted alkanediyl$_{(C\leq4)}$;
A" is -$CHR_k$-, -C(O)O-, or -C(O)$NR_l$-;

$R_l$ is hydrogen, alkyl$_{(C\leq6)}$, or substituted alkyl$_{(C\leq6)}$; and
$R_k$ is hydrogen, halo, hydroxy, acyloxy$_{(C\leq24)}$, or substituted acyloxy$_{(C\leq24)}$; and

$R_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and
r is 1, 2, 3, or 4;

$R_a$, $R_3$, and $R_4$ are each independently hydrogen, alkyl$_{(C\leq6)}$, or substituted alkyl$_{(C\leq6)}$; and
m, n, and p are each independently an integer selected from 0, 1, 2, 3, 4, 5, or 6;

or a pharmaceutically acceptable salt thereof;
wherein when a term is used with the "substituted" modifier, or the phrase "substituted version" is used, one or more hydrogen atom has been independently replaced by -OH, -F, -Cl, -Br, -I, -$NH_2$, -$NO_2$, -$CO_2H$, -$CO_2CH_3$, -CN, -SH, -$OCH_3$, -$OCH_2CH_3$, -C(O)$CH_3$, -$NHCH_3$, -$NHCH_2CH_3$, -$N(CH_3)_2$, -C(O)$NH_2$, -C(O)$NHCH_3$, -C(O)$N(CH_3)_2$, -OC(O)$CH_3$, -NHC(O)$CH_3$, -$S(O)_2OH$, or -$S(O)_2NH_2$.

**7.** The composition for use according to either claim 3 or claim 4, wherein the terminating group is further defined by the formula:

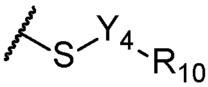 (VIII)

wherein:

$Y_4$ is alkanediyl$_{(C\leq18)}$, alkenediyl$_{(C\leq18)}$, or a substituted version of either of these groups; such as wherein $Y_4$ is alkanediyl$_{(C\leq18)}$; and
$R_{10}$ is hydrogen.

**8.** The composition for use according to any one of claims 3, 4 and 7, wherein the core is further defined by the formula:

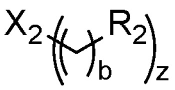 (III)

wherein:

$X_2$ is $N(R_5)_y$;

$R_5$ is hydrogen or alkyl$_{(C\leq8)}$, or substituted alkyl$_{(C\leq18)}$; and
y is 0, 1, or 2, provided that the sum of y and z is 3;

$R_2$ is amino, hydroxy, or mercapto, or alkylamino$_{(C\leq12)}$, dialkylamino$_{(C\leq12)}$, or a substituted version of either of these groups;
b is 1, 2, 3, 4, 5, or 6; and
z is 1, 2, 3; provided that the sum of z and y is 3.

**9.** The composition for use of claim 8, wherein:

(i) $X_2$ is $NR_5$ and $R_5$ is hydrogen or alkyl$_{(C\leq8)}$;
optionally wherein $R_5$ is methyl; and/or
(ii) z is 3; and/or
(iii) $R_2$ is amino; and/or
(iv) b is 2 or 3; and/or
(v) wherein the core is further defined as:

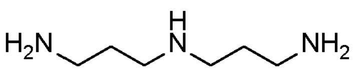, 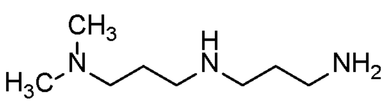, 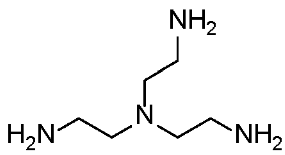,

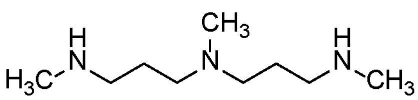, 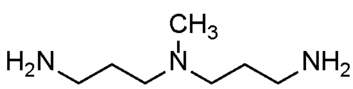, 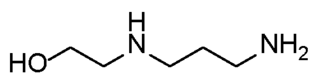, or

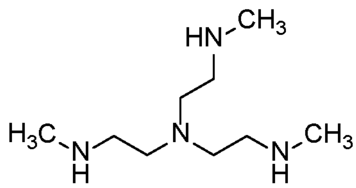.

**10.** The composition for use according to any one of claims 3, 4 and 7, wherein the core is further defined as:

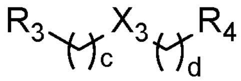
(IV)

wherein:
$X_3$ is $-NR_6-$, -O-, or alkylaminodiyl$_{(C\leq8)}$, alkoxydiyl$_{(C\leq8)}$, arenediyl$_{(C\leq8)}$, heteroarenediyl$_{(C\leq8)}$, heterocycloalkanediyl$_{(C\leq8)}$, or a substituted version of any of these groups, wherein $R_6$ is hydrogen, alkyl$_{(C\leq8)}$, or substituted alkyl$_{(C\leq8)}$;

$R_3$ and $R_4$ are each independently amino, hydroxy, or mercapto, or alkylamino$_{(C\leq12)}$, dialkylamino$_{(C\leq12)}$, wherein, optionally, either of these groups is substituted by one or more substituents selected from the group consisting of: -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$, and $-S(O)_2NH_2$;
wherein:
c and d are each independently 1, 2, 3, 4, 5, or 6;

optionally wherein the core is further defined as:

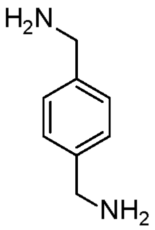,
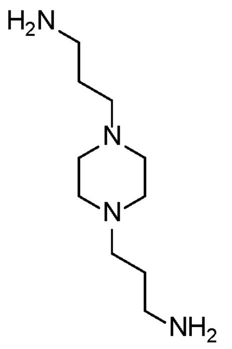,
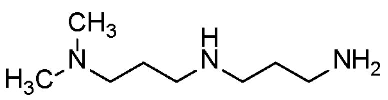,
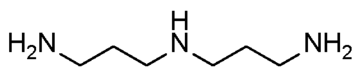,

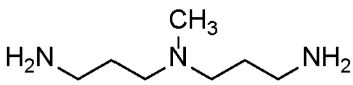,
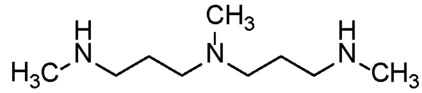,
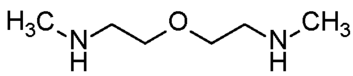,

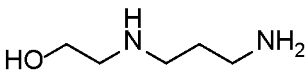,
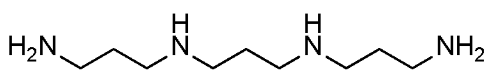,

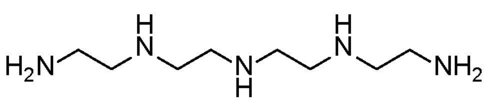,
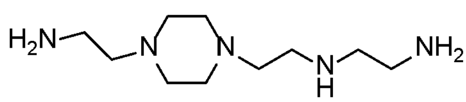,

or

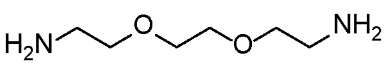.

**11.** The composition for use of claim 2, wherein the aminolipid delivery compound is further defined as:

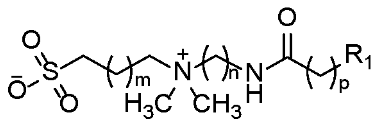
(II)

wherein:

$R_1$ is a group of the formula:

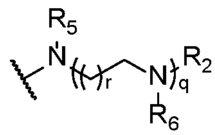

wherein:

$R_5$, $R_6$, and $R_2$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:

R' and R" are each independently hydrogen, alkyl$_{(C\leq8)}$, substituted alkyl$_{(C\leq8)}$, or -$Z_2$A'$R_7$; wherein:

$Z_2$ is alkanediyl$_{(C\leq4)}$ or substituted alkanediyl$_{(C\leq4)}$;
A' is -$CHR_j$-, -C(O)O-, or -C(O)$NR_b$-;

$R_b$ is hydrogen, alkyl$_{(C\leq6)}$, or substituted alkyl$_{(C\leq6)}$; and
$R_j$ is hydrogen, halo, hydroxy, acyloxy$_{(C\leq24)}$, or substituted acyloxy$_{(C\leq24)}$;

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $R_2$ are each independently -$Z_3$A"$R_8$; wherein:

$Z_3$ is alkanediyl$_{(C\leq4)}$ or substituted alkanediyl$_{(C\leq4)}$;
A" is -$CHR_k$-, -C(O)O-, or -C(O)$NR_l$-;

$R_l$ is hydrogen, alkyl$_{(C\leq6)}$, or substituted alkyl$_{(C\leq6)}$; and
$R_k$ is hydrogen, halo, hydroxy, acyloxy$_{(C\leq24)}$, or substituted acyloxy$_{(C\leq24)}$; and

$R_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and
r is 1, 2, 3, or 4; and

m, n, and p are each independently an integer selected from 0, 1, 2, 3, 4, 5, or 6;

or a pharmaceutically acceptable salt thereof;
optionally wherein the aminolipid delivery compound is further defined as:

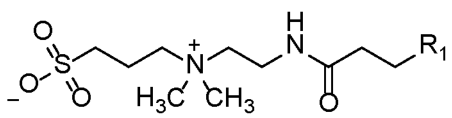
(III)

wherein:

$R_1$ is a group of the formula:

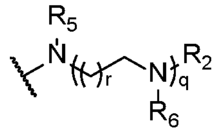

wherein:

$R_5$, $R_6$, and $R_2$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:
R' and R" are each independently are each independently hydrogen, alkyl$_{(C\leq8)}$, substituted alkyl$_{(C\leq8)}$, or -$Z_2$A'$R_7$; wherein:

$Z_2$ is alkanediyl$_{(C\leq4)}$ or substituted alkanediyl$_{(C\leq4)}$;
A' is -$CHR_j$-, -C(O)O-, or -C(O)$NR_b$-;

$R_b$ is hydrogen, alkyl$_{(C\leq6)}$, or substituted alkyl$_{(C\leq6)}$; and
$R_j$ is hydrogen, halo, hydroxy, acyloxy$_{(C\leq24)}$, or substituted acyloxy$_{(C\leq24)}$;

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $R_2$ are each independently -$Z_3$A"$R_8$; wherein:

$Z_3$ is alkanediyl$_{(C\leq4)}$ or substituted alkanediyl$_{(C\leq4)}$;
A" is -$CHR_k$-, -C(O)O-, or -C(O)$NR_l$-;

$R_l$ is hydrogen, alkyl$_{(C\leq6)}$, or substituted alkyl$_{(C\leq6)}$; and
$R_k$ is hydrogen, halo, hydroxy, acyloxy$_{(C\leq24)}$, or substituted acyloxy$_{(C\leq24)}$; and

$R_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and
r is 1, 2, 3, or 4;

or a pharmaceutically acceptable salt thereof;
wherein when a term is used with the "substituted" modifier, or the phrase "substituted version" is used, one or more hydrogen atom has been independently replaced by -OH, -F, -Cl, -Br, -I, -$NH_2$, -$NO_2$, -$CO_2H$, -$CO_2CH_3$, -CN, -SH, -$OCH_3$, -$OCH_2CH_3$, -C(O)$CH_3$, -$NHCH_3$, -$NHCH_2CH_3$, -$N(CH_3)_2$, -C(O)$NH_2$, -C(O)$NHCH_3$, -C(O)$N(CH_3)_2$, -OC(O)$CH_3$, -NHC(O)$CH_3$, -$S(O)_2OH$, or -$S(O)_2NH_2$.

**12.** The composition for use according to claim 6, wherein:

(i) $R_3$ is alkyl$_{(C\leq8)}$ or substituted alkyl$_{(C\leq8)}$; and/or
(ii) $R_4$ is alkyl$_{(C\leq8)}$ or substituted alkyl$_{(C\leq8)}$; and/or
(iii) m is 1 or 2; and/or
(iv) n is 2 or 3; and/or
(v) p is 1, 2, or 3; and/or
(vi) $R_1$ is a group of the formula:

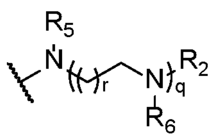

wherein:

$R_5$, $R_6$, and $R_2$ are each independently hydrogen or alkyl$_{(C\leq8)}$, -alkanediyl$_{(C\leq6)}$-$NH_2$, -alkanediyl$_{(C\leq6)}$-alkylamino$_{(C\leq8)}$, -alkanediyl$_{(C\leq6)}$-dialkylamino$_{(C\leq12)}$, -alkanediyl$_{(C\leq6)}$-NR'R", or a substituted version of any of these groups wherein:
R' and R" are each independently hydrogen, alkyl$_{(C\leq8)}$, substituted alkyl$_{(C\leq8)}$, -$Z_2$A'$R_7$; wherein:

$Z_2$ is alkanediyl$_{(C\leq4)}$ or substituted alkanediyl$_{(C\leq4)}$;
A' is -$CHR_j$-, -C(O)O-, or -C(O)$NR_b$-;

$R_b$ is hydrogen, alkyl$_{(C\leq6)}$, or substituted alkyl$_{(C\leq6)}$; and
$R_j$ is hydrogen, halo, hydroxy, acyloxy$_{(C\leq24)}$, or substituted acyloxy$_{(C\leq24)}$;

$R_7$ is alkyl$_{(C6-24)}$, substituted alkyl$_{(C6-24)}$, alkenyl$_{(C6-24)}$, substituted alkenyl$_{(C6-24)}$; or

$R_5$, $R_6$, and $X_1$ are each independently -$Z_3$A"$R_8$; wherein:

$Z_3$ is alkanediyl$_{(C\leq4)}$ or substituted alkanediyl$_{(C\leq4)}$;
A" is -$CHR_k$-, -C(O)O-, or -C(O)$NR_l$-;

$R_l$ is hydrogen, alkyl$_{(C\leq6)}$, or substituted alkyl$_{(C\leq6)}$; and
$R_k$ is hydrogen, halo, hydroxy, acyloxy$_{(C\leq24)}$, or substituted acyloxy$_{(C\leq24)}$; and

$R_8$ is alkyl$_{(C6-24)}$, substituted alkyl$_{(C6-24)}$, alkenyl$_{(C6-24)}$, substituted alkenyl$_{(C6-24)}$;

q is 1, 2, or 3; and
r is 1, 2, 3, or 4;
preferably wherein:

(a) q is 1 or 2; and/or
(b) $R_5$ is -$Z_3$A"$R_8$; wherein:

$Z_3$ is alkanediyl$_{(C\leq4)}$ or substituted alkanediyl$_{(C\leq4)}$;
A" is -$CHR_k$-, -C(O)O-, or -C(O)$NR_l$-;
$R_l$ is hydrogen, alkyl$_{(C\leq6)}$, or substituted alkyl$_{(C\leq6)}$; and
$R_k$ is hydrogen, halo, hydroxy, acyloxy$_{(C\leq24)}$, or substituted acyloxy$_{(C\leq24)}$; and
$R_8$ is alkyl$_{(C6-24)}$, substituted alkyl$_{(C6-24)}$, alkenyl$_{(C6-24)}$, substituted alkenyl$_{(C6-24)}$; and/or

(c) $R_6$ is -$Z_3$A"$R_8$; wherein:

$Z_3$ is alkanediyl$_{(C\leq4)}$ or substituted alkanediyl$_{(C\leq4)}$;
A" is -$CHR_k$-, -C(O)O-, or -C(O)$NR_l$-;
$R_l$ is hydrogen, alkyl$_{(C\leq6)}$, or substituted alkyl$_{(C\leq6)}$; and
$R_k$ is hydrogen, halo, hydroxy, acyloxy$_{(C\leq24)}$, or substituted acyloxy$_{(C\leq24)}$; and
$R_8$ is alkyl$_{(C6-24)}$, substituted alkyl$_{(C6-24)}$, alkenyl$_{(C6-24)}$, substituted alkenyl$_{(C6-24)}$; and/or

(d) $R_2$ is -$Z_3$A"$R_8$; wherein:

$Z_3$ is alkanediyl$_{(C\leq4)}$ or substituted alkanediyl$_{(C\leq4)}$;
A" is -$CHR_k$-, -C(O)O-, or -C(O)$NR_l$-;
$R_l$ is hydrogen, alkyl$_{(C\leq6)}$, or substituted alkyl$_{(C\leq6)}$; and
$R_k$ is hydrogen, halo, hydroxy, acyloxy$_{(C\leq24)}$, or substituted acyloxy$_{(C\leq24)}$; and
$R_8$ is alkyl$_{(C6-24)}$, substituted alkyl$_{(C6-24)}$, alkenyl$_{(C6-24)}$, substituted alkenyl$_{(C6-24)}$.

**13.** The composition for use according to any one of claims 2 to 12, wherein the tRNA is a suppressor tRNA; optionally wherein

the tRNA is a tRNA that delivers an amino acid into a protein instead of terminating translation; or

the tRNA is a tRNA amber suppressor, a tRNA opal suppressor, a tRNA ochre suppressor, or a tRNA frameshift suppressor; or
the tRNA corrects a nonsense mutation in cystic fibrosis transmembrane conductance regulator protein.

**14.** The composition for use according to any one of claims 2 to 13, wherein the composition further comprises a steroid or steroid derivative; and/or

wherein the composition further comprises a phospholipid; and/or
wherein the composition further comprises a PEG lipid.

**15.** A composition according to claim 1, or a composition for use according to any of claims 2 to 14, for use in one or more of the following:

(i) restoring expression of p53 protein;
(ii) delivering an amino acid into a protein instead of terminating translation; and
(iii) correcting a nonsense mutation in a cystic fibrosis transmembrane conductance regulator (CFTR) protein.

**Patentansprüche**

**1.** Zusammensetzung, umfassend:

(A) eine Transferribonukleinsäure (tRNA); und
(B) eine Verbindung zur Aminolipidabgabe;
wobei die Verbindung zur Aminolipidabgabe ein Nanopartikel bildet und die Verbindung zur Aminolipidabgabe ist:

(i) ein Dendrimer mit der Formel:

Kern-(Wiederholungseinheit)$_n$-terminierende Gruppe (I)

wobei der Kern mit der Wiederholungseinheit verbunden ist, indem ein oder mehrere Wasserstoffatome aus dem Kern entfernt und durch die Wiederholungseinheit ersetzt werden und wobei:

der Kern die folgende Formel aufweist:

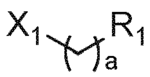
(II)

wobei:

$X_1$ Amino oder Alkylamino$_{(C\leq12)}$, Dialkylamino$_{(C\leq12)}$, Heterocycloalkyl$_{(C\leq12)}$, Heteroaryl$_{(C\leq12)}$ oder eine substituierte Version davon ist;
$R_1$ Amino, Hydroxy oder Mercapto oder Alkylamino$_{(C\leq12)}$, Dialkylamino$_{(C\leq12)}$ oder eine substituierte Version einer dieser Gruppen ist; und
a 1, 2, 3, 4, 5 oder 6 ist; oder

der Kern die folgende Formel aufweist:

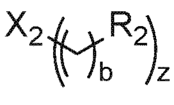
(III)

wobei:

$X_2$ $N(R_5)_y$ ist;
$R_5$ Wasserstoff, $Alkyl_{(C\leq18)}$ oder substituiertes $Alkyl_{(C\leq18)}$ ist; und
y 0, 1 oder 2 ist, vorausgesetzt, dass die Summe von y und z 3 ist;
$R_2$ Amino, Hydroxy oder Mercapto oder $Alkylamino_{(C\leq12)}$, $Dialkylamino_{(C\leq12)}$ oder eine substituierte Version einer dieser Gruppen ist;
b 1, 2, 3, 4, 5 oder 6 ist; und
z 1, 2, 3 ist; vorausgesetzt, dass die Summe von z und y 3 ist; oder

der Kern die folgende Formel aufweist:

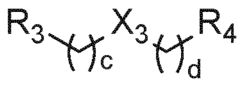
(IV)

wobei:

$X_3$ $-NR_6-$, -O- oder $Alkylaminodiyl_{(C\leq8)}$, $Alkoxydiyl_{(C\leq8)}$, $Arendiyl_{(C\leq8)}$, $Heteroarendiyl_{(C\leq8)}$, $Heterocycloalkandiyl_{(C\leq8)}$ oder eine substituierte Version einer dieser Gruppen ist, wobei $R_6$ Wasserstoff, $Alkyl_{(C\leq8)}$ oder substituiertes $Alkyl_{(C\leq8)}$ ist;
$R_3$ und $R_4$ jeweils unabhängig voneinander Amino, Hydroxy oder Mercapto oder $Alkylamino_{(C\leq12)}$, $Dialkylamino_{(C\leq12)}$ sind, wobei optional jede dieser Gruppen durch einen oder mehrere Substituenten substituiert ist, die ausgewählt sind aus der Gruppe bestehend aus: -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, - $NHCH_3$, $-NHCH_2CH_3$, $-N(CH_3)_2$, $-C(O)NH_2$, - $C(O)NHCH_3$, $-C(O)N(CH_3)_2$, $-OC(O)CH_3$, - $NHC(O)CH_3$, $-S(O)_2OH$ und $-S(O)_2NH_2$;

wobei:
c und d jeweils unabhängig 1, 2, 3, 4, 5 oder 6 sind; oder
der Kern $Alkylamin_{(C\leq18)}$, $Dialkylamin_{(C\leq36)}$, $Heterocycloalkan_{(C\leq12)}$ oder eine substituierte Version einer dieser Gruppen ist;
wobei die Wiederholungseinheit eine abbaubare Diacylgruppe und eine Linkergruppe umfasst;
die abbaubare Diacylgruppe die folgende Formel aufweist:

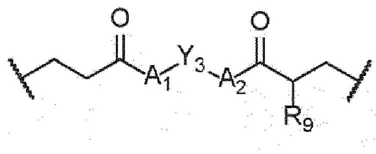
(VII)

wobei:

$A_1$ und $A_2$ jeweils unabhängig voneinander -O-, -S- oder $-NR_a-$ sind, wobei:
$R_2$ Wasserstoff, $Alkyl_{(C\leq6)}$ oder substituiertes $Alkyl_{(C\leq6)}$ ist;
$Y_3$ $Alkanediyl_{(C\leq12)}$, $Alkendiyl_{(C\leq12)}$, $Arendiyl_{(C\leq12)}$ oder eine substituierte Version einer dieser Gruppen ist; oder eine Gruppe mit der Formel:

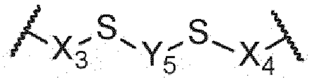
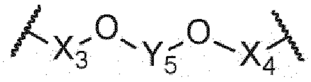

oder

wobei:

$X_3$ und $X_4$ $Alkanediyl_{(C\leq12)}$, $Alkendiyl_{(C\leq12)}$, $Arendiyl_{(C\leq12)}$ oder eine substituierte Version einer dieser Gruppen sind;
$Y_5$ eine kovalente Bindung, $Alkandiyl_{(C\leq12)}$, $Alkendiyl_{(C\leq12)}$, $Arendiyl_{(C\leq12)}$ oder eine substituierte Version einer dieser Gruppen ist; und

$R_9$ Alkyl$_{(C\leq 8)}$ oder substituiertes Alkyl$_{(C\leq 8)}$ ist;

die Linkergruppe die folgende Formel aufweist:

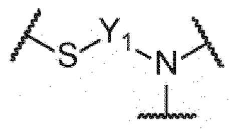
(VI)

wobei:

$Y_1$ Alkanediyl $_{(C\leq 12)}$, Alkendiyl $_{(C\leq 12)}$, Arendiyl$_{(C\leq 12)}$ oder eine substituierte Version einer dieser Gruppen ist; und
wobei, wenn die Wiederholungseinheit eine Linkergruppe umfasst, die Linkergruppe an eine abbaubare Diacylgruppe sowohl an den Stickstoff- als auch an den Schwefelatomen der Linkergruppe gebunden ist, wobei die erste Gruppe in der Wiederholungseinheit eine abbaubare Diacylgruppe ist, wobei für jede Linkergruppe, die nächste Gruppe zwei abbaubare Diacylgruppen umfasst, die an das Stickstoffatom der Linkergruppe gebunden sind; und wobei n die Anzahl der Linkergruppen ist, die in der sich wiederholenden Einheit vorhanden sind; und

die terminierende Gruppe die folgende Formel aufweist:

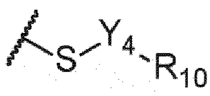
(VIII)

wobei:

$Y_4$ Alkanediyl$_{(C\leq 18)}$, Alkenediyl$_{(C\leq 18)}$ oder eine substituierte Version einer dieser Gruppen ist;
$R_{10}$ Wasserstoff, Carboxy, Hydroxy oder
Aryl$_{(C\leq 12)}$, Alkylamino$_{(C\leq 12)}$, Dialkylamino$_{(C\leq 12)}$, *N*-Heterocycloalkyl$_{(C\leq 12)}$, -C(O)N($R_{11}$)-Alkanediyl$_{(C\leq 6)}$-Heterocycloalkyl$_{(C\leq 12)}$, - C(O)-Alkylamino$_{(C\leq 12)}$, -C(O)-Dialkylamino$_{(C\leq 12)}$, -C(O)-N-Heterocycloalkyl$_{(C\leq 12)}$ ist, wobei:
$R_{11}$ Wasserstoff, Alkyl$_{(C\leq 6)}$ oder substituiertes Alkyl$_{(C\leq 6)}$ ist;
wobei die letzte abbaubare Diacylgruppe in der Kette an eine terminierende Gruppe angebunden ist;
n 0, 1, 2, 3, 4, 5 oder 6 ist; oder (ii)eine Verbindung der Formel:

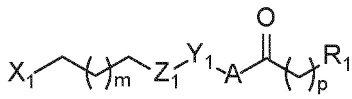
(I)

wobei:
$X_1$ $-S(O)_2O^-$, $-OP(O)OR_eO^-$, $-(CHR_f)_ZC(O)O^-$ oder - $NR_gR_hR_i^+$ ist, wobei:

$R_e$, $R_g$, $R_h$ und $R_i$ jeweils unabhängig voneinander Wasserstoff, Alkyl$_{(C\leq 6)}$ oder substituiertes Alkyl$_{(C\leq 6)}$ sind;
$R_f$ Wasserstoff, Amino, Hydroxy oder Alkyl$_{(C\leq 12)}$, Aryl$_{(C\leq 12)}$, Aralkyl$_{(C\leq 12)}$, Heteroaryl$_{(C\leq 12)}$, Acyl$_{(C\leq 12)}$, Alkoxy$_{(C\leq 12)}$, Acyloxy$_{(C\leq 12)}$, Amido$_{(C\leq 12)}$, Alkoxy$_{(C\leq 12)}$, Alkoxy$_{(C\leq 12)}$ oder eine substituierte Version einer der letzten zehn Gruppen ist; und
z 1, 2, 3 oder 4 ist;

$Y_1$ Alkanediyl$_{(C\leq 12)}$, Alkendiyl$_{(C\leq 12)}$, Arendiyl$_{(C\leq 12)}$, Heteroarendiyl$_{(C12)}$, Heterocycloalkandiyl$_{(C\leq 12)}$, -Alkandiyl$_{(C\leq 8)}$-heterocycloalkandiyl$_{(C\leq 12)}$, -Alkandiyl$_{(C\leq 8)}$-heterocycloalkandiyl$_{(C\leq 12)}$ -alkandiyl$_{(C\leq 8)}$, - Alkandiyl$_{(C\leq 8)}$ -heteroarenediyl$_{(C\leq 12)}$, - Alkandiyl$_{(C\leq 8)}$ -heteroaren-diyl$_{(C\leq 12)}$-alkandiyl$_{(C\leq 8)}$ oder eine substituierte Version einer dieser Gruppen ist;

$Z_1$ $-N^+R_3R_4-$ oder $-OP(O)O^-O-$
A $-NR_a-$, -S- oder -O- ist; wobei:
$R_a$ Wasserstoff, Alkyl$_{(C\leq6)}$ oder substituiertes Alkyl$_{(C\leq6)}$ ist oder $R_a$ zusammen mit entweder $R_3$ oder $R_4$ genommen wird und Alkandiyl$_{(C\leq8)}$, Alkenediyl$_{(C\leq8)}$, Alkoxydiyl$_{(C\leq8)}$, Alkylaminodiyl$_{(C\leq8)}$ oder eine substituierte Version einer dieser Gruppen ist;
R,_ eine Gruppe der folgenden Formel ist:

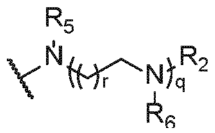

wobei:
$R_5$, $R_6$ und $R_2$ jeweils unabhängig voneinander Wasserstoff oder Alkyl $_{(C\leq8)}$, - Alkandiyl$_{(C\leq6)}$-$NH_2$, -Alkandiyl$_{(C\leq6)}$-alkylamino$_{(C\leq8)}$, -Alkandiyl$_{(C\leq6)}$-dialkylamino$_{(C\leq12)}$, -Alkandiyl$_{(C\leq6)}$-NR'R" oder eine substituierte Version einer dieser Gruppen sind, wobei:
R' und R" jeweils unabhängig voneinander Wasserstoff, Alkyl$_{(C\leq8)}$, substituiertes Alkyl$_{(C\leq8)}$ oder $-Z_2A'R_7$ sind; wobei:

$Z_2$ Alkanediyl$_{(C\leq6)}$, substituiertes Alkanediyl$_{(C\leq6)}$ oder eine Gruppe der folgenden Formel ist:

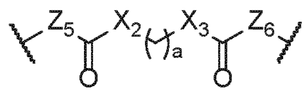

wobei:

$Z_5$ und $Z_6$ jeweils unabhängig voneinander Alkandiyl$_{(C\leq6)}$ oder substituiertes Alkandiyl$_{(C\leq6)}$ sind;
$X_2$ und $X_3$ jeweils unabhängig voneinander -O-, -S- oder $-NR_m-$ sind; wobei:
$R_m$ Wasserstoff, Alkyl$_{(C\leq6)}$ oder substituiertes Alkyl$_{(C\leq6)}$ ist; und
a 0, 1, 2, 3, 4, 5 oder 6 ist;

A' $-CHR_j$, -C(O)O- oder $-C(O)NR_b-$ ist;

$R_b$ Wasserstoff, Alkyl$_{(C\leq6)}$ oder substituiertes Alkyl$_{(C\leq6)}$ ist; und
$R_j$ Wasserstoff, Halogen, Hydroxy, Acyloxy$_{(C\leq24)}$ oder substituiertes Acyloxy$_{(C\leq24)}$ ist;

$R_7$ Alkyl$_{(C6-24)}$, substituiertes Alkyl$_{(C6-24)}$, Alkenyl$_{(C6-24)}$, substituiertes Alkenyl$_{(C6-24)}$ ist; oder
$R_5$, $R_6$ und $R_2$ jeweils unabhängig voneinander $-Z_3A''R_8$ sind; wobei:

$Z_3$ Alkanediyl$_{(C\leq6)}$, substituiertes Alkanediyl$_{(C\leq6)}$ oder eine Gruppe der folgenden Formel ist:

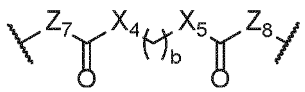

wobei:

$Z_7$ und $Z_8$ jeweils unabhängig voneinander Alkandiyl$_{(C<6)}$ oder substituiertes Alkandiyl$_{(C\leq6)}$ sind;
$X_4$ und $X_5$ jeweils unabhängig voneinander -O-, -S- oder $-NR_n-$ sind; wobei:
$R_n$ Wasserstoff, Alkyl$_{(C\leq6)}$ oder substituiertes Alkyl$_{(C\leq6)}$ ist; und
b 0, 1, 2, 3, 4, 5 oder 6 ist;

A" $-CHR_k-$, -S-, -C(O)O- oder $-C(O)NR_1$-ist;

$R_1$ Wasserstoff, Alkyl$_{(C\leq6)}$ oder substituiertes Alkyl$_{(C\leq6)}$ ist; und
$R_k$ Wasserstoff, Halogen, Hydroxy, Acyloxy$_{(C\leq24)}$ oder substituiertes Acyloxy$_{(C\leq24)}$ ist; und

$R_8$ Alkyl$_{(C6-24)}$, substituiertes Alkyl$_{(C6-24)}$, Alkenyl$_{(C6-24)}$, substituiertes Alkenyl$_{(C6-24)}$ ist;

q 1, 2 oder 3 ist; und
r 1, 2, 3 oder 4 ist;

$R_1$ eine Gruppe der folgenden Formel ist:

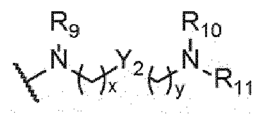

wobei:

$Y_2$ Arendiyl$_{(C\leq12)}$, Heterocycloalkandiyl$_{(C\leq12)}$, Heteroarendiyl$_{(C\leq12)}$, Alkoxydiyl$_{(C\leq12)}$ oder eine substituierte Version einer dieser Gruppen ist;
$R_9$, $R_{10}$ und $R_{11}$ jeweils unabhängig voneinander Wasserstoff, Alkyl$_{(C\leq8)}$, substituiertes Alkyl$_{(C\leq8)}$ oder -$Z_4$A' "$R_{12}$ sind; wobei:

$Z_4$ Alkanediyl$_{(C\leq6)}$, substituiertes Alkanediyl$_{(C\leq6)}$ oder eine Gruppe der folgenden Formel ist:

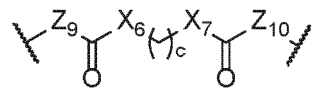

wobei:

$Z_9$ und $Z_{10}$ jeweils unabhängig voneinander Alkandiyl$_{(C\leq6)}$ oder substituiertes Alkandiyl$_{(C\leq6)}$ sind;
$X_6$ und $X_7$ jeweils unabhängig voneinander -O-, -S- oder -$NR_o$- sind; wobei:
$R_o$ Wasserstoff, Alkyl$_{(C\leq6)}$ oder substituiertes Alkyl$_{(C\leq6)}$ ist; und
c 0, 1, 2, 3, 4, 5 oder 6 ist;

A'" -$CHR_k$-, -S-, -C(O)O- oder -C(O)$NR_1$-ist;

$R_1$ Wasserstoff, Alkyl$_{(C\leq6)}$ oder substituiertes Alkyl$_{(C\leq6)}$ ist; und
$R_k$ Wasserstoff, Halogen, Hydroxy, Acyloxy$_{(C\leq24)}$ oder substituiertes Acyloxy$_{(C\leq24)}$ ist; und

$R_{12}$ Alkyl$_{(C6-24)}$, substituiertes Alkyl$_{(C6-24)}$, Alkenyl$_{(C6-24)}$, substituiertes Alkenyl$_{(C6-24)}$ ist; und
x und y 0, 1, 2, 3 oder 4 sind;

$R_3$ und $R_4$ jeweils unabhängig voneinander Wasserstoff, Alkyl$_{(C\leq6)}$ oder substituiertes Alkyl$_{(C\leq6)}$ sind oder $R_3$ oder $R_4$ zusammen mit $R_a$ genommen werden und Alkandiyl$_{(C\leq8)}$, Alkenediyl$_{(C\leq8)}$, Alkoxydiyl$_{(C\leq8)}$, Alkylaminodiyl$_{(C\leq8)}$ oder eine substituierte Version einer dieser Gruppen sind; und
m, n und p jeweils unabhängig voneinander eine ganze Zahl, ausgewählt aus 0, 1, 2, 3, 4, 5 oder 6, sind; vorausgesetzt, wenn $X_1$ positiv geladen ist, dann ist $Z_1$ negativ geladen und wenn $X_1$ negativ geladen ist, dann ist $Z_1$ positiv geladen;

oder ein pharmazeutisch verträgliches Salz davon;
wobei, wenn ein Begriff mit dem Modifikator "substituiert" verwendet wird oder der Ausdruck "substituierte Version" verwendet wird, ein oder mehrere Wasserstoffatome unabhängig voneinander durch -OH, -F, -Cl, -Br, -I, -$NH_2$, -$NO_2$, -$CO_2H$, -$CO_2CH_3$, -CN, -SH, -$OCH_3$, -$OCH_2CH_3$, -C(O)$CH_3$, -$NHCH_3$, -NHCH2$CH_3$, -N$(CH_3)_2$, -C(O)$NH_2$, -C(O)$NHCH_3$, -C(O)N$(CH_3)$ $_2$, -OC(O)$CH_3$, -NHC(O)$CH_3$, -S$(O)_2$OH oder -S$(O)_2NH_2$ ersetzt worden sind.

**2.** Zusammensetzung zur Verwendung in der Medizin, wobei die Zusammensetzung verwendet wird, um eine Transfer-RNA (tRNA) in eine Zelle abzugeben, die Zusammensetzung umfassend:

(a) die tRNA; und

(b) eine Verbindung zur Aminolipidabgabe;

wobei die Verbindung zur Aminolipidabgabe ein Nanopartikel bildet.

**3.** Zusammensetzung zur Verwendung nach Anspruch 2, wobei die Verbindung zur Aminolipidabgabe ein Dendrimer mit der folgenden Formel ist:

Kern-(Wiederholungseinheit)n-terminierende Gruppe (I)

wobei der Kern mit der Wiederholungseinheit verbunden ist, indem ein oder mehrere Wasserstoffatome aus dem Kern entfernt und durch die Wiederholungseinheit ersetzt werden und wobei:

der Kern die folgende Formel aufweist:

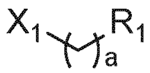
(II)

wobei:

$X_1$ Amino oder Alkylamino$_{(C\leq12)}$, Dialkylamino$_{(C\leq12)}$, Heterocycloalkyl$_{(C\leq12)}$, Heteroaryl$_{(C\leq12)}$ oder eine substituierte Version davon ist;
$R_1$ Amino, Hydroxy oder Mercapto oder Alkylamino$_{(C\leq12)}$, Dialkylamino$_{(C\leq12)}$ oder eine substituierte Version einer dieser Gruppen ist; und
a 1, 2, 3, 4, 5 oder 6 ist; oder

der Kern die folgende Formel aufweist:

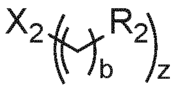
(III)

wobei:
$X_2$ $N(R_5)_y$ ist:

$R_5$ Wasserstoff, Alkyl$_{(C\leq18)}$ oder substituiertes Alkyl$_{(C\leq18)}$ ist; und
y 0, 1 oder 2 ist, vorausgesetzt, dass die Summe von y und z 3 ist;
$R_2$ Amino, Hydroxy oder Mercapto oder Alkylamino$_{(C\leq12)}$, Dialkylamino$_{(C\leq12)}$ oder eine substituierte Version einer dieser Gruppen ist;
b 1, 2, 3, 4, 5 oder 6 ist; und
z 1, 2, 3 ist; vorausgesetzt, dass die Summe von z und y 3 ist; oder

der Kern die folgende Formel aufweist:

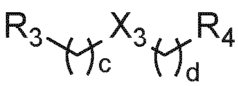
(IV)

wobei:

$X_3$ $-NR_6-$, -O- oder Alkylaminodiyl$_{(C\leq8)}$, Alkoxydiyl$_{(C\leq8)}$, Arendiyl$_{(C\leq8)}$, Heteroarendiyl$_{(C\leq8)}$, Heterocycloalkandiyl$_{(C\leq8)}$ oder eine substituierte Version einer dieser Gruppen ist, wobei $R_6$ Wasserstoff, Alkyl$_{(C\leq8)}$ oder substituiertes Alkyl$_{(C\leq8)}$ ist;
$R_3$ und $R_4$ jeweils unabhängig voneinander Amino, Hydroxy oder Mercapto oder Alkylamino$_{(C\leq12)}$, Dialky-

lamino$_{(C\leq 12)}$ sind, wobei optional jede dieser Gruppen durch einen oder mehrere Substituenten substituiert ist, die ausgewählt sind aus der Gruppe bestehend aus: -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, - $NHCH_3$, $-NHCH_2CH_3$, $-N(CH3)_2$, $-C(O)NH_2$, - $C(O)NHCH_3$, $-C(O)N(CH_3)_2$, $-OC(O)CH_3$, - $NHC(O)CH_3$, $-S(O)_2OH$ und $-S(O)_2NH_2$;
wobei:
c und d jeweils unabhängig 1, 2, 3, 4, 5 oder 6 sind; oder

der Kern Alkylamin$_{(C\leq 18)}$, Dialkylamin$_{(C\leq 36)}$, Heterocycloalkan$_{(C\leq 12)}$ oder eine substituierte Version einer dieser Gruppen ist;
wobei die Wiederholungseinheit eine abbaubare Diacylgruppe und eine Linkergruppe umfasst;
die abbaubare Diacylgruppe die folgende Formel aufweist:

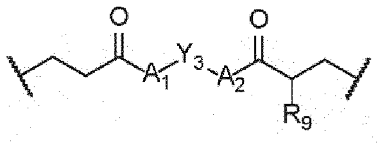 (VII)

wobei:

$A_1$ und $A_2$ jeweils unabhängig voneinander -O-, -S- oder $-NR_a$- sind, wobei:
$R_a$ Wasserstoff, Alkyl$_{(C\leq 6)}$ oder substituiertes Alkyl$_{(C\leq 6)}$ ist;
$Y_3$ Alkandiyl$_{(C\leq 12)}$, Alkendiyl$_{(C\leq 12)}$, Arendiyl$_{(C\leq 12)}$ oder eine substituierte Version einer dieser Gruppen ist; oder eine Gruppe der folgenden Formel ist:

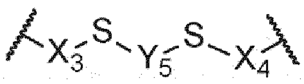 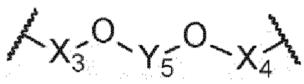

oder

wobei:

$X_3$ und $X_4$ Alkandiyl$_{(C\leq 12)}$, Alkendiyl$_{(C\leq 12)}$, Arendiyl$_{(C\leq 12)}$, oder eine substituierte Version einer dieser Gruppen sind;
$Y_5$ eine kovalente Bindung, Alkandiyl$_{(C\leq 12)}$, Alkendiyl$_{(C\leq 12)}$, Arendiyl$_{(C\leq 12)}$ oder eine substituierte Version einer dieser Gruppen ist; und
$R_9$ Alkyl$_{(C\leq 8)}$ oder substituiertes Alkyl$_{(C\leq 8)}$ ist;

die Linkergruppe die folgende Formel aufweist:

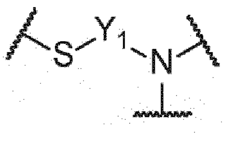 (VI)

wobei:

$Y_1$ Alkandiyl$_{(C\leq 12)}$, Alkendiyl$_{(C\leq 12)}$, Arendiyl$_{(C\leq 12)}$ oder eine substituierte Version einer dieser Gruppen ist; und
wobei, wenn die Wiederholungseinheit eine Linkergruppe umfasst, die Linkergruppe an eine abbaubare Diacylgruppe sowohl an den Stickstoff- als auch an den Schwefelatomen der Linkergruppe gebunden ist, wobei die erste Gruppe in der Wiederholungseinheit eine abbaubare Diacylgruppe ist, wobei für jede Linkergruppe, die nächste Gruppe zwei abbaubare Diacylgruppen umfasst, die an das Stickstoffatom der Linkergruppe gebunden sind; und wobei n die Anzahl der Linkergruppen ist, die in der sich wiederholenden

Einheit vorhanden sind; und

die terminierende Gruppe die folgende Formel aufweist:

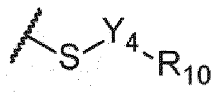 (VIII)

wobei:

$Y_4$ Alkanediyl$_{(C\leq18)}$, Alkenediyl$_{(C\leq18)}$ oder eine substituierte Version jeder Gruppe ist;
$R_{10}$ Wasserstoff, Carboxy, Hydroxy oder Aryl$_{(C\leq12)}$, Alkylamino$_{(C\leq12)}$, Dialkylamino$_{(C\leq12)}$, *N*-Heterocycloalkyl$_{(C\leq12)}$, -C(O)N($R_{11}$)-Alkandiyl$_{(C\leq6)}$-heterocycloalkyl$_{(C\leq12)}$, -C(O)-Alkylamino$_{(C\leq12)}$, -C(O)-Dialkylamino$_{(C\leq12)}$, -C(O)-*N*-Heterocycloalkyl$_{(C\leq12)}$ ist, wobei:
$R_{11}$ Wasserstoff, Alkyl$_{(C\leq6)}$ oder substituiertes Alkyl$_{(C\leq6)}$ ist;
wobei die letzte abbaubare Diacylgruppe in der Kette an eine terminierende Gruppe angebunden ist;
n 0, 1, 2, 3, 4, 5 oder 6 ist;

oder ein pharmazeutisch verträgliches Salz davon;
wobei, wenn ein Begriff mit dem Modifikator "substituiert" verwendet wird oder der Ausdruck "substituierte Version" verwendet wird, ein oder mehrere Wasserstoffatome unabhängig voneinander durch -OH, -F, -Cl, -Br, -I, $-NH_2$, $-NO_2$, $-CO_2H$, $-CO_2CH_3$, -CN, -SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, $-OC(O)CH_3$, $-NHC(O)CH_3$, $-S(O)_2OH$ oder $-S(O)_2NH_2$ ersetzt worden sind.

**4.** Zusammensetzung zur Verwendung nach Anspruch 3, wobei die Verbindung zur Aminolipidabgabe ein Dendrimer mit der folgenden Formel ist:

Kern-(Wiederholungseinheit)*n*-terminierende Gruppe (I)

wobei der Kern mit der Wiederholungseinheit verbunden ist, indem ein oder mehrere Wasserstoffatome aus dem Kern entfernt und durch die Wiederholungseinheit ersetzt werden und wobei:

der Kern die folgende Formel aufweist:

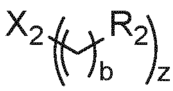 (III)

wobei:

$X_2$ $N(R_5)_y$ ist;

$R_5$ Wasserstoff oder Alkyl$_{(C\leq8)}$ oder substituiertes Alkyl$_{(C\leq18)}$ ist; und
y 0, 1 oder 2 ist, vorausgesetzt, dass die Summe von y und z 3 ist;

$R_2$ Amino, Hydroxy oder Mercapto oder Alkylamino$_{(C\leq12)}$, Dialkylamino$_{(C\leq12)}$ oder eine substituierte Version einer dieser Gruppen ist;
b 1, 2, 3, 4, 5 oder 6 ist; und
z 1, 2, 3 ist; vorausgesetzt, dass die Summe von z und y 3 ist;

wobei die Wiederholungseinheit eine abbaubare Diacylgruppe und eine Linkergruppe umfasst;

die abbaubare Diacylgruppe die folgende Formel aufweist:

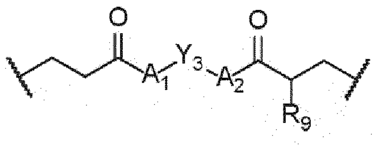 (VII)

wobei:

$A_1$ und $A_2$ jeweils unabhängig voneinander -O- oder $-NR_a-$ sind,
wobei:
$R_a$ Wasserstoff, $Alkyl_{(C\leq6)}$ oder substituiertes $Alkyl_{(C\leq6)}$ ist;
$Y_3$ $Alkandiyl_{(C\leq12)}$, $Alkendiyl_{(C\leq12)}$, $Arendiyl_{(C\leq12)}$ oder eine substituierte Version einer dieser Gruppen ist; oder eine Gruppe der folgenden Formel ist:

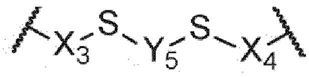 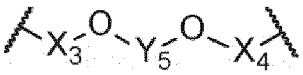
oder

wobei:

$X_3$ und $X_4$ $Alkandiyl_{(C\leq12)}$, $Alkendiyl_{(C\leq12)}$, $Arendiyl_{(C\leq12)}$ oder eine substituierte Version einer dieser Gruppen sind;
$Y_5$ eine kovalente Bindung, $Alkandiyl_{(C\leq12)}$, $Alkendiyl_{(C\leq12)}$, $Arendiyl_{(C\leq12)}$ oder eine substituierte Version einer dieser Gruppen ist; und

$R_9$ $Alkyl_{(C\leq8)}$ oder substituiertes $Alkyl_{(C\leq8)}$ ist;

die Linkergruppe die folgende Formel aufweist:

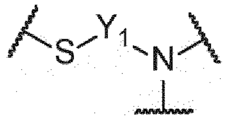 (VI)

wobei:
$Y_1$ $Alkandiyl_{(C\leq12)}$, $Alkendiyl_{(C\leq12)}$, $Arendiyl_{(C\leq12)}$ oder eine substituierte Version einer dieser Gruppen ist; und
wobei, wenn die Wiederholungseinheit eine Linkergruppe umfasst, die Linkergruppe an eine abbaubare Diacylgruppe sowohl an den Stickstoff- als auch an den Schwefelatomen der Linkergruppe gebunden ist, wobei die erste Gruppe in der Wiederholungseinheit eine abbaubare Diacylgruppe ist, wobei für jede Linkergruppe, die nächste Gruppe zwei abbaubare Diacylgruppen umfasst, die an das Stickstoffatom der Linkergruppe gebunden sind; und wobei n die Anzahl der Linkergruppen ist, die in der sich wiederholenden Einheit vorhanden sind; und
die terminierende Gruppe, wobei die terminierende Gruppe die folgende Formel aufweist:

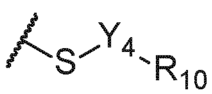 (VIII)

wobei:

$Y_4$ $Alkanediyl_{(C\leq18)}$, $Alkenediyl_{(C\leq18)}$ oder eine substituierte Version jeder Gruppe ist;
$R_{10}$ Wasserstoff, Carboxy, Hydroxy oder
$Aryl_{(C\leq12)}$, $Alkylamino_{(C\leq12)}$, $Dialkylamino_{(C\leq12)}$, *N*-$Heterocycloalkyl_{(C\leq12)}$, -C(O)N(R11)-$Alkandiyl_{(C\leq6)}$-$heterocycloalkyl_{(C\leq12)}$, -C(O)-$Alkylamino_{(C\leq12)}$, -C(O)-$Dialkylamino_{(C\leq12)}$, -C(O)-N-Heterocyc-

loalkyl$_{(C\leq12)}$ ist, wobei:

wobei die letzte abbaubare Diacylgruppe in der Kette an eine terminierende Gruppe angebunden ist; n 0, 1, 2, 3, 4, 5 oder 6 ist;

oder ein pharmazeutisch verträgliches Salz davon; oder wobei die Verbindung zur Aminolipidabgabe ein Dendrimer mit der folgenden Formel ist:

Kern-(Wiederholungseinheit)*n*-terminierende Gruppe (I)

wobei der Kern mit der Wiederholungseinheit verbunden ist, indem ein oder mehrere Wasserstoffatome aus dem Kern entfernt und durch die Wiederholungseinheit ersetzt werden und wobei:

der Kern die folgende Formel aufweist:

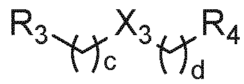

(IV)

wobei:

$X_3$ $-NR_6-$, -O- oder Alkylaminodiyl$_{(C\leq8)}$, Alkoxydiyl$_{(C\leq8)}$, Arendiyl$_{(C\leq8)}$, Heteroarendiyl$_{(C\leq8)}$, Heterocycloalkandiyl$_{(C\leq8)}$ oder eine substituierte Version einer dieser Gruppen ist, wobei $R_6$ Wasserstoff, Alkyl$_{(C\leq8)}$ oder substituiertes Alkyl$_{(C\leq8)}$ ist;
$R_3$ und $R_4$ jeweils unabhängig voneinander Amino, Hydroxy oder Mercapto oder Alkylamino$_{(C\leq12)}$, Dialkylamino$_{(C\leq12)}$ sind, wobei optional jede dieser Gruppen durch einen oder mehrere Substituenten substituiert ist, die ausgewählt sind aus der Gruppe bestehend aus: -OH, -F, -Cl, -Br,-I, $-NH_2$, $-NO_2$, $-CO_2H$, $-CO_2CH_3$, -CN,-SH, $-OCH_3$, $-OCH_2CH_3$, -C(O)CH3, $-NHCH_3$, -NHCH2CH$_3$, $-N(CH_3)_2$, $-C(O)NH_2$,$-C(O)NHCH_3$, -C(O)N(CH3)$_2$, $-OC(O)CH_3$,$-NHC(O)CH_3$, $-S(O)_2OH$ und $-S(O)_2NH_2$;
wobei:
c und d jeweils unabhängig 1, 2, 3, 4, 5 oder 6 sind; und

wobei die Wiederholungseinheit eine abbaubare Diacylgruppe und eine Linkergruppe umfasst; die abbaubare Diacylgruppe weist die folgende Formel auf:

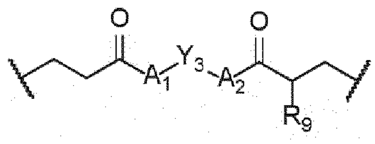

(VII)

wobei:

$A_1$ und $A_2$ jeweils unabhängig voneinander -O- oder $-NR_a$-sind, wobei:
$R_a$ Wasserstoff, Alkyl$_{(C\leq6)}$ oder substituiertes Alkyl$_{(C\leq6)}$ ist;
$Y_3$ Alkandiyl$_{(C\leq12)}$, Alkendiyl$_{(C\leq12)}$, Arendiyl$_{(C\leq12)}$ oder eine substituierte Version einer dieser Gruppen ist; oder eine Gruppe der folgenden Formel ist:

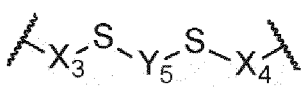

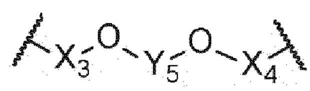

oder

wobei:

$X_3$ und $X_4$ Alkandiyl$_{(C\leq12)}$, Alkendiyl$_{(C\leq12)}$, Arendiyl$_{(C\leq12)}$ oder eine substituierte Version einer dieser Gruppen sind;
$Y_5$ eine kovalente Bindung, Alkandiyl$_{(C\leq12)}$, Alkendiyl$_{(C\leq12)}$, Arendiyl$_{(C\leq12)}$ oder eine substituierte Version einer dieser Gruppen ist; und

$R_9$ Alkyl$_{(C\leq8)}$ oder substituiertes Alkyl$_{(C\leq8)}$ ist;
die Linkergruppe die folgende Formel aufweist:

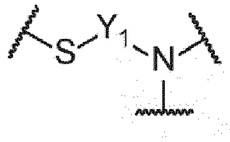
(VI)

wobei:
$Y_1$ Alkandiyl$_{(C\leq12)}$, Alkendiyl$_{(C\leq12)}$, Arendiyl$_{(C\leq12)}$ oder eine substituierte Version einer dieser Gruppen ist; und
wobei, wenn die Wiederholungseinheit eine Linkergruppe umfasst, die Linkergruppe an eine abbaubare Diacylgruppe sowohl an den Stickstoff- als auch an den Schwefelatomen der Linkergruppe gebunden ist, wobei die erste Gruppe in der Wiederholungseinheit eine abbaubare Diacylgruppe ist, wobei für jede Linkergruppe, die nächste Gruppe zwei abbaubare Diacylgruppen umfasst, die an das Stickstoffatom der Linkergruppe gebunden sind; und wobei n die Anzahl der Linkergruppen ist, die in der sich wiederholenden Einheit vorhanden sind; und
die terminierende Gruppe, wobei die terminierende Gruppe die folgende Formel aufweist:

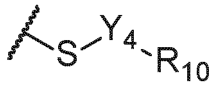
(VIII)

wobei:

$Y_4$ Alkanediyl$_{(C\leq18)}$ Alkenediyl$_{(C\leq18)}$ oder eine substituierte Version jeder Gruppe ist;
$R_{10}$ Wasserstoff, Carboxy, Hydroxy oder
Aryl$_{(C\leq12)}$, Alkylamino$_{(C\leq12)}$, Dialkylamino$_{(C\leq12)}$, *N*-Heterocycloalkyl$_{(C\leq12)}$, -C(O)N($R_{11}$)-Alkandiyl$_{(C\leq6)}$-heterocycloalkyl$_{(C\leq12)}$, -C(O)-Alkylamino$_{(C\leq12)}$, -C(O)-Dialkylamino$_{(C\leq12)}$, -C(O)-N-Heterocycloalkyl$_{(C\leq12)}$ ist, wobei:
$R_{11}$ Wasserstoff, Alkyl$_{(C\leq6)}$ oder substituiertes Alkyl$_{(C\leq6)}$ ist;

wobei die letzte abbaubare Diacylgruppe in der Kette an eine terminierende Gruppe angebunden ist;
n 0, 1, 2, 3, 4, 5 oder 6 ist;

oder ein pharmazeutisch verträgliches Salz davon.

**5.** Zusammensetzung zur Verwendung nach Anspruch 2, wobei die Verbindung zur Aminolipidabgabe ferner definiert ist als:

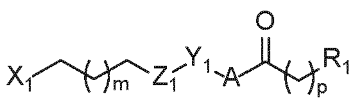
(I)

wobei:
$X_1$ $-S(O)_2O^-$, $-OP(O)OR_eO^-$, $-(CHR_f)_zC(O)O^-$ oder - $NR_gR_hR_i^+$ ist, wobei:

$R_e$, $R_g$, $R_h$ und $R_i$ jeweils unabhängig voneinander Wasserstoff, Alkyl$_{(C\leq6)}$ oder substituiertes Alkyl$_{(C\leq6)}$ sind;
$R_f$ Wasserstoff, Amino, Hydroxy oder Alkyl$_{(C\leq12)}$, Aryl$_{(C\leq12)}$, Aralkyl$_{(C\leq12)}$, Heteroaryl$_{(C\leq12)}$, Acyl$_{(C\leq12)}$, Alkoxy$_{(C\leq12)}$, Acyloxy$_{(C\leq12)}$, Amido$_{(C\leq12)}$, Alkoxy$_{(C\leq12)}$, Alkoxy$_{(C\leq12)}$ oder eine substituierte Version einer der letzten

zehn Gruppen ist; und

z 1, 2, 3 oder 4 ist;

$Y_1$ Alkanediyl$_{(C\leq12)}$, Alkendiyl$_{(C\leq12)}$, Arendiyl$_{(C\leq12)}$, Heteroarendiyl$_{(C\leq12)}$, Heterocycloalkandiyl$_{(C\leq12)}$, -Alkandiyl$_{(C\leq8)}$-heterocycloalkandiyl$_{(C\leq12)}$, -Alkandiyl$_{(C\leq8)}$-heterocycloalkandiyl$_{(C\leq12)}$-alkandiyl$_{(C\leq8)}$, - Alkandiyl$_{(C\leq8)}$-heteroarenediyl$_{(C\leq12)}$, - Alkandiyl$_{(C\leq8)}$-Heteroaren-diyl$_{(C\leq12)}$-alkandiyl$_{(C\leq8)}$ oder eine substituierte Version einer dieser Gruppen ist;

$Z_1$ -$N^+R_3R_4$- oder -OP(O)$O^-$O-

A -$NR_a$-, -S- oder -O- ist; wobei:

$R_a$ Wasserstoff, Alkyl$_{(C\leq6)}$ oder substituiertes Alkyl$_{(C\leq6)}$ ist oder $R_a$ zusammen mit entweder $R_3$ oder $R_4$ genommen wird und Alkandiyl$_{(C\leq8)}$, Alkenediyl$_{(C\leq8)}$, Alkoxydiyl$_{(C\leq8)}$, Alkylaminodiyl$_{(C\leq8)}$ oder eine substituierte Version einer dieser Gruppen ist;

$R_1$ eine Gruppe der folgenden Formel ist:

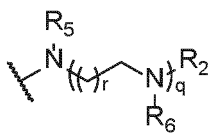

wobei:

$R_5$, $R_6$ und $R_2$ jeweils unabhängig voneinander Wasserstoff oder Alkyl$_{(C\leq8)}$, - Alkandiyl$_{(C\leq6)}$-$NH_2$, -Alkandiyl$_{(C\leq6)}$-alkylamino$_{(C\leq8)}$, -Alkandiyl$_{(C\leq6)}$-dialkylamino$_{(C\leq12)}$, -Alkandiyl$_{(C\leq6)}$-NR'R'' oder eine substituierte Version einer dieser Gruppen sind, wobei:

R' und R" jeweils unabhängig voneinander Wasserstoff, Alkyl$_{(C\leq8)}$, substituiertes Alkyl$_{(C\leq8)}$ oder -$Z_2A'R_7$ sind; wobei:

$Z_2$ Alkanediyl$_{(C\leq6)}$, substituiertes Alkanediyl$_{(C\leq6)}$ oder eine Gruppe der folgenden Formel ist:

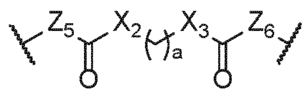

wobei:

$Z_5$ und $Z_6$ jeweils unabhängig voneinander Alkandiyl$_{(C\leq6)}$ oder substituiertes Alkandiyl$_{(C\leq6)}$ sind;

$X_2$ und $X_3$ jeweils unabhängig voneinander -O-, -S- oder -$NR_m$- sind; wobei:

$R_m$ Wasserstoff, Alkyl$_{(C\leq6)}$ oder substituiertes Alkyl$_{(C\leq6)}$ ist; und

a 0, 1, 2, 3, 4, 5 oder 6 ist;

A' -$CHR_j$, -C(O)O- oder -C(O)$NR_b$- ist;

$R_b$ Wasserstoff, Alkyl$_{(C\leq6)}$ oder substituiertes Alkyl$_{(C\leq6)}$ ist; und

$R_j$ Wasserstoff, Halogen, Hydroxy, Acyloxy$_{(C\leq24)}$ oder substituiertes Acyloxy$_{(C\leq24)}$ ist;

$R_7$ Alkyl$_{(C6-24)}$, substituiertes Alkyl$_{(C6-24)}$, Alkenyl$_{(C6-24)}$, substituiertes Alkenyl$_{(C6-24)}$ ist; oder

$R_5$, $R_6$ und $R_2$ jeweils unabhängig voneinander -$Z_3A''R_8$ sind; wobei:

$Z_3$ Alkanediyl$_{(C\leq6)}$, substituiertes Alkanediyl$_{(C\leq6)}$ oder eine Gruppe der folgenden Formel ist:

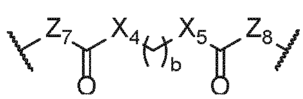

wobei:

$Z_7$ und $Z_8$ jeweils unabhängig voneinander Alkandiyl$_{(C\leq6)}$ oder substituiertes Alkandiyl$_{(C\leq6)}$ sind;

$X_4$ und $X_5$ jeweils unabhängig voneinander -O-, -S- oder -$NR_n$- sind; wobei:

$R_n$ Wasserstoff, Alkyl$_{(C\leq6)}$ oder substituiertes Alkyl$_{(C\leq6)}$ ist; und

b 0, 1, 2, 3, 4, 5 oder 6 ist;

A" -$CHR_k$-, -S-, -C(O)O- oder -C(O)$NR_1$-ist;

$R_1$ Wasserstoff, Alkyl$_{(C\leq6)}$ oder substituiertes Alkyl$_{(C\leq6)}$ ist; und
$R_k$ Wasserstoff, Halogen, Hydroxy, Acyloxy$_{(C\leq24)}$ oder substituiertes Acyloxy$_{(C\leq24)}$) ist; und

$R_8$ Alkyl$_{(C6-24)}$, substituiertes Alkyl$_{(C6-24)}$, Alkenyl$_{(C6-24)}$, substituiertes Alkenyl$_{(C6-24)}$ ist;
q 1, 2 oder 3 ist; und
r 1, 2, 3 oder 4 ist;

R,_ eine Gruppe der folgenden Formel ist:

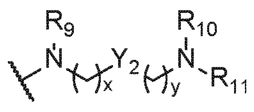

wobei:

$Y_2$ Arendiyl$_{(C\leq12)}$, Heterocycloalkandiyl$_{(C\leq12)}$, Heteroarendiyl$_{(C\leq12)}$, Alkoxydiyl$_{(C\leq12)}$ oder eine substituierte Version einer dieser Gruppen ist;
$R_9$, $R_{10}$ und $R_{11}$ jeweils unabhängig voneinander Wasserstoff, Alkyl$_{(C\leq8)}$, substituiertes Alkyl$_{(C\leq8)}$ oder -$Z_4$A'"$R_{12}$ sind; wobei:

$Z_4$ Alkanediyl$_{(C\leq6)}$, substituiertes Alkanediyl$_{(C\leq6)}$ oder eine Gruppe der folgenden Formel ist:

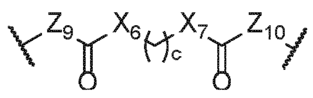

wobei:

$Z_9$ und $Z_{10}$ jeweils unabhängig voneinander Alkandiyl$_{(C\leq6)}$ oder substituiertes Alkandiyl$_{(C\leq6)}$ sind;
$X_6$ und $X_7$ jeweils unabhängig voneinander -O-, -S- oder -NRo- sind; wobei:
$R_o$ Wasserstoff, Alkyl$_{(C\leq6)}$ oder substituiertes Alkyl$_{(C\leq6)}$ ist; und
c 0, 1, 2, 3, 4, 5 oder 6 ist;

A'" -$CHR_k$-, -S-, -C(O)O- oder -C(O)$NR_1$-ist;

$R_1$ Wasserstoff, Alkyl$_{(C\leq6)}$ oder substituiertes Alkyl$_{(C\leq6)}$ ist; und
$R_k$ Wasserstoff, Halogen, Hydroxy, Acyloxy$_{(C\leq24)}$ oder substituiertes Acyloxy$_{(C\leq24)}$ ist; und

$R_{12}$ Alkyl$_{(C6-24)}$, substituiertes Alkyl$_{(C6-24)}$, Alkenyl$_{(C6-24)}$, substituiertes Alkenyl$_{(C6-24)}$ ist; und

x und y 0, 1, 2, 3 oder 4 sind;

$R_3$ und $R_4$ jeweils unabhängig voneinander Wasserstoff, Alkyl$_{(C\leq6)}$ oder substituiertes Alkyl$_{(C\leq6)}$ sind oder $R_3$ oder $R_4$ zusammen mit Ra genommen sind und Alkandiyl$_{(C\leq8)}$, Alkenediyl$_{(C\leq8)}$, Alkoxydiyl$_{(C\leq8)}$, Alkylaminodiyl$_{(C\leq8)}$ oder eine substituierte Version einer dieser Gruppen sind; und
m, n und p jeweils unabhängig voneinander eine ganze Zahl, ausgewählt aus 0, 1, 2, 3, 4, 5 oder 6, sind;
vorausgesetzt, wenn $X_1$ positiv geladen ist, dann ist $Z_1$ negativ geladen und wenn $X_1$ negativ geladen ist, dann ist $Z_1$ positiv geladen;
oder ein pharmazeutisch verträgliches Salz davon;
wobei, wenn ein Begriff mit dem Modifikator "substituiert" verwendet wird oder der Ausdruck "substituierte Version" verwendet wird, ein oder mehrere Wasserstoffatome unabhängig voneinander durch -OH, -F, -Cl, -Br, -I, -$NH_2$, -$NO_2$, -$CO_2H$, -$CO_2CH_3$, -CN, -SH, -$OCH_3$, -$OCH_2CH_3$, -C(O)$CH_3$, -$NHCH_3$, -$NHCH_2CH_3$, -N$(CH_3)_2$, -C(O)$NH_2$, -C(O)$NHCH_3$, -C(O)N$(CH_3)_2$, -OC(O)$CH_3$, -NHC(O)$CH_3$, -S$(O)_2$OH oder -S$(O)_2NH_2$ ersetzt worden

sind.

**6.** Zusammensetzung zur Verwendung nach Anspruch 2, wobei die Verbindung zur Aminolipidabgabe eine Verbindung der folgenden Formel ist:

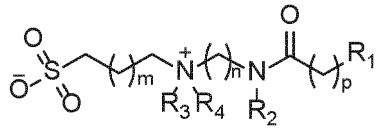

(II)

wobei:

$R_1$ eine Gruppe der folgenden Formel ist:

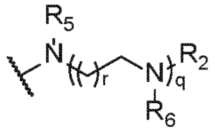

wobei:
$R_5$, $R_6$ und $R_2$ jeweils unabhängig voneinander Wasserstoff oder Alkyl$_{(C\leq 8)}$, -Alkandiyl$_{(C\leq 6)}$-NH2, - Alkandiyl$_{(C\leq 6)}$-alkylamino$_{(C\leq 8)}$, - Alkandiyl$_{(C\leq 6)}$-dialkylamino$_{(C\leq 12)}$, -Alkandiyl$_{(C\leq 6)}$-NR'R" oder eine substituierte Version einer dieser Gruppen sind, wobei:

R' und R" jeweils unabhängig voneinander Wasserstoff, Alkyl$_{(C\leq 8)}$, substituiertes Alkyl$_{(C\leq 8)}$ oder -$Z_2$A'$R_7$ sind; wobei:

$Z_2$ Alkanediyl$_{(C\leq 4)}$ oder substituiertes Alkanediyl$_{(C\leq 4)}$ ist;
A' -CH$R_j$, -C(O)O- oder -C(O)N$R_b$-ist;

$R_b$ Wasserstoff, Alkyl$_{(C\leq 6)}$ oder substituiertes Alkyl$_{(C\leq 6)}$ ist; und
$R_j$ Wasserstoff, Halogen, Hydroxy, Acyloxy$_{(C\leq 24)}$ oder substituiertes Acyloxy$_{(C\leq 24)}$ ist;

$R_7$ Alkyl$_{(C6\text{-}24)}$, substituiertes Alkyl$_{(C6\text{-}24)}$, Alkenyl$_{(C6\text{-}24)}$, substituiertes Alkenyl$_{(C6\text{-}24)}$ ist; oder

$R_5$, $R_6$ und $R_2$ jeweils unabhängig voneinander -$Z_3$A"$R_8$ sind; wobei:

$Z_3$ Alkandiyl$_{(C\leq 4)}$ oder substituiertes Alkandiyl$_{(C\leq 4)}$ ist; A" -CH$R_k$-, -C(O)O- oder -C(O)N$R_1$-ist;

$R_1$ Wasserstoff, Alkyl$_{(C\leq 6)}$ oder substituiertes Alkyl$_{(C\leq 6)}$ ist; und
$R_k$ Wasserstoff, Halogen, Hydroxy, Acyloxy$_{(C\leq 24)}$ oder substituiertes Acyloxy$_{(C\leq 24)}$ ist; und

$R_8$ Alkyl$_{(C6\text{-}24)}$, substituiertes Alkyl$_{(C6\text{-}24)}$, Alkenyl$_{(C6\text{-}24)}$, substituiertes Alkenyl$_{(C6\text{-}24)}$ ist;

q 1, 2 oder 3 ist; und
r 1, 2, 3 oder 4 ist;

$R_a$, $R_3$ und $R_4$ jeweils unabhängig voneinander Wasserstoff, Alkyl$_{(C\leq 6)}$ oder substituiertes Alkyl$_{(C\leq 6)}$ sind; und
m, n und p jeweils unabhängig voneinander eine ganze Zahl, ausgewählt aus 0, 1, 2, 3, 4, 5 oder 6, sind;
oder ein pharmazeutisch verträgliches Salz davon;
wobei, wenn ein Begriff mit dem Modifikator "substituiert" verwendet wird oder der Ausdruck "substituierte Version" verwendet wird, ein oder mehrere Wasserstoffatome unabhängig voneinander durch -OH, -F, -Cl, -Br, -I, -$NH_2$, -$NO_2$, -$CO_2H$, -$CO_2CH_3$, -CN, -SH, -$OCH_3$, -$OCH_2CH_3$, -C(O)$CH_3$, -$NHCH_3$, -$NHCH_2CH_3$, -N(CH3)$_2$, -C(O)$NH_2$, -C(O)$NHCH_3$, -C(O)N(CH3)$_2$, -OC(O)$CH_3$, -NHC(O)$CH_3$, -S(O)$_2$OH oder -S(O)$_2NH_2$ ersetzt worden sind.

**7.** Zusammensetzung zur Verwendung nach Anspruch 3 oder 4, wobei die terminierende Gruppe ferner durch die folgende Formel definiert ist:

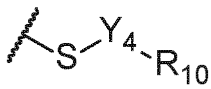 (VIII)

wobei:

$Y_4$ Alkanediyl$_{(C\leq18)}$, Alkenediyl$_{(C\leq18)}$ oder eine substituierte Version einer dieser Gruppen ist; wobei beispielsweise $Y_4$ Alkanediyl$_{(C\leq18)}$ ist; und
$R_{10}$ Wasserstoff ist.

**8.** Zusammensetzung zur Verwendung nach einem der Ansprüche 3, 4 und 7, wobei der Kern ferner durch die folgende Formel definiert ist:

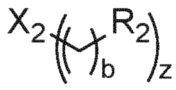 (III)

wobei:

$X_2$ $N(R_5)_y$ ist;

$R_5$ Wasserstoff oder Alkyl$_{(C\leq8)}$ oder substituiertes Alkyl$_{(C\leq18)}$ ist; und
y 0, 1 oder 2 ist, vorausgesetzt, dass die Summe von y und z 3 ist;

$R_2$ Amino, Hydroxy oder Mercapto oder Alkylamino$_{(C\leq12)}$, Dialkylamino$_{(C\leq12)}$ oder eine substituierte Version einer dieser Gruppen ist;
b 1, 2, 3, 4, 5 oder 6 ist; und
z 1, 2, 3 ist; vorausgesetzt, die Summe von z und y ist 3.

**9.** Zusammensetzung zur Verwendung nach Anspruch 8, wobei:

(i) $X_2$ $NR_5$ ist und $R_5$ Wasserstoff oder Alkyl$_{(C\leq8)}$ ist;
optional wobei $R_5$ Methyl ist; und/oder
(ii) z 3 ist; und/oder
(iii) $R_2$ Amino ist; und/oder
(iv) b 2 oder 3 ist; und/oder
(v) wobei der Kern ferner definiert ist als:

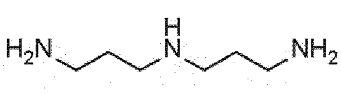, 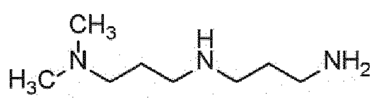, 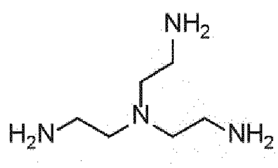,

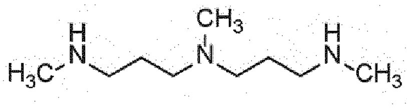, 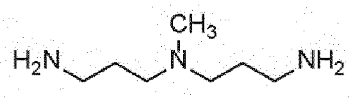, 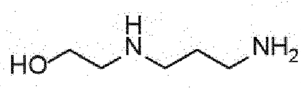,

oder

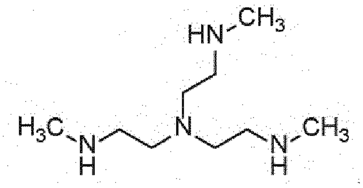

**10.** Zusammensetzung zur Verwendung nach einem der Ansprüche 3, 4 und 7, wobei der Kern ferner definiert ist als:

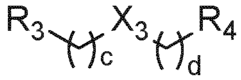
(IV)

wobei:

$X_3$ -$NR_6$-, -O- oder Alkylaminodiyl$_{(C\leq8)}$, Alkoxydiyl$_{(C\leq8)}$, Arendiyl$_{(C\leq8)}$, Heteroarendiyl$_{(C\leq8)}$, Heterocycloalkandiyl$_{(C\leq8)}$ oder eine substituierte Version einer dieser Gruppen ist, wobei $R_6$ Wasserstoff, Alkyl$_{(C\leq8)}$ oder substituiertes Alkyl$_{(C\leq8)}$ ist; $R_3$ und $R_4$ jeweils unabhängig voneinander Amino, Hydroxy oder Mercapto oder Alkylamino$_{(C\leq12)}$, Dialkylamino$_{(C\leq12)}$ sind, wobei optional jede dieser Gruppen durch einen oder mehrere Substituenten substituiert ist, die ausgewählt sind aus der Gruppe bestehend aus: -OH, -F, - Cl, -Br, -I, -$NH_2$, -$NO_2$, -$CO_2H$, - $CO_2CH_3$, -CN, -SH, -$OCH_3$, -OCH2$CH_3$, -C(O)$CH_3$, -$NHCH_3$, -$NHCH_2CH_3$, - N$(CH_3)$ $_2$, -C(O)$NH_2$, -C(O)$NHCH_3$, - C(O)N$(CH_3)_2$, -OC(O)$CH_3$, - NHC(O)$CH_3$, -S$(O)_2$OH und -S$(O)_2NH_2$; wobei:

c und d jeweils unabhängig voneinander 1, 2, 3, 4, 5 oder 6 sind;

optional wobei der Kern ferner definiert ist als:

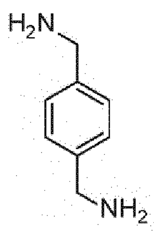, 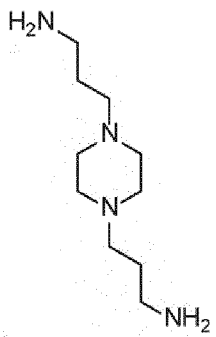, 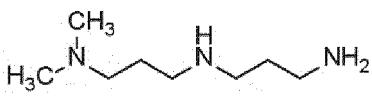, 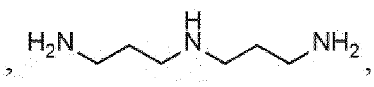,

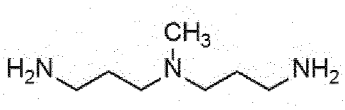, 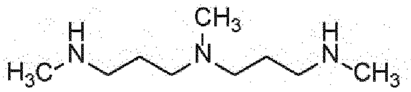, 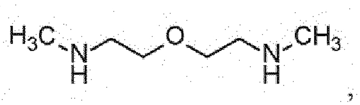,

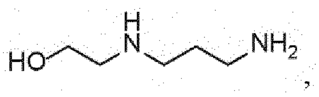, 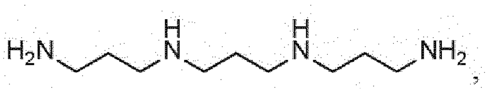,

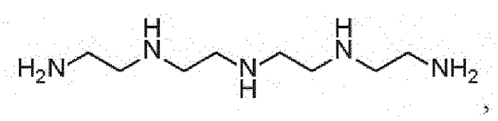, 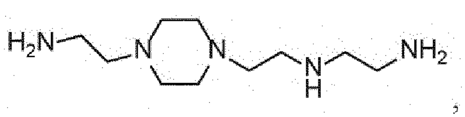,

oder

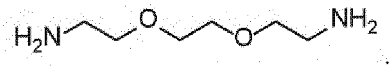.

**11.** Zusammensetzung zur Verwendung nach Anspruch 2, wobei die Verbindung zur Aminolipidabgabe ferner definiert ist als:

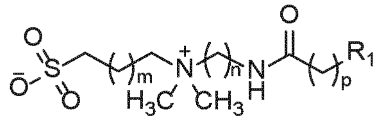

(II)

wobei:

$R_1$ eine Gruppe der folgenden Formel ist:
wobei:

$R_5$, $R_6$ und $R_2$ jeweils unabhängig voneinander Wasserstoff oder Alkyl$_{(C\leq 8)}$, -Alkandiyl$_{(C\leq 6)}$-$NH_2$, - Alkandiyl$_{(C\leq 6)}$-alkylamino$_{(C\leq 8)}$, - Alkandiyl$_{(C\leq 6)}$-dialkylamino$_{(C\leq 12)}$, -Alkandiyl$_{(C\leq 6)}$-NR'R" oder eine substituierte Version einer dieser Gruppen sind, wobei:

R' und R" jeweils unabhängig voneinander Wasserstoff, Alkyl$_{(C\leq 8)}$, substituiertes Alkyl$_{(C\leq 8)}$ oder -$Z_2$A'$R_7$ sind; wobei:

$Z_2$ Alkanediyl$_{(C\leq 4)}$ oder substituiertes Alkanediyl$_{(C\leq 4)}$ ist;
A' -$CHR_j$, -C(O)O- oder - C(O)$NR_b$- ist;

$R_b$ Wasserstoff, Alkyl$_{(C\leq 6)}$ oder substituiertes Alkyl$_{(C\leq 6)}$ ist; und
$R_j$ Wasserstoff, Halogen, Hydroxy, Acyloxy$_{(C\leq 24)}$ oder substituiertes Acyloxy$_{(C\leq 24)}$ ist;

$R_7$ Alkyl$_{(C6-24)}$, substituiertes Alkyl$_{(C6-24)}$, Alkenyl$_{(C6-24)}$, substituiertes Alkenyl$_{(C6-24)}$ ist; oder

$R_5$, $R_6$ und $R_2$ jeweils unabhängig voneinander -$Z_3$A"$R_8$ sind; wobei:

$Z_3$ Alkanediyl$_{(C\leq 4)}$ oder substituiertes Alkanediyl$_{(C\leq 4)}$ ist;
A" -$CHR_k$-, -C(O)O- oder -C(O)$NR_1$-ist;

$R_1$ Wasserstoff, Alkyl$_{(C\leq 6)}$ oder substituiertes Alkyl$_{(C\leq 6)}$ ist; und
$R_k$ Wasserstoff, Halogen, Hydroxy, Acyloxy$_{(C\leq 24)}$ oder substituiertes Acyloxy$_{(C\leq 24)}$ ist; und

$R_8$ Alkyl$_{(C6-24)}$, substituiertes Alkyl$_{(C6-24)}$, Alkenyl$_{(C6-24)}$, substituiertes Alkenyl$_{(C6-24)}$ ist;

q 1, 2 oder 3 ist; und
r 1, 2, 3 oder 4 ist; und

m, n und p jeweils unabhängig voneinander eine ganze Zahl, ausgewählt aus 0, 1, 2, 3, 4, 5 oder 6, sind;
oder ein pharmazeutisch verträgliches Salz davon; optional wobei die Verbindung zur Aminolipidabgabe ferner definiert ist als:

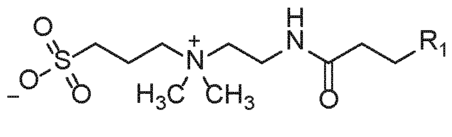

(III)

wobei:

$R_1$ eine Gruppe der folgenden Formel ist:

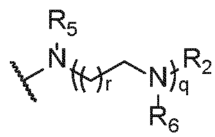

wobei:

$R_5$, $R_6$ und $R_2$ jeweils unabhängig voneinander Wasserstoff oder Alkyl$_{(C\leq8)}$, -Alkandiyl$_{(C\leq6)}$-$NH_2$, - Alkandiyl$_{(C\leq6)}$ -alkylamino$_{(C\leq8)}$, - Alkandiyl$_{(C\leq6)}$-dialkylamino$_{(C\leq12)}$, -Alkandiyl$_{(C\leq6)}$-NR'R" oder eine substituierte Version einer dieser Gruppen sind, wobei:

R' und R" jeweils unabhängig voneinander jeweils unabhängig voneinander Wasserstoff, Alkyl$_{(C\leq8)}$, substituiertes Alkyl$_{(C\leq8)}$ oder -$Z_2$A'$R_7$ sind; wobei:

$Z_2$ Alkandiyl$_{(C\leq4)}$ oder substituiertes Alkandiyl$_{(C\leq4)}$ ist; A' -CHR$_j$, -C(O)O- oder -C(O)NR$_b$-ist;

$R_b$ Wasserstoff, Alkyl$_{(C\leq6)}$ oder substituiertes Alkyl$_{(C\leq6)}$ ist; und
$R_j$ Wasserstoff, Halogen, Hydroxy, Acyloxy$_{(C\leq24)}$ oder substituiertes Acyloxy$_{(C\leq24)}$ ist;

$R_7$ Alkyl$_{(C6\leq24)}$, substituiertes Alkyl$_{(C6\leq24)}$, Alkenyl$_{(C6-24)}$, substituiertes Alkenyl$_{(C6-24)}$ ist; oder

$R_5$, $R_6$ und $R_2$ jeweils unabhängig voneinander -$Z_3$A"$R_8$ sind; wobei:

$Z_3$ Alkanediyl$_{(C\leq4)}$ oder substituiertes Alkanediyl$_{(C\leq4)}$ ist;
A" -CHRk-, -C(O)O- oder -C(O)NR,-ist;

R,_ Wasserstoff, Alkyl$_{(C\leq6)}$ oder substituiertes Alkyl$_{(C\leq6)}$ ist; und
$R_k$ Wasserstoff, Halogen, Hydroxy, Acyloxy$_{(C\leq24)}$ oder substituiertes Acyloxy$_{(C\leq24)}$ ist; und

$R_8$ Alkyl$_{(C6\leq24)}$, substituiertes Alkyl$_{(C6\leq24)}$, Alkenyl$_{(C6\leq24)}$, substituiertes Alkenyl$_{(C6-24)}$ ist;

q 1, 2 oder 3 ist; und
r 1, 2, 3 oder 4 ist;

oder ein pharmazeutisch verträgliches Salz davon;
wobei, wenn ein Begriff mit dem Modifikator "substituiert" verwendet wird oder der Ausdruck "substituierte Version" verwendet wird, ein oder mehrere Wasserstoffatome unabhängig voneinander durch -OH, -F, -Cl, -Br, -I, -$NH_2$, -$NO_2$, -$CO_2H$, -$CO_2CH_3$, -CN, -SH, -$OCH_3$, -$OCH_2CH_3$, -C(O)$CH_3$, -$NHCH_3$, -$NHCH_2CH_3$, -N$(CH_3)_2$, -C(O)$NH_2$, -C(O)$NHCH_3$, -C(O)N$(CH_3)_2$, -OC(O)$CH_3$, -NHC(O)$CH_3$, -S(O)$_2$OH oder -S(O)$_2NH_2$ ersetzt worden sind.

**12.** Zusammensetzung zur Verwendung nach Anspruch 6, wobei:

(i) $R_3$ Alkyl$_{(C\leq8)}$ oder substituiertes Alkyl$_{(C\leq8)}$ ist; und/oder
(ii) $R_4$ Alkyl$_{(C\leq8)}$ oder substituiertes Alkyl$_{(C\leq8)}$ ist; und/oder
(iii) m 1 oder 2 ist; und/oder
(iv) n 2 oder 3 ist; und/oder
(v) p 1, 2 oder 3 ist; und/oder
(vi) $R_1$ eine Gruppe der folgenden Formel ist:

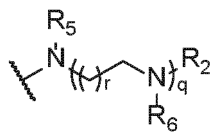

wobei:

$R_5$, $R_6$ und $R_2$ jeweils unabhängig voneinander Wasserstoff oder Alkyl$_{(C\leq8)}$, -Alkandiyl$_{(C\leq6)}$-$NH_2$, -Alkandiyl$_{(C\leq6)}$ -alkylamino$_{(C\leq8)}$,-Alkandiyl$_{(C\leq6)}$-dialkylamino$_{(C\leq12)}$, - Alkandiyl$_{(C\leq6)}$-NR'R" oder eine substituierte Version einer dieser Gruppen sind, wobei:
R' und R" jeweils unabhängig voneinander Wasserstoff, Alkyl$_{(C\leq8)}$, substituiertes Alkyl$_{(C\leq8)}$, -$Z_2$A'$R_7$ sind; wobei:

$Z_2$ Alkanediyl$_{(C\leq4)}$ oder substituiertes Alkanediyl$_{(C\leq4)}$ ist;
A' -$CHR_k$, -C(O)O- oder -C(O)$NR_b$- ist;
$R_b$ Wasserstoff, Alkyl$_{(C\leq6)}$ oder substituiertes Alkyl$_{(C\leq6)}$ ist; und
$R_j$ Wasserstoff, Halogen, Hydroxy, Acyloxy$_{(C\leq24)}$ oder substituiertes Acyloxy$_{(C\leq24)}$ ist;

$R_7$ Alkyl$_{(C6\leq24)}$, substituiertes Alkyl$_{(C6-24)}$, Alkenyl$_{(C6\leq24)}$, substituiertes Alkenyl$_{(C6-24)}$ ist; oder

$R_5$, $R_6$ und $X_1$ jeweils unabhängig voneinander -$Z_3$A"$R_8$ sind; wobei:

$Z_3$ Alkanediyl$_{(C\leq4)}$ oder substituiertes Alkanediyl$_{(C\leq4)}$ ist;
A" -$CHR_k$-, -C(O)O- oder -C(O)$NR_l$- ist;

$R_1$ Wasserstoff, Alkyl$_{(C\leq6)}$ oder substituiertes Alkyl$_{(C\leq6)}$ ist; und
$R_k$ Wasserstoff, Halogen, Hydroxy, Acyloxy$_{(C\leq24)}$ oder substituiertes Acyloxy$_{(C\leq24)}$ ist; und

$R_8$ Alkyl$_{(C6\leq24)}$, substituiertes Alkyl$_{(C6\leq24)}$, Alkenyl$_{(C6\leq24)}$, substituiertes Alkenyl$_{(C6-24)}$ ist;

q 1, 2 oder 3 ist; und
r 1, 2, 3 oder 4 ist;
vorzugsweise wobei:

(a) q 1 oder 2 ist; und/oder
(b) $R_5$ -$Z_3$A"$R_5$ ist; wobei:

$Z_3$ Alkanediyl$_{(C\leq4)}$ oder substituiertes Alkanediyl$_{(C\leq4)}$ ist;
A" -$CHR_k$-, -C(O)O- oder -C(O)NRl- ist;
$R_1$ Wasserstoff, Alkyl$_{(C\leq6)}$ oder substituiertes Alkyl$_{(C\leq6)}$ ist; und $R_k$ Wasserstoff, Halogen, Hydroxy, Acyloxy$_{(C\leq24)}$ oder substituiertes Acyloxy$_{(C\leq24)}$ ist; und
$R_8$ Alkyl$_{(C6\leq24)}$, substituiertes Alkyl$_{(C6-24)}$, Alkenyl$_{(C6\leq24)}$, substituiertes Alkenyl$_{(C6-24}$) ist; und/oder

(c) $R_6$ -$Z_3$A"$R_8$ ist; wobei:

$Z_3$ Alkanediyl$_{(C\leq4)}$ oder substituiertes Alkanediyl$_{(C\leq4)}$ ist;
A" -$CHR_k$-, -C(O)O- oder -C(O)$NR_l$- ist; $R_1$ Wasserstoff, Alkyl$_{(C\leq6)}$ oder substituiertes Alkyl$_{(C\leq6)}$ ist; und
$R_k$ Wasserstoff, Halogen, Hydroxy, Acyloxy$_{(C\leq24)}$ oder substituiertes Acyloxy$_{(C\leq24)}$ ist; und
$R_8$ Alkyl$_{(C6\leq24)}$, substituiertes Alkyl$_{(C6-24)}$, Alkenyl$_{(C6\leq24)}$, substituiertes Alkenyl$_{(C6\leq24)}$ ist; und/oder

(d) $R_2$ -$Z_3$A"$R_8$ ist; wobei:

$Z_3$ Alkanediyl$_{(C\leq4)}$ oder substituiertes Alkanediyl$_{(C\leq4)}$ ist;
A" -$CHR_k$-, -C(O)O- oder -C(O)$NR_l$- ist;
R,_ Wasserstoff, Alkyl$_{(C\leq6)}$ oder substituiertes Alkyl$_{(C\leq6)}$ ist; und
$R_k$ Wasserstoff, Halogen, Hydroxy, Acyloxy$_{(C\leq24)}$ oder substituiertes Acyloxy$_{(C\leq24)}$ ist; und
$R_8$ Alkyl$_{(C6\leq24)}$, substituiertes Alkyl$_{(C6-24)}$, Alkenyl$_{(C6\leq24)}$, substituiertes Alkenyl$_{(C6-24)}$ ist.

**13.** Zusammensetzung zur Verwendung nach einem der Ansprüche 2 bis 12, wobei die tRNA eine SuppressortRNA ist; optional wobei

die tRNA eine tRNA ist, die eine Aminosäure in ein Protein liefert, anstatt die Translation zu beenden; oder
die tRNA ein tRNA-Amber-Suppressor, ein tRNA-Opal-Suppressor, ein tRNA-Ocker-Suppressor oder ein tRNA-Frameshift-Suppressor ist; oder
die tRNA eine Nonsense-Mutation im Mukoviszidose-Transmembranleitfähigkeitsregulator-Protein korrigiert.

**14.** Zusammensetzung zur Verwendung nach einem der Ansprüche 2 bis 13, wobei die Zusammensetzung ferner ein Steroid oder Steroidderivat umfasst; und/oder

wobei die Zusammensetzung ferner ein Phospholipid umfasst; und/oder
wobei die Zusammensetzung ferner ein PEG-Lipid umfasst.

**15.** Zusammensetzung nach Anspruch 1 oder eine Zusammensetzung zur Verwendung nach einem der Ansprüche 2 bis 14, zur Verwendung in einem oder mehreren der folgenden:

(i) Wiederherstellen der Expression des p53-Proteins;
(ii)Abgeben einer Aminosäure in ein Protein anstelle des Beendens der Translation; und
(iii) Korrigieren einer Nonsense-Mutation in einem Protein des zystischen Fibrose-Transmembranleitwerts (CFTR).

**Revendications**

**1.** Composition comprenant :

(A) un acide ribonucléique de transfert (ARNt) ; et
(B) un composé d'administration d'aminolipides ;
dans laquelle le composé d'administration d'aminolipides forme une nanoparticule et le composé d'administration d'aminolipides est :

(i) un dendrimère de formule :

$$\text{Noyau-(Groupe récurrent)}_n\text{-Groupe de terminaison (I)}$$

dans laquelle le noyau est lié au motif récurrent en éliminant un ou plusieurs atomes d'hydrogène du noyau et en les remplaçant par le motif récurrent et dans laquelle :

le noyau a la formule :

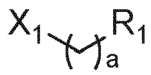
(II)

dans laquelle :

$X_1$ est un groupe amino ou alkylamino$_{(C\leq12)}$, dialkylamino$_{(C<12)}$, hétérocycloalkyle$_{(C\leq12)}$, hétéroaryle$_{(C\leq12)}$ ou une version substituée de celui-ci ;
$R_1$ est un groupe amino, hydroxy ou mercapto, ou alkylamino$_{(C\leq12)}$, dialkylamino$_{(C\leq12)}$, ou une version substituée de l'un ou l'autre de ces groupes ; et
a vaut 1, 2, 3, 4, 5 ou 6 ; ou

le noyau a la formule :

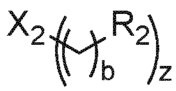
(III)

dans laquelle :

$X_2$ est $N(R_5)_y$ ;
$R_5$ est un hydrogène, un groupe alkyle$_{(C\leq18)}$ ou alkyle$_{(C\leq18)}$ substitué ; et

y vaut 0, 1 ou 2, à condition que la somme de y et z soit égale à 3 ;
$R_2$ est un groupe amino, hydroxy ou mercapto, ou alkylamino$_{(C\leq12)}$, dialkylamino$_{(C\leq12)}$, ou une version substituée de l'un ou l'autre de ces groupes ;
b vaut 1, 2, 3, 4, 5 ou 6 ; et
z vaut 1, 2, 3 ; à condition que la somme de z et y soit égale à 3 ; ou

le noyau a la formule :

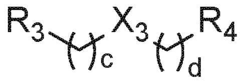
(IV)

dans laquelle :

$X_3$ est $-NR_6-$, -O-, ou un groupe alkylaminodiyle$_{(C\leq8)}$, alcoxydiyle$_{(C\leq8)}$, arènediyle$_{(C\leq8)}$, hétéroarènediyle$_{(C\leq8)}$, hétérocycloalcanediyle$_{(C\leq8)}$ ou une version substituée de l'un quelconque de ces groupes, dans laquelle $R_6$ est un hydrogène, un groupe alkyle$_{(C\leq8)}$ ou alkyle$_{(C\leq8)}$ substitué ;
$R_3$ et $R_4$ sont chacun indépendamment un groupe amino, hydroxy ou mercapto, ou alkylamino$_{(C\leq12)}$, dialkylamino$_{(C\leq12)}$, dans laquelle, éventuellement, l'un ou l'autre de ces groupes est substitué par un ou plusieurs substituants choisis dans le groupe constitué de : -OH, -F, -Cl, -Br, -I, $-NH_2$, $-NO_2$, $-CO_2H$, $-CO_2CH_3$, -CN, -SH, - $OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, - $C(O)N(CH_3)_2$, $-OC(O)CH_3$, $-NHC(O)CH_3$, - $S(O)_2OH$ et $-S(O)_2NH_2$ ;
dans laquelle :
c et d valent chacun indépendamment 1, 2, 3, 4, 5 ou 6 ; ou

le noyau est une alkylamine$_{(C\leq18)}$, une dialkylamine$_{(C\leq36)}$, un hétérocycloalcane$_{(C\leq12)}$ ou une version substituée de l'un quelconque de ces groupes ;
dans laquelle le motif récurrent comprend un groupe diacyle dégradable et un groupe de liaison ;
le groupe diacyle dégradable a la formule :

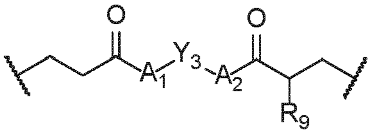
(VII)

dans laquelle :

$A_1$ et $A_2$ sont chacun indépendamment -O-, -S- ou $-NR_a-$, dans laquelle :
$R_a$ est un hydrogène, un groupe alkyle$_{(C\leq6)}$ ou alkyle$_{(C\leq6)}$ substitué ;
$Y_3$ est un groupe alcanediyle$_{(C\leq12)}$, alcènediyle$_{(C\leq12)}$, arènediyle$_{(C\leq12)}$ ou une version substituée de l'un quelconque de ces groupes ; ou un groupe de formule :

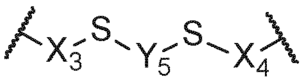
ou
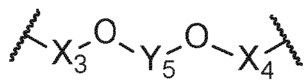

dans laquelle :

$X_3$ et $X_4$ sont un groupe alcanediyle$_{(C\leq12)}$, alcènediyle$_{(C\leq12)}$, arènediyle$_{(C\leq12)}$ ou une version substituée de l'un quelconque de ces groupes ;
$Y_5$ est une liaison covalente, un groupe alcanediyle$_{(C\leq12)}$, alcènediyle$_{(C\leq12)}$, arènediyle$_{(C\leq12)}$ ou une version substituée de l'un quelconque de ces groupes ; et
$R_9$ est un groupe alkyle$_{(C\leq8)}$ ou alkyle$_{(C\leq8)}$ substitué ;

le groupe de liaison a la formule :

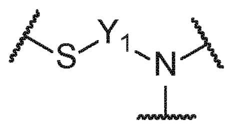
(VI)

dans laquelle :

$Y_1$ est un groupe alcanediyle$_{(C\leq12)}$, alcènediyle$_{(C\leq12)}$, arènediyle$_{(C\leq12)}$ ou une version substituée de l'un quelconque de ces groupes ; et
dans laquelle, lorsque le motif récurrent comprend un groupe de liaison, alors le groupe de liaison est attaché à un groupe diacyle dégradable à la fois sur les atomes d'azote et de soufre du groupe de liaison, dans laquelle le premier groupe dans le motif récurrent est un groupe diacyle dégradable, dans laquelle pour pour chaque groupe de liaison, le groupe suivant comprend deux groupes diacyle dégradables attachés à l'atome d'azote du groupe de liaison ; et dans lequel n est le nombre de groupes de liaison présents dans le motif récurrent ; et

le groupe de terminaison a la formule :

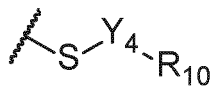
(VIII)

dans laquelle :

$Y_4$ est un groupe alcanediyle$_{(C\leq18)}$, alcènediyle$_{(C\leq18)}$ ou une version substituée de l'un ou l'autre groupe ;
$R_{10}$ est un hydrogène, un groupe carboxy, hydroxy ou
aryle$_{(C\leq12)}$, alkylamino$_{(C\leq12)}$, dialkylamino$_{(C\leq12)}$, *N*-hétérocycloalkyle$_{(C\leq12)}$, -C(O)N($R_{11}$)-alcanediy$_{(C\leq6)}$-hétérocycloalkyle$_{(C\leq12)}$, - C(O)-alkylamino$_{(C\leq12)}$, -C(O)-dialkylamino$_{(C\leq12)}$, -C(O)-*N*-hétérocycloalkyle$_{(C\leq12)}$, dans laquelle :
$R_{11}$ est un hydrogène, un groupe alkyle$_{(C\leq6)}$ ou alkyle$_{(C\leq6)}$ substitué ;
dans laquelle le groupe diacyle dégradable final dans la chaîne est attaché à un groupe de terminaison ;
n vaut 0, 1, 2, 3, 4, 5 ou 6 ; ou

(ii) un composé de formule :

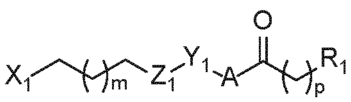
(I)

dans laquelle :

$X_1$ est $-S(O)_2O^-$, $-OP(O)OR_eO^-$, $-(CHR_f)_zC(O)O^-$ ou $-NR_gR_hR_i^+$, dans laquelle :

$R_e$, $R_g$, $R_h$ et $R_i$ sont chacun indépendamment un hydrogène, un groupe alkyle$_{(C\leq6)}$ ou alkyle$_{(C\leq6)}$ substitué ;
$R_f$ est un hydrogène, un groupe amino, hydroxy ou alkyle$_{(C\leq12)}$, aryle$_{(C\leq12)}$, aralkyle$_{(C\leq12)}$, hétéroaryle$_{(C\leq12)}$, acyle$_{(C\leq12)}$, alcoxy$_{(C\leq12)}$, acyloxy$_{(C\leq12)}$, amido$_{(C\leq12)}$, alcoxy$_{(C\leq12)}$, alcoxy$_{(C\leq12)}$, ou une version substituée de l'un quelconque des dix derniers groupes ; et
z vaut 1, 2, 3 ou 4 ;

$Y_1$ est un groupe alcanediyle$_{(C\leq12)}$, alcènediyle$_{(C\leq12)}$, arènediyle$_{(C\leq12)}$, hétéroarènediyle$_{(C\leq12)}$, hétérocycloalcanediyle$_{(C\leq12)}$, - alcanediyl$_{(C\leq8)}$-hétérocycloalcanediyle$_{(C\leq 12)}$, -alcanediyl$_{(C\leq8)}$-hétérocycloalcanediyl$_{(C\leq12)}$-alcanediyle$_{(C\leq8)}$, -alcanediyl$_{(C\leq8)}$-hétéroarènediyle$_{(C\leq12)}$, - alca-

nediyl$_{(C\leq 8)}$-hétéroarènediyl$_{(C\leq 12)}$ - alcanediyle$_{(C\leq 8)}$, ou une version substituée de l'un quelconque de ces groupes ;

$Z_1$ est $-N^+R_3R_4-$ ou $-OP(O)O^-O-$

A est -NR$_a$-, -S- ou -O- ; dans laquelle :

$R_a$ est un hydrogène, un groupe alkyle$_{(C\leq 6)}$ ou alkyle$_{(C\leq 6)}$ substitué, ou $R_a$ est pris ensemble avec soit $R_3$ ou $R_4$ et est un groupe alcanediyle$_{(C\leq 8)}$, alcènediyle$_{(C\leq 8)}$, alcoxydiyle$_{(C\leq 8)}$, alkylaminodiyle$_{(C\leq 8)}$, ou une version substituée de l'un quelconque de ces groupes ;

$R_1$ est un groupe de formule :

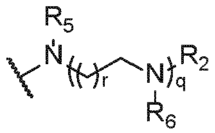

dans laquelle :

$R_5$, $R_6$ et $R_2$ sont chacun indépendamment un atome d'hydrogène ou un groupe alkyle$_{(C\leq 8)}$, -alcanediyl$_{(C\leq 6)}$-NH$_2$, - alcanediyl$_{(C\leq 6)}$-alkylamino$_{(C\leq 8)}$, - alcanediyl$_{(C\leq 6)}$-dialkylamino$_{(C\leq 12)}$, - alcanediyl$_{(C\leq 6)}$-NR'R", ou une version substituée de l'un quelconque de ces groupes dans laquelle :

R' et R" sont chacun indépendamment un hydrogène, un groupe alkyle (c≤8), alkyle$_{(C\leq 8)}$ substitué ou $-Z_2A'R_7$ ; dans laquelle :

$Z_2$ est un groupe alcanediyle$_{(C\leq 6)}$, alcanediyle$_{(C\leq 6)}$ substitué ou un groupe de formule :

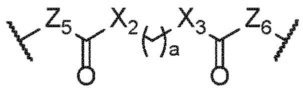

dans laquelle :

$Z_5$ et $Z_6$ sont chacun indépendamment un groupe alcanediyle$_{(C\leq 6)}$ ou alcanediyle$_{(C\leq 6)}$ substitué ;

$X_2$ et $X_3$ sont chacun indépendamment -O-, -S- ou -NR$_m$- ; dans laquelle : $R_m$ est un hydrogène, un groupe alkyle$_{(C\leq 6)}$ ou alkyle$_{(C\leq 6)}$ substitué ; et

a vaut 0, 1, 2, 3, 4, 5 ou 6 ;

A' est -CHR$_j$-, -C(O)O- ou -C(O)NR$_b$- ;

$R_b$ est un hydrogène, un groupe alkyle$_{(C\leq 6)}$ ou alkyle$_{(C\leq 6)}$ substitué ; et

$R_j$ est un hydrogène, un groupe halogéno, hydroxy, acyloxy$_{(C\leq 24)}$ ou acyloxy$_{(C\leq 24)}$ substitué ;

$R_7$ est un groupe alkyle$_{(C6-24)}$, alkyle$_{(C6-24)}$ substitué, alcényle$_{(C6-24)}$, alcényle$_{(C6-24)}$ substitué ; ou

$R_5$, $R_6$ et $R_2$ sont chacun indépendamment - $Z_3A''R_8$ ; dans laquelle :

$Z_3$ est un groupe alcanediyle$_{(C\leq 6)}$, alcanediyle$_{(C\leq 6)}$ substitué ou un groupe de formule :

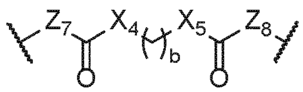

dans laquelle :

$Z_7$ et $Z_8$ sont chacun indépendamment un groupe alcanediyle$_{(C\leq 6)}$ ou alcanediyle$_{(C\leq 6)}$ substitué ;

$X_4$ et $X_5$ sont chacun indépendamment -O-, -S- ou -$NR_n$- ; dans laquelle :

$R_n$ est un hydrogène, un groupe alkyle$_{(C\leq6)}$ ou alkyle$_{(C\leq6)}$ substitué ; et

b vaut 0, 1, 2, 3, 4, 5 ou 6 ;

A" est -$CHR_k$-, -S-, -C(O)O- ou - C(O)$NR_1$- ;

$R_1$ est un hydrogène, un groupe alkyle$_{(C\leq6)}$ ou alkyle$_{(C\leq6)}$ substitué ; et

$R_k$ est un hydrogène, un groupe halogéno, hydroxy, acyloxy$_{(C\leq24)}$ ou acyloxy$_{(C\leq24)}$ substitué ; et

$R_8$ est un groupe alkyle$_{(C6-24)}$, alkyle$_{(C6-24)}$ substitué, alcényle$_{(C6-24)}$, alcényle$_{(C6-24)}$ substitué ;

q vaut 1, 2 ou 3 ; et

r vaut 1, 2, 3 ou 4 ;

R,_ est un groupe de formule :

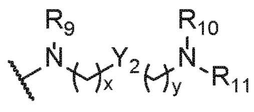

dans laquelle :

$Y_2$ est un groupe arènediyle$_{(C\leq12)}$, hétérocycloalcanediyle$_{(C\leq12)}$, hétéroarènediyle$_{(C\leq12)}$, alcoxydiyle$_{(C\leq12)}$ ou une version substituée de l'un quelconque de ces groupes ;

$R_9$, $R_{10}$ et $R_{11}$ sont chacun indépendamment un hydrogène, un groupe alkyle$_{(C\leq8)}$, alkyle$_{(C\leq8)}$ substitué ou -$Z_4A'''R_{12}$ ; dans laquelle :

$Z_4$ est un groupe alcanediyle$_{(C\leq6)}$, alcanediyle$_{(C\leq6)}$ substitué ou un groupe de formule :

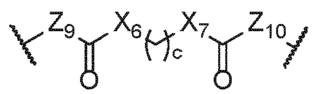

dans laquelle :

$Z_9$ et $Z_{10}$ sont chacun indépendamment un groupe alcanediyle$_{(C\leq6)}$ ou alcanediyle$_{(C\leq6)}$ substitué ;

$X_6$ et $X_7$ sont chacun indépendamment -O-, -S- ou -$NR_o$- ; dans laquelle :

$R_o$ est un hydrogène, un groupe alkyle$_{(C\leq6)}$ ou alkyle$_{(C\leq6)}$ substitué ; et

c vaut 0, 1, 2, 3, 4, 5 ou 6 ;

A''' est -$CHR_k$-, -S-, -C(O)O- ou - C(O)$NR_1$- ;

$R_1$ est un hydrogène, un groupe alkyle$_{(C\leq6)}$ ou alkyle$_{(C\leq6)}$ substitué ; et

$R_k$ est un hydrogène, un groupe halogéno, hydroxy, acyloxy$_{(C\leq24)}$ ou acyloxy$_{(C\leq24)}$ substitué ; et

$R_{12}$ est un groupe alkyle$_{(C6-24)}$, alkyle$_{(C6-24)}$ substitué, alcényle$_{(C6-24)}$, alcényle$_{(C6-24)}$ substitué ; et

x et y valent 0, 1, 2, 3 ou 4 ;

$R_3$ et $R_4$ sont chacun indépendamment un hydrogène, un groupe alkyle$_{(C\leq6)}$ ou alkyle$_{(C\leq6)}$ substitué, ou $R_3$ ou $R_4$ sont pris ensemble avec $R_a$ et représente un groupe alcanediyle$_{(C\leq8)}$, alcènediyle$_{(C\leq8)}$, alcoxydiyle$_{(C\leq8)}$, alkylaminodiyle$_{(C\leq8)}$, ou une version substituée de l'un quelconque de ces

groupes ; et
m, n et p sont chacun indépendamment un nombre entier choisi parmi 0, 1, 2, 3, 4, 5 ou 6 ;
à condition que si $X_1$ est chargé positivement, alors $Z_1$ est chargé négativement, et si $X_1$ est chargé négativement, alors $Z_1$ est chargé positivement ;

ou un sel pharmaceutiquement acceptable de celui-ci ;
dans laquelle, lorsqu'un terme est utilisé avec le modificateur « substitué », ou que l'expression « version substituée » est utilisée, un ou plusieurs atomes d'hydrogène ont été remplacés indépendamment par -OH, -F, -Cl, -Br, -I, $-NH_2$, $-NO_2$, $-CO_2H$, $-CO_2CH_3$, -CN, - SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, - $OC(O)CH_3$, $-NHC(O)CH_3$, $-S(O)_2OH$ ou $-S(O)_2NH_2$.

**2.** Composition destinée à être utilisée en médecine, dans laquelle la composition est utilisée pour administrer un ARN de transfert (ARNt) dans une cellule, la composition comprenant :

(a) l'ARNt ; et
(b) un composé d'administration d'aminolipides ;

dans laquelle le composé d'administration d'aminolipides forme une nanoparticule.

**3.** Composition destinée à être utilisée selon la revendication 2, dans laquelle le composé d'administration d'aminolipides est un dendrimère de formule :

$$\text{Noyau-(Groupe récurrent)}_n\text{-Groupe de terminaison (I)}$$

dans laquelle le noyau est lié au motif récurrent en éliminant un ou plusieurs atomes d'hydrogène du noyau et en les remplaçant par le motif récurrent et dans laquelle :

le noyau a la formule :

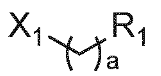
(II)

dans laquelle :

$X_1$ représente un groupe amino ou alkylamino$_{(C\leq 12)}$, dialkylamino$_{(C\leq 12)}$, hétérocycloalkyle$_{(C\leq 12)}$, hétéroaryle$_{(C\leq 12)}$ ou une version substituée de celui-ci ;
$R_1$ est un groupe amino, hydroxy ou mercapto, ou alkylamino$_{(C\leq 12)}$, dialkylamino$_{(C\leq 12)}$, ou une version substituée de l'un ou l'autre de ces groupes ; et
a vaut 1, 2, 3, 4, 5 ou 6 ; ou

le noyau a la formule :

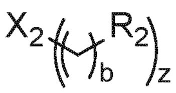
(III)

dans laquelle :

$X_2$ est $N(R_5)_y$ ;
$R_5$ est un hydrogène, un groupe alkyle$_{(C\leq 18)}$ ou alkyle$_{(C\leq 18)}$ substitué ; et
y vaut 0, 1 ou 2, à condition que la somme de y et z soit égale à 3 ;
$R_2$ est un groupe amino, hydroxy ou mercapto, ou alkylamino$_{(C\leq 12)}$, dialkylamino$_{(C\leq 12)}$, ou une version substituée de l'un ou l'autre de ces groupes ;
b vaut 1, 2, 3, 4, 5 ou 6 ; et

z vaut 1, 2, 3 ; à condition que la somme de z et y soit égale à 3 ; ou

le noyau a la formule :

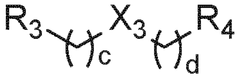
(IV)

dans laquelle :

$X_3$ est $-NR_6-$, -O-, ou un groupe alkylaminodiyle$_{(C\leq8)}$, alcoxydiyle$_{(C\leq8)}$, arènediyle$_{(C\leq8)}$, hétéroarènediyle$_{(C\leq8)}$, hétérocycloalcanediyle$_{(C\leq8)}$ ou une version substituée de l'un quelconque de ces groupes, dans laquelle $R_6$ est un hydrogène, un groupe alkyle$_{(C\leq8)}$ ou alkyle$_{(C\leq8)}$ substitué ;
$R_3$ et $R_4$ sont chacun indépendamment un groupe amino, hydroxy ou mercapto, ou alkylamino$_{(C\leq12)}$, dialkylamino$_{(C\leq12)}$, dans laquelle, éventuellement, l'un ou l'autre de ces groupes est substitué par un ou plusieurs substituants choisis dans le groupe constitué de : -OH, -F, -Cl, -Br, -I, $-NH_2$, $-NO_2$, $-CO_2H$, $-CO_2CH_3$, -CN, -SH, - $OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, - $C(O)N(CH_3)_2$, $-OC(O)CH_3$, $-NHC(O)CH_3$, - $S(O)_2OH$ et $-S(O)_2NH_2$ ;
dans laquelle :
c et d valent chacun indépendamment 1, 2, 3, 4, 5 ou 6 ; ou

le noyau est une alkylamine$_{(C\leq18)}$, une dialkylamine$_{(C\leq36)}$, un hétérocycloalcane$_{(C\leq12)}$ ou une version substituée de l'un quelconque de ces groupes ;
dans laquelle le motif récurrent comprend un groupe diacyle dégradable et un groupe de liaison ;
le groupe diacyle dégradable a la formule :

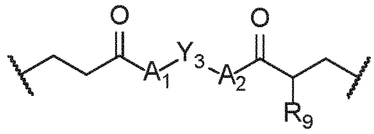
(VII)

dans laquelle :

$A_1$ et $A_2$ sont chacun indépendamment -O-, -S- ou $-NR_a-$, dans laquelle :
$R_a$ est un hydrogène, un groupe alkyle$_{(C\leq6)}$ ou alkyle$_{(C\leq6)}$ substitué ;
$Y_3$ est un groupe alcanediyle$_{(C\leq12)}$, alcènediyle$_{(C\leq12)}$, arènediyle$_{(C\leq12)}$ ou une version substituée de l'un quelconque de ces groupes ; ou un groupe de formule :

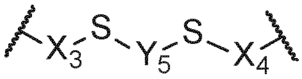
ou
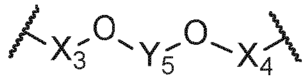

dans laquelle :

$X_3$ et $X_4$ sont un groupe alcanediyle$_{(C\leq12)}$, alcènediyle$_{(C\leq12)}$, arènediyle$_{(C\leq12)}$ ou une version substituée de l'un quelconque de ces groupes ;
$Y_5$ est une liaison covalente, un groupe alcanediyle$_{(C\leq12)}$, alcènediyle$_{(C\leq12)}$, arènediyle$_{(C\leq12)}$ ou une version substituée de l'un quelconque de ces groupes ; et
$R_9$ est un groupe alkyle$_{(C\leq8)}$ ou alkyle$_{(C\leq8)}$ substitué ;

le groupe de liaison a la formule :

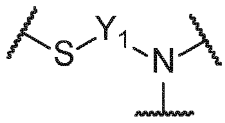

(VI)

dans laquelle :

$Y_1$ est groupe alcanediyle$_{(C\leq 12)}$, alcènediyle$_{(C\leq 12)}$, arènediyle$_{(C\leq 12)}$ ou une version substituée de l'un quelconque de ces groupes ; et
dans laquelle, lorsque le motif récurrent comprend un groupe de liaison, alors le groupe de liaison est attaché à un groupe diacyle dégradable à la fois sur les atomes d'azote et de soufre du groupe de liaison, dans laquelle le premier groupe dans le motif récurrent est un groupe diacyle dégradable, dans laquelle pour pour chaque groupe de liaison, le groupe suivant comprend deux groupes diacyle dégradables attachés à l'atome d'azote du groupe de liaison ; et dans lequel n est le nombre de groupes de liaison présents dans le motif récurrent ; et

le groupe de terminaison a la formule :

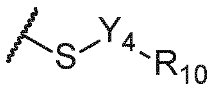

(VIII)

dans laquelle :

$Y_4$ est un groupe alcanediyle$_{(C\leq 18)}$, alcènediyle$_{(C\leq 18)}$ ou une version substituée de l'un ou l'autre groupe ;
$R_{10}$ est un hydrogène, un groupe carboxy, hydroxy ou
aryle$_{(C\leq 12)}$, alkylamino$_{(C\leq 12)}$, dialkylamino$_{(C\leq 12)}$, *N*-hétérocycloalkyle$_{(C\leq 12)}$, -C(O)N($R_{11}$)-alcanediyl$_{(C\leq 6)}$-hétérocycloalkyle$_{(C\leq 12)}$, - C(O)-alkylamino$_{(C\leq 12)}$, -C(O)-dialkylamino$_{(C\leq 12)}$, -C(O)-*N*-hétérocycloalkyle$_{(C\leq 12)}$, dans laquelle :
$R_{11}$ est un hydrogène, un groupe alkyle$_{(C\leq 6)}$ ou alkyle$_{(C\leq 6)}$ substitué ;
dans laquelle le groupe diacyle dégradable final dans la chaîne est attaché à un groupe de terminaison ;
n vaut 0, 1, 2, 3, 4, 5 ou 6 ;

ou un sel pharmaceutiquement acceptable de celui-ci ;
dans laquelle, lorsqu'un terme est utilisé avec le modificateur « substitué », ou que l'expression « version substituée » est utilisée, un ou plusieurs atomes d'hydrogène ont été remplacés indépendamment par -OH, -F, -Cl, -Br, -I, $-NH_2$, $-NO_2$, $-CO_2H$, $-CO_2CH_3$, -CN, - SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, - $OC(O)CH_3$, $-NHC(O)CH_3$, $-S(O)_2OH$ ou $-S(O)_2NH_2$.

**4.** Composition destinée à être utilisée selon la revendication 3, dans laquelle le composé d'administration d'aminolipides est un dendrimère de formule :

Noyau-(Groupe récurrent)$_n$-Groupe de terminaison (I)

dans laquelle le noyau est lié au motif récurrent en éliminant un ou plusieurs atomes d'hydrogène du noyau et en les remplaçant par le motif récurrent et dans laquelle :

le noyau a la formule :

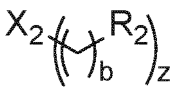

(III)

dans laquelle :

$X_2$ est $N(R_5)_y$ ;

$R_5$ est un hydrogène, un groupe alkyle$_{(C\leq 8)}$ ou alkyle$_{(C\leq 18)}$ substitué ; et
y vaut 0, 1 ou 2, à condition que la somme de y et z soit égale à 3 ;

$R_2$ est un groupe amino, hydroxy ou mercapto, ou alkylamino$_{(C\leq 12)}$, dialkylamino$_{(C\leq 12)}$, ou une version substituée de l'un ou l'autre de ces groupes ;
b vaut 1, 2, 3, 4, 5 ou 6 ; et
z vaut 1, 2, 3 ; à condition que la somme de z et y soit égale à 3 ; ou

dans laquelle le motif récurrent comprend un groupe diacyle dégradable et un groupe de liaison ;

le groupe diacyle dégradable a la formule :

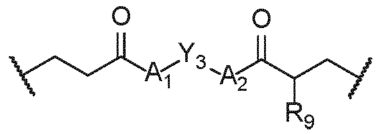
(VII)

dans laquelle :

$A_1$ et $A_2$ sont chacun indépendamment - O- ou $-NR_a-$, dans laquelle :
$R_a$ est un hydrogène, un groupe alkyle$_{(C\leq 6)}$ ou alkyle$_{(C\leq 6)}$ substitué ;
$Y_3$ est un groupe alcanediyle$_{(C\leq 12)}$, alcènediyle $_{(C\leq 12)}$, arènediyle$_{(C\leq 12)}$ ou une version substituée de l'un quelconque de ces groupes ; ou un groupe de formule :

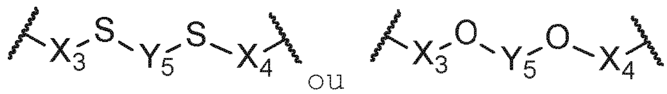

dans laquelle :

$X_3$ et $X_4$ sont un groupe alcanediyle$_{(C\leq 12)}$, alcènediyle$_{(C\leq 12)}$, arènediyle$_{(C\leq 12)}$ ou une version substituée de l'un quelconque de ces groupes ;
$Y_5$ est une liaison covalente, un groupe alcanediyle$_{(C\leq 12)}$, alcènediyle$_{(C\leq 12)}$, arènediyle$_{(C\leq 12)}$ ou une version substituée de l'un quelconque de ces groupes ; et

$R_9$ est un groupe alkyle$_{(C\leq 8)}$ ou alkyle$_{(C\leq 8)}$ substitué ;

le groupe de liaison a la formule :

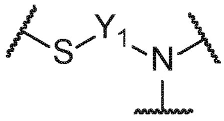
(VI)

dans laquelle :
$Y_1$ est un groupe alcanediyle$_{(C\leq 12)}$, alcènediyle$_{(C\leq 12)}$, arènediyle$_{(C\leq 12)}$ ou une version substituée de l'un quelconque de ces groupes ; et

dans laquelle, lorsque le motif récurrent comprend un groupe de liaison, alors le groupe de liaison est attaché à un groupe diacyle dégradable à la fois sur les atomes d'azote et de soufre du groupe de liaison, dans laquelle le premier groupe dans le motif récurrent est un groupe diacyle dégradable, dans laquelle pour pour chaque groupe de liaison, le groupe suivant comprend deux groupes diacyle dégradables attachés à l'atome d'azote du groupe de liaison ; et dans lequel n est le nombre de groupes de liaison présents dans

le motif récurrent ; et

le groupe de terminaison, dans laquelle le groupe de terminaison a la formule :

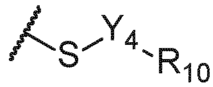
(VIII)

dans laquelle :

$Y_4$ est un groupe alcanediyle$_{(C\leq18)}$, alcènediyle$_{(C\leq18)}$ ou une version substituée de l'un ou l'autre groupe ; $R_{10}$ est un hydrogène, un groupe carboxy, hydroxy ou aryle$_{(C\leq12)}$, alkylamino$_{(C\leq12)}$, dialkylamino$_{(C\leq12)}$, N-hétérocycloalkyle$_{(C\leq12)}$, -C(O)N($R_{11}$)-alcanediyl$_{(C\leq6)}$-hétérocycloalkyle$_{(C\leq12)}$, -C(O)-alkylamino$_{(C\leq12)}$, -C(O)-dialkylamino$_{(C\leq12)}$, -C(O)-*N*-hétérocycloalkyle$_{(C\leq12)}$, dans laquelle :

dans laquelle le groupe diacyle dégradable final dans la chaîne est attaché à un groupe de terminaison ;

n vaut 0, 1, 2, 3, 4, 5 ou 6 ;

ou un sel pharmaceutiquement acceptable de celui-ci ;

ou dans laquelle le composé d'administration d'aminolipides est un dendrimère de formule :

Noyau-(Groupe récurrent)$_n$-Groupe de terminaison (I)

dans laquelle le noyau est lié au motif récurent en éliminant un ou plusieurs atomes d'hydrogène du noyau et en les remplaçant par le motif récurrent et dans laquelle :

le noyau a la formule :

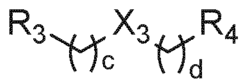
(IV)

dans laquelle :

$X_3$ est -$NR_6$-, -O-, ou un groupe alkylaminodiyle$_{(C\leq8)}$, alcoxydiyle$_{(C\leq8)}$, arènediyle$_{(C\leq8)}$, hétéroarènediyle$_{(C\leq8)}$, hétérocycloalcanediyle$_{(C\leq8)}$ ou une version substituée de l'un quelconque de ces groupes, dans laquelle $R_6$ est un hydrogène, un groupe alkyle$_{(C\leq8)}$ ou alkyle$_{(C\leq8)}$ substitué ;

$R_3$ et $R_4$ sont chacun indépendamment un groupe amino, hydroxy ou mercapto, ou alkylamino$_{(C\leq12)}$, dialkylamino$_{(C\leq12)}$, dans laquelle, éventuellement, l'un ou l'autre de ces groupes est substitué par un ou plusieurs substituants choisis dans le groupe constitué de : -OH, -F, -Cl, -Br, - I, -$NH_2$, -$NO_2$, -$CO_2H$, -$CO_2CH_3$, -CN, - SH, -$OCH_3$, -$OCH_2CH_3$, -C(O)$CH_3$, -$NHCH_3$, -$NHCH_2CH_3$, -N$(CH_3)_2$, -C(O)$NH_2$, -C(O)$NHCH_3$, -C(O)N$(CH_3)_2$, -OC(O)$CH_3$, - NHC(O)$CH_3$, -S(O)$_2$OH et -S(O)$_2NH_2$ ;

dans laquelle :

c et d valent chacun indépendamment 1, 2, 3, 4, 5 ou 6 ; et

dans laquelle le motif récurrent comprend un groupe diacyle dégradable et un groupe de liaison ;

le groupe diacyle dégradable a la formule :

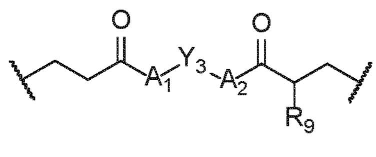
(VII)

dans laquelle :

$A_1$ et $A_2$ sont chacun indépendamment - O- ou -$NR_a$-, dans laquelle :
$R_a$ est un hydrogène, un groupe alkyle$_{(C\leq 6)}$ ou alkyle$_{(C\leq 6)}$ substitué ; et
$Y_3$ est un groupe alcanediyle$_{(C\leq 12)}$, alcènediyle$_{(C\leq 12)}$, arènediyle$_{(C\leq 12)}$ ou une version substituée de l'un quelconque de ces groupes ; ou un groupe de formule :

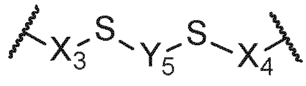
ou
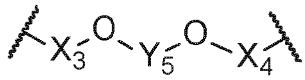

dans laquelle :

$X_3$ et $X_4$ sont un groupe alcanediyle$_{(C\leq 12)}$, alcènediyle$_{(C\leq 12)}$, arènediyle$_{(C\leq 12)}$ ou une version substituée de l'un quelconque de ces groupes ;
$Y_5$ est une liaison covalente, un groupe alcanediyle$_{(C\leq 12)}$, alcènediyle$_{(C\leq 12)}$, arènediyle$_{(C\leq 12)}$ ou une version substituée de l'un quelconque de ces groupes ; et

$R_9$ est un groupe alkyle$_{(C\leq 8)}$ ou alkyle$_{(C\leq 8)}$ substitué ;

le groupe de liaison a la formule :

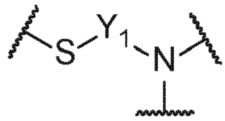
(VI)

dans laquelle :
Y,_ est un groupe alcanediyle$_{(C\leq 12)}$, alcènediyle$_{(C\leq 12)}$, arènediyle$_{(C\leq 12)}$ ou une version substituée de l'un quelconque de ces groupes ; et

dans laquelle, lorsque le motif récurrent comprend un groupe de liaison, alors le groupe de liaison est attaché à un groupe diacyle dégradable à la fois sur les atomes d'azote et de soufre du groupe de liaison, dans laquelle le premier groupe dans le motif récurrent est un groupe diacyle dégradable, dans laquelle pour pour chaque groupe de liaison, le groupe suivant comprend deux groupes diacyle dégradables attachés à l'atome d'azote du groupe de liaison ; et dans lequel n est le nombre de groupes de liaison présents dans le motif récurrent ; et
le groupe de terminaison, dans laquelle le groupe de terminaison a la formule :

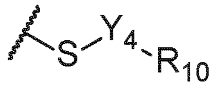
(VIII)

dans laquelle :

$Y_4$ est un groupe alcanediyle$_{(C\leq 18)}$, alcènediyle$_{(C\leq 18)}$ ou une version substituée de l'un ou l'autre groupe ;
$R_{10}$ est un hydrogène, un groupe carboxy, hydroxy ou
aryle$_{(C\leq 12)}$, alkylamino$_{(C\leq 12)}$, dialkylamino$_{(C\leq 12)}$, N-hétérocycloalkyle$_{(C\leq 12)}$, -C(O)N($R_{11}$)-alcanediyl$_{(C\leq 6)}$-hétérocycloalkyle$_{(C\leq 12)}$, -C(O)-alkylamino$_{(C\leq 12)}$, -C(O)-dialkylamino$_{(C\leq 12)}$, -C(O)-*N*-hétérocycloalkyle$_{(C\leq 12)}$, dans laquelle :
$R_{11}$ est un hydrogène, un groupe alkyle$_{(C\leq 6)}$ ou alkyle$_{(C\leq 6)}$ substitué ;

dans laquelle le groupe diacyle dégradable final dans la chaîne est attaché à un groupe de terminaison ;
n vaut 0, 1, 2, 3, 4, 5 ou 6 ;

ou un sel pharmaceutiquement acceptable de celui-ci.

**5.** Composition destinée à être utilisée selon la revendication 2, dans laquelle le composé d'administration d'aminolipides est en outre défini comme :

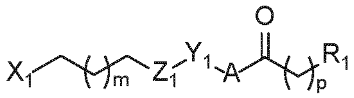

(I)

dans laquelle :

$X_1$ est $-S(O)_2O^-$, $-OP(O)OR_eO^-$, $-(CHR_f)_zC(O)O^-$, ou $-NR_gR_hR_i+$, dans laquelle :

$R_e$, $R_g$, $R_h$ et $R_i$ sont chacun indépendamment un hydrogène, un groupe alkyle$_{(C\leq6)}$ ou alkyle$_{(C\leq6)}$ substitué ;
$R_f$ est un hydrogène, un groupe amino, hydroxy ou alkyle$_{(C\leq12)}$, aryle$_{(C\leq12)}$, aralkyle$_{(C\leq12)}$, hétéroaryle$_{(C\leq12)}$, acyle$_{(C\leq12)}$, alcoxy$_{(C\leq12)}$, acyloxy$_{(C\leq12)}$, amido$_{(C\leq12)}$, alcoxy$_{(C\leq12)}$, alcoxy$_{(C\leq12)}$, ou une version substituée de l'un des dix derniers groupes ; et
z vaut 1, 2, 3 ou 4 ;

$Y_1$ est un groupe alcanediyle$_{(C\leq12)}$, alcènediyle$_{(C\leq12)}$, arènediyle$_{(C\leq12)}$, hétéroarènediyle$_{(C\leq12)}$, hétérocycloalcanediyle$_{(C\leq12)}$, - alcanediyl$_{(C\leq8)}$-hétérocycloalcanediyle$_{(C\leq12)}$, -alcanediyl$_{(C\leq8)}$-hétérocycloalcanediyl$_{(C\leq12)}$-alcanediyle$_{(C\leq8)}$, -alcanediyl$_{(C\leq8)}$-hétéroarènediyle$_{(C\leq12)}$, - alcanediyl$_{(C\leq8)}$-hétéroarènediyl$_{(C\leq12)}$-alcanediyle$_{(C\leq8)}$, ou une version substituée de l'un quelconque de ces groupes ;
$Z_1$ est $-N^+R_3R_4-$ ou $-OP(O)O^-O-$
A est $-NR_a-$, -S- ou -O- ; dans laquelle :
$R_a$ est un hydrogène, un groupe alkyle$_{(C\leq6)}$ ou alkyle$_{(C\leq6)}$ substitué, ou $R_a$ est pris ensemble avec soit $R_3$ ou $R_4$ et est un groupe alcanediyle$_{(C\leq8)}$, alcènediyle$_{(C\leq8)}$, alcoxydiyle$_{(C\leq8)}$, alkylaminodiyle$_{(C\leq8)}$ ou une version substituée de l'un quelconque de ces groupes ;
$R_1$ est un groupe de formule :

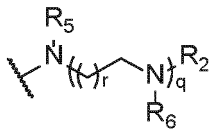

dans laquelle :
$R_5$, $R_6$ et $R_2$ sont chacun indépendamment un atome d'hydrogène ou un groupe alkyle$_{(C\leq8)}$, -alcanediyl$_{(C\leq6)}$-$NH_2$, - alcanediyl$_{(C\leq6)}$-alkylamino$_{(C\leq8)}$, - alcanediyl$_{(C\leq6)}$-dialkylamino$_{(C\leq12)}$, - alcanediyl$_{(C\leq6)}$-NR'R", ou une version substituée de l'un quelconque de ces groupes dans laquelle :

R' et R" sont chacun indépendamment un hydrogène, un groupe alkyle$_{(C\leq8)}$, alkyle$_{(C\leq8)}$ substitué ou $-Z_2A'R_7$ ; dans laquelle :

$Z_2$ est un groupe alcanediyle$_{(C\leq6)}$, alcanediyle$_{(C\leq6)}$ substitué ou un groupe de formule :

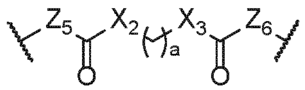

dans laquelle :

$Z_5$ et $Z_6$ sont chacun indépendamment un groupe alcanediyle$_{(C\leq6)}$ ou alcanediyle$_{(C\leq6)}$ substitué ;
$X_2$ et $X_3$ sont chacun indépendamment -O-, -S- ou $-NR_m-$ ; dans laquelle :
$R_m$ est un hydrogène, un groupe alkyle$_{(C\leq6)}$ ou alkyle$_{(C\leq6)}$ substitué ; et
a vaut 0, 1, 2, 3, 4, 5 ou 6 ;

A' est $-CHR_j-$, -C(O)O- ou $-C(O)NR_b-$ ;

$R_b$ est un hydrogène, un groupe alkyle$_{(C\leq6)}$ ou alkyle$_{(C\leq6)}$ substitué ; et
$R_j$ est un hydrogène, un groupe halogéno, hydroxy, acyloxy$_{(C\leq24)}$ ou acyloxy$_{(C\leq24)}$ substitué ;

$R_7$ est un groupe alkyle$_{(C6-24)}$, alkyle$_{(C6-24)}$ substitué, alcényle$_{(C6-24)}$, alcényle$_{(C6-24)}$ substitué ; ou

$R_5$, $R_6$ et $R_2$ sont chacun indépendamment - $Z_3A''R_8$ ; dans laquelle :

$Z_3$ est un groupe alcanediyle$_{(C\leq6)}$, alcanediyle$_{(C\leq6)}$ substitué ou un groupe de formule :

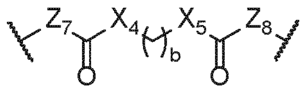

dans laquelle :

$Z_7$ et $Z_8$ sont chacun indépendamment un groupe alcanediyle$_{(C\leq6)}$ ou alcanediyle$_{(C\leq6)}$ substitué ;
$X_4$ et $X_5$ sont chacun indépendamment -O-, -S- ou -$NR_n$- ; dans laquelle :
$R_n$ est un hydrogène, un groupe alkyle$_{(C\leq6)}$ ou alkyle$_{(C\leq6)}$ substitué ; et
b vaut 0, 1, 2, 3, 4, 5 ou 6 ;

A" est -$CHR_k$-, -S-, -C(O)O- ou - C(O)$NR_1$- ;

$R_1$ est un hydrogène, un groupe alkyle$_{(C\leq6)}$ ou alkyle$_{(C\leq6)}$ substitué ; et
$R_k$ est un hydrogène, un groupe halogéno, hydroxy, acyloxy$_{(C\leq24)}$ ou acyloxy$_{(C\leq24)}$ substitué ; et

$R_8$ est un groupe alkyle$_{(C6-24)}$, alkyle($_{C6-24}$) substitué, alcényle$_{(C6-24)}$, alcényle$_{(C6-24)}$ substitué ;

q vaut 1, 2 ou 3 ; et
r vaut 1, 2, 3 ou 4 ;

R,_ est un groupe de formule :

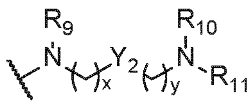

dans laquelle :

$Y_2$ est un groupe arènediyle$_{(C\leq12)}$, hétérocycloalcanediyle$_{(C\leq12)}$, hétéroarènediyle$_{(C\leq12)}$, alcoxydiyle$_{(C\leq12)}$ ou une version substituée de l'un quelconque de ces groupes ;
$R_9$, $R_{10}$ et $R_{11}$ sont chacun indépendamment un hydrogène, un groupe alkyle$_{(C\leq8)}$, alkyle$_{(C\leq8)}$ substitué ou -$Z_4A'''R_{12}$ ; dans laquelle :

$Z_4$ est un groupe alcanediyle$_{(C\leq6)}$, alcanediyle$_{(C\leq6)}$ substitué ou un groupe de formule :

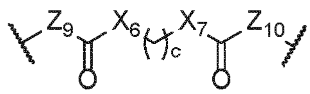

dans laquelle :

$Z_9$ et $Z_{10}$ sont chacun indépendamment un groupe alcanediyle$_{(C\leq6)}$ ou alcanediyle$_{(C\leq6)}$ substitué ;
$X_6$ et $X_7$ sont chacun indépendamment -O-, -S- ou -$NR_o$- ; dans laquelle :
$R_o$ est un hydrogène, un groupe alkyle$_{(C\leq6)}$ ou alkyle$_{(C\leq6)}$ substitué ; et c vaut 0, 1, 2, 3, 4, 5 ou 6 ;

A''' est -$CHR_k$-, -S-, -C(O)O- ou - C(O)$NR_1$- ;

$R_1$ est un hydrogène, un groupe alkyle$_{(C\leq6)}$ ou alkyle$_{(C\leq6)}$ substitué ; et
$R_k$ est un hydrogène, un groupe halogéno, hydroxy, acyloxy$_{(C\leq24)}$ ou acyloxy$_{(C\leq24)}$ substitué ; et

$R_{12}$ est un groupe alkyle$_{(C6-24)}$, alkyle$_{(C6-24)}$ substitué, alcényle$_{(C6-24)}$, alcényle$_{(C6-24)}$ substitué ; et

x et y valent 0, 1, 2, 3 ou 4 ;

$R_3$ et $R_4$ sont chacun indépendamment un hydrogène, un groupe alkyle$_{(C\leq6)}$ ou alkyle$_{(C\leq6)}$ substitué, ou $R_3$ ou $R_4$ sont pris ensemble avec $R_a$ et représente un groupe alcanediyle$_{(C\leq8)}$, alcènediyle$_{(C\leq8)}$, alcoxydiyle$_{(C\leq8)}$, alkylaminodiyle$_{(C\leq8)}$, ou une version substituée de l'un quelconque de ces groupes ; et
m, n et p sont chacun indépendamment un nombre entier choisi parmi 0, 1, 2, 3, 4, 5 ou 6 ;
à condition que si $X_1$ est chargé positivement, alors $Z_1$ est chargé négativement, et si $X_1$ est chargé négativement, alors $Z_1$ est chargé positivement ;
ou un sel pharmaceutiquement acceptable de celui-ci ;
dans laquelle, lorsqu'un terme est utilisé avec le modificateur « substitué », ou que l'expression « version substituée » est utilisée, un ou plusieurs atomes d'hydrogène ont été remplacés indépendamment par -OH, -F, -Cl, -Br, -I, $-NH_2$, $-NO_2$, $-CO_2H$, $-CO_2CH_3$, -CN, - SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, - $OC(O)CH_3$, $-NHC(O)CH_3$, $-S(O)_2OH$ ou $-S(O)_2NH_2$.

**6.** Composition destinée à être utilisée selon la revendication 2, dans laquelle le composé d'administration d'aminolipides est un composé de formule :

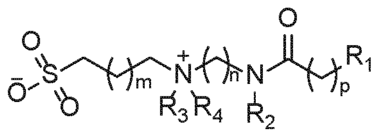
(II)

dans laquelle :

$R_1$ est un groupe de formule :

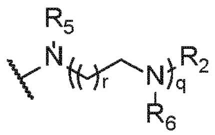

dans laquelle :
$R_5$, $R_6$ et $R_2$ sont chacun indépendamment un atome d'hydrogène ou un groupe alkyle$_{(C\leq8)}$, -alcanediyl$_{(C\leq6)}$-$NH_2$, - alcanediyl$_{(C\leq6)}$-alkylamino$_{(C\leq8)}$, - alcanediyl$_{(C\leq6)}$-dialkylamino$_{(C\leq12)}$, - alcanediyl$_{(C\leq6)}$-NR'R", ou une version substituée de l'un quelconque de ces groupes dans laquelle :

R' et R" sont chacun indépendamment un hydrogène, un groupe alkyle$_{(C\leq8)}$, alkyle$_{(C\leq8)}$ substitué ou $-Z_2A'R_7$ ; dans laquelle :

$Z_2$ est un groupe alcanediyle$_{(C\leq4)}$ ou alcanediyle$_{(C\leq4)}$ substitué ;
A' est $-CHR_j-$, -C(O)O- ou $-C(O)NR_b-$ ;

$R_b$ est un hydrogène, un groupe alkyle$_{(C\leq6)}$ ou alkyle$_{(C\leq6)}$ substitué ; et
$R_j$ est un hydrogène, groupe halogéno, hydroxy, acyloxy$_{(C\leq24)}$ ou acyloxy$_{(C\leq24)}$ substitué ;

$R_7$ est un groupe alkyle$_{(C6-24)}$, alkyle$_{(C6-24)}$ substitué, alcényle$_{(C6-24)}$, alcényle$_{(C6-24)}$ substitué ; ou

$R_5$, $R_6$ et $R_2$ sont chacun indépendamment - $Z_3A''R_8$ ; dans laquelle :

$Z_3$ est un groupe alcanediyle$_{(C\leq4)}$ ou alcanediyle$_{(C\leq4)}$ substitué ;
A" est $-CHR_k-$, -C(O)O- ou $-C(O)NR_1-$ ;

$R_1$ est un hydrogène, un groupe alkyle$_{(C\leq6)}$ ou alkyle$_{(C\leq6)}$ substitué ; et
$R_k$ est un hydrogène, un groupe halogéno, hydroxy, acyloxy$_{(C\leq24)}$ ou acyloxy$_{(C\leq24)}$ substitué ; et

$R_8$ est un groupe alkyle$_{(C6-24)}$, alkyle$_{(C6-24)}$ substitué, alcényle$_{(C6-24)}$, alcényle ($_{C6-24}$) substitué ;

q vaut 1, 2 ou 3 ; et
r vaut 1, 2, 3 ou 4 ;

$R_a$, $R_3$ et $R_4$ sont chacun indépendamment un hydrogène, un groupe alkyle$_{(C\leq6)}$ ou alkyle$_{(C\leq6)}$ substitué ; et
m, n et p sont chacun indépendamment un nombre entier choisi parmi 0, 1, 2, 3, 4, 5 ou 6 ;
ou un sel pharmaceutiquement acceptable de celui-ci ;
dans laquelle, lorsqu'un terme est utilisé avec le modificateur « substitué », ou que l'expression « version substituée » est utilisée, un ou plusieurs atomes d'hydrogène ont été remplacés indépendamment par -OH, -F, -Cl, -Br, -I, $-NH_2$, $-NO_2$, $-CO_2H$, $-CO_2CH_3$, -CN, - SH, $-OCH_3$, $-OCH_2CH_3$, $-C(O)CH_3$, $-NHCH_3$, $-NHCH_2CH_3$, $-N(CH_3)_2$, $-C(O)NH_2$, $-C(O)NHCH_3$, $-C(O)N(CH_3)_2$, - $OC(O)CH_3$, $-NHC(O)CH_3$, $-S(O)_2OH$ ou $-S(O)_2NH_2$.

**7.** Composition destinée à être utilisée selon la revendication3 ou la revendication 4, dans laquelle le groupe de terminaison est en outre défini par la formule :

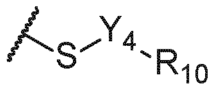
(VIII)

dans laquelle :

$Y_4$ est un groupe alcanediyle$_{(C\leq18)}$, alcènediyle$_{(C\leq18)}$ ou une version substituée de l'un ou l'autre de ces groupes ;
telle que dans laquelle $Y_4$ est un groupe alcanediyle$_{(C\leq18)}$ ; et
$R_{10}$ est un hydrogène.

**8.** Composition destinée à être utilisée selon l'une quelconque des revendications 3, 4 et 7, dans laquelle le noyau est en outre défini par la formule :

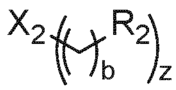
(III)

dans laquelle :

$X_2$ est $N(R_5)_y$ ;

$R_5$ est un hydrogène, un groupe alkyle$_{(C\leq8)}$ ou alkyle$_{(C\leq18)}$ substitué ; et
y vaut 0, 1 ou 2, à condition que la somme de y et z soit égale à 3 ;

$R_2$ est un groupe amino, hydroxy ou mercapto, ou alkylamino$_{(C\leq12)}$, dialkylamino$_{(C\leq12)}$, ou une version substituée de l'un ou l'autre de ces groupes ;
b vaut 1, 2, 3, 4, 5 ou 6 ; et
z vaut 1, 2, 3 ; à condition que la somme de z et y soit égale à 3.

**9.** Composition destinée à être utilisée selon la revendication 8, dans laquelle :

(i) $X_2$ est $NR_5$ et $R_5$ est un hydrogène ou un groupe alkyle$_{(C\leq8)}$ ;
éventuellement dans laquelle $R_5$ est un groupe méthyle ; et/ou
(ii) z vaut 3 ; et/ou
(iii) $R_2$ est un groupe amino ; et/ou
(iv) b vaut 2 ou 3 ; et/ou

(v) dans laquelle le noyau est en outre défini comme :

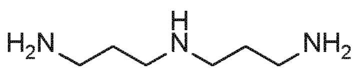,
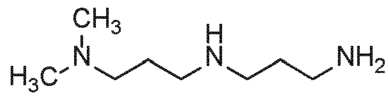,

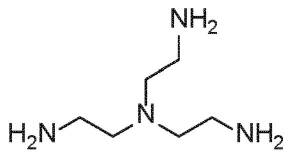,
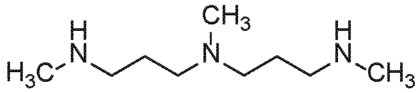,

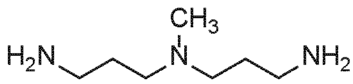,
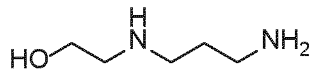,

ou

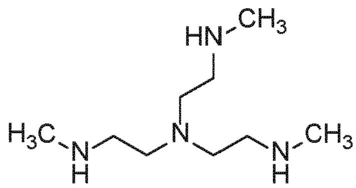.

**10.** Composition destinée à être utilisée selon l'une quelconque des revendications 3, 4 et 7, dans laquelle le noyau est en outre défini comme :

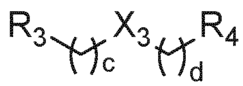
(IV)

dans laquelle :

$X_3$ est $-NR_6-$, -O-, ou un groupe alkylaminodiyle$_{(C\leq8)}$, alcoxydiyle$_{(C\leq8)}$, arènediyle$_{(C\leq8)}$, hétéroarènediyle$_{(C\leq8)}$, hétérocycloalcanediyle$_{(C\leq8)}$ ou une version substituée de l'un quelconque de ces groupes, dans laquelle $R_6$ est un hydrogène, un groupe alkyle$_{(C\leq8)}$ ou alkyle$_{(C\leq8)}$ substitué ;

$R_3$ et $R_4$ sont chacun indépendamment un groupe amino, hydroxy ou mercapto, ou alkylamino$_{(C\leq12)}$, dialkylamino$_{(C\leq12)}$, dans laquelle, éventuellement, l'un ou l'autre de ces groupes est substitué par un ou plusieurs substituants choisis dans le groupe constitué de : -OH, -F, -Cl, -Br, - I, $-NH_2$, $-NO_2$, $-CO_2H$, $-CO_2CH_3$, -CN, - SH, $-OCH_3$, $-OCH_2CH_3$, $-C(O)CH_3$, $-NHCH_3$, $-NHCH_2CH_3$, $-N(CH_3)_2$, $-C(O)NH_2$, - $C(O)NHCH_3$, $-C(O)N(CH_3)_2$, $-OC(O)CH_3$, - $NHC(O)CH_3$, $-S(O)_2OH$ et $-S(O)_2NH_2$ ; dans laquelle :

c et d valent chacun indépendamment 1, 2, 3, 4, 5 ou 6 ;

éventuellement, dans laquelle le noyau est en outre défini comme :

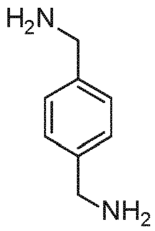, 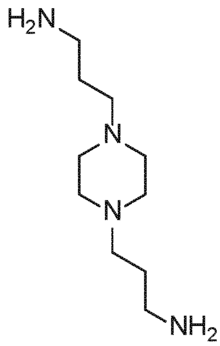, 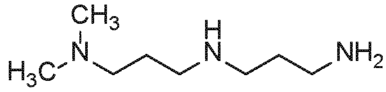,

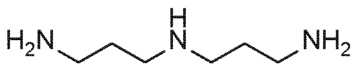, 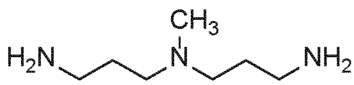,

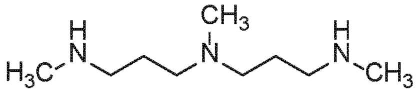, 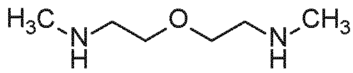,

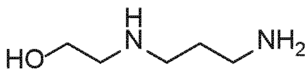, 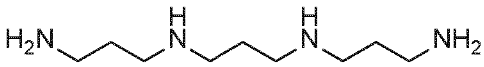,

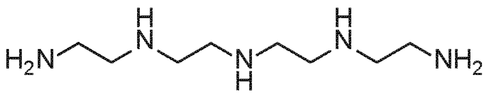, 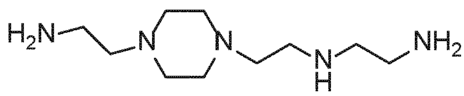,

ou

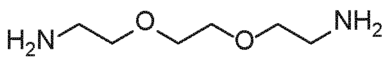.

**11.** Composition destinée à être utilisée selon la revendication 2, dans laquelle le composé d'administration d'aminolipides est en outre défini comme :

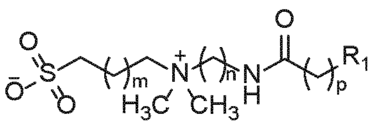 (II)

dans laquelle :

$R_1$ est un groupe de formule :

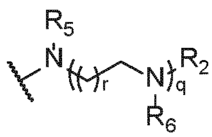

dans laquelle :

$R_5$, $R_6$ et $R_2$ sont chacun indépendamment un atome d'hydrogène ou un groupe alkyle$_{(C\leq 8)}$, -alcanediyl$_{(C\leq 6)}$-$NH_2$, - alcanediyl$_{(C\leq 6)}$-alkylamino$_{(C\leq 8)}$, - alcanediyl$_{(C\leq 6)}$-dialkylamino$_{(C\leq 12)}$, - alcanediyl$_{(C\leq 6)}$ -NR'R", ou une version substituée de l'un quelconque de ces groupes dans laquelle :
R' et R" sont chacun indépendamment un hydrogène, un groupe alkyle$_{(C\leq 8)}$, alkyle$_{(C\leq 8)}$ substitué ou -$Z_2$A'$R_7$ ; dans laquelle :

$Z_2$ est un groupe alcanediyle$_{(C\leq 4)}$ ou alcanediyle$_{(C\leq 4)}$ substitué ;
A' est -CH$R_j$-, -C(O)O- ou - C(O)N$R_b$- ;

$R_b$ est un hydrogène, un groupe alkyle$_{(C\leq 6)}$ ou alkyle$_{(C\leq 6)}$ substitué ; et
$R_j$ est un hydrogène, un groupe halogéno, hydroxy, acyloxy$_{(C\leq 24)}$ ou acyloxy$_{(C\leq 24)}$ substitué ;

$R_7$ est un groupe alkyle$_{(C6-24)}$, alkyle ($_{C6-24}$) substitué, alcényle$_{(C-24)}$, alcényle$_{(C6-24)}$ substitué ; ou

$R_5$, $R_6$ et $R_2$ sont chacun indépendamment - $Z_3$A"$R_8$ ; dans laquelle :

$Z_3$ est un groupe alcanediyle$_{(C\leq 4)}$ ou alcanediyle$_{(C\leq 4)}$ substitué ;
A" est -CH$R_k$-, -C(O)O- ou -C(O)N$R_1$- ;

$R_1$ est un hydrogène, un groupe alkyle$_{(C\leq 6)}$ ou alkyle$_{(C\leq 6)}$ substitué ; et
$R_k$ est un hydrogène, un groupe halogéno, hydroxy, acyloxy$_{(C\leq 24)}$ ou acyloxy$_{(C\leq 24)}$ substitué ; et

$R_8$ est un groupe alkyle$_{(C6-24)}$, alkyle$_{(C6-24)}$ substitué, alcényle$_{(C6-24)}$, alcényle$_{(C6-24)}$ substitué ;

q vaut 1, 2 ou 3 ; et
r vaut 1, 2, 3 ou 4 ; et

m, n et p sont chacun indépendamment un nombre entier choisi parmi 0, 1, 2, 3, 4, 5 ou 6 ;
ou un sel pharmaceutiquement acceptable de celui-ci ;
éventuellement, dans lequel le composé d'administration d'aminolipides est en outre défini comme :

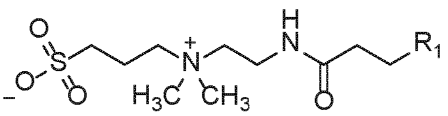

(III)

dans laquelle :

$R_1$ est un groupe de formule :

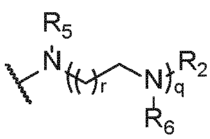

dans laquelle :
$R_5$, $R_6$ et $R_2$ sont chacun indépendamment un atome d'hydrogène ou un groupe alkyle$_{(C\leq 8)}$, -alcanediyl$_{(C\leq 6)}$-$NH_2$, - alcanediyl$_{(C\leq 6)}$-alkylamino$_{(C\leq 8)}$, - alcanediyl$_{(C\leq 6)}$-dialkylamino$_{(C\leq 12)}$, - alcanediyl$_{(C\leq 6)}$-NR'R", ou une version substituée de l'un quelconque de ces groupes dans laquelle :

R' et R" sont chacun indépendamment un hydrogène, un groupe alkyle$_{(C\leq 8)}$, alkyle$_{(C\leq 8)}$ substitué ou -$Z_2$A'$R_7$ ; dans laquelle :

$Z_2$ est un groupe alcanediyle$_{(C\leq 4)}$ ou alcanediyle$_{(C\leq 4)}$ substitué ;
A' est -CH$R_j$-, -C(O)O- ou -C(O)N$R_b$- ;

$R_b$ est un hydrogène, un groupe alkyle$_{(C\leq6)}$ ou alkyle$_{(C\leq6)}$ substitué ; et
$R_j$ est un hydrogène, un groupe halogéno, hydroxy, acyloxy$_{(C\leq24)}$ ou acyloxy$_{(C\leq24)}$ substitué ;

$R_7$ est un groupe alkyle$_{(C6-24)}$, alkyle$_{(C6-24)}$ substitué, alcényle$_{(C6-24)}$, alcényle$_{(C6-24)}$ substitué ; ou

$R_5$, $R_6$ et $R_2$ sont chacun indépendamment - $Z_3A''R_8$ ; dans laquelle :

$Z_3$ est un groupe alcanediyle$_{(C\leq4)}$ ou alcanediyle$_{(C\leq4)}$ substitué ;
A" est -$CHR_k$-, -C(O)O- ou -C(O)$NR_1$- ;

$R_1$ est un hydrogène, un groupe alkyle$_{(C\leq6)}$ ou alkyle$_{(C\leq6)}$ substitué ; et
$R_k$ est un hydrogène, un groupe halogéno, hydroxy, acyloxy$_{(C\leq24)}$ ou acyloxy$_{(C\leq24)}$ substitué ; et

$R_8$ est un groupe alkyle$_{(C6-24)}$, alkyle$_{(C6-24)}$ substitué, alcényle$_{(C6-24)}$, alcényle$_{(C6-24)}$ substitué ;

q vaut 1, 2 ou 3 ; et
r vaut 1, 2, 3 ou 4 ;

ou un sel pharmaceutiquement acceptable de celui-ci ;
dans laquelle, lorsqu'un terme est utilisé avec le modificateur « substitué », ou que l'expression « version substituée » est utilisée, un ou plusieurs atomes d'hydrogène ont été remplacés indépendamment par -OH, -F, -Cl, -Br, -I, -$NH_2$, -$NO_2$, -$CO_2H$, -$CO_2CH_3$, -CN, - SH, -$OCH_3$, -$OCH_2CH_3$, -C(O)$CH_3$, -$NHCH_3$, -$NHCH_2CH_3$, -$N(CH_3)_2$, -C(O)$NH_2$, -C(O)$NHCH_3$, -C(O)$N(CH_3)_2$, - OC(O)$CH_3$, -NHC(O)$CH_3$, -$S(O)_2OH$ ou -$S(O)_2NH_2$.

**12.** Composition destinée à être utilisée selon la revendication 6, dans laquelle :

(i) $R_3$ est un groupe alkyle$_{(C\leq8)}$ ou alkyle$_{(C\leq8)}$ substitué ; et/ou
(ii) $R_4$ est un groupe alkyle$_{(C\leq8)}$ ou alkyle$_{(C\leq8)}$ substitué ; et/ou
(iii) m vaut 1 ou 2 ; et/ou
(iv) n vaut 2 ou 3 ; et/ou
(v) p vaut 1, 2 ou 3 ; et/ou
(vi) $R_1$ est un groupe de formule :

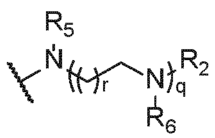

dans laquelle :

$R_5$, $R_6$ et $R_2$ sont chacun indépendamment un atome d'hydrogène ou un groupe alkyle$_{(C\leq8)}$, - alcanediyl$_{(C\leq6)}$-$NH_2$, -alcanediyl$_{(C\leq6)}$-alkylamino$_{(C\leq8)}$, -alcanediyl$_{(C\leq6)}$-dialkylamino$_{(C\leq12)}$, -alcanediyl$_{(C\leq6)}$-NR'R", ou une version substituée de l'un quelconque de ces groupes dans laquelle :
R' et R" sont chacun indépendamment un hydrogène, un groupe alkyle$_{(C\leq8)}$, alkyle$_{(C\leq8)}$ substitué, -$Z_2A'R_7$ ; dans laquelle :

$Z_2$ est un groupe alcanediyle$_{(C\leq4)}$ ou alcanediyle$_{(C\leq4)}$ substitué ;
A' est -$CHR_j$-, -C(O)O- ou -C(O)$NR_b$- ;

$R_b$ est un hydrogène, un groupe alkyle$_{(C\leq6)}$ ou alkyle$_{(C\leq6)}$ substitué ; et
$R_j$ est un hydrogène, un groupe halogéno, hydroxy, acyloxy$_{(c\leq24)}$ ou acyloxy$_{(c\leq24)}$ substitué ;

$R_7$ est un groupe alkyle$_{(c6-24)}$, alkyle$_{(c6-24)}$ substitué, alcényle$_{(c6-24)}$, alcényle$_{(c6-24)}$ substitué ; ou

$R_5$, $R_6$ et $X_1$ sont chacun indépendamment -$Z_3A''R_8$ ; dans laquelle :

$Z_3$ est un groupe alcanediyle$_{(c\leq4)}$ ou alcanediyle$_{(c\leq4)}$ substitué ;

A" est $-CHR_k-$, -C(O)O- ou $-C(O)NR_1-$ ;

$R_1$ est un hydrogène, un groupe alkyle$_{(c\leq6)}$ou alkyle$_{(c\leq6)}$ substitué ; et
$R_k$ est un hydrogène, un groupe halogéno, hydroxy, acyloxy$_{(c\leq24)}$ ou acyloxy$_{(c\leq24)}$ substitué ; et

Rs est un groupe alkyle$_{(c6-24)}$, alkyle$_{(c6-24)}$ substitué, alcényle$_{(c6-24)}$, alcényle$_{(C6-24}$) substitué ;

q vaut 1, 2 ou 3 ; et
r vaut 1, 2, 3 ou 4 ;
de préférence dans laquelle :

(a) q vaut 1 ou 2 ; et/ou
(b) $R_5$ est $-Z_3A''R_8$ ; dans laquelle :

$Z_3$ est un groupe alcanediyle $_{(c\leq4)}$ ou alcanediyle $_{(c\leq4)}$ substitué ;
A" est $-CHR_k-$, -C(O)O- ou $-C(O)NR_1-$ ; R1 est un hydrogène, un groupe alkyle$_{(c\leq6)}$ou alkyle$_{(c\leq6)}$ substitué ; et
$R_k$ est un hydrogène, un groupe halogéno, hydroxy, acyloxy$_{(c\leq24)}$ ou acyloxy$_{(c\leq24)}$ substitué ; et
$R_8$ est un groupe alkyle$_{(c6-24)}$, alkyle$_{(c6-24)}$ substitué, alcényle$_{(c6-24)}$, alcényle$_{(c6-24)}$ substitué ; et/ou

(c) $R_6$ est $-Z_3A''R_8$ ; dans laquelle :

$Z_3$ est un groupe alcanediyle$_{(c\leq4)}$ ou alcanediyle$_{(c\leq4)}$ substitué ;
A'' est $-CHR_k-$, -C(O)O- ou $-C(O)NR_1-$ ; $R_1$ est un hydrogène, un groupe alkyle$_{(c\leq6)}$ ou alkyle$_{(c\leq6)}$ substitué ; et
$R_k$ est un hydrogène, un groupe halogéno, hydroxy, acyloxy$_{(c\leq24)}$ ou acyloxy$_{(c\leq24)}$; substitué ; et
$R_8$ est un groupe alkyle$_{(c6-24)}$, alkyle$_{(c6-24)}$ substitué, alcényle$_{(c6-24)}$, alcényle$_{(c6-24)}$ substitué ; et/ou

(d) $R_2$ est $-Z_3A''R_8$ ; dans laquelle :

$Z_3$ est un groupe alcanediyle$_{(c\leq4)}$ ou alcanediyle$_{(c\leq4)}$ substitué ;
A" est $-CHR_k-$, -C(O)O- ou $-C(O)NR_1-$ ; R1 est un hydrogène, un groupe alkyle$_{(c\leq6)}$ ou alkyle$_{(c\leq6)}$ substitué ; et
$R_k$ est un hydrogène, un groupe halogéno, hydroxy, acyloxy$_{(c\leq24)}$ ou acyloxy$_{(c\leq24)}$ substitué ; et
$R_8$ est un groupe alkyle$_{(c6-24)}$, alkyle$_{(c6-24)}$ substitué, alcényle$_{(c6-24)}$, alcényle$_{(c6-24)}$ substitué .

**13.** Composition destinée à être utilisée selon l'une quelconque des revendications 2 à 12, dans laquelle l'ARNt est un ARNt suppresseur ;
éventuellement dans laquelle

l'ARNt est un ARNt qui administre un acide aminé dans une protéine au lieu de terminer la traduction ; ou
l'ARNt est un ARNt suppresseur ambre, un ARNt suppresseur opale, un ARNt suppresseur ocre ou un ARNt suppresseur de décalage du cadre de lecture ; ou
l'ARNt corrige une mutation non-sens de la protéine régulatrice de la conductance transmembranaire de la mucoviscidose.

**14.** Composition destinée à être utilisée selon l'une quelconque des revendications 2 à 13, dans laquelle la composition comprend en outre un stéroïde ou un dérivé de stéroïde ; et/ou

dans laquelle la composition comprend en outre un phospholipide ; et/ou
dans laquelle la composition comprend en outre un lipide PEG.

**15.** Composition selon la revendication 1, ou composition destinée à être utilisée selon l'une quelconque des revendications 2 à 14, destinée à être utilisée dans un ou plusieurs des éléments suivants :

(i) restauration de l'expression de la protéine p53 ;
(ii) administration d'un acide aminé dans une protéine au lieu de terminer la traduction ; et
(iii) correction d'une mutation non-sens dans une protéine régulatrice de la conductance transmembranaire de

la mucoviscidose (CFTR).

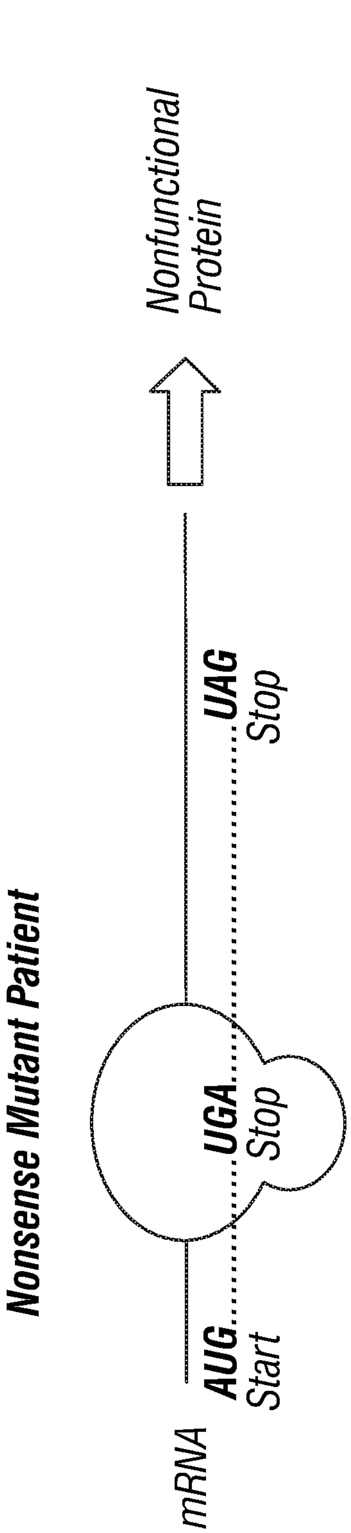
Nonsense Mutant Patient
mRNA
AUG
Start
UGA
Stop
UAG
Stop
Nonfunctional
Protein

FIG. 1

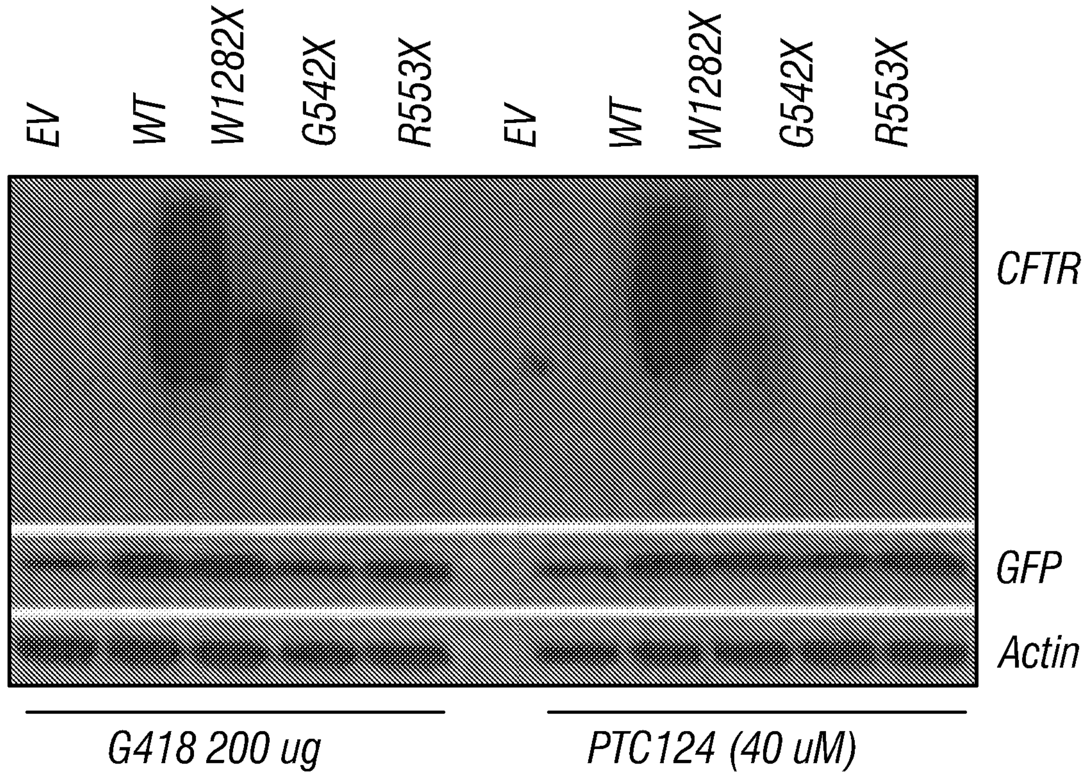
EV
WT
W1282X
G542X
R553X
EV
WT
W1282X
G542X
R553X
CFTR
GFP
Actin
G418 200 ug
PTC124 (40 uM)

FIG. 2A

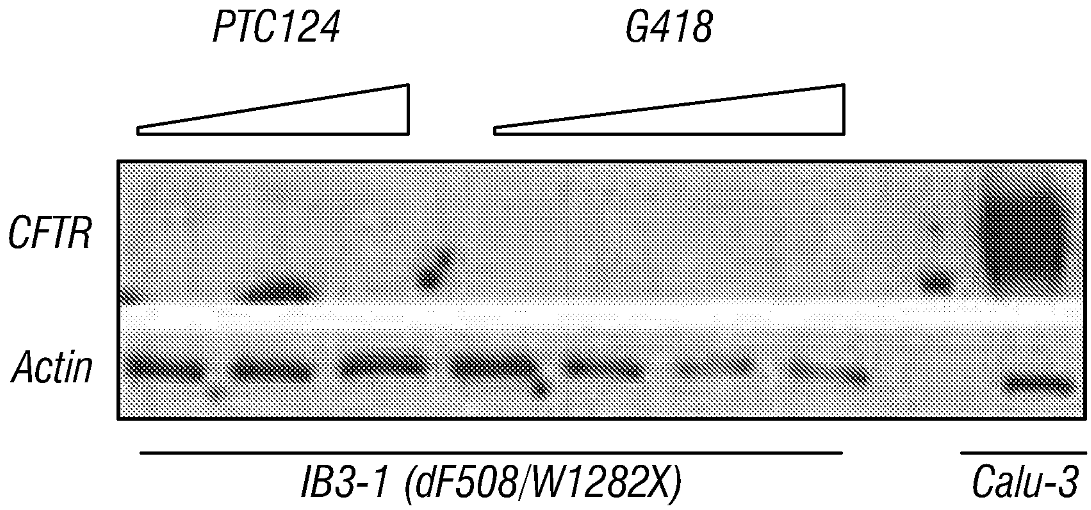
PTC124
G418
CFTR
Actin
IB3-1 (dF508/W1282X)
Calu-3

FIG. 2B

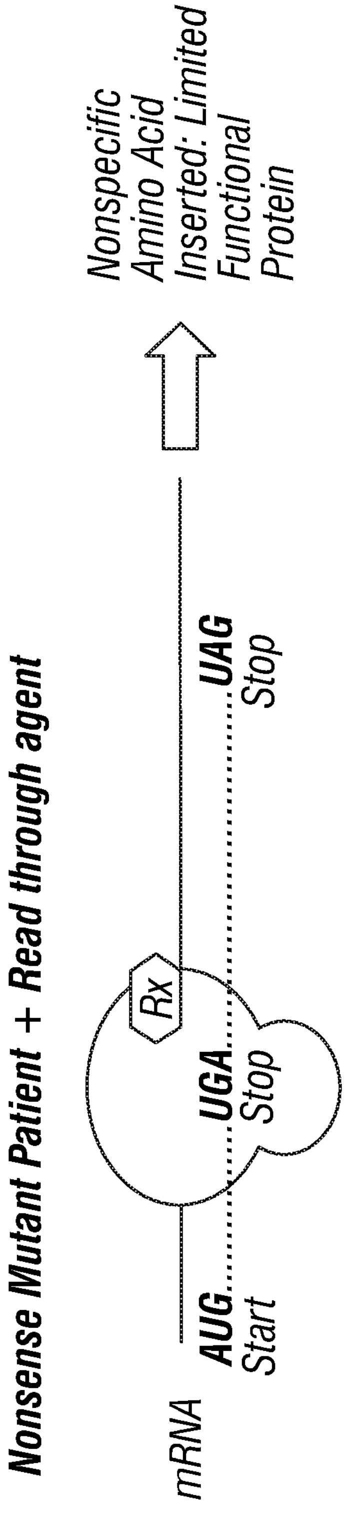
Nonsense Mutant Patient + Read through agent
mRNA
AUG
Start
Rx
UGA
Stop
UAG
Stop
Nonspecific Amino Acid Inserted: Limited Functional Protein

FIG. 2C

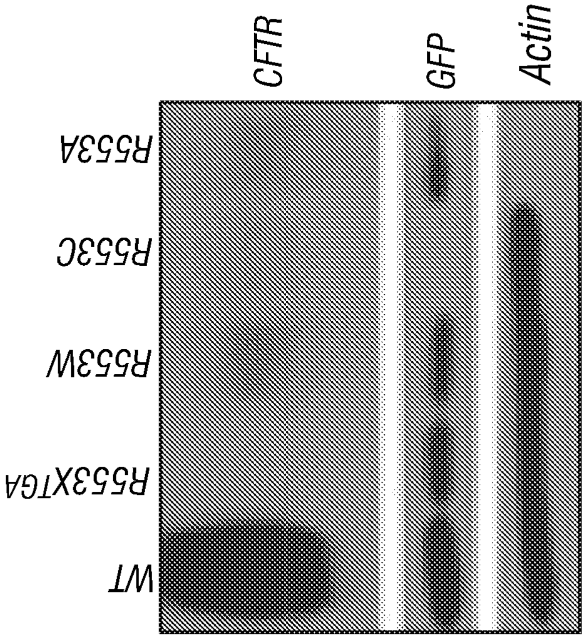
CFTR
GFP
Actin
R553A
R553C
R553W
R553XTGA
WT

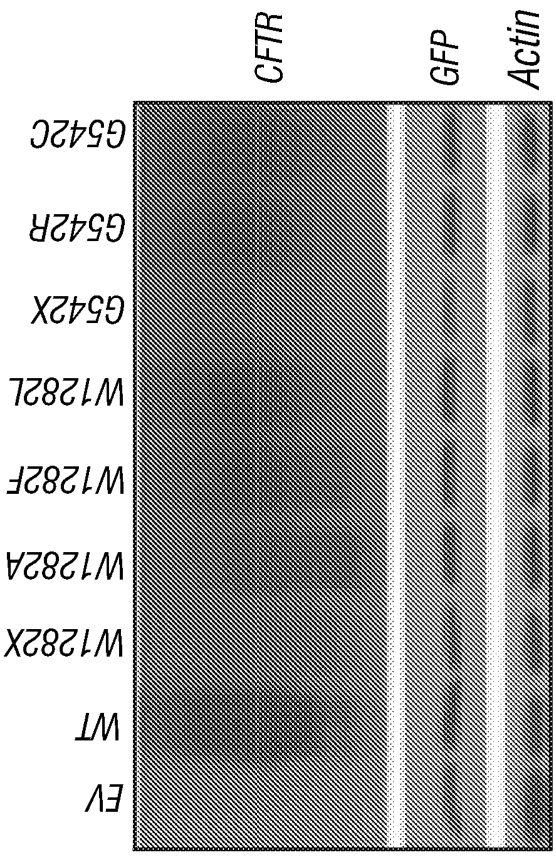
CFTR
GFP
Actin
G542C
G542R
G542X
W1282L
W1282F
W1282A
W1282X
WT
EV

FIG. 3A

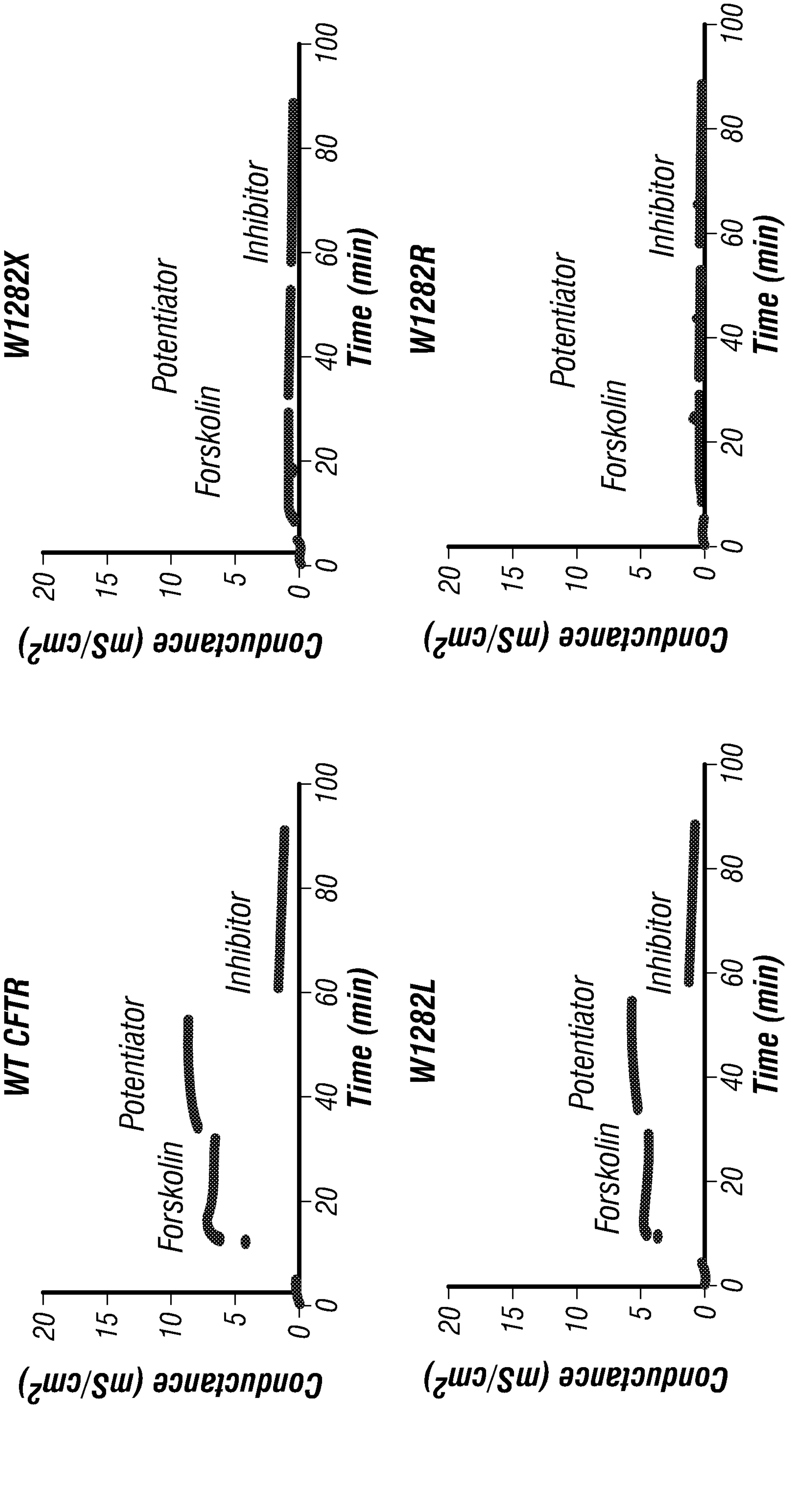
W1282X
Conductance (mS/cm2)
Forskolin
Potentiator
Inhibitor
Time (min)
W1282R
Conductance (mS/cm2)
Forskolin
Potentiator
Inhibitor
Time (min)
WT CFTR
Conductance (mS/cm2)
Forskolin
Potentiator
Inhibitor
Time (min)
W1282L
Conductance (mS/cm2)
Forskolin
Potentiator
Inhibitor
Time (min)

FIG. 3B

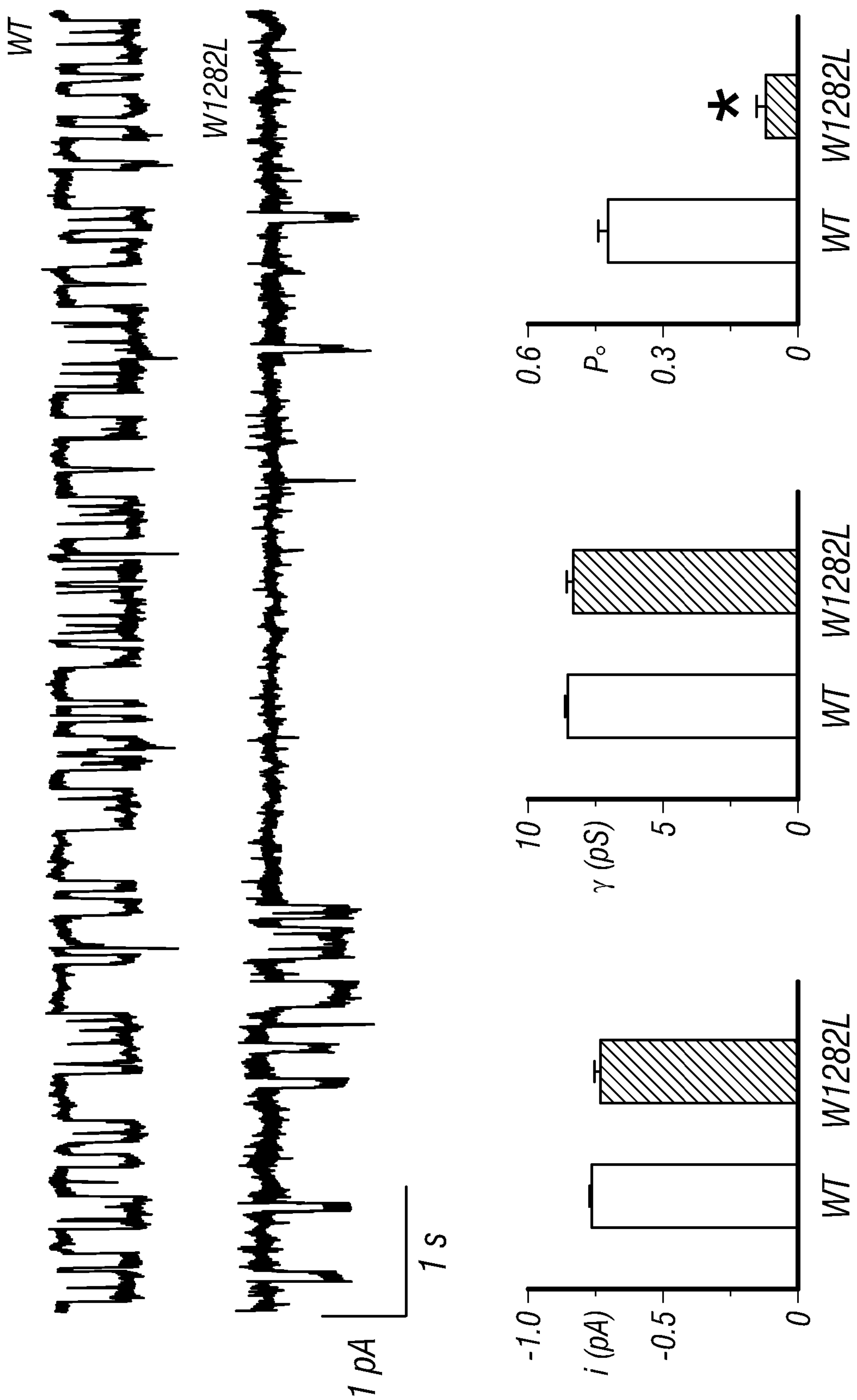
WT
W1282L
1 pA
1 s
i (pA)
-1.0
-0.5
0
WT
W1282L
γ (pS)
10
5
0
WT
W1282L
$P_o$
0.6
0.3
0
WT
W1282L
*

FIG. 4A

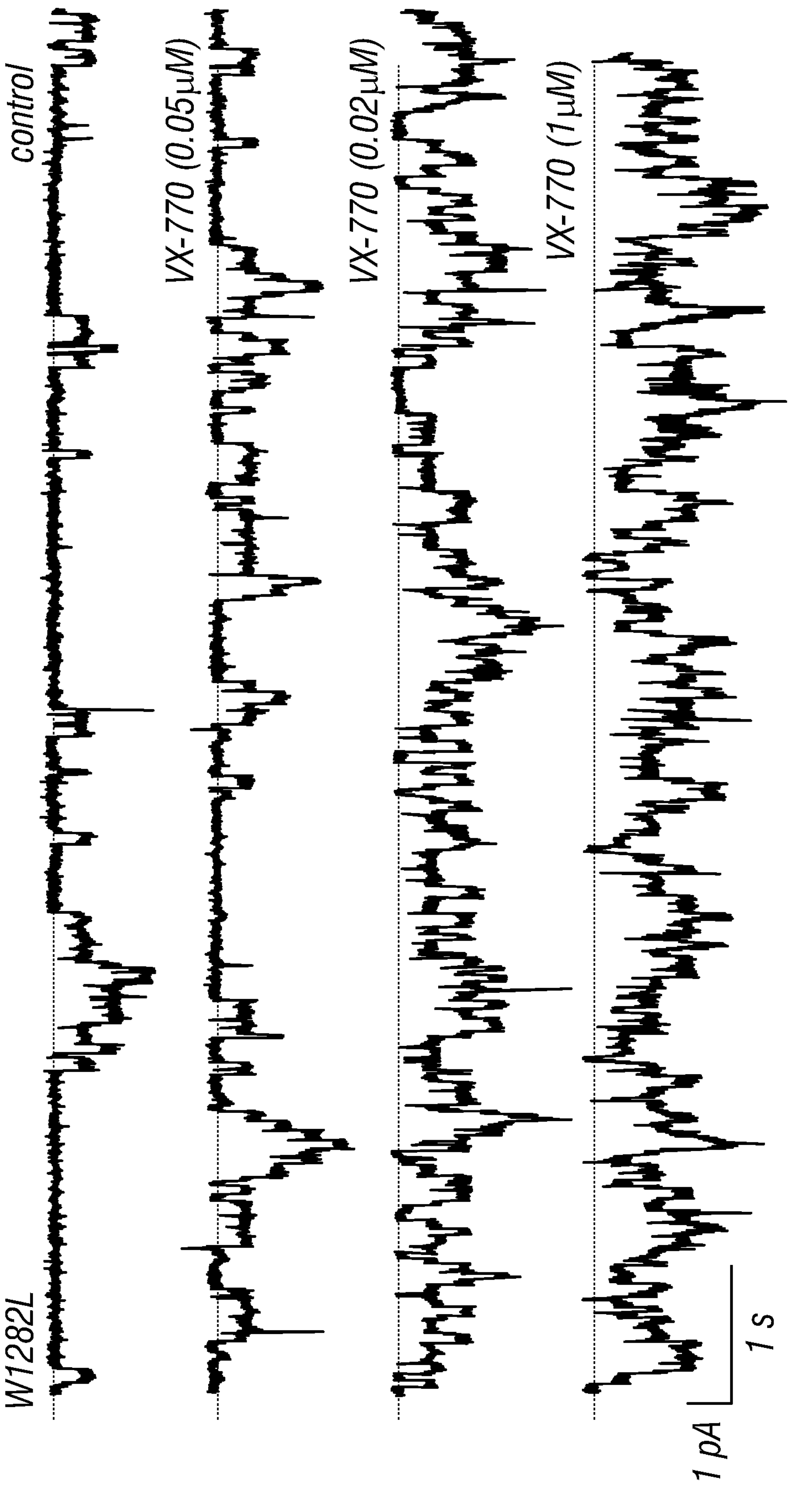


FIG. 4B

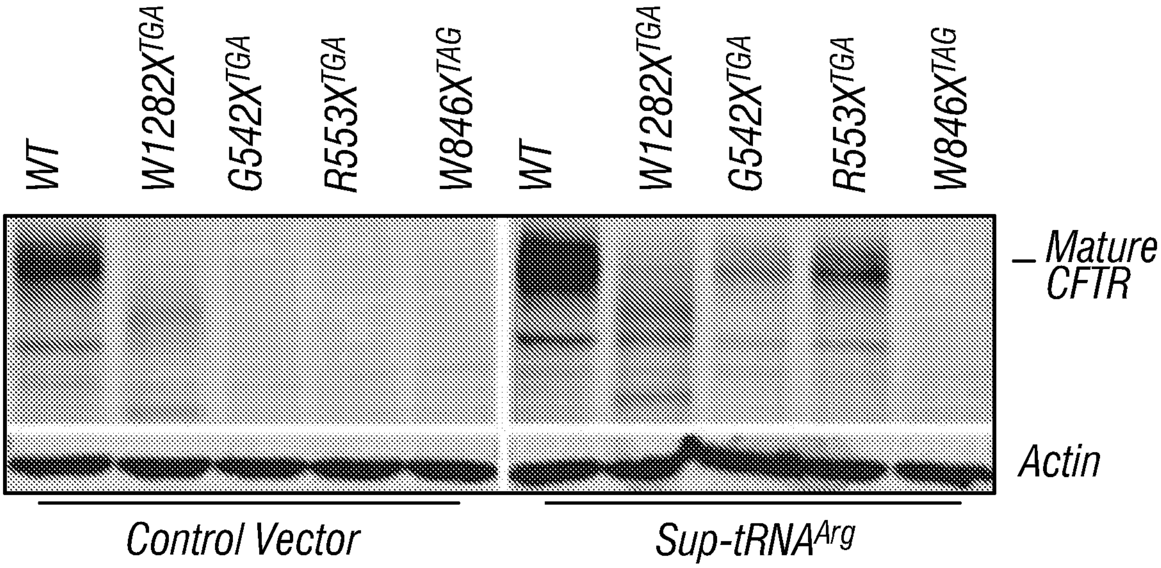
WT
W1282X^TGA
G542X^TGA
R553X^TGA
W846X^TAG
WT
W1282X^TGA
G542X^TGA
R553X^TGA
W846X^TAG
Mature CFTR
Actin
Control Vector
Sup-tRNA^Arg

FIG. 5A

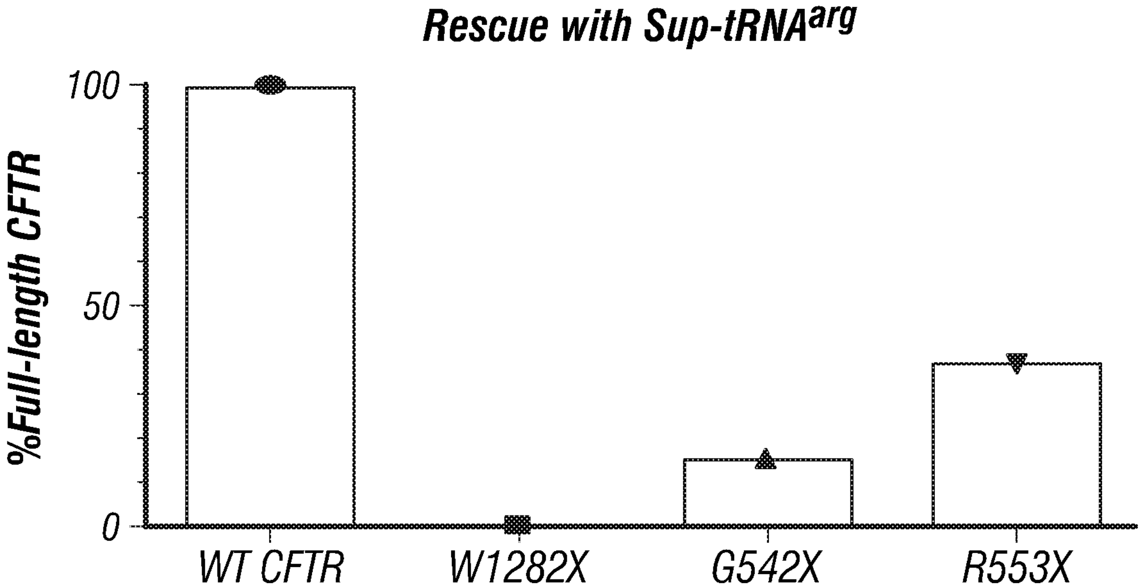
Rescue with Sup-tRNA^arg
%Full-length CFTR
100
50
0
WT CFTR
W1282X
G542X
R553X

FIG. 5B

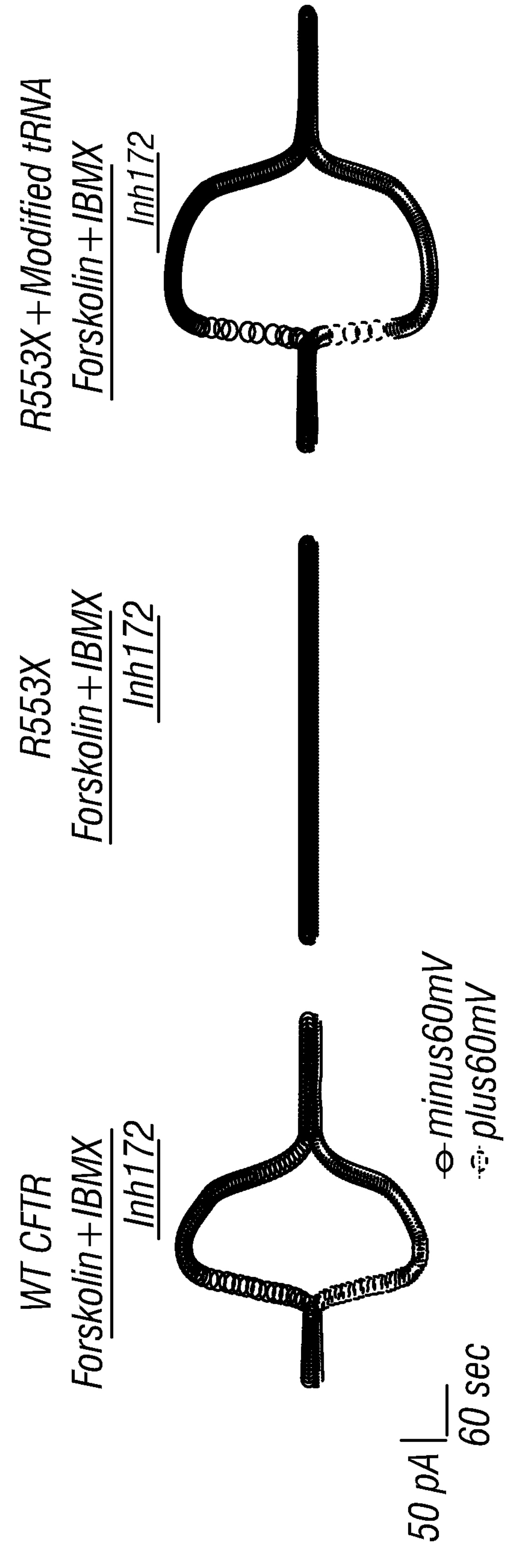
R553X+Modified tRNA
Forskolin+IBMX
Inh172
R553X
Forskolin+IBMX
Inh172
WT CFTR
Forskolin+IBMX
Inh172
minus60mV
plus60mV
50 pA
60 sec

FIG. 5C

**RCT-Z100**

*Cell membrane*
*Labeled tRNA*
*Cell nuclei*

*Labeled tRNA*

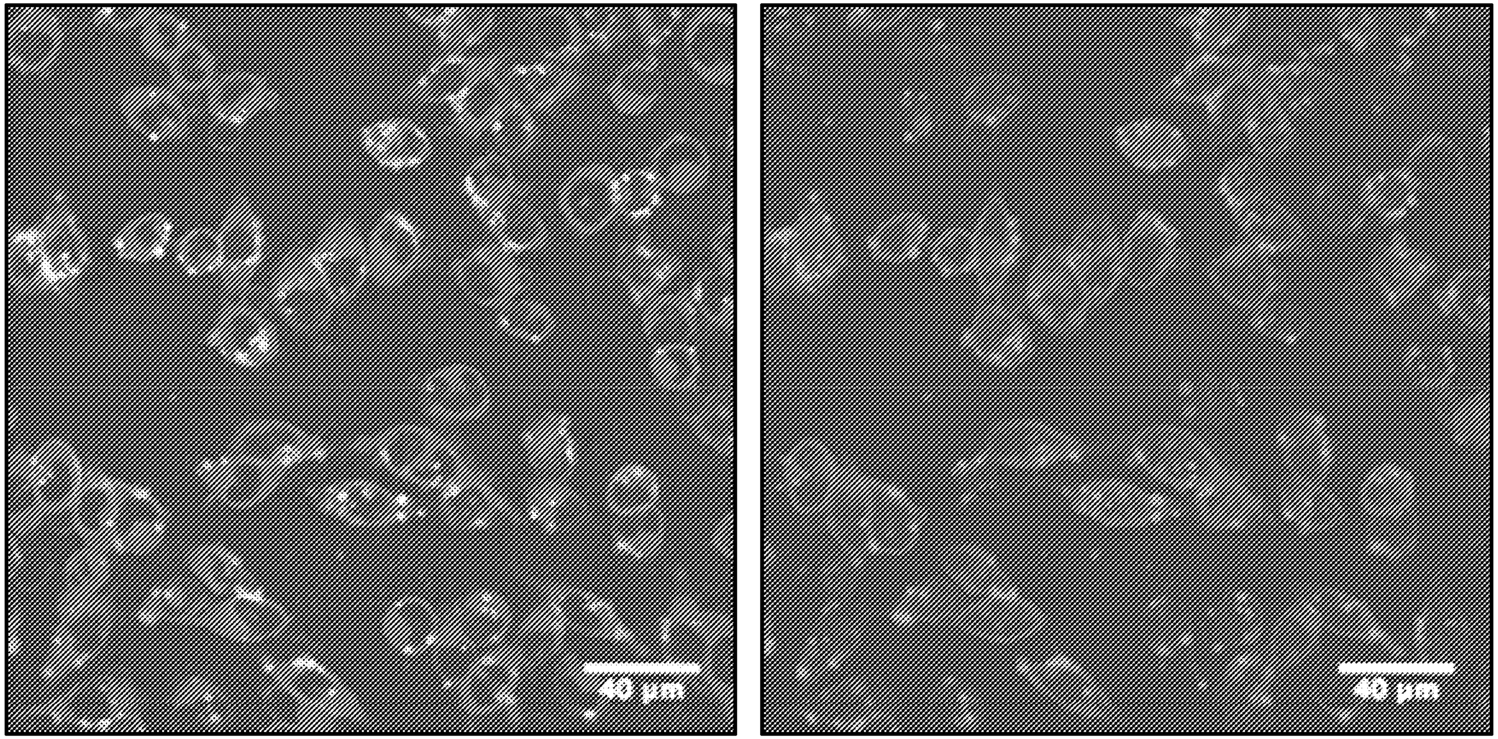


*Calu6 cells, 0.9 μg/mL, tRNA, 6 h*

**FIG. 6A**

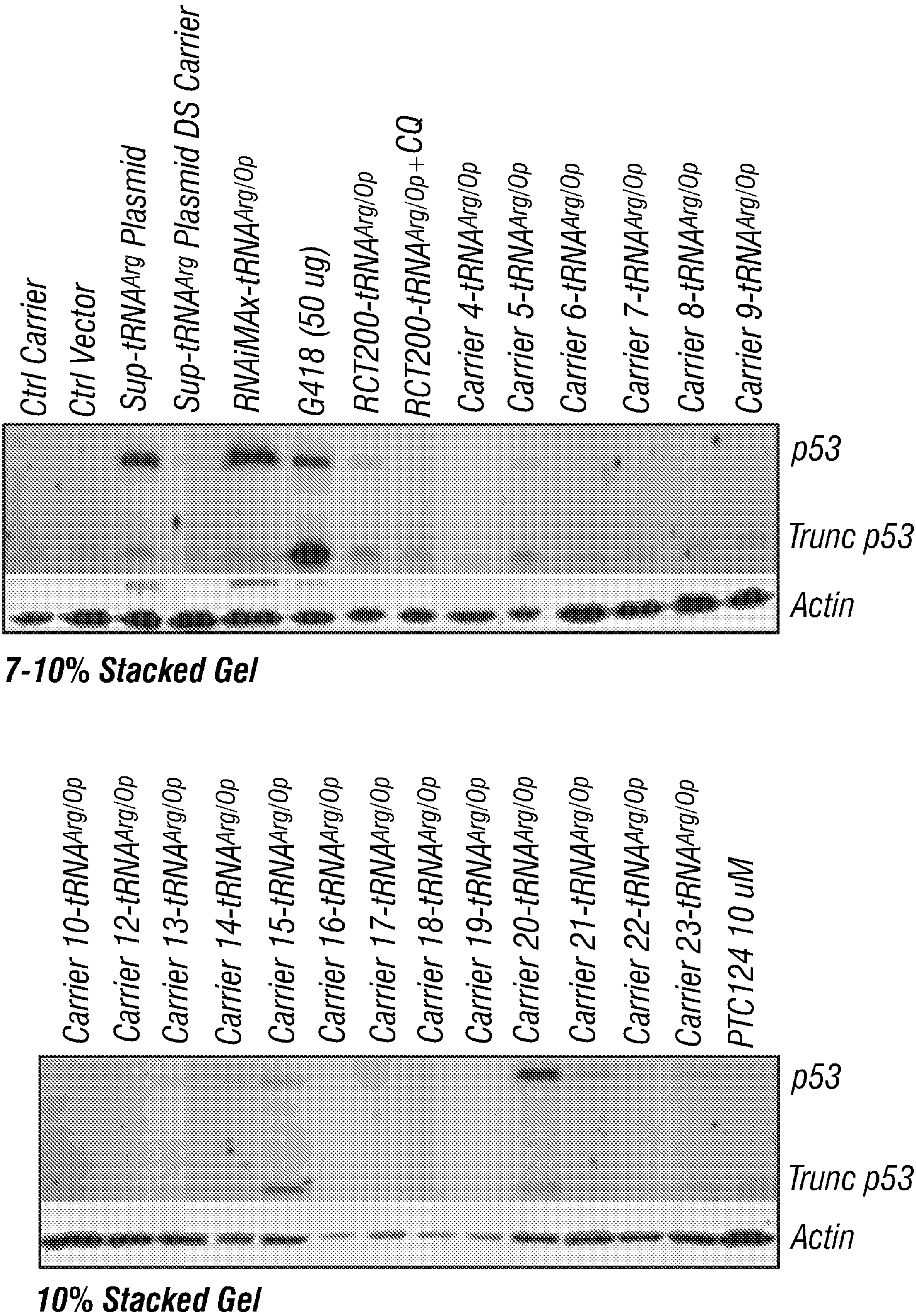


*Calu6 cells P53 196X (TGA)*
*48hr Post Transfection (4ug tRNA dose)*

**FIG. 6B**

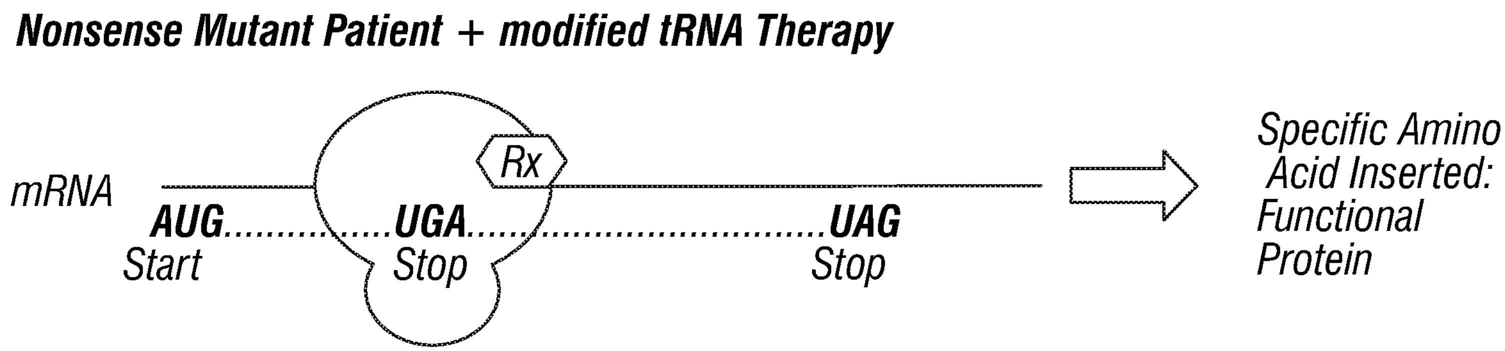
Nonsense Mutant Patient + modified tRNA Therapy
mRNA
Rx
AUG
Start
UGA
Stop
UAG
Stop
Specific Amino Acid Inserted: Functional Protein

FIG. 6C

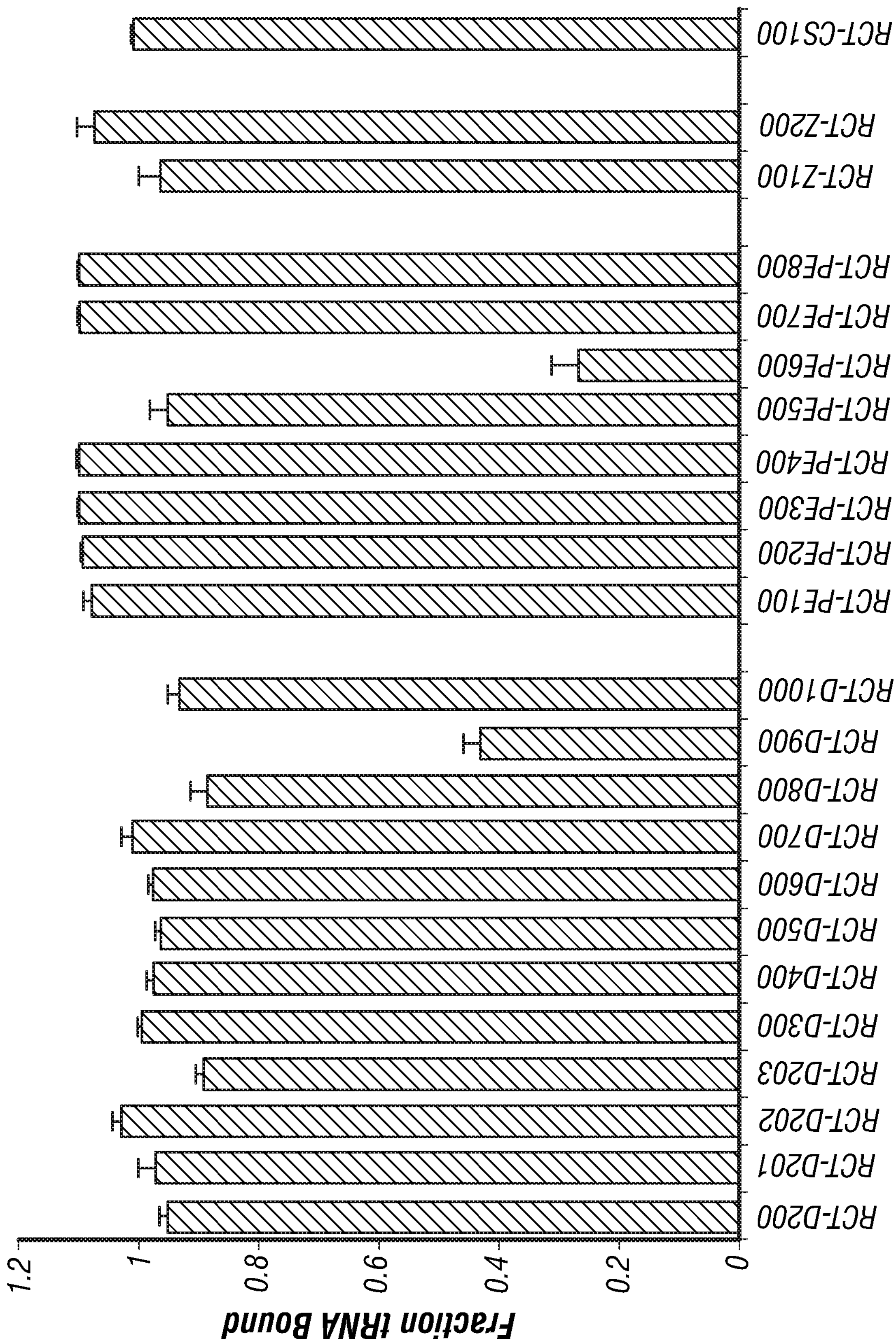
Fraction tRNA Bound
0
0.2
0.4
0.6
0.8
1
1.2
RCT-D200
RCT-D201
RCT-D202
RCT-D203
RCT-D300
RCT-D400
RCT-D500
RCT-D600
RCT-D700
RCT-D800
RCT-D900
RCT-D1000
RCT-PE100
RCT-PE200
RCT-PE300
RCT-PE400
RCT-PE500
RCT-PE600
RCT-PE700
RCT-PE800
RCT-Z100
RCT-Z200
RCT-CS100

FIG. 7

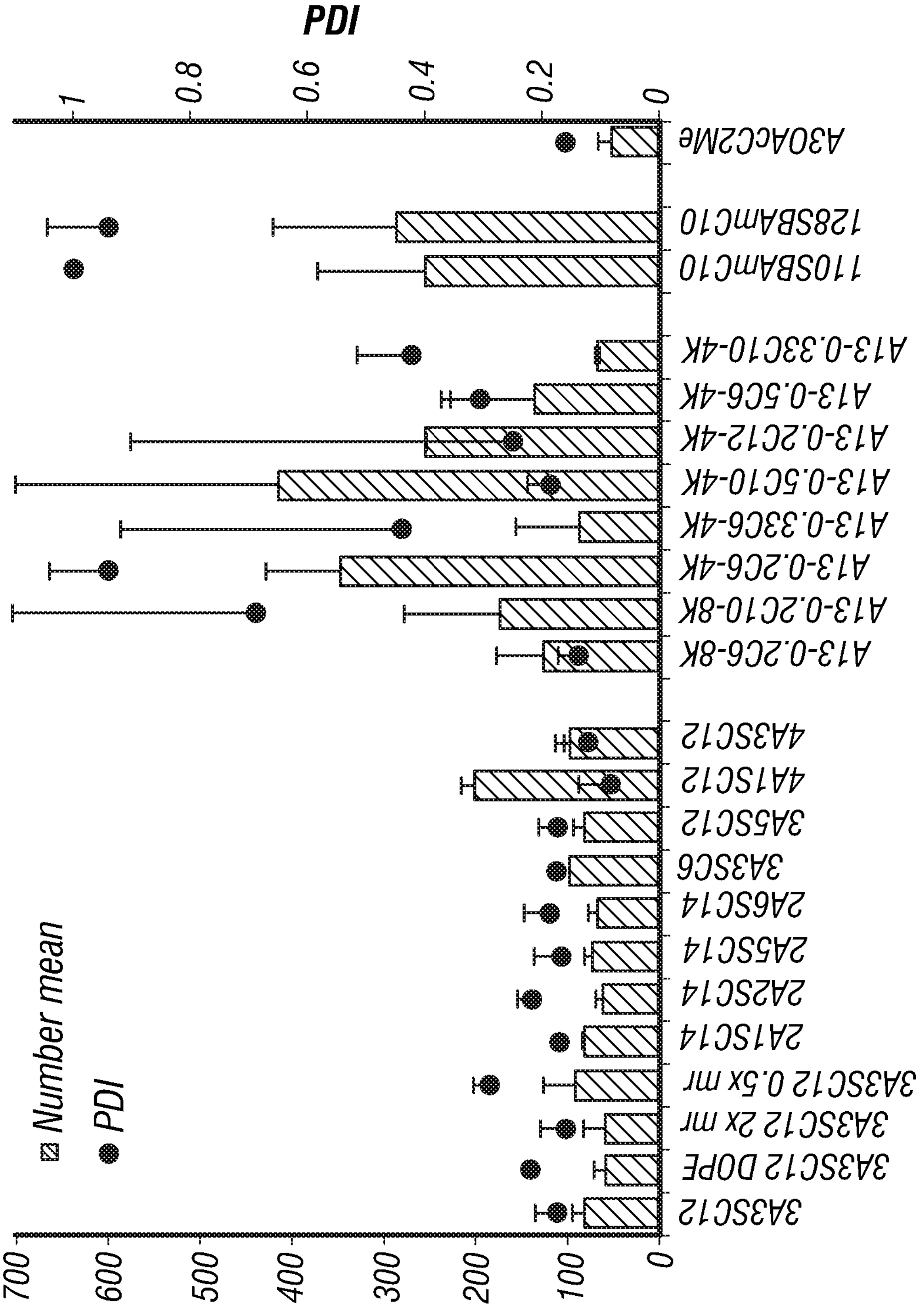
PDI
0
0.2
0.4
0.6
0.8
1
Dh.nm
0
100
200
300
400
500
600
700
Number mean
PDI
3A3SC12
3A3SC12 DOPE
3A3SC12 2x mr
3A3SC12 0.5x mr
2A1SC14
2A2SC14
2A5SC14
2A6SC14
3A3SC6
3A5SC12
4A1SC12
4A3SC12
A13-0.2C6-8K
A13-0.2C10-8K
A13-0.2C6-4K
A13-0.33C6-4K
A13-0.5C10-4K
A13-0.2C12-4K
A13-0.5C6-4K
A13-0.33C10-4K
110SBAmC10
128SBAmC10
A30AcC2Me

FIG. 8

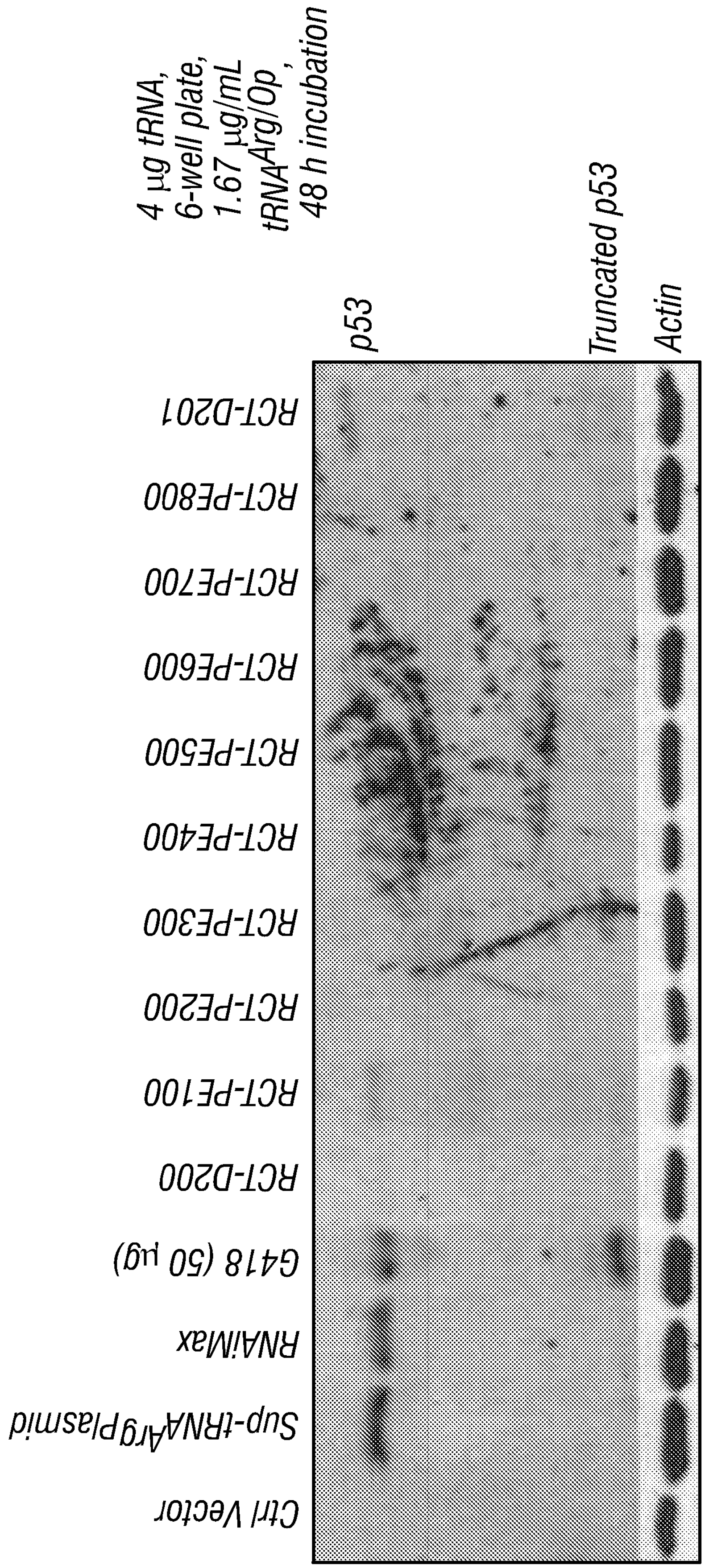
4 μg tRNA,
6-well plate,
1.67 μg/mL
tRNA Arg/Op ,
48 h incubation
p53
Truncated p53
Actin
Ctrl Vector
Sup-tRNA Arg Plasmid
RNAiMax
G418 (50 μg)
RCT-D200
RCT-PE100
RCT-PE200
RCT-PE300
RCT-PE400
RCT-PE500
RCT-PE600
RCT-PE700
RCT-PE800
RCT-D201

FIG. 9

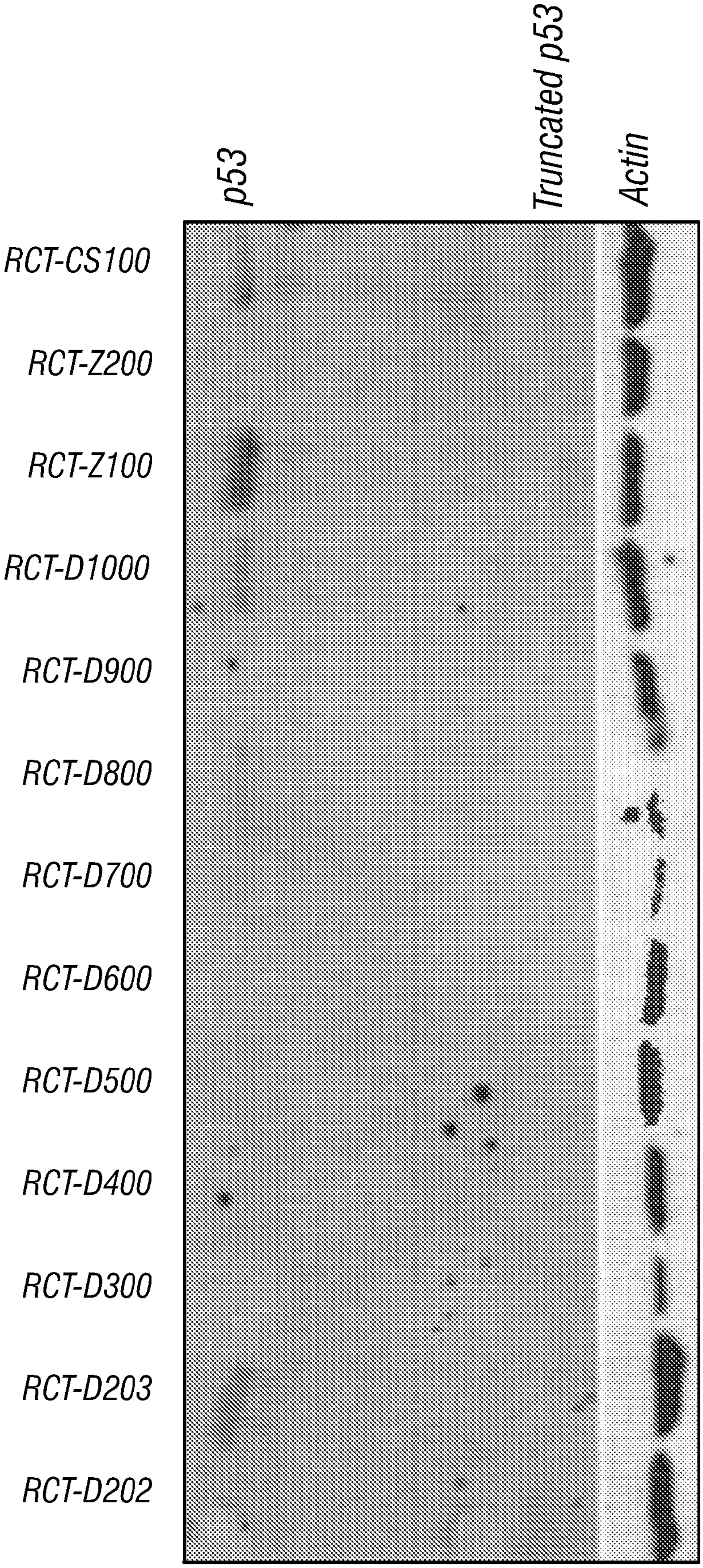
p53
Truncated p53
Actin
RCT-CS100
RCT-Z200
RCT-Z100
RCT-D1000
RCT-D900
RCT-D800
RCT-D700
RCT-D600
RCT-D500
RCT-D400
RCT-D300
RCT-D203
RCT-D202

FIG. 9 (Cont'd)

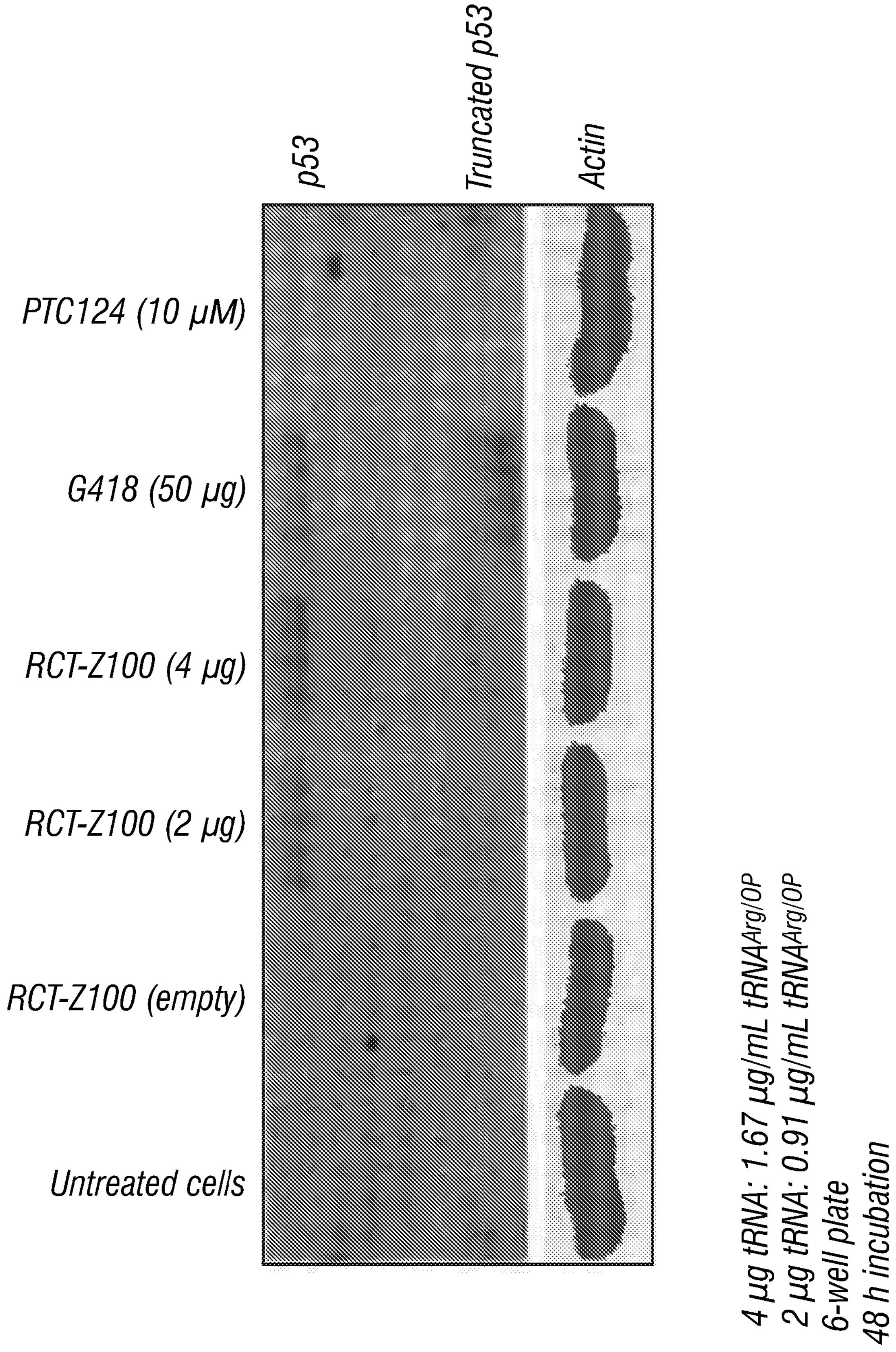
p53
Truncated p53
Actin
PTC124 (10 μM)
G418 (50 μg)
RCT-Z100 (4 μg)
RCT-Z100 (2 μg)
RCT-Z100 (empty)
Untreated cells
4 μg tRNA: 1.67 μg/mL tRNAArg/OP
2 μg tRNA: 0.91 μg/mL tRNAArg/OP
6-well plate
48 h incubation

FIG. 10

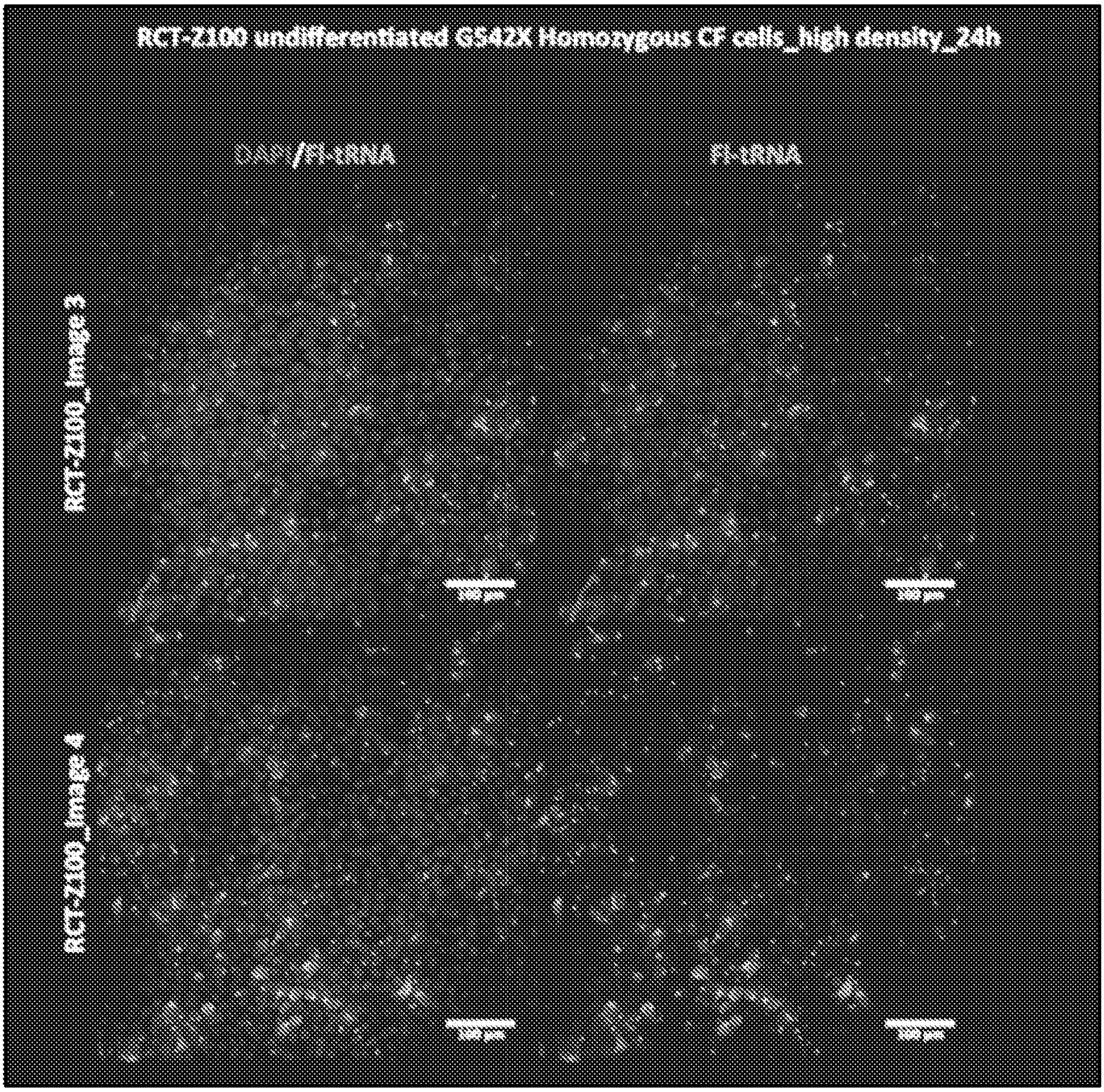
RCT-Z100 undifferentiated G542X Homozygous CF cells_high density_24h
DAPI/Fl-tRNA
Fl-tRNA
RCT-Z100_Image 3
RCT-Z100_Image 4

*FIG. 11*

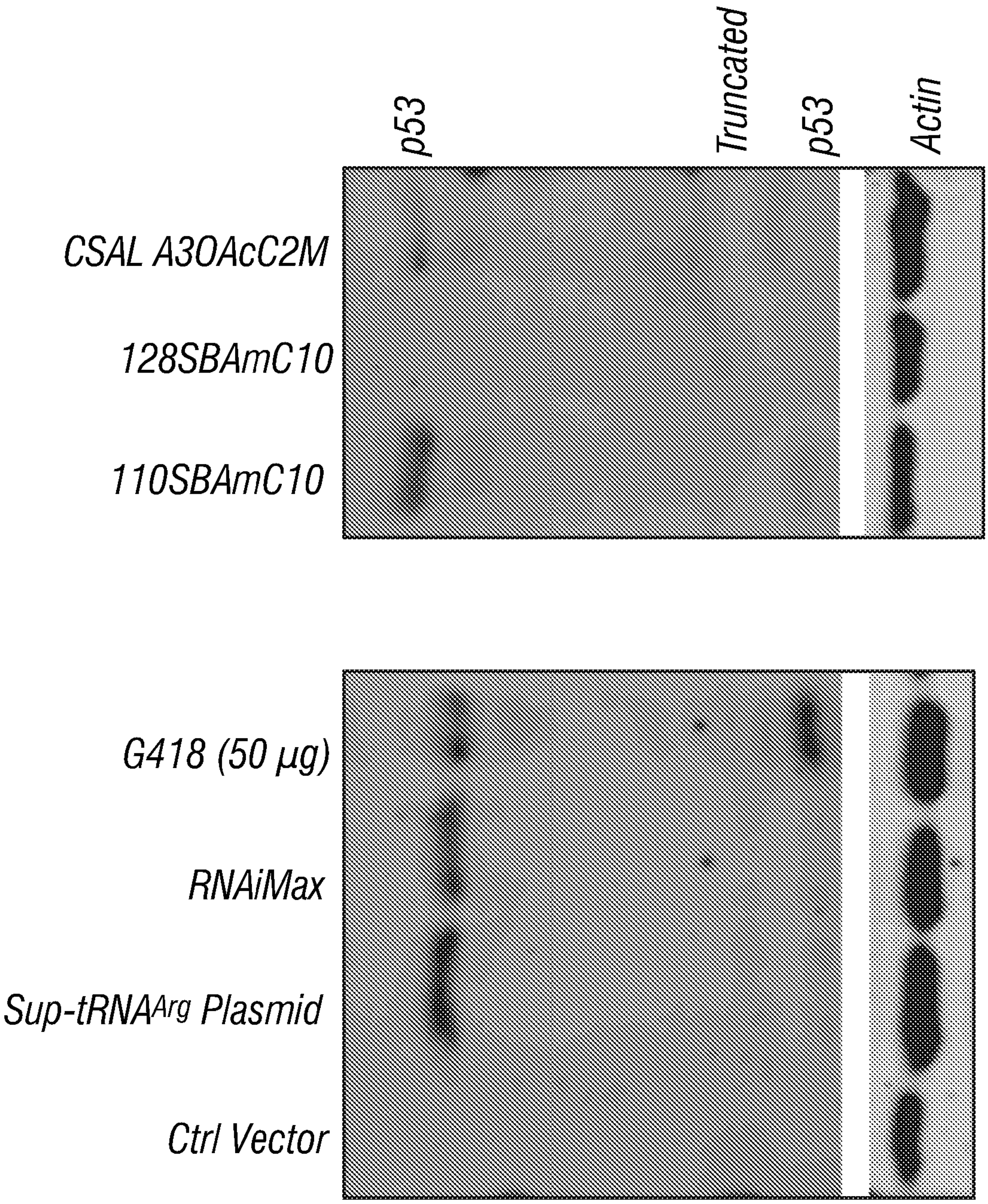
p53
Truncated p53
Actin
CSAL A30AcC2M
128SBAmC10
110SBAmC10
G418 (50 µg)
RNAiMax
Sup-tRNAArg Plasmid
Ctrl Vector

FIG. 12

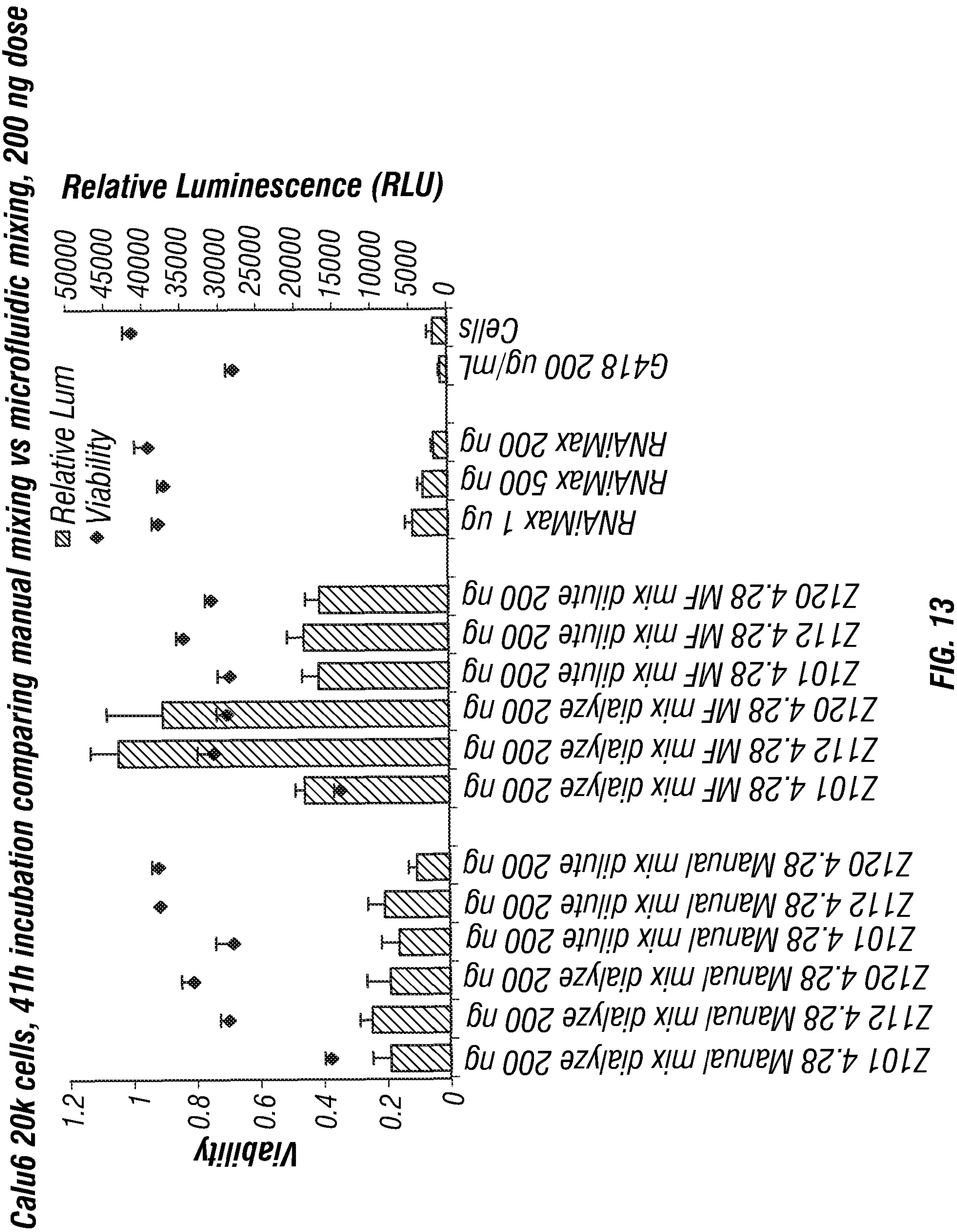
Calu6 20k cells, 41h incubation comparing manual mixing vs microfluidic mixing, 200 ng dose
Relative Luminescence (RLU)
50000
45000
40000
35000
30000
25000
20000
15000
10000
5000
0
Viability
1.2
1
0.8
0.6
0.4
0.2
0
Relative Lum
Viability
Cells
G418 200 ug/mL
RNAiMax 200 ng
RNAiMax 500 ng
RNAiMax 1 ug
Z120 4.28 MF mix dilute 200 ng
Z112 4.28 MF mix dilute 200 ng
Z101 4.28 MF mix dilute 200 ng
Z120 4.28 MF mix dialyze 200 ng
Z112 4.28 MF mix dialyze 200 ng
Z101 4.28 MF mix dialyze 200 ng
Z120 4.28 Manual mix dilute 200 ng
Z112 4.28 Manual mix dilute 200 ng
Z101 4.28 Manual mix dilute 200 ng
Z120 4.28 Manual mix dialyze 200 ng
Z112 4.28 Manual mix dialyze 200 ng
Z101 4.28 Manual mix dialyze 200 ng

FIG. 13

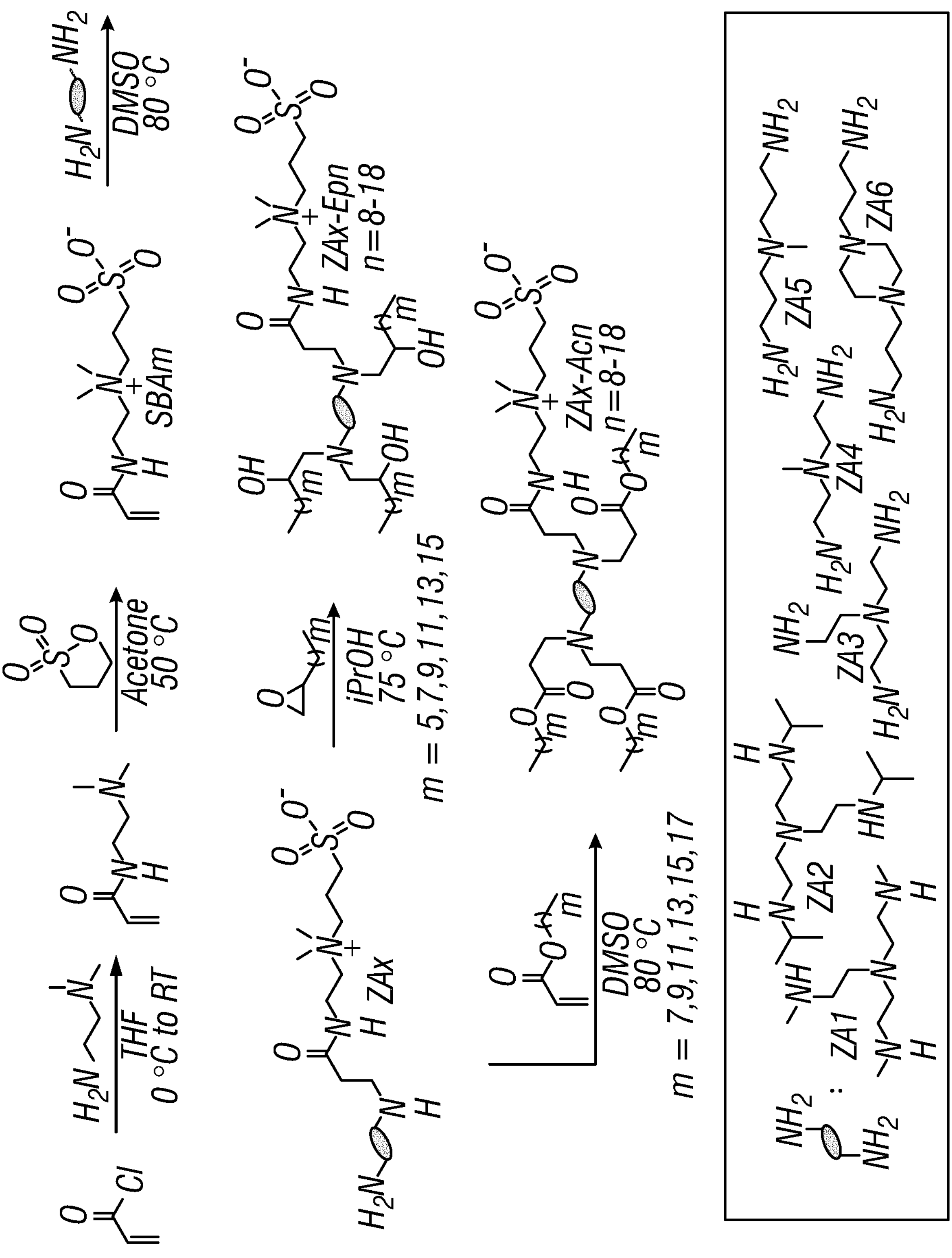
THF
0 °C to RT
Acetone
50 °C
SBAm
DMSO
80 °C
ZAx
iPrOH
75 °C
m = 5,7,9,11,13,15
ZAx-Epn
n=8-18
DMSO
80 °C
m = 7,9,11,13,15,17
ZAx-Acn
n=8-18
ZA1
ZA2
ZA3
ZA4
ZA5
ZA6

**FIG. 14**

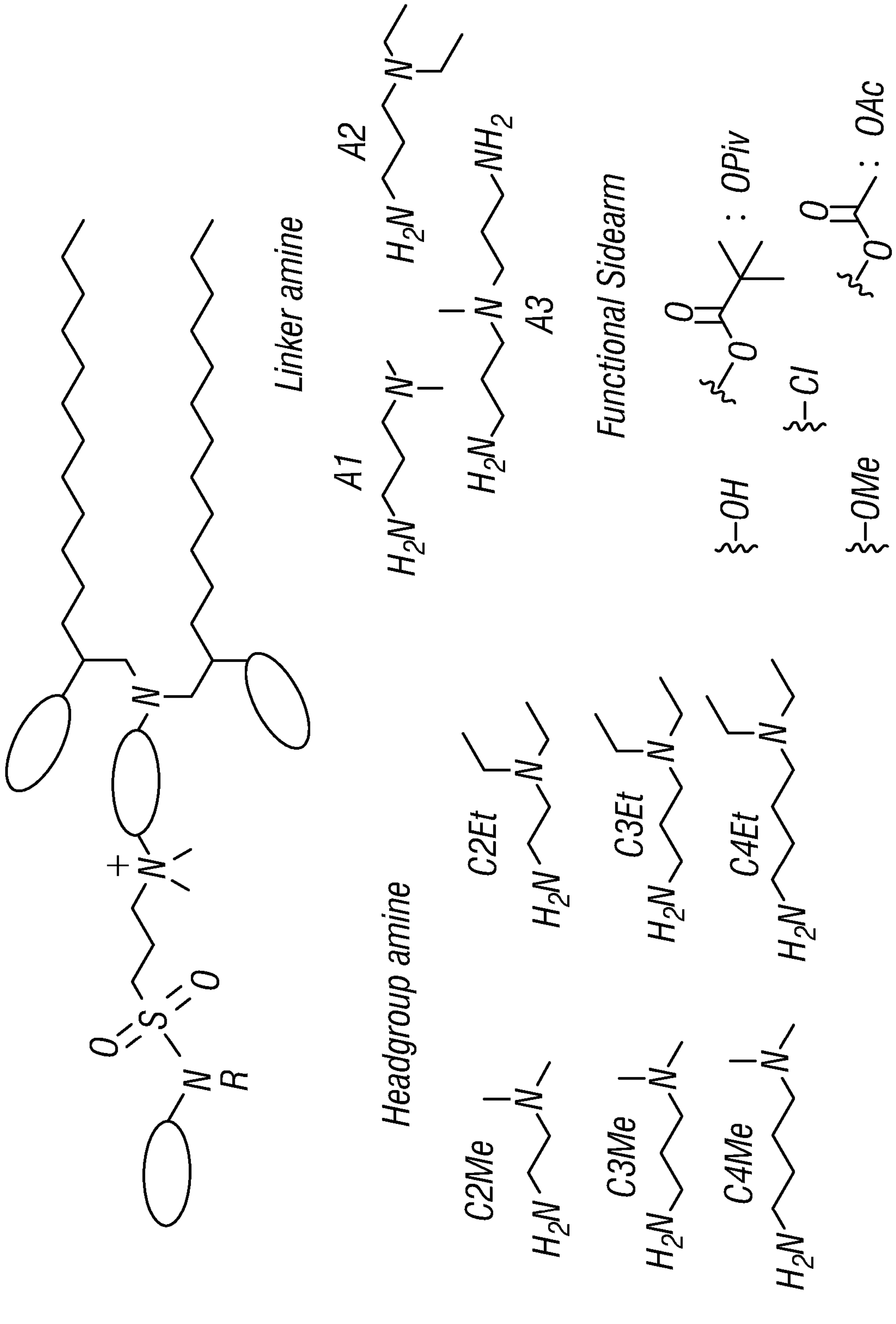

Headgroup amine

C2Me

C3Me

C4Me

C2Et

C3Et

C4Et

Linker amine

A1

A2

A3

Functional Sidearm

: OPiv

: OAc

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20150297749 A
- WO 2016094342 A
- WO 2017048789 A
- US 2017032950 W
- US 5820873 A
- WO 2010141069 A
- US 8450298 B
- US 5801005 A
- US 5739169 A
- US 5830880 A
- US 5846945 A
- US 5824311 A


### Non-patent literature cited in the description


- *March's Advanced Organic Chemistry: Reactions, Mechanisms, and Structure,* 2007
- Handbook of Pharmaceutical Salts: Properties, and Use. 2002
- Remington's Pharmaceutical Sciences. 1035-1038, 1570-1580
- Handbook of Pharmaceutical Salts: Properties, and Use. Verlag Helvetica Chimica Acta, 2002
- **SÖLL et al.** tRNA: Structure, Biosynthesis, and Function. ASM Press, 1995
- **EL YACOUBI et al.** *Annu. Rev. Genet.,* 2012, vol. 46, 69-95
- **GROSJEAN ; BENNE.** Modification and Editing of RNA. ASM Press, 1998
- **HENDRICKSON et al.** *Annu. Rev. Biochem.,* 2004, vol. 73, 147-176
- **IBBA ; SOIL.** *Annu. Rev. Biochem.,* 2000, vol. 69, 617-650
- **JOHNSON et al.** *Cold Spring Harbor Symp. Quant. Biol.,* 1995, vol. 60, 71-82
- **JOHNSON et al.** *J. Mol. Biol.,* 1982, vol. 156, 113-140
- **CROWLEY et al.** *Cell,* 1994, vol. 78, 61-71
- **BEIER ; GRIMM.** *Nucleic Acids Res.,* 2001, vol. 29, 4767-4782
- **TORRES et al.** *Trends Mol. Med.,* 2014, vol. 20, 306-314
- **BJÖRK et al.** *Annu. Rev. Biochem.,* 1987, vol. 56, 263-287
- **GREEN ; SAMBROOK.** Molecular Cloning: A Laboratory Manual. CHSL Press, 2012
- **RIO et al.** RNA: A Laboratory Manual. CHSL Press, 2011
- **FLANAGAN et al.** *J. Biol. Chem.,* 2003, vol. 278, 18628-18637
- **JANIAK et al.** *Biochemistry,* 1992, vol. 31, 5830-5840
- **YAN et al.** *Proc. Natl. Acad. Sci., USA,* 2016, vol. 113 (39), E5702-E5710